# EUROPEAN PATENT SPECIFICATION

(11) **EP 4 262 808 B1**
(45) Date of publication and mention of the grant of the patent: **13.08.2025**
(21) Application number: 21907590.0
(22) Date of filing: 14.12.2021
(51) Int. Cl.: C07D 403/06, C07D 403/14, C07D 413/14, C07D 487/04, C07D 487/10, C07D 519/00, A61P 31/22, A61K 31/416, A61K 31/4162, A61K 31/5377, A61K 31/4439, A61K 31/506, A61K 31/422, A61K 31/427, A61K 31/4178, A61K 31/517, A61K 31/4985, A61K 45/06

(54) **FUSED BICYCLIC PYRAZOLE DERIVATIVES AND METHODS OF USE THEREOF FOR THE TREATMENT OF HERPESVIRUSES**
KONDENSIERTE BICYCLISCHE PYRAZOLDERIVATE UND VERFAHREN ZUR VERWENDUNG DAVON ZUR BEHANDLUNG VON HERPESVIREN
DÉRIVÉS BICYCLIQUES FUSIONNÉS DE PYRAZOLE ET LEURS MÉTHODES D'UTILISATION POUR LE TRAITEMENT DE VIRUS HERPÉTIQUES

(30) Priority: 18.12.2020 US 202063127537 P
(43) Date of publication of application: 25.10.2023
(73) Proprietor: Merck Sharp & Dohme LLC, Rahway, New Jersey 07065 (US)
(72) Inventor: CHANG, Ronald, K., West Point, Pennsylvania 19486 (US); COOKE, Andrew, J., Jr., Broughty Ferry, Scotland DD5 3JA (GB); COX, Christopher, Douglas, West Point, Pennsylvania 19486 (US); LABROLI, Marc, West POint, Pennsylvania 19486 (US); RAHEEM, Izzat, Tiedje, West Point, Pennsylvania 19486 (US); SCOTT, Jack, D., Kenilworth, New Jersey 07033 (US); SCHUBERT, Jeffrey, W., West Point, Pennsylvania 19486 (US); SKUDLAREK, Jason, W., West Point, Pennsylvania 19486 (US); TAN, Zheng, Kenilworth, New Jersey 07033 (US); TONG, Ling, Kenilworth, New Jersey 07033 (US)
(74) Representative: Merck Sharp & Dohme LLC
(86) International application number: PCT/US2021/063192
(87) International publication number: WO 2022/132686

(56) References cited:
- WO-A1-2012/031197
- WO-A1-2018/232154
- WO-A2-2006/023844
- CN-A- 107 674 029
- DATABASE PubChem 26 April 2019 (2019-04-26), ANONYMOUS : "SUBSTANCE RECORD SID 354475584", XP055953185, Database accession no. SID 354475584
- DAWIDOWSKI MACIEJ, KALEL VISHAL C., NAPOLITANO VALERIA, FINO ROBERTO, SCHORPP KENJI, EMMANOUILIDIS LEONIDAS, LENHART DOMINIK, OSTE: "Structure–Activity Relationship in Pyrazolo[4,3-c]pyridines, First Inhibitors of PEX14–PEX5 Protein–Protein Interaction with Trypanocidal Activity", JOURNAL OF MEDICINAL CHEMISTRY, vol. 63, no. 2, 23 January 2020 (2020-01-23), US , pages 847 - 879, XP055953186, ISSN: 0022-2623, DOI: 10.1021/acs.jmedchem.9b01876

## Description

### FIELD OF THE INVENTION

The present invention relates to novel Fused Bicyclic Pyrazole Derivatives, compositions comprising at least one Fused Bicyclic Pyrazole Derivative, and methods of using the Fused Bicyclic Pyrazole Derivatives for treating or preventing herpesvirus infection in a patient. The invention is set out in the appended set of claims. In addition, any reference to methods of treatment in the subsequent paragraphs of this description is to be interpreted as references to the compounds, pharmaceutical compositions and medicaments of the present invention for use in a method for treatment of the human or animal body by therapy.

### BACKGROUND OF THE INVENTION

Human herpes viruses (*Herpesviridae*) are responsible for causing a wide variety of diseases in humans. Infection with herpes viruses can occur early in life and by adulthood over 95% of the population is infected by at least one herpes virus. These viruses establish a persistent life-long infection through viral latency in neuronal, lymphoid, or myeloid cells. Recurrent episodes of herpes virus disease can be triggered by numerous stimuli, including concurrent viral infections, stress, fatigue, allergies, pregnancy, sunlight or fever. Herpes virus infection in immune competent individuals generally causes mild self-limiting disease, such as: oral (HSV-1), and genital (HSV-2) ulcers, chicken pox (VZV), flu-like syndrome (CMV), and mononucleosis (EBV). In immunocompromised individuals however, primary infection with, or reactivation of an existing herpes virus infection is a major cause of disease and death. Key at-risk immunocompromised populations include patients undergoing solid organ or stem cell transplants, individuals with HIV/AIDS, and ICU patients.

Herpesviridae comprise a diverse family of double-stranded DNA viruses that are classified into three subfamilies (*i.e.,* α, β, and γ) based upon biological characteristics such as cell tropism, diseases caused, viral life-cycle, and site of viral persistence and latency. The family consists of eight members: Herpes Simplex Virus type 1 and 2 (HSV-1, HSV-2), Varicella Zoster Virus (VZV), Epstein-Barr virus (EBV), Cytomegalovirus (CMV), and human herpes viruses 6-8 (HHV6-8).

α-herpes viruses include herpes simplex virus types 1 and 2 (HSV1 and HSV2), and varicella-zoster virus (VZV). HSV1 causes orofacial lesions, commonly known as fever blisters or cold sores. Approximately 30% of the United States population suffers from recurrent episodes of HSV1. HSV2, which is less common than HSV1, causes genital lesions. Primary infection with VZV causes varicella, commonly known as chicken pox. Reactivation of latent VZV manifests as herpes zoster or shingles. Cytomegalovirus (CMV) is a prototypical β herpes virus. Seroprevalance to CMV in the adult population is ~60%, but certain endemic areas of the world have rates closer to 100%. CMV represents the leading viral cause of morbidity and mortality in at-risk immunocompromised patients. EBV, a γ herpes virus, causes infectious mononucleosis and is responsible for lymphoid cancers such as Burkitt's and Hodgkin's lymphoma.

Presently, there is no cure for herpes. Medicines have been developed that can prevent or shorten outbreaks, but there is a need for improved therapies for treating herpes virus infection and inhibiting viral replication. The current standard of care for immunocompromised patients at risk for herpes virus disease is pre-emptive treatment with high-dose nucleoside/nucleotide analog drugs such as acyclovir, (val)ganciclovir, and cidofovir, all of which target the viral DNA polymerase. **In** general, current treatments are virus specific (not broad spectrum), and in the case of (val)ganciclovir and cidofovir cannot be administered prophylactically due to dose-related toxicities including bone marrow suppression and renal toxicity. Although efficacious in many settings, the current nucleos(t)ide drugs are also limited by drug-resistant viral variants and existing cross-resistant variants which may lead to treatment failure. Therefore, there is an urgent medical need for improved, well-tolerated anti-herpes agents. Anti-herpes compounds have been disclosed, for example in WO 2012/031197, CN 107674029 and WO 2006/023844.

### SUMMARY OF THE INVENTION

In one aspect, the present invention provides Compounds of Formula (I) 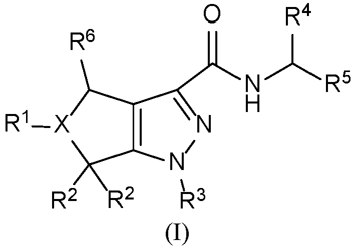 or a pharmaceutically acceptable salt thereof, wherein:
X is N or C(R⁹);
R¹ is selected from -(C₁-C₆ alkylene)-(4 to 7-membered monocyclic heterocycloalkyl), - (C₁-C₆ alkylene)-(6 to 10-membered bicyclic heterocycloalkyl), and -(C₁-C₆ alkylene)-(5 or 6-membered monocyclic heteroaryl), wherein said 4 to 7-membered monocyclic heterocycloalkyl group, said 6 to 10-membered bicyclic heterocycloalkyl group, and said 5 or 6-membered monocyclic heteroaryl group, can each be optionally substituted with one or more R^{A} groups, which can be the same or different;
each R² is independently selected from H, C₁-C₆ alkyl, -(C₁-C₆ alkylene)ₘ-O-C(O)-(C₁-C₆ alkyl), and C₁-C₆ hydroxyalkyl, or both R² groups, together with the carbon atom to which they are attached, combine to form a spirocyclic C₃-C₆ cycloalkyl group;
R³ is selected from C₁-C₆ alkyl, , C₁-C₆ hydroxyalkyl, --CD³, -(C₁-C₆ alkylene)ₘ-C₃-C₆ monocyclic cycloalkyl -(C₁-C₆ alkylene)-N(R⁷)₂ C₁-C₆ haloalkyl, -(C₁-C₆ alkylene)ₘ-(3 to 7-membered monocyclic heterocycloalkyl), -(C₁-C₆ alkylene)ₘ-(5 or 6-membered monocyclic heteroaryl), and 9 or 10-membered bicyclic heteroaryl, wherein said C₃-C₆ monocyclic cycloalkyl group, said 3 to 7-membered monocyclic heterocycloalkyl group, said 5 or 6-membered monocyclic heteroaryl group, and said 9 or 10-membered bicyclic heteroaryl group can each be optionally substituted with one or more R^{B} groups, which can be the same or different;
each occurrence of R⁴ is independently H or C₁-C₆ alkyl;
R⁵ is phenyl, which can be optionally substituted with one or more groups, which can be the same or different, and are selected from: halo, CN, and NO₂;
R⁶ is H or C₁-C₆ alkyl;
each occurrence of R⁷ is independently selected from H, C₁-C₆ alkyl, and -C(O)R⁸;
each occurrence of R⁸ is independently selected from H, C₁-C₆ alkyl, and C₃-C₆ monocyclic cycloalkyl, wherein C₃-C₆ monocyclic cycloalkyl can be optionally substituted with a group selected from C₁-C₆ alkyl, halo, and -OH;
each occurrence of R⁹ is independently selected from H, C₁-C₆ alkyl and -OH;
each occurrence of R^{A} is independently selected from oxo, halo, C₁-C₆ alkyl, C₁-C₆ alkenyl and -C(O)-C₁-C₆ alkyl;
each occurrence of R^{B} is independently selected from C₁-C₆ alkyl, -OH, -O-(C₁-C₆ alkyl), halo, -C₁-C₆ haloalkyl, -C₁-C₆ hydroxyalkyl, phenyl, and -(C₁-C₆ alkylene)ₘ-(3 to 6-membered monocyclic cycloalkyl); and
occurrence of m is independently 0 or 1.

The Compounds of Formula (I) (also referred to herein as the "Fused Bicyclic Pyrazole Derivatives"), and pharmaceutically acceptable salts thereof can be useful, for example, for inhibiting herpesvirus viral replication or activity, and for treating or preventing herpesvirus infection in a patient. Without being bound by any specific theory, it is believed that the Fused Bicyclic Pyrazole Derivatives inhibit herpesvirus viral replication by inhibiting herpesvirus polymerase.

Accordingly, the present invention provides methods for treating or preventing herpesvirus infection in a patient, comprising administering to the patient an effective amount of at least one Fused Bicyclic Pyrazole Derivative.

The details of the invention are set forth in the accompanying detailed description below.

Although any methods and materials similar to those described herein can be used in the practice or testing of the present invention, illustrative methods and materials are now described. Other embodiments, aspects and features of the present invention are either further described in or will be apparent from the ensuing description, examples and appended claims.

### DETAILED DESCRIPTION OF THE INVENTION

The present invention relates to novel Fused Bicyclic Pyrazole Derivatives, compositions comprising at least one Fused Bicyclic Pyrazole Derivative, and methods of using the Fused Bicyclic Pyrazole Derivatives for treating or preventing herpesvirus infection in a patient.

### Definitions and Abbreviations

The terms used herein have their ordinary meaning and the meaning of such terms is independent at each occurrence thereof. That notwithstanding and except where stated otherwise, the following definitions apply throughout the specification and claims. Chemical names, common names, and chemical structures may be used interchangeably to describe the same structure. If a chemical compound is referred to using both a chemical structure and a chemical name and an ambiguity exists between the structure and the name, the structure predominates. These definitions apply regardless of whether a term is used by itself or in combination with other terms, unless otherwise indicated. Hence, the definition of "alkyl" applies to "alkyl" as well as the "alkyl" portions of "hydroxyalkyl," "haloalkyl," "-O-alkyl," etc...

As used herein, and throughout this disclosure, the following terms, unless otherwise indicated, shall be understood to have the following meanings:
A "patient" is a human or non-human mammal. In one embodiment, a patient is a human.

The term "effective amount" as used herein, refers to an amount of Fused Bicyclic Pyrazole Derivative and/or an additional therapeutic agent, or a composition thereof that is effective in producing the desired therapeutic, ameliorative, inhibitory or preventative effect when administered to a patient suffering from a viral infection or virus-related disorder. In the combination therapies of the present invention, an effective amount can refer to each individual agent or to the combination as a whole, wherein the amounts of all agents administered are together effective, but wherein the component agent of the combination may not be present individually in an effective amount.

The term "preventing," as used herein with respect to a herpesvirus viral infection or herpesvirus-virus related disorder, refers to reducing the likelihood of herpesvirus infection.

The term "alkyl," as used herein, refers to an aliphatic hydrocarbon group having one of its hydrogen atoms replaced with a bond. An alkyl group may be straight or branched and contain from about 1 to about 20 carbon atoms. In one embodiment, an alkyl group contains from about 1 to about 12 carbon atoms. In different embodiments, an alkyl group contains from 1 to 6 carbon atoms (C₁-C₆ alkyl) or from about 1 to about 4 carbon atoms (C₁-C₄ alkyl). Non-limiting examples of alkyl groups include methyl, ethyl, n-propyl, isopropyl, n-butyl, sec-butyl, isobutyl, tert-butyl, n-pentyl, neopentyl, isopentyl, n-hexyl, isohexyl and neohexyl. An alkyl group may be unsubstituted or substituted by one or more substituents which may be the same or different, each substituent being independently selected from the group consisting of halo, alkenyl, alkynyl, aryl, cycloalkyl, cyano, hydroxy, -O-alkyl, -O-aryl, -alkylene-O-alkyl, alkylthio, -NH₂, -NH(alkyl), -N(alkyl)₂, NH(cycloalkyl), -O-C(O)-alkyl, -O-C(O)-aryl, -O-C(O)-cycloalkyl, -C(O)OH and -C(O)O-alkyl. In one embodiment, an alkyl group is linear. In another embodiment, an alkyl group is branched. Unless otherwise indicated, an alkyl group is unsubstituted.

The term "alkenyl," as used herein, refers to an aliphatic hydrocarbon group containing at least one carbon-carbon double bond and having one of its hydrogen atoms replaced with a bond. An alkenyl group may be straight or branched and contain from about 2 to about 15 carbon atoms. In one embodiment, an alkenyl group contains from about 2 to about 12 carbon atoms. In another embodiment, an alkenyl group contains from about 2 to about 6 carbon atoms. Non-limiting examples of alkenyl groups include ethenyl, propenyl, n-butenyl, 3-methylbut-2-enyl, n-pentenyl, octenyl and decenyl. An alkenyl group may be unsubstituted or substituted by one or more substituents which may be the same or different, each substituent being independently selected from the group consisting of halo, alkenyl, alkynyl, aryl, cycloalkyl, cyano, hydroxy, - O-alkyl, -O-aryl, -alkylene-O-alkyl, alkylthio, -NH₂, -NH(alkyl), -N(alkyl)₂, -NH(cycloalkyl), - O-C(O)-alkyl, -O-C(O)-aryl, -O-C(O)-cycloalkyl, -C(O)OH and -C(O)O-alkyl. The term "C₂-C₆ alkenyl" refers to an alkenyl group having from 2 to 6 carbon atoms. Unless otherwise indicated, an alkenyl group is unsubstituted.

The term "alkynyl," as used herein, refers to an aliphatic hydrocarbon group containing at least one carbon-carbon triple bond and having one of its hydrogen atoms replaced with a bond. An alkynyl group may be straight or branched and contain from about 2 to about 15 carbon atoms. In one embodiment, an alkynyl group contains from about 2 to about 12 carbon atoms. In another embodiment, an alkynyl group contains from about 2 to about 6 carbon atoms. Non-limiting examples of alkynyl groups include ethynyl, propynyl, 2-butynyl and 3-methylbutynyl. An alkynyl group may be unsubstituted or substituted by one or more substituents which may be the same or different, each substituent being independently selected from the group consisting of halo, alkenyl, alkynyl, aryl, cycloalkyl, cyano, hydroxy, -O-alkyl, -O-aryl, -alkylene-O-alkyl, alkylthio, -NH₂, -NH(alkyl), -N(alkyl)₂, -NH(cycloalkyl), -O-C(O)-alkyl, -O-C(O)-aryl, -O-C(O)-cycloalkyl, -C(O)OH and -C(O)O-alkyl. The term "C₂-C₆ alkynyl" refers to an alkynyl group having from 2 to 6 carbon atoms. Unless otherwise indicated, an alkynyl group is unsubstituted.

The term "alkylene," as used herein, refers to an alkyl group, as defined above, wherein one of the alkyl group's hydrogen atoms has been replaced with a bond. Non-limiting examples of alkylene groups include -CH₂-, -CH₂CH₂-, -CH₂CH₂CH₂-, -CH₂CH₂CH₂CH₂-, - CH(CH₃)CH₂CH₂-, -CH(CH₃)- and -CH₂CH(CH₃)CH₂-. In one embodiment, an alkylene group has from 1 to about 6 carbon atoms. In another embodiment, an alkylene group is branched. In another embodiment, an alkylene group is linear. In one embodiment, an alkylene group is - CH₂-. The term "C₁-C₆ alkylene" refers to an alkylene group having from 1 to 6 carbon atoms.

The term "aryl," as used herein, refers to an aromatic monocyclic or multicyclic ring system comprising from about 6 to about 14 carbon atoms. In one embodiment, an aryl group contains from about 6 to about 10 carbon atoms. An aryl group can be optionally substituted with one or more "ring system substituents" which may be the same or different and are as defined herein below. In one embodiment, an aryl group can be optionally fused to a cycloalkyl or cycloalkanoyl group. Non-limiting examples of aryl groups include phenyl and naphthyl. In one embodiment, an aryl group is phenyl. In another embodiment, an aryl group is napthalene. Unless otherwise indicated, an aryl group is unsubstituted.

The term "cycloalkyl," as used herein, refers to a non-aromatic mono- or multicyclic ring system comprising from about 3 to about 10 ring carbon atoms. In one embodiment, a cycloalkyl contains from about 5 to about 10 ring carbon atoms. In another embodiment, a cycloalkyl contains from about 3 to about 7 ring atoms. In another embodiment, a cycloalkyl contains from about 5 to about 6 ring atoms. Non-limiting examples of monocyclic cycloalkyls include cyclopropyl, cyclobutyl, cyclopentyl, cyclohexyl, cycloheptyl and cyclooctyl. Non-limiting examples of multicyclic cycloalkyls include 1-decalinyl, norbornyl and adamantyl. A cycloalkyl group can be optionally substituted with one or more "ring system substituents" which may be the same or different and are as defined herein below. Unless otherwise indicated, a cycloalkyl group is unsubstituted. In one embodiment, a cycloalkyl group is unsubstituted. The term "3 to 6-membered cycloalkyl" refers to a cycloalkyl group having from 3 to 6 ring carbon atoms. A ring carbon atom of a cycloalkyl group may be functionalized as a carbonyl group. An illustrative example of such a cycloalkyl group (also referred to herein as a "cycloalkanoyl" group) includes, but is not limited to, cyclobutanoyl:

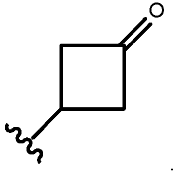

The term "cycloalkenyl," as used herein, refers to a non-aromatic mono- or multicyclic ring system comprising from about 4 to about 10 ring carbon atoms and containing at least one endocyclic double bond. In one embodiment, a cycloalkenyl contains from about 4 to about 7 ring carbon atoms. In another embodiment, a cycloalkenyl contains 5 or 6 ring atoms. Non-limiting examples of monocyclic cycloalkenyls include cyclopentenyl, cyclohexenyl, cyclohepta-1,3-dienyl, and the like. A cycloalkenyl group can be optionally substituted with one or more "ring system substituents" which may be the same or different and are as defined herein below. A ring carbon atom of a cycloalkyl group may be functionalized as a carbonyl group. In one embodiment, a cycloalkenyl group is cyclopentenyl. In another embodiment, a cycloalkenyl group is cyclohexenyl. The term "4 to 6-membered cycloalkenyl" refers to a cycloalkenyl group having from 4 to 6 ring carbon atoms.

The term "halo," as used herein, means -F, -Cl, -Br or -I.

The term "haloalkyl," as used herein, refers to an alkyl group as defined above, wherein one or more of the alkyl group's hydrogen atoms has been replaced with a halogen. In one embodiment, a haloalkyl group has from 1 to 6 carbon atoms. In another embodiment, a haloalkyl group is substituted with from 1 to 3 F atoms. Non-limiting examples of haloalkyl groups include -CH₂F, -CHF₂, -CF₃, -CH₂Cl and -CCl₃.

The term "C₁-C₆ haloalkyl" refers to a haloalkyl group having from 1 to 6 carbon atoms.

The term "hydroxyalkyl," as used herein, refers to an alkyl group as defined above, wherein one or more of the alkyl group's hydrogen atoms has been replaced with an -OH group. In one embodiment, a hydroxyalkyl group has from 1 to 6 carbon atoms. Non-limiting examples of hydroxyalkyl groups include -CH₂OH, -CH₂CH₂OH, -CH₂CH₂CH₂OH and - CH₂CH(OH)CH₃. The term "C₁-C₆ hydroxyalkyl" refers to a hydroxyalkyl group having from 1 to 6 carbon atoms.

The term "heteroaryl," as used herein, refers to an aromatic monocyclic or multicyclic ring system comprising about 5 to about 14 ring atoms, wherein from 1 to 4 of the ring atoms is independently O, N or S and the remaining ring atoms are carbon atoms. In one embodiment, a heteroaryl group has 5 to 10 ring atoms. In another embodiment, a heteroaryl group is monocyclic and has 5 or 6 ring atoms. In another embodiment, a heteroaryl group is bicyclic and had 9 or 10 ring atoms. A heteroaryl group can be optionally substituted by one or more "ring system substituents" which may be the same or different and are as defined herein below. Unless otherwise indicated, a heteroaryl group is unsubstituted. A heteroaryl group is joined via a ring carbon atom, and any nitrogen atom of a heteroaryl can be optionally oxidized to the corresponding N-oxide. The term "heteroaryl" also encompasses a heteroaryl group, as defined above, which is fused to a benzene ring. Non-limiting examples of heteroaryls include pyridyl, pyrazinyl, furanyl, thienyl, pyrimidinyl, pyridone (including N-substituted pyridones), isoxazolyl, isothiazolyl, oxazolyl, oxadiazolyl, thiazolyl, pyrazolyl, furazanyl, pyrrolyl, triazolyl, 1,2,4-thiadiazolyl, pyrazinyl, pyridazinyl, quinoxalinyl, phthalazinyl, oxindolyl, imidazo[1,2-a]pyridinyl, imidazo[2,1-b]thiazolyl, benzofurazanyl, indolyl, azaindolyl, benzimidazolyl, benzothienyl, quinolinyl, imidazolyl, benzimidazolyl, thienopyridyl, quinazolinyl, thienopyrimidyl, pyrrolopyridyl, imidazopyridyl, isoquinolinyl, benzoazaindolyl, 1,2,4-triazinyl, benzothiazolyl and the like, and all isomeric forms thereof. The term "heteroaryl" also refers to partially saturated heteroaryl moieties such as, for example, tetrahydroisoquinolyl, tetrahydroquinolyl and the like. In one embodiment, a heteroaryl group is a 5-membered heteroaryl. In another embodiment, a heteroaryl group is a 6-membered heteroaryl. In another embodiment, a "9- or 10-membered bicyclic heteroaryl" group comprises a 5- to 6-membered heterocycloalkyl group fused to a benzene ring, such as:

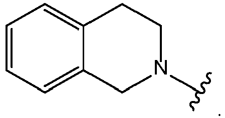

In still another embodiment, a "9- or 10-membered bicyclic heteroaryl" group comprises a 5- to 6-membered heteroaryl group fused to a cycloalkyl ring or a heterocycloalkyl ring, such as:

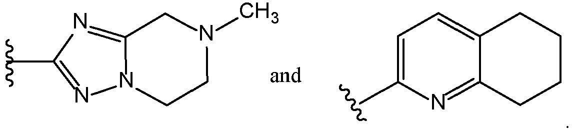

The term "heteroarylene," as used herein, refers to a bivalent group derived from an heteroaryl group, as defined above, by removal of a hydrogen atom from a ring carbon or ring heteroatom of a heteroaryl group. A heteroarylene group can be derived from a monocyclic or multicyclic ring system comprising about 5 to about 14 ring atoms, wherein from 1 to 4 of the ring atoms are each independently O, N or S and the remaining ring atoms are carbon atoms. A heteroarylene group can be optionally substituted by one or more "ring system substituents" which may be the same or different and are as defined herein below. Unless otherwise indicated, a heteroarylene l group is unsubstituted. A heteroarylene group is joined via a ring carbon atom or by a nitrogen atom with an open valence, and any nitrogen atom of a heteroarylene can be optionally oxidized to the corresponding N-oxide. The term "heteroarylene" also encompasses a heteroarylene group, as defined above, which is fused to a benzene ring. Non-limiting examples of heteroarylenes include pyridylene, pyrazinylene, furanylene, thienylene, pyrimidinylene, pyridonylene (including those derived from N-substituted pyridonyls), isoxazolylene, isothiazolylene, oxazolylene, oxadiazolylene, thiazolylene, pyrazolylene, thiophenylene, furazanylene, pyrrolylene, triazolylene, 1,2,4-thiadiazolylene, pyrazinylene, pyridazinylene, quinoxalinylene, phthalazinylene, oxindolylene, imidazo[1,2-a]pyridinylene, imidazo[2,1-b]thiazolylene, benzofurazanylene, indolylene, azaindolylene, benzimidazolylene, benzothienylene, quinolinylene, imidazolylene, benzimidazolylene, thienopyridylene, quinazolinylene, thienopyrimidylene, pyrrolopyridylene, imidazopyridylene, isoquinolinylene, benzoazaindolylene, 1,2,4-triazinylene, benzothiazolylene and the like, and all isomeric forms thereof. The term "heteroarylene" also refers to partially saturated heteroarylene moieties such as, for example, tetrahydroisoquinolylene, tetrahydroquinolylene, and the like. A heteroarylene group is divalent and unless specified ohterwise, either available bond on a heteroarylene ring can connect to either group flanking the heteroarylene group. For example, the group "A-heteroarylene-B," wherein the heteroarylene group is: 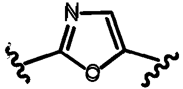 is understood to represent both:

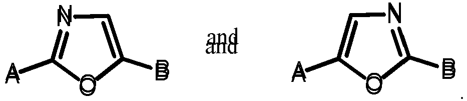

In one embodiment, a heteroarylene group is a monocyclic heteroarylene group or a bicyclic heteroarylene group. In another embodiment, a heteroarylene group is a monocyclic heteroarylene group. In another embodiment, a heteroarylene group is a bicyclic heteroarylene group. In still another embodiment, a heteroarylene group has from about 5 to about 10 ring atoms. In another embodiment, a heteroarylene group is monocyclic and has 5 or 6 ring atoms. In another embodiment, a heteroarylene group is bicyclic and has 9 or 10 ring atoms. In another embodiment, a heteroarylene group is a 5-membered monocyclic heteroarylene. In another embodiment, a heteroarylene group is a 6-membered monocyclic heteroarylene. In another embodiment, a bicyclic heteroarylene group comprises a 5 or 6-membered monocyclic heteroarylene group fused to a benzene ring. In still another embodiment, a heteroaryl group comprises a 5- to 6-membered monocyclic heteroarylene group fused to a cycloalkyl ring or a heterocycloalkyl ring.

The term "heterocycloalkyl," as used herein, refers to a non-aromatic saturated monocyclic or multicyclic ring system comprising 3 to about 11 ring atoms, wherein from 1 to 4 of the ring atoms are independently O, S, N or Si, and the remainder of the ring atoms are carbon atoms. A heterocycloalkyl group can be joined via a ring carbon, ring silicon atom or ring nitrogen atom. In one embodiment, a heterocycloalkyl group is monocyclic. In one embodiment, a heterocycloalkyl group is monocyclic and has from about 3 to about 7 ring atoms ("3 to 7-membered bicyclic heterocycloalkyl"). In another embodiment, a heterocycloalkyl group is monocyclic has from about 4 to about 7 ring atoms ("4 to 7-membered bicyclic heterocycloalkyl"). In still another embodiment, a heterocycloalkyl group is monocyclic and has 5 or 6 ring atoms ("5 or 6-membered monocyclic heterocycloalkyl"). In one embodiment, a heterocycloalkyl group is bicyclic. In another embodiment, a heterocycloalkyl group is bicyclic and has from about 6 to about 10 ring atoms ("6 to 10-membered bicyclic heterocycloalkyl"). There are no adjacent oxygen and/or sulfur atoms present in the ring system. Any -NH group in a heterocycloalkyl ring may exist protected such as, for example, as an -N(BOC), -N(CBz), - N(Tos) group and the like; such protected heterocycloalkyl groups are considered part of this invention. A heterocycloalkyl group can be optionally substituted by one or more "ring system substituents" which may be the same or different and are as defined herein below. The nitrogen or sulfur atom of the heterocycloalkyl can be optionally oxidized to the corresponding N-oxide, S-oxide or S,S-dioxide. Non-limiting examples of monocyclic heterocycloalkyl rings include oxetanyl, piperidyl, pyrrolidinyl, piperazinyl, morpholinyl, thiomorpholinyl, thiazolidinyl, 1,4-dioxanyl, tetrahydrofuranyl, tetrahydrothiophenyl, delta-lactam, delta-lactone, silacyclopentane, silapyrrolidine and the like, and all isomers thereof. Non-limiting illustrative examples of a silyl-containing heterocycloalkyl group include:

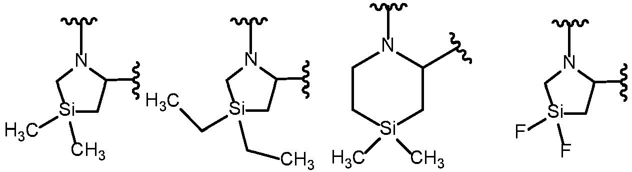

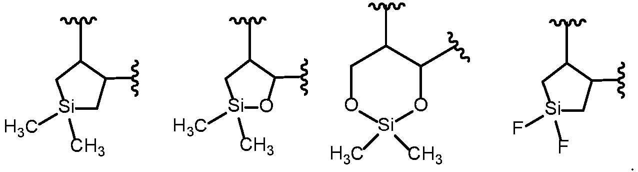

A ring carbon atom of a heterocycloalkyl group may be functionalized as a carbonyl group. Illustrative examples of such a heterocycloalkyl group include, but are not limited to:

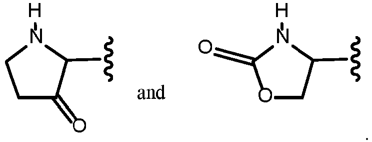

A ring sulfur atom of a heterocycloalkyl group may also be functionalized as a sulfonyl group. An example of such a heterocycloalkyl group is:

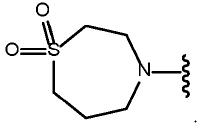

In one embodiment, a heterocycloalkyl group is a 5-membered monocyclic heterocycloalkyl. In another embodiment, a heterocycloalkyl group is a 6-membered monocyclic heterocycloalkyl.

A multicyclic heterocycloalkyl group may have rings that are fused, rings that are joined in a spirocyclic manner, and rings that are bridged. In one embodiment, a heterocycloalkyl group can be a bicyclic spirocyclic heteroaryl group having from 7 to 9 ring atoms. Illustrative examples of such a bicyclic heteroycloalkyl group include:

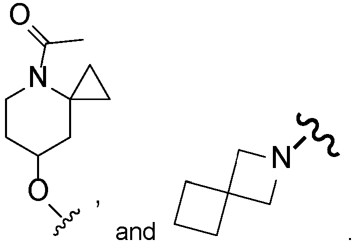

In another embodiment, a heterocycloalkyl group can be a bicyclic fused heterocycloalkyl group having from 6 to 10 ring atoms. Illustrative examples of such a fused bicyclic heteroycloalkyl group include:

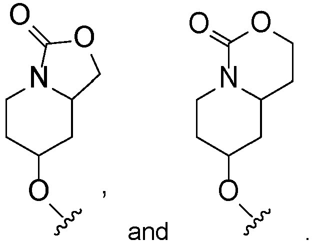

In another embodiment, a heterocycloalkyl group can be a bridged bridged heterocycloalkyl group having from 6 to 10 ring atoms. Illustrative examples of such a bridged bicyclic heteroycloalkyl group include:

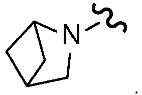

The term "heterocycloalkenyl," as used herein, refers to a heterocycloalkyl group, as defined above, wherein the heterocycloalkyl group contains from 4 to 10 ring atoms, and at least one endocyclic carbon-carbon or carbon-nitrogen double bond. A heterocycloalkenyl group can be joined via a ring carbon or ring nitrogen atom. In one embodiment, a heterocycloalkenyl group has from 4 to 6 ring atoms. In another embodiment, a heterocycloalkenyl group is monocyclic and has 5 or 6 ring atoms. In another embodiment, a heterocycloalkenyl group is bicyclic. A heterocycloalkenyl group can optionally substituted by one or more ring system substituents, wherein "ring system substituent" is as defined above. The nitrogen or sulfur atom of the heterocycloalkenyl can be optionally oxidized to the corresponding N-oxide, S-oxide or S,S-dioxide. A ring carbon atom of a heterocycloalkenyl group may be functionalized as a carbonyl group. Non-limiting examples of heterocycloalkenyl groups include 1,2,3,4-tetrahydropyridinyl, 1,2-dihydropyridinyl, 1,4-dihydropyridinyl, 1,2,3,6-tetrahydropyridinyl, 1,4,5,6-tetrahydropyrimidinyl, 2-pyrrolinyl, 3-pyrrolinyl, 2-imidazolinyl, 2-pyrazolinyl, dihydroimidazolyl, dihydrooxazolyl, dihydrooxadiazolyl, dihydrothiazolyl, 3,4-dihydro-2H-pyranyl, dihydrofuranyl, fluoro-substituted dihydrofuranyl, 7-oxabicyclo[2.2.1]heptenyl, dihydrothiophenyl, dihydrothiopyranyl, and the like and the like. In one embodiment, a heterocycloalkenyl group is a 5-membered heterocycloalkenyl. In another embodiment, a heterocycloalkenyl group is a 6-membered heterocycloalkenyl. The term "4 to 6-membered heterocycloalkenyl" refers to a heterocycloalkenyl group having from 4 to 6 ring atoms.

The term "substituted" means that one or more hydrogens on the designated atom is replaced with a selection from the indicated group, provided that the designated atom's normal valency under the existing circumstances is not exceeded, and that the substitution results in a stable compound. Combinations of substituents and/or variables are permissible only if such combinations result in stable compounds. By "stable compound' or "stable structure" is meant a compound that is sufficiently robust to survive isolation to a useful degree of purity from a reaction mixture, and formulation into an efficacious therapeutic agent.

The term "in substantially purified form," as used herein, refers to the physical state of a compound after the compound is isolated from a synthetic process (*e.g*., from a reaction mixture), a natural source, or a combination thereof. The term "in substantially purified form," also refers to the physical state of a compound after the compound is obtained from a purification process or processes described herein or well-known to the skilled artisan (*e.g*., chromatography, recrystallization and the like), in sufficient purity to be characterizable by standard analytical techniques described herein or well-known to the skilled artisan.

It should also be noted that any carbon as well as heteroatom with unsatisfied valences in the text, schemes, examples and tables herein is assumed to have the sufficient number of hydrogen atom(s) to satisfy the valences.

When a functional group in a compound is termed "protected", this means that the group is in modified form to preclude undesired side reactions at the protected site when the compound is subjected to a reaction. Suitable protecting groups will be recognized by those with ordinary skill in the art as well as by reference to standard textbooks such as, for example, T. W. Greene et al, Protective Groups in Organic Synthesis (1991), Wiley, New York.

Examples of "ring system substituents" include, but are not limited to, alkyl, alkenyl, alkynyl, aryl, heteroaryl, -alkylene-aryl, -arylene-alkyl, -alkylene-heteroaryl,-alkenylene-heteroaryl, -alkynylene-heteroaryl, -OH, hydroxyalkyl, haloalkyl, -O-alkyl, -O-haloalkyl, - alkylene-O-alkyl, -O-aryl, -O-alkylene-aryl, acyl, - C(O)-aryl, halo, -NO₂, -CN, -SF₅, -C(O)OH, -C(O)O-alkyl, -C(O)O-aryl, -C(O)O-alkylene-aryl, -S(O)-alkyl, -S(O)₂-alkyl, -S(O)-aryl, -S(O)₂-aryl, -S(O)-heteroaryl, -S(O)z-heteroaryl, -S-alkyl, -S-aryl, -S-heteroaryl, -S-alkylene-aryl, -S-alkyleneheteroaryl, -S(O)₂-alkylene-aryl, -S(O)₂-alkylene-heteroaryl, -Si(alkyl)₂, -Si(aryl)₂, Si(heteroaryl)₂ -Si(alkyl)( aryl), -Si(alkyl)(cycloalkyl), -Si(alkyl)(heteroaryl), cycloalkyl, heterocycloalkyl, -O-C(O)-alkyl, -O-C(O)-aryl, -O-C(O)-cycloalkyl, -C(=N-CN)-NH₂, - C(=NH)-NH₂, -C(=NH)-NH(alkyl), -N(Y¹)(Y²), -alkylene-N(Y¹)(Y²), -C(O)N(Y¹)(y²), and - S(O)₂N(Y¹)(Y²), wherein Y¹ and Y² can be the same or different and are independently selected from the group consisting of hydrogen, alkyl, aryl, cycloalkyl, and -alkylene-aryl. "Ring system substituent" may also mean a single moiety which simultaneously replaces two available hydrogens on two adjacent carbon atoms (one H on each carbon) on a ring system. Examples of such moiety are methylenedioxy, ethylenedioxy, -C(CH₃)₂- and the like which form moieties such as, for example:

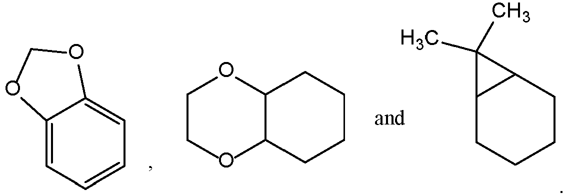

When any substituent or variable (*e.g.,* R¹, m, etc.) occurs more than one time in any constituent or in Formula (I), its definition on each occurrence is independent of its definition at every other occurrence, unless otherwise indicated.

As used herein, the term "composition" is intended to encompass a product comprising the specified ingredients in the specified amounts, as well as any product which results from combination of the specified ingredients in the specified amounts.

Prodrugs and solvates of the compounds of the invention are also contemplated herein. but not constitute part of the invention. A discussion of prodrugs is provided in T. Higuchi and V. Stella, Pro-drugs as Novel Delivery Systems (1987) 14 of the A.C.S. Symposium Series, and in Bioreversible Carriers in Drug Design, (1987) Edward B. Roche, ed., American Pharmaceutical Association and Pergamon Press. The term "prodrug" means a compound (*e.g.,* a drug precursor) that is transformed *in vivo* to provide an Fused Bicyclic Pyrazole Derivative or a pharmaceutically acceptable salt or solvate of the compound. The transformation may occur by various mechanisms (*e.g*., by metabolic or chemical processes), such as, for example, through hydrolysis in blood.

For example, if an Fused Bicyclic Pyrazole Derivative or a pharmaceutically acceptable salt, hydrate or solvate of the compound contains a carboxylic acid functional group, a prodrug can comprise an ester formed by the replacement of the hydrogen atom of the acid group with a group such as, for example, (C₁-C₈)alkyl, (C₂-C₁₂)alkanoyloxymethyl, 1-(alkanoyloxy)ethyl having from 4 to 9 carbon atoms, 1-methyl-1-(alkanoyloxy)-ethyl having from 5 to 10 carbon atoms, alkoxycarbonyloxymethyl having from 3 to 6 carbon atoms, 1-(alkoxycarbonyloxy)ethyl having from 4 to 6 carbon atoms, 1-methyl-1-(alkoxycarbonyloxy)ethyl having from 5 to 8 carbon atoms, N-(alkoxycarbonyl)aminomethyl having from 3 to 9 carbon atoms, 1-(N-(alkoxycarbonyl)amino)ethyl having from 4 to 10 carbon atoms, 3-phthalidyl, 4-crotonolactonyl, gamma-butyrolacton-4-yl, di-N,N-(C₁-C₂)alkylamino(C₂-C₃)alkyl (such as β-dimethylaminoethyl), carbamoyl-(C₁-C₂)alkyl, N,N-di (C₁-C₂)alkylcarbamoyl-(C₁-C₂)alkyl and piperidino-, pyrrolidino- or morpholino(C₂-C₃)alkyl, and the like.

Similarly, if an Fused Bicyclic Pyrazole Derivative contains an alcohol functional group, a prodrug can be formed by the replacement of the hydrogen atom of the alcohol group with a group such as, for example, (C₁-C₆)alkanoyloxymethyl, 1-((C₁-C₆)alkanoyloxy)ethyl, 1-methyl-1-((C₁-C₆)alkanoyloxy)ethyl, (C₁-C₆)alkoxycarbonyloxymethyl, N-(C₁-C₆)alkoxycarbonylaminomethyl, succinoyl, (C₁-C₆)alkanoyl, α-amino(C₁-C₄)alkyl, α-amino(C₁-C₄)alkylene-aryl, arylacyl and α-aminoacyl, or α-aminoacyl-α-aminoacyl, where each α-aminoacyl group is independently selected from the naturally occurring L-amino acids, - P(O)(OH)₂, -P(O)(O(C₁-C₆)alkyl)₂ or glycosyl (the radical resulting from the removal of a hydroxyl group of the hemiacetal form of a carbohydrate), and the like. If a Fused Bicyclic Pyrazole Derivative incorporates an amine functional group, a prodrug can be formed by the replacement of a hydrogen atom in the amine group with a group such as, for example, R-carbonyl-, RO-carbonyl-, NRR'-carbonyl- wherein R and R' are each independently (C₁-C₁₀)alkyl, (C₃-C₇) cycloalkyl, benzyl, a natural α-aminoacyl, -C(OH)C(O)OY¹ wherein Y¹ is H, (C₁-C₆)alkyl or benzyl, -C(OY²)Y³ wherein Y² is (C₁-C₄) alkyl and Y³ is (C₁-C₆)alkyl; carboxy (C₁-C₆)alkyl; amino(C₁-C₄)alkyl or mono-N- or di-N,N-(C₁-C₆)alkylaminoalkyl; -C(Y⁴)Y⁵ wherein Y⁴ is H or methyl and Y⁵ is mono-N- or di-N,N-(C₁-C₆)alkylamino morpholino; piperidin-1-yl or pyrrolidin-1-yl, and the like.

Pharmaceutically acceptable esters of the present compounds include the following groups: (1) carboxylic acid esters obtained by esterification of the hydroxy group of a hydroxyl compound, in which the non-carbonyl moiety of the carboxylic acid portion of the ester grouping is selected from straight or branched chain alkyl (*e.g*., methyl, ethyl, n-propyl, isopropyl, t-butyl, sec-butyl or n-butyl), alkoxyalkyl (*e.g.,* methoxymethyl), aralkyl (*e.g.,* benzyl), aryloxyalkyl (for example, phenoxymethyl), aryl (*e.g*., phenyl optionally substituted with, for example, halogen, C₁₋₄alkyl, -O-(C₁₋₄alkyl) or amino); (2) sulfonate esters, such as alkyl- or aralkylsulfonyl (for example, methanesulfonyl); (3) amino acid esters (*e.g*., L-valyl or L-isoleucyl); (4) phosphonate esters and (5) mono-, di- or triphosphate esters. The phosphate esters may be further esterified by, for example, a C₁₋₂₀ alcohol or reactive derivative thereof, or by a 2,3-di (C₆₋₁₄)acyl glycerol.

One or more compounds of the invention may exist in unsolvated as well as solvated forms with pharmaceutically acceptable solvents such as water, ethanol, and the like, and it is intended that the disclosure embrace both solvated and unsolvated forms. "Solvate" means a physical association of a compound of this invention with one or more solvent molecules. This physical association involves varying degrees of ionic and covalent bonding, including hydrogen bonding. In certain instances the solvate will be capable of isolation, for example when one or more solvent molecules are incorporated in the crystal lattice of the crystalline solid. "Solvate" encompasses both solution-phase and isolatable solvates. Non-limiting examples of solvates include ethanolates, methanolates, and the like. A "hydrate" is a solvate wherein the solvent molecule is water.

One or more compounds of the invention may optionally be converted to a solvate. Preparation of solvates is generally known. Thus, for example, M. Caira et al, J. Pharmaceutical Sci., 93(3), 601-611 (2004) describe the preparation of the solvates of the antifungal fluconazole in ethyl acetate as well as from water. Similar preparations of solvates, hemisolvate, hydrates and the like are described by E. C. van Tonder et al, AAPS PharmSciTechours., 5(1), article 12 (2004); and A. L. Bingham et al, Chem. Commun., 603-604 (2001). A typical, non-limiting, process involves dissolving the inventive compound in desired amounts of the desired solvent (organic or water or mixtures thereof) at a higher than room temperature, and cooling the solution at a rate sufficient to form crystals which are then isolated by standard methods. Analytical techniques such as, for example IR spectroscopy, show the presence of the solvent (or water) in the crystals as a solvate (or hydrate).

The Fused Bicyclic Pyrazole Derivatives can form salts which are also within the scope of this invention. The term "salt(s)", as employed herein, denotes acidic salts formed with inorganic and/or organic acids, as well as basic salts formed with inorganic and/or organic bases. In addition, when an Fused Bicyclic Pyrazole Derivative contains both a basic moiety, such as, but not limited to a pyridine or imidazole, and an acidic moiety, such as, but not limited to a carboxylic acid, zwitterions ("inner salts") may be formed and are included within the term "salt(s)" as used herein. In one embodiment, the salt is a pharmaceutically acceptable (*i.e.,* nontoxic, physiologically acceptable) salt. In another embodiment, the salt is other than a pharmaceutically acceptable salt. Salts of the Compounds of Formula (I) may be formed, for example, by reacting an Fused Bicyclic Pyrazole Derivative with an amount of acid or base, such as an equivalent amount, in a medium such as one in which the salt precipitates or in an aqueous medium followed by lyophilization.

Exemplary acid addition salts include acetates, ascorbates, benzoates, benzenesulfonates, bisulfates, borates, butyrates, citrates, camphorates, camphorsulfonates, fumarates, hydrochlorides, hydrobromides, hydroiodides, lactates, maleates, methanesulfonates, naphthalenesulfonates, nitrates, oxalates, phosphates, propionates, salicylates, succinates, sulfates, tartarates, thiocyanates, toluenesulfonates (also known as tosylates), and the like. Additionally, acids which are generally considered suitable for the formation of pharmaceutically useful salts from basic pharmaceutical compounds are discussed, for example, by P. Stahl et al, Camille G. (eds.) Handbook of Pharmaceutical Salts. Properties, Selection and Use. (2002) Zurich: Wiley-VCH; S. Berge et al, Journal of Pharmaceutical Sciences (1977) 66(1) 1-19; P. Gould, International J. of Pharmaceutics (1986) 33 201-217; Anderson et al, The Practice of Medicinal Chemistry (1996), Academic Press, New York; and in The Orange Book (Food & Drug Administration, Washington, D.C. on their website).

Exemplary basic salts include ammonium salts, alkali metal salts such as sodium, lithium, and potassium salts, alkaline earth metal salts such as calcium and magnesium salts, salts with organic bases (for example, organic amines) such as dicyclohexylamine, t-butyl amine, choline, and salts with amino acids such as arginine, lysine and the like. Basic nitrogen-containing groups may be quarternized with agents such as lower alkyl halides (*e.g*., methyl, ethyl, and butyl chlorides, bromides and iodides), dialkyl sulfates (*e.g.*, dimethyl, diethyl, and dibutyl sulfates), long chain halides (*e.g.,* decyl, lauryl, and stearyl chlorides, bromides and iodides), aralkyl halides (*e.g*., benzyl and phenethyl bromides), and others.

All such acid salts and base salts are intended to be pharmaceutically acceptable salts within the scope of the invention and all acid and base salts are considered equivalent to the free forms of the corresponding compounds for purposes of the invention.

Diastereomeric mixtures can be separated into their individual diastereomers on the basis of their physical chemical differences by methods well-known to those skilled in the art, such as, for example, by chromatography and/or fractional crystallization. Enantiomers can be separated by converting the enantiomeric mixture into a diastereomeric mixture by reaction with an appropriate optically active compound (*e.g*., chiral auxiliary such as a chiral alcohol or Mosher's acid chloride), separating the diastereomers and converting (*e.g*., hydrolyzing) the individual diastereomers to the corresponding pure enantiomers. Sterochemically pure compounds may also be prepared by using chiral starting materials or by employing salt resolution techniques. Also, some of the Fused Bicyclic Pyrazole Derivatives may be atropisomers (*e.g*., substituted biaryls), and are considered as part of this invention. Enantiomers can also be directly separated using chiral chromatographic techniques.

It is also possible that the Fused Bicyclic Pyrazole Derivatives may exist in different tautomeric forms, and all such forms are embraced within the scope of the invention. For example, all keto-enol and imine-enamine forms of the compounds are included in the invention.

All stereoisomers (for example, geometric isomers, optical isomers and the like) of the present compounds (including those of the salts) such as those which may exist due to asymmetric carbons on various substituents, including enantiomeric forms (which may exist even in the absence of asymmetric carbons), rotameric forms, atropisomers, and diastereomeric forms, are contemplated within the scope of this invention. If an Fused Bicyclic Pyrazole Derivative incorporates a double bond or a fused ring, both the cis- and trans-forms, as well as mixtures, are embraced within the scope of the invention.

Individual stereoisomers of the compounds of the invention may, for example, be substantially free of other isomers, or may be admixed, for example, as racemates or with all other, or other selected, stereoisomers. The chiral centers of the present invention can have the S or R configuration as defined by the *IUPAC* 1974 Recommendations. The use of the terms "salt", "solvate", "ester", "prodrug" and the like, is intended to apply equally to the salt, solvate, ester and prodrug of enantiomers, stereoisomers, rotamers, tautomers, positional isomers, racemates or prodrugs of the inventive compounds.

In the Compounds of Formula (I), the atoms may exhibit their natural isotopic abundances, or one or more of the atoms may be artificially enriched in a particular isotope having the same atomic number, but an atomic mass or mass number different from the atomic mass or mass number predominantly found in nature. The present invention is meant to include all suitable isotopic variations of the compounds of generic Formula I. For example, different isotopic forms of hydrogen (H) include protium (¹H), and deuterium (²H). Protium is the predominant hydrogen isotope found in nature. Enriching for deuterium may afford certain therapeutic advantages, such as increasing *in vivo* half-life or reducing dosage requirements, or may provide a compound useful as a standard for characterization of biological samples. Isotopically-enriched Compounds of Formula (I) can be prepared without undue experimentation by conventional techniques well known to those skilled in the art or by processes analogous to those described in the Schemes and Examples herein using appropriate isotopically-enriched reagents and/or intermediates. In one embodiment, a Compound of Formula (I) has one or more of its hydrogen atoms replaced with deuterium.

Polymorphic forms of the Fused Bicyclic Pyrazole Derivatives, and of the salts, solvates, hydrates, esters and prodrugs of the Fused Bicyclic Pyrazole Derivatives, are intended to be included in the present invention.

The following abbreviations are used below and have the following meanings: AcOH is acetic acid; ATCC is American Type Culture Collection; Dess-Martin Periodinane is Tris(acetyloxy)-1,1-dihydro-1,2-benziodoxol-3-(1H)-one; DCM is dichloromethane; DIEA is diisopropylethylamine; DMF is NN-dimethylformamide; DMSO is dimethylsulfoxide; EDTA is ethylenediaminetetraacetic acid; ESI is electrospray ionization; EtOAc is ethyl acetate; EtOH is ethanol; HATU is 1-[Bis(dimethylamino)methylene]-1*H*-1,2,3-triazolo[4,5-*b*]pyridinium 3-oxid hexafluorophosphate; HEPES is 2-[4-(2-hydroxyethyl)piperazin-1-yl]ethanesulfonic acid; HPLC is high performance liquid chromatography; IPA is isopropanol; Ir[dF(CF₃)ppy]₂(dtbbpy)PF₆ is [4,4'-*Bis*(1,1-dimethylethyl)-2,2'-bipyridine-*N*1,*N*1*'*]*bis*[3,5-difluoro-2-[5-(trifluoromethyl)-2-pyridinyl-*N*]phenyl-C]Iridium(III) hexafluorophosphate; LCMS is liquid chromatography/mass spectrometry; LED is light-emitting diode; Me is methyl; MeOH is methanol; MS is mass spectrometry; NMP is N-methylpyrrolidinone; PSI is pounds/square inch; SFC is supercritical fluid chromatography; t-butyl is tertiary butyl; TFA is trifluoroacetic acid; THF is tetrahydrofuran; and TLC is thin-layer chromatography.

### The Compounds of Formula (I)

The present invention provides Fused Bicyclic Pyrazole Derivatives of Formula (I): 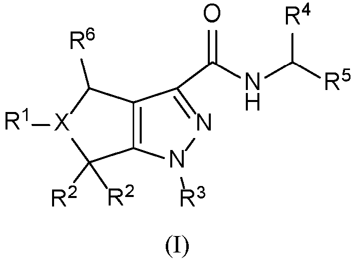 and pharmaceutically acceptable salts thereof, wherein X, R¹, R², R³, R⁴, R⁵ and R⁶ are as defined above for the Compounds of Formula (I).

In one embodiment, X is N.

In another embodiment, X is CH.

In another embodiment, X is C(OH).

In still another embodiment, X is C(CH₃).

In one embodiment, R¹ is selected from:

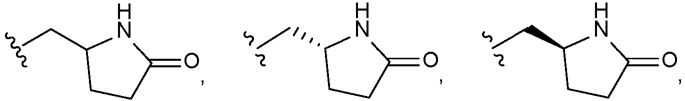

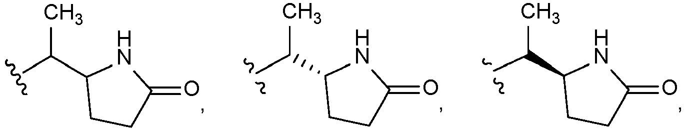

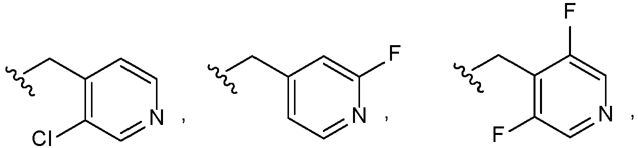

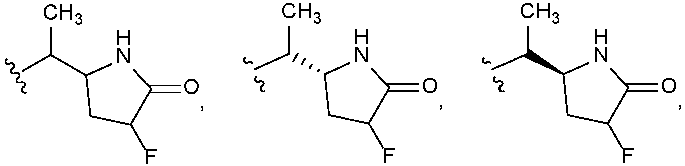

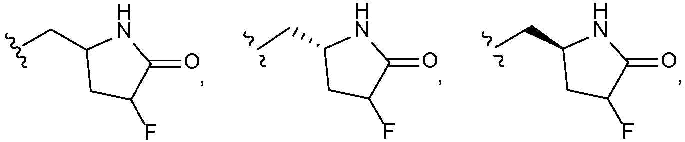

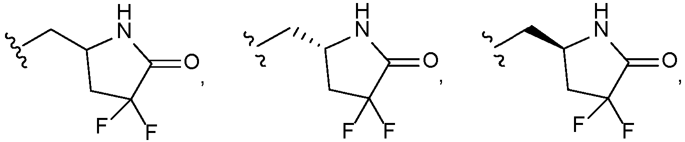

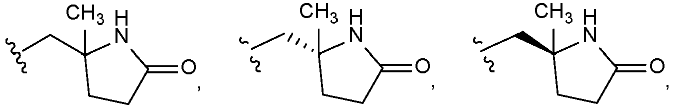

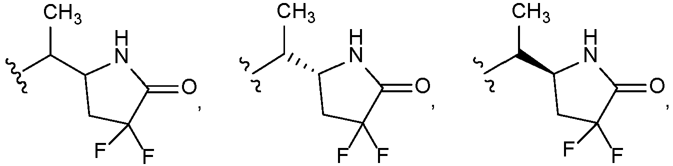

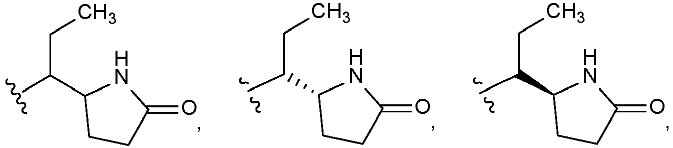

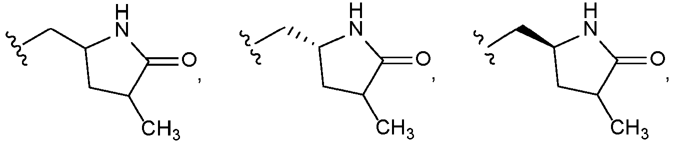

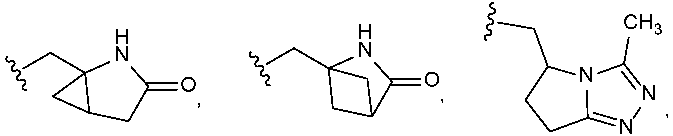

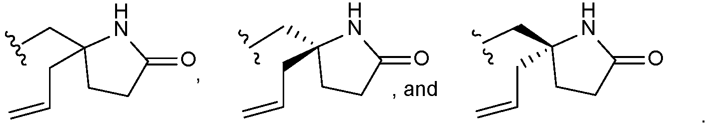

In another embodiment, R¹ is selected from

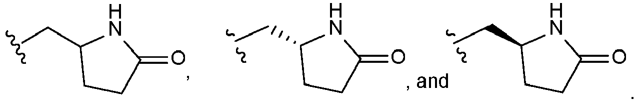

In another embodiment, X is N, and R¹ is selected from

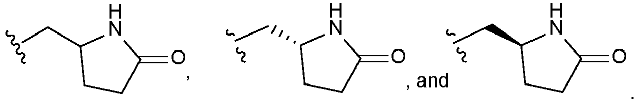

In another embodiment, X is CH, and R¹ is selected from

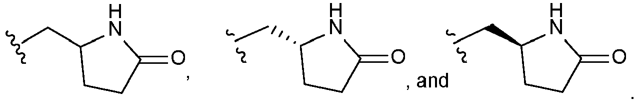

In one embodiment, R² is selected from H, methyl, ethyl, isopropyl, -CH₂OC(O)CH₃, - CH₂OH, or both R² groups, together with the carbon atom to which they are attached, combine to form a spirocyclic cyclobutyl group.

In another embodiment, each R² is H.

In another embodiment, one occurrence of R² is H, and the other occurrence of R² is other than H.

In one embodiment, R³ is selected from H, methyl, ethyl, isopropyl, -CHF₂, - CH₂CH(OH)CH(CH₃)₂, -CH₂C(OH)(CH₃)₂, -CD₃, -CH₂CH₂NHC(O)CH(CH₃)₂, thiazolyl, pyridyl, pyrimidinyl, indazolyl, 1,2,5-thiadiazolyl, oxazolyl, -CH₂CH₂-morpholinyl, pyrazolyl, - CH₂CH₂-pyrazolyl, thiazolyl, tetrahydrofuranyl, -CH₂CH₂-tetrahydrofuranyl, -CH₂-thiazolyl, tetrahydropyranyl, cyclopentanyl, azetidinyl, -CH₂-oxetanyl, -CH₂CH₂NHC(O)-cyclobutanyl, - CH₂CH₂N(CH₃)C(O)-cyclobutanyl, -CH₂CH₂N(CH₃)C(O)-cyclopropanyl, -CH₂CH₂-azetidinyl, and 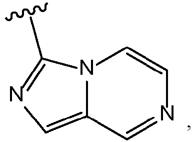 wherein said thiazolyl group, said pyridyl group, said pyrimidinyl group, said indazolyl group, said 1,2,5-thiadiazolyl group, said oxazolyl group, said pyrazolyl group, said tetrahydrofuranyl group, said -CH₂-thiazolyl group, said tetrahydropyranyl group, said cyclopentanyl group, said azetidinyl group, said oxetanyl group, said cyclobutanyl group, and said cyclopropanyl group can be optionally substituted with one or more of the following groups, which can be the same or different: F, Cl, methyl, ethyl, isopropyl, isobutyl, t-butyl, ethoxy, -OH, -CH₂OH, -CH₂F, -CH₂-cyclopropanyl, and phenyl.

In another embodiment, R³ is selected from methyl, CH₂CH₂NHC(O)-cyclobutanyl, - CH₂CH₂N(CH₃)C(O)-cyclobutanyl, and -CH₂CH₂N(CH₃)C(O)-cyclopropanyl.

In one embodiment, R⁴ is H.

In one embodiment, R⁴ is methyl.

In one embodiment, R⁵ is phenyl, which is substituted with one or more of the following groups, which can be the same or different: F, Cl and CN.

In another embodiment, R⁵ is:

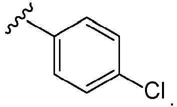

In one embodiment, R⁶ is H.

In another embodiment, R⁶ is methyl.

In one embodiment:
X is N;
R¹ is selected from: 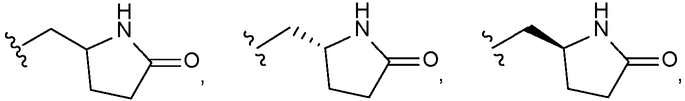 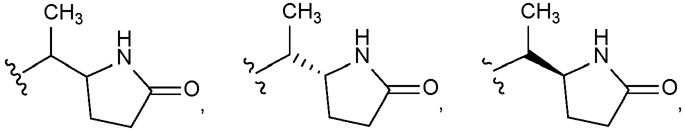 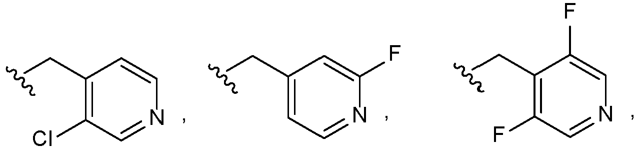 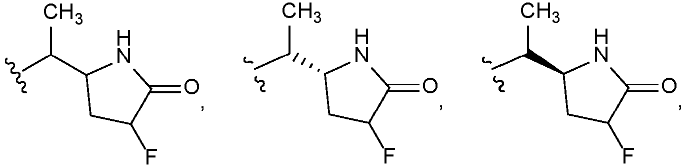 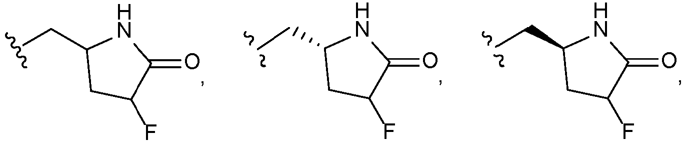 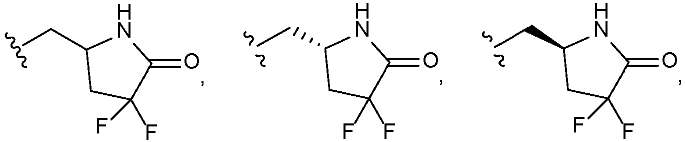 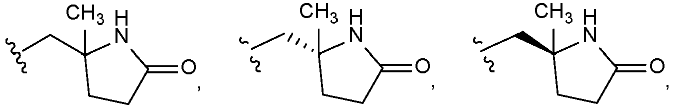 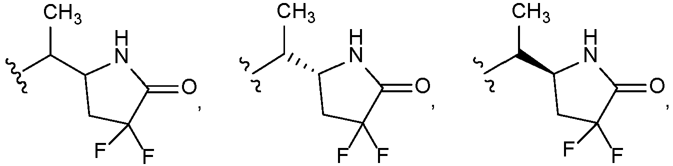 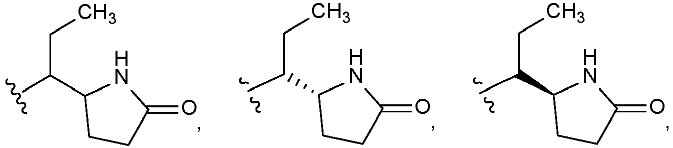 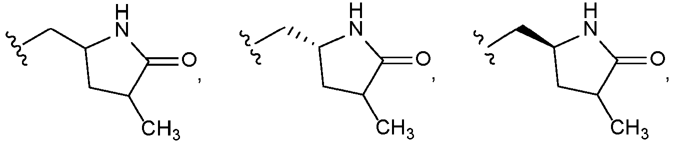 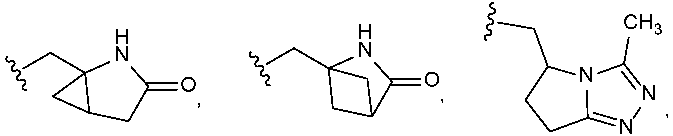 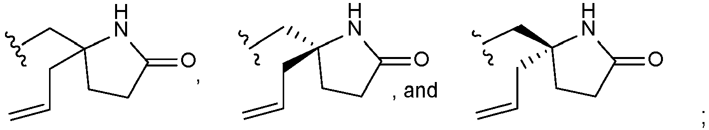
R² is selected from H, methyl, ethyl, isopropyl, -CH₂OC(O)CH₃, -CH₂OH, or both R² groups, together with the carbon atom to which they are attached, combine to form a spirocyclic cyclobutyl group;
R³ is selected from H, methyl, ethyl, isopropyl, -CHF₂, -CH₂CH(OH)CH(CH₃)₂, - CH₂C(OH)(CH₃)₂, -CD₃, -CH₂CH₂NHC(O)CH(CH₃)₂, thiazolyl, pyridyl, pyrimidinyl, indazolyl, 1,2,5-thiadiazolyl, oxazolyl, -CH₂CH₂-morpholinyl, pyrazolyl, -CH₂CH₂-pyrazolyl, thiazolyl, tetrahydrofuranyl, -CH₂CH₂-tetrahydrofuranyl, -CH₂-thiazolyl, tetrahydropyranyl, cyclopentanyl, azetidinyl, -CH₂-oxetanyl, -CH₂CH₂NHC(O)-cyclobutanyl, - CH₂CH₂N(CH₃)C(O)-cyclobutanyl, -CH₂CH₂N(CH₃)C(O)-cyclopropanyl, -CH₂CH₂-azetidinyl, and 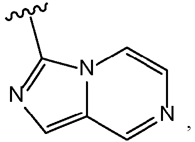 wherein said thiazolyl group, said pyridyl group, said pyrimidinyl group, said indazolyl group, said 1,2,5-thiadiazolyl group, said oxazolyl group, said pyrazolyl group, said tetrahydrofuranyl group, said -CH₂-thiazolyl group, said tetrahydropyranyl group, said cyclopentanyl group, said azetidinyl group, said oxetanyl group, said cyclobutanyl group, and said cyclopropanyl group can be optionally substituted with one or more of the following groups, which can be the same or different: F, Cl, methyl, ethyl, isopropyl, isobutyl, t-butyl, ethoxy, -OH, -CH₂OH, -CH₂F, -CH₂-cyclopropanyl, and phenyl;
R⁴ is H;
R⁵ is: 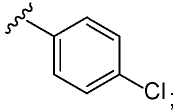 and
R⁶ is H.

In another embodiment:
X is N;
R¹ is selected from: 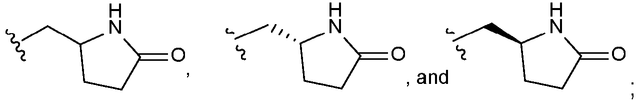
R² is selected from H, methyl, ethyl, isopropyl, -CH₂OC(O)CH₃, -CH₂OH, or both R² groups, together with the carbon atom to which they are attached, combine to form a spirocyclic cyclobutyl group;
R³ is selected from methyl, CH₂CH₂NHC(O)-cyclobutanyl, -CH₂CH₂N(CH₃)C(O)-cyclobutanyl, and -CH₂CH₂N(CH₃)C(O)-cyclopropanyl;
R⁴ is H;
R⁵ is: 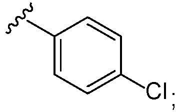 and
R⁶ is H.

It is understood that the present invention includes any combination of two or more of the above embodiments.

It is understood that the present invention encompasses compounds of formula (I) in isolated and purified form.

Other embodiments of the present invention include the following:
(a) A pharmaceutical composition comprising an effective amount of a Compound of Formula (I) or a pharmaceutically acceptable salt thereof, and a pharmaceutically acceptable carrier.
(b) The pharmaceutical composition of (a), further comprising a second therapeutic agent selected from the group consisting of anti-herpes agents and immunomodulators.
(c) The pharmaceutical composition of (b), wherein the anti-herpes agent is selected from the group consisting of herpesvirus polymerase inhibitors, and CMV terminase inhibitors.
(d) A pharmaceutical combination that is (i) a Compound of Formula (I), and (ii) a second therapeutic agent selected from the group consisting of anti-herpes agents and immunomodulators; wherein the Compound of Formula (I), and the second therapeutic agent are each employed in an amount that renders the combination effective for inhibiting herpesvirus replication, or for treating herpesvirus infection and/or reducing the likelihood or severity of symptoms of herpesvirus infection.
(e) The combination of (d), wherein the anti-herpes agent is selected from the group consisting of herpesvirus polymerase inhibitors, and CMV terminase inhibitors.
(f) A method of inhibiting herpesvirus replication in a subject in need thereof which comprises administering to the subject an effective amount of a Compound of Formula (I).
(g) A method of treating herpesvirus infection and/or reducing the likelihood or severity of symptoms of herpesvirus infection in a subject in need thereof which comprises administering to the subject an effective amount of a Compound of Formula (I).
(h) The method of (g), wherein the Compound of Formula (I) is administered in combination with an effective amount of at least one second therapeutic agent selected from the group consisting of anti-herpes agents and immunomodulators.
(i) The method of (h), wherein the anti-herpes agent is selected from the group consisting of herpesvirus polymerase inhibitors, and CMV terminase inhibitors.
(j) A method of inhibiting herpesvirus replication in a subject in need thereof which comprises administering to the subject the pharmaceutical composition of (a), (b) or (c) or the combination of (d) or (e).
(k) A method of treating herpesvirus infection and/or reducing the likelihood or severity of symptoms of herpesvirus infection in a subject in need thereof which comprises administering to the subject the pharmaceutical composition of (a), (b) or (c) or the combination of (d) or (e).

The present invention also includes a compound of the present invention for use (i) in, (ii) as a medicament for, or (iii) in the preparation of a medicament for: (a) medicine; (b) inhibiting herpesvirus replication or (c) treating herpesvirus infection and/or reducing the likelihood or severity of symptoms of herpesvirus infection. In these uses, the compounds of the present invention can optionally be employed in combination with one or more second therapeutic agents selected from anti-herpes agents, anti-infective agents, and immunomodulators.

Additional embodiments of the invention include the pharmaceutical compositions, combinations and methods set forth in (a)-(k) above and the uses set forth in the preceding paragraph, wherein the compound of the present invention employed therein is a compound of one of the embodiments, aspects, classes, sub-classes, or features of the compounds described above. In all of these embodiments, the compound may optionally be used in the form of a pharmaceutically acceptable salt or hydrate as appropriate.

It is further to be understood that the embodiments of compositions and methods provided as (a) through (k) above are understood to include all embodiments of the compounds, including such embodiments as result from combinations of embodiments.

Non-limiting examples of the Compounds of Formula (I) include compounds **1-140,** as set forth in the Examples below, and pharmaceutically acceptable salts thereof.

### Methods For Making the Compounds of Formula (I)

The Compounds of Formula (I) may be prepared from known or readily prepared starting materials, following methods known to one skilled in the art of organic synthesis. Methods useful for making the Compounds of Formula (I) are set forth in the Examples below Alternative synthetic pathways and analogous structures will be apparent to those skilled in the art of organic synthesis.

One skilled in the art of organic synthesis will recognize that the synthesis of multicyclic heterocycle cores contained in Compounds of Formula (I) may require protection of certain functional groups (*i.e.,* derivatization for the purpose of chemical compatibility with a particular reaction condition). Suitable protecting groups for the various functional groups of these Compounds and methods for their installation and removal are well known in the art of organic chemistry. A summary of many of these methods can be found in Greene et al., Protective Groups in Organic Synthesis, Wiley-Interscience, New York, (1999).

One skilled in the art of organic synthesis will also recognize that one route for the synthesis of the multicyclic heterocycle cores of the Compounds of Formula (I) may be more desirable depending on the choice of appendage substituents.

Additionally, one skilled in the art will recognize that in some cases the order of reactions may differ from that presented herein to avoid functional group incompatibilities and thus adjust the synthetic route accordingly.

The preparation of multicyclic intermediates useful for making the multicyclic heterocycle cores of the Compounds of Formula (I) have been described in the literature and in compendia such as "Comprehensive Heterocyclic Chemistry" editions I, II and III, published by Elsevier and edited by A.R. Katritzky & R. JK Taylor. Manipulation of the required substitution patterns have also been described in the available chemical literature as summarized in compendia such as "Comprehensive Organic Chemistry" published by Elsevier and edited by DH R. Barton and W. D. Ollis; "Comprehensive Organic Functional Group Transformations" edited by edited by A.R. Katritzky & R. JK Taylor and "Comprehensive Organic Transformation" published by Wiley-CVH and edited by R. C. Larock.

The starting materials used, and the intermediates prepared using the methods set forth in the Examples below may be isolated and purified if desired using conventional techniques, including but not limited to filtration, distillation, crystallization, chromatography and alike. Such materials can be characterized using conventional means, including physical constants and spectral data.

One skilled in the art will be aware of standard formulation techniques as set forth in the open literature as well as in textbooks such as Zheng, "Formulation and Analytical Development for Low-dose Oral Drug Products," Wiley, 2009, ISBN.

### EXAMPLES

### General Methods

Solvents, reagents, and intermediates that are commercially available were used as received. Reagents and intermediates that are not commercially available were prepared in the manner as described below. ¹H NMR spectra are reported as ppm downfield from (CH₃)₄Si with number of protons, multiplicities, and coupling constants in Hertz indicated parenthetically. Where LC/MS data are presented, the observed parent ion is given. Flash column chromatography was performed using pre-packed normal phase silica or bulk silica, typically using a gradient elution of hexanes or petroleum ether and ethyl acetate, from 100% hexanes/petroleum ether to 100% ethyl acetate. In cases where diastereomers or enantiomers are separated, diastereomeric and enantiomeric excess (%de and %ee, respectively) typically exceeds 95%. In cases where absolute stereochemistry is not shown, the tags "chiral" and "single isomer" are applied.

### Example 1

### Preparation of Int-1

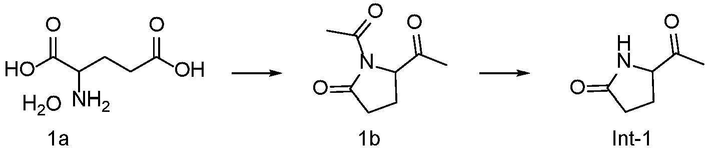

### Step A - synthesis of compound 1a

A mixture of 2-aminopentanedioic acid hydrate (5.00 g, 30.3 mmol), and 4-dimethylaminopyridine (0.370 g, 3.03 mmol) in acetic anhydride (16.0 mL, 170 mmol), and triethylamine (16.0 mL, 115 mmol) was heated to 60 °C, and allowed to stir for about 15 hours. The reaction mixture was concentrated in vacuo and diluted with dichloromethane. The organic phase was then washed three times with water, dried over Na₂SO₄, filtered, and concentrated in vacuo, and the resulting residue was purified using silica gel chromatography eluting with 0-70% (3:1 EtOAc:EtOH)/heptanes to provide **1b** as an oil. MS: *m*/*z* 170.0 = [M+H].

### Step B - synthesis of Int-1

To a mixture of 1b (2.10 g, 12.4 mmol), and sodium carbonate (4.30 g, 40.6 mmol) in water (30 mL) was allowed to stir at room temperature for 4 hours. The reaction mixture was concentrated in vacuo, and the resulting residue was slurried in CH₂Cl₂ (100 mL), and filtered. The filtrate was concentrated in vacuo to provide Int-1 as an oil, which was used without further purification. MS: *m*/*z* 255.1 = [Mx2+H]. ¹H NMR (400 MHz, CDCl₃, ppm): δ 7.03 (s, 1H), 4.23-4.28 (m, 1H), 2.42-2.56 (m, 1H), 2.31-2.41 (m, 2H), 2.23 (s, 3H), 2.05-2.12 (m, 1H).

### Example 2

### Preparation of Compounds 1 and 2

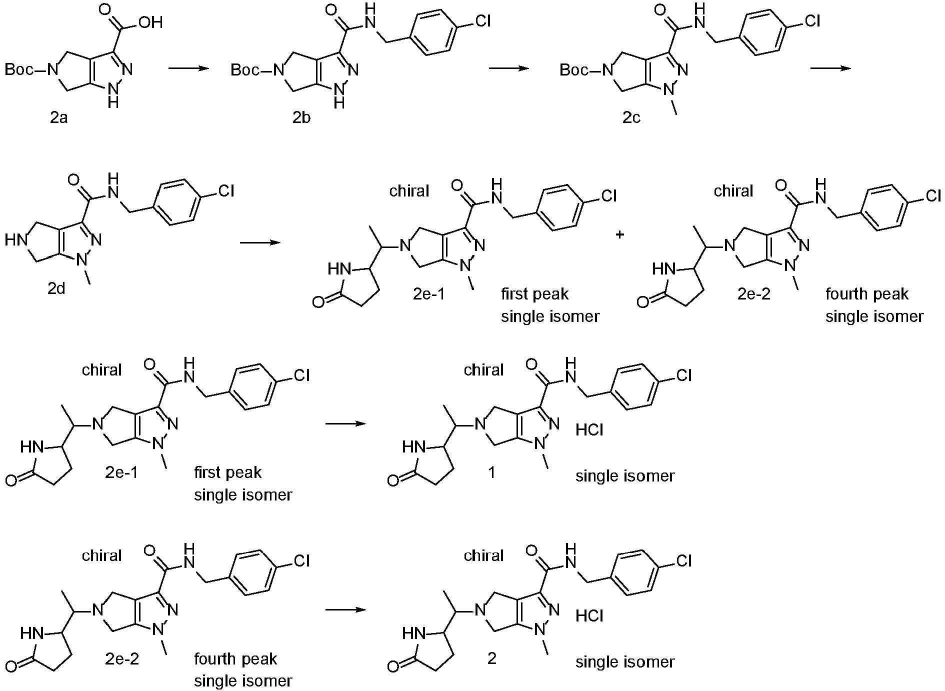

### Step A - synthesis of compound 2b

To a mixture of 2a (6.50 g, 25.7 mmol) in DMF (150 mL) was added 2-(3H-[1,2,3]triazolo[4,5-b]pyridin-3-yl)-1,1,3,3-tetramethylisouronium hexafluorophosphate(V) (HATU, 10.7 g, 28.2 mmol), and the resulting reaction was allowed to stir at room temperature for 30 minutes . (4-chlorophenyl)methanamine (4.36 g, 30.8 mmol), and triethylamine (5.19 g, 51.3 mmol) were added, and the resulting reaction was stirred an additional 2 hours. The reaction mixture was diluted with water, extracted with ethyl acetate, washed with brine, dried over Na₂SO₄, and concentrated in vacuo. The resulting residue was purified using silica gel chromatography eluting with 0-10% MeOH/CH₂Cl₂ to provide **2b** as a solid. MS: *m*/*z* 377.2 = [M+H].

### Step B - synthesis of compound 2c

To a mixture of 2b (5.00 g, 13.3 mmol), and tribasic potassium phosphate (8.45 g, 39.8 mmol) in DMF (30 mL) was added iodomethane (3.77 g, 26.5 mmol) at 0 °C, and the resulting reaction was allowed to heat to room temperature, and allowed to stir at this temperature for 3 hours. The reaction mixture was diluted with water, extracted with ethyl acetate, washed with brine, dried over Na₂SO₄, and concentrated in vacuo. The resulting residue was purified using silica gel chromatography eluting with 0-100% EtOAc/petroleum ether to provide **2c** as a solid. MS: *m*/*z* 391.2 = [M+H].

### Step C - synthesis of compound 2d

To a mixture of **2c** (4.20 g, 10.8 mmol) in dichloromethane (12.0 mL) was added trifluoroacetic acid (8.0 mL) at 0 °C. The mixture was allowed to stir at room temperature for 1.5 hours, then concentrated in vacuo, and diluted with dichloromethane (100 mL). The resulting solution was adjusted to pH 10 with saturated, aq. NaHCO₃ (~100 mL), and filtered. The collected solid was dried in vacuo to provide **2d** as a solid, which was used without further purification. MS: *m*/*z* 291.1 = [M+H].

### Step D - synthesis of compounds 2e-1 and 2e-2

To a mixture of Int-1 (861 mg, 6.78 mmol), and 2d (657 mg, 2.26 mmol) in acetic acid (814 mg, 13.6 mmol) was added sodium cyanoborohydride (3.53 g, 56.1 mmol), and diluted with 1,2-dichloroethane (22.6 mL). The reaction was allowed to stir for 16 hours at 110 °C, then the reaction mixture was diluted with water (25 mL) and extracted with dichloromethane. The organic extract was washed with brine, dried over Na₂SO₄, filtered, and concentrated in vacuo. The residue obtained was purified using preparative HPLC (reverse-phase C-18), eluting with acetonitrile/water + 0.1% ammonium bicarbonate 10-60% to provide a mixture of isomers. MS: *m*/*z* 402.2 = [M+H].

Chiral resolution by Prep-SFC using an AS-H (2x15 cm) column, eluting with 40% MeOH (+0.1% diethylamine) in CO₂ at 100 bar, 50 mL/min, provided 2e-1 (first peak).

Chiral resolution using an OJ-H (2x25 cm) column, eluting with 30% MeOH (+0.1% diethylamine) in CO₂ at 100 bar, 60 mL/min, provided 2e-2 (fourth peak).

### Step E - synthesis of compound 1

To a solution of 2e-1 (99.0 mg, 0.246 mmol) in dioxane (2500 µL), and water (500 µL) was added HCl (4 M in dioxane, 61.6 µL). The reaction mixture was allowed to stir at room temperature for 1 hour, then concentrated in vacuo to provide 1 as a solid. MS: *m*/*z* 402.2 and 404.2 = [M+H for ³⁵Cl and ³⁷Cl]. ¹H NMR (600 MHz, DMSO-*d₆*, ppm): δ 7.32 (d, *J* = 8.4 Hz, 2H), 7.25 (d, *J* = 8.4 Hz, 2H), 4.89 (d, *J* = 7.8 Hz, < 1H), 4.73 (dd, *J* = 31.8, 13.8 Hz, 1H), 4.48-4.58 (m, 2H), 4.33 (s, 2H), 4.13 (d, *J* = 45.0 Hz, 1H), 3.82 (s, 3H), 3.71 (d, *J* = 6.6 Hz, 1H), 3.41-3.68 (m, 1H), 2.08-2.26 (m, 3H), 1.71 (br s, 1H), 1.19-1.24 (m, 3H).

### Step F - synthesis of compound 2

To a solution of 2e-2 (91.0 mg, 0.226 mmol) in dioxane (2500 µL), and water (500 µL) was added HCl (4 M in dioxane, 56.6 µL). The reaction mixture was allowed to stir at room temperature for 1 hour, then was concentrated in vacuo to provide 2 as a solid. MS: *m*/*z* 402.2 and 404.2 = [M+H for ³⁵Cl and ³⁷Cl]. ¹H NMR (600 MHz, DMSO-*d₆*, ppm): δ 7.32 (d, *J* = 8.4 Hz, 2H), 7.25 (d, *J* = 8.4 Hz, 2H), 4.89 (d, *J* = 7.8 Hz, < 1H), 4.72 (dd, *J* = 39.6, 13.8 Hz, 1H), 4.48-4.58 (m, 2H), 4.33 (s, 2H), 4.13 (dt, *J* = 48.0, 7.2 Hz, 1H), 3.82 (s, 3H), 3.71 (d, *J* = 6.6 Hz, 1H), 3.41-3.68 (m, 1H), 2.10-2.27 (m, 3H), 1.70 (br s, 1H), 1.19-1.24 (m, 3H).

### Example 3

### Preparation of Int-2 and Int-3

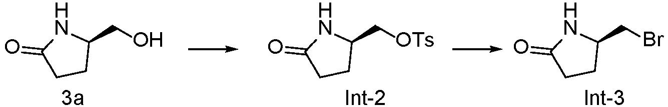

### Step A - synthesis of Int-2

To a mixture of 3a (4.24 g, 34.7 mmol), and triethylamine (229.0 g, 2258 mmol) in dichloromethane (2000 mL) was added 4-methylbenzene-1-sulfonyl chloride (397.0 g, 2085 mmol). The reaction mixture was allowed to stir for 16 hours at room temperature. The reaction was quenched with saturated, aqueous NH₄Cl (1000 mL), and then extracted with dichloromethane, washed with brine, dried over Na₂SO₄, and concentrated in vacuo. The resulting residue was purified using silica gel chromatography eluting with 30-60% EtOAc/petroleum ether to provide Int-2. MS: *m*/*z* 270.1 = [M+H].

### Step B - synthesis of Int-3

A mixture of Int-2 (40.0 g, 134 mmol), lithium bromide (58.0 g, 668 mmol), and acetone (800 mL) was allowed to stir for 16 hours at 60 °C. The reaction mixture was concentrated in vacuo, diluted with water (500 mL), and extracted with ethyl acetate. The organic extract was washed with saturated, aqueous sodium thiosulfate (2 × 500 mL), and water (1000 mL), dried over Na₂SO₄, and concentrated in vacuo to provide Int-3 which was used without further purification. MS: *m*/*z* 178.0 and 180.0 = [M+H for ⁷⁹Br and ⁸¹Br].

### Example 4

### Preparation of Compound 3

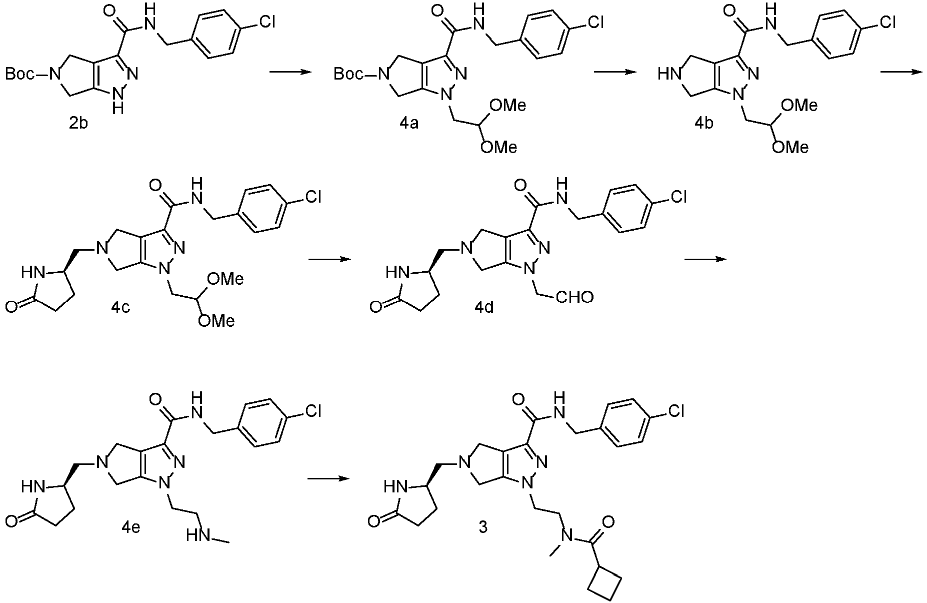

### Step A - synthesis of compound 4a

To a mixture of 2b (300 mg, 0.798 mmol), and tribasic potassium phosphate (338 mg, 1.60 mmol) in acetonitrile (4 mL) was added 2-bromo-1,1-dimethoxyethane (148 mg, 0.878 mmol), and the resulting reaction was allowed to stir for 5 hours at 80 °C. The reaction mixture was concentrated in vacuo, and the resulting residue was purified using silica gel chromatography eluting with 10-60% EtOAc/petroleum ether to provide 4a. MS: *m*/*z* 465.3 = [M+H].

### Step B - synthesis of compound 4b

To a mixture of 4a (330 mg, 0.647 mmol) in dichloromethane (2 mL) was added trifluoroacetic acid (1 mL, 0.366 mmol), and the resulting reaction was allowed to stir for 30 minutes at room temperature. The reaction mixture was diluted with saturated, aqueous Na₂CO₃ (30 mL), extracted with dichloromethane, dried over Na₂SO₄, and concentrated in vacuo to provide 4b, which was used without further purification. MS: *m*/*z* 365.1 = [M+H].

### Step C - synthesis of compound 4c

To a mixture of 4b (240 mg, 0.658 mmol), and tribasic potassium phosphate (279 mg, 1.32 mmol) in acetonitrile (3 mL) was added Int-2 (195 mg, 0.724 mmol), and the resulting reaction was allowed to stir for 5 hours at 80 °C. The reaction mixture was concentrated in vacuo, and the resulting residue was purified using silica gel chromatography eluting with 10-60% EtOAc/petroleum ether to provide 4c. MS: *m*/*z* 462.2 = [M+H].

### Step D - synthesis of compound 4d

To a mixture of 4c (200 mg, 0.433 mmol) in acetone (2.5 mL) was added hydrochloric acid (6 N, 2.5 mL), and the resulting reaction was allowed to stir for 2 hours at 60 °C. The reaction mixture was concentrated in vacuo to provide 4d, which was used without further purification. MS: *m*/*z* 416.2 = [M+H].

### Step E - synthesis of compound 4e

To a mixture of 4d (100 mg, 0.240 mmol), and methanamine (37 mg, 1.2 mmol) in methanol (3 mL) was added acetic acid (30 µL, 0.52 mmol), and sodium cyanotrihydroborate (23 mg, 0.36 mmol), and the resulting reaction was allowed to stir for 1 hour at room temperature. The reaction mixture was concentrated in vacuo, diluted with water (20 mL), and extracted with ethyl acetate. The organic extract was washed with brine, dried over Na₂SO₄, and concentrated in vacuo to provide 4e, which was used without further purification. MS: *m*/*z* 431.3 = [M+H].

### Step F - synthesis of compound 3

A mixture of cyclobutanecarboxylic acid (19 mg, 0.20 mmol), and 2HATU (74 mg, 0.20 mmol) in DMF (1.5 mL) was allowed to stir for 30 minutes at room temperature. Diisopropylethylamine (42 mg, 0.33 mmol), and 4e (70 mg, 0.16 mmol) were then added, and the resulting reaction was allowed to stir for 2 hours at room temperature. The reaction mixture was diluted with water (15 mL), extracted with ethyl acetate, and the organic extract was washed with brine, dried over Na₂SO₄, filtered and concentrated in vacuo. The resulting residue was purified using preparative HPLC (reverse-phase C-18), eluting with acetonitrile/water + 10 mmol/L ammonium bicarbonate 40-90% to provide **3.** MS: *m*/*z* 542.3 = [M+H]. ¹H NMR (400 MHz, methanol-*d*₄, ppm): δ 7.33 (s, 4H), 5.10-4.98 (m, 1H), 4.81-4.57 (m, 2H), 4.50 (s, 2H), 4.42-4.37 (m, 2H), 4.23-4.19 (m, 1H), 3.85-3.63 (m, 4H), 3.35-3.25 (m, 2H), 2.97 (s, 3H), 2.58-2.33 (m, 3H), 2.10-1.83 (m, 6H), 1.72-1.60 (s, 1H).

The following compound of the present invention were made using methodology described in Example 4, Step E above, and substituting the appropriate reactants and/or reagents:

| Compound | Structure | MS [M+H] |
|---|---|---|
| 4 | 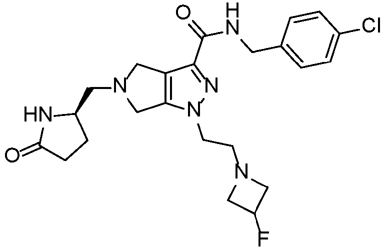 | 475.2 |

### Example 5

### Preparation of Int-4 and Int-5

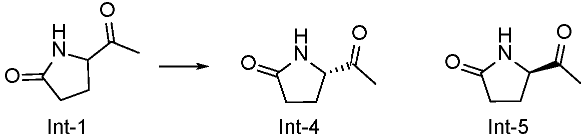

### Step A - resolution of Int-4 and Int-5

Int-1 was resolved using Prep-SFC using an AD-H (5x25 cm) column, eluting with 40% MeOH (+0.1% diethylamine) in CO₂ at 100 bar, 160 g/min, to provide Int-4 (fast peak, 99.6% ee), and Int-5 (slow peak, 99.6% ee), as yellow oils.

For Int-4: MS: *m*/*z* 128.1 = [M+H]. ¹H NMR (300MHz, CDCl₃, ppm): δ 7.22 (s, 1H), 4.24-4.29 (m, 1H), 2.46-2.56 (m, 1H), 2.35-2.45 (m, 2H), 2.22 (s, 3H), 2.04-2.36 (m, 1H). [α]D = +28.67° (c = 0.645 g/100 mL in MeOH, T = 16.5 °C).

The absolute configuration of Int-4 was assigned to be (S) using Vibrational Circular Dichroism (VCD) spectroscopy; analysis was performed by comparing experimental data to the calculated VCD and IR spectra of the (*R*) configuration. The experimental VCD spectrum of Int-4 coincided with the mirror image of the calculated Int-5 spectrum over the region from 1000-1600 cm-1, resulting in an assignment of (S).

For Int-5: MS: *m*/*z* 128.1 = [M+H]. ¹H NMR (400 MHz, CDCl₃, ppm): δ 7.05 (s, 1H), 4.25-4.28 (m, 1H), 2.50-2.55 (m, 1H), 2.38-2.50 (m, 2H), 2.37 (s, 3H), 2.04-2.37 (m, 1H). [α]D = -4.1° (c = 0.39 g/100 mL in CHCl₃, T = 20.1 °C).

### Example 6

### Preparation of Compounds 5 and 6

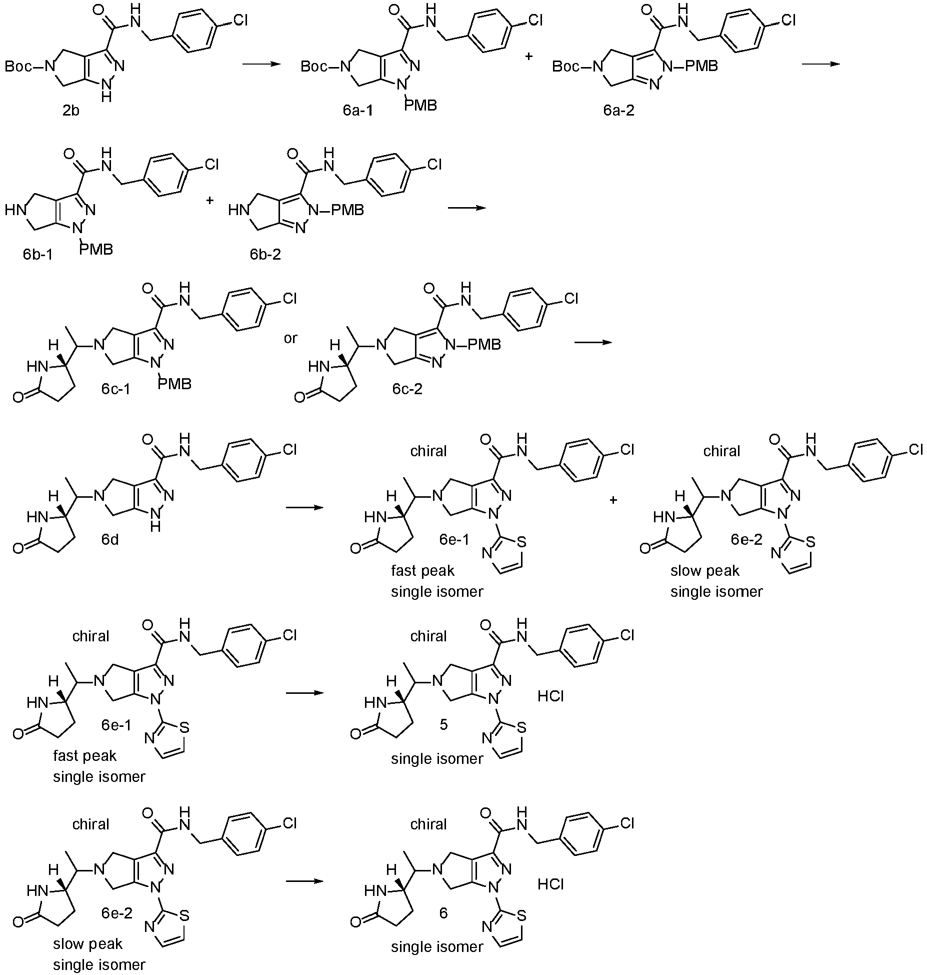

### Step A - synthesis of compounds 6a-1 and 6a-2

To a mixture of 2b (16.2 g, 43.0 mmol), and tribasic potassium phosphate (27.4 g, 129 mmol) in DMF (172 mL) was added dropwise 4-methoxybenzyl chloride (8.78 mL, 64.5 mmol) at room temperature, and the resulting reaction was allowed to stir for about 15 hours. The reaction mixture was diluted with water (500 mL), extracted with ethyl acetate, and the organic extract was washed with brine, dried over MgSO₄, filtered, and concentrated in vacuo. The resulting residue was purified using silica gel chromatography eluting with 0-65% EtOAc/hexanes to provide a mixture of 6a-1 and 6a-2 as a solid. MS: *m*/*z* 497.4 = [M+H].

### Step B - synthesis of compound 6b-1 and 6b-2

To a mixture of 6a-1 and 6a-2 (15.0 g, 30.2 mmol) in dioxane (101 mL) was cooled at 0 ° C, and HCl (4 M in dioxane, 14.89 mL) was added. The resulting reaction was allowed to stir at room temperature for 2.5 hours, then more HCl (45 mL) was added, and stirring continued for about 15 additional hours. The reaction mixture was cooled at 0 °C, and basified to pH 9-10 with saturated, aqueous Na₂CO₃ (~45 mL). The mixture was extracted with ethyl acetate (aqueous phase was not discarded), washed with brine, dried over MgSO₄, and concentrated in vacuo to provide a mixture of 6b-1 and 6b-2, which was used without further purification. MS: *m*/*z* 397.4 = [M+H].

The collected aqueous phase was filtered, and the collected solid was washed with water and dried to provide either 6b-1 or 6b-2 as a single regioisomer, which was used without further purification. MS: *m*/*z* 397.4 = [M+H].

### Step C - synthesis of compound 6c-1 or 6c-2

A mixture of 6b-1 or 6b-2 (single regioisomer isolated from aqueous mixture in Step B, 3.90 g, 9.83 mmol), and Int-4 (3.75 g, 29.5 mmol) in 1,2-dichloroethane (82 mL), and acetic acid (3.38 mL, 59.0 mmol) was sonicated to provide a solution, which was divided into 7 equal portions. Each portion was combined with MP-cyanoborohydride (Biotage, 3.2 mmol/g, 0.88 g, 2.82 mmol) then capped and irradiated at 120 ° C for 10 minutes. The 7 reaction mixtures were then combined and diluted with dichloromethane, filtered, and concentrated in vacuo. The resulting residue was twice azeotroped with heptane to remove bulk acetic acid, and the resulting residue was diluted with dichloromethane and basified using saturated, aqueous NaHCO₃. The aqueous phase was back-extracted with dichloromethane, and the combined organic extracts were washed with brine (with some NaHCO₃ added), dried over MgSO4, filtered, and concentrated in vacuo. The resulting residue was purified using silica gel chromatography eluting with 0-60% (3:1 EtOAc:EtOH)/hexanes to provide 6c-1 or 6c-2 as a pinkish foam (mixture of diastereomers). MS: *m*/*z* 508.4 = [M+H].

### Step D - synthesis of compound 6d

A solution of 6c-1 or 6c-2 (2.92 g, 5.75 mmol) in trifluoroacetic acid (14.37 mL) was flushed subsurface with nitrogen and cooled in an ice bath. Anisole (0.063 mL, 0.58 mmol) was added, purging continued momentarily, and then the reaction was heated at 80 °C and allowed to stir at this temperature for 3.5 hours. The reaction mixture was concentrated in vacuo, and then carefully (at 0 °C) basified to pH 8 by addition of saturated, aqueous NaHCO₃. The mixture was extracted with ethyl acetate, and the organic extract was washed with brine (with some NaHCO₃ added), dried over MgSO₄, filtered, and concentrated in vacuo. The resulting residue was purified using silica gel chromatography eluting with 0-100% (3:1 EtOAc:EtOH)/hexanes to provide 6d as a foam (mixture of diastereomers). MS: *m*/*z* 388.3 = [M+H].

### Step E - synthesis of compounds 6e-1 and 6e-2

A mixture of 6d (diastereomeric mixture, 150 mg, 0.387 mmol), 2-bromothiazole (139 µL, 1.55 mmol), and tribasic potassium phosphate (246 mg, 1.16 mmol) in dioxane (3.2 mL) was purged subsurface with nitrogen. (+/-)-*Trans*-1,2-diaminocyclohexane (93 µL, 0.77 mmol), and copper (I) iodide (73.7 mg, 0.387 mmol) were added, purging continued for 5 minutes, and then the reaction was capped and heated at 105 ° C for 24 hours. The reaction mixture was cooled to room temperature, concentrated in vacuo, and the resulting residue was purified using preparative HPLC (reverse-phase C-18), eluting with acetonitrile/water + 0.1% TFA 10-60%. Fractions containing the product were combined, basified with saturated, aqueous NaHCO₃, and extracted with ethyl acetate. The organic extract was washed with brine, dried over MgSO₄, and concentrated in vacuo to provide a mixture of diastereomers as a solid. The diastereomers were resolved using Prep-SFC using an OJ-H (2x25 cm) column, eluting with 35% MeOH (+0.1% diethylamine) in CO₂ at 100 bar, 60 mL/min, to provide 6e-1 (fast peak), and 6e-2 (slow peak). MS: *m*/*z* 471.4 = [M+H] for both compounds.

### Step F - synthesis of compound 5

Compound 5 was made from 6e-1, using the method described in Example 2, step E, and substituting the appropriate reactants and/or reagents. MS: *m*/*z* 471.4 = [M+H]. ¹H NMR (500 MHz, CDCl₃, ppm): δ 7.55 (d, *J* = 3.5 Hz, 1H), 7.31-7.35 (m, 4H), 7.23 (t, *J* = 6.0 Hz, 1H), 7.13 (d, *J* = 3.5 Hz, 1H), 5.88 (s, 1H), 4.61 (d, *J* = 6.0 Hz, 2H), 4.32 (d, *J* = 14.0 Hz, 1H), 4.23 (d, *J* = 14.0 Hz, 1H), 4.12 (d, *J* = 12.5 Hz, 1H), 4.04 (d, *J* = 12.5 Hz, 1H), 3.92-3.95 (m, 1H), 2.83-2.88 (m, 1H), 2.35-2.42 (m, 2H), 2.23-2.33 (m, 1H), 1.90-2.01 (m, 1H), 1.15 (d, *J* = 6.5 Hz, 3H).

### Step G - synthesis of compound 6

Compound 6 was made from 6e-2, using the method described in Example 2, step E, and substituting the appropriate reactants and/or reagents. MS: *m*/*z* 471.4 = [M+H]. ¹H NMR (500 MHz, CDCl₃, ppm): δ 7.55 (d, *J* = 3.5 Hz, 1H), 7.31-7.36 (m, 4H), 7.23 (t, *J* = 5.5 Hz, 1H), 7.14 (d, *J* = 3.5 Hz, 1H), 6.19 (s, 1H), 4.62 (d, *J* = 6.5 Hz, 2H), 4.33 (d, *J* = 14.0 Hz, 1H), 4.22 (d, *J* = 14.0 Hz, 1H), 4.13 (d, *J* = 12.5 Hz, 1H), 4.02 (d, *J* = 12.5 Hz, 1H), 3.59-3.64 (m, 1H), 2.76-2.82 (m, 1H), 2.36-2.50 (m, 2H), 2.20-2.25 (m, 1H), 1.66-1.79 (m, 1H), 1.06 (d, *J* = 6.5 Hz, 3H).

### Example 7

### Preparation of Compound 7

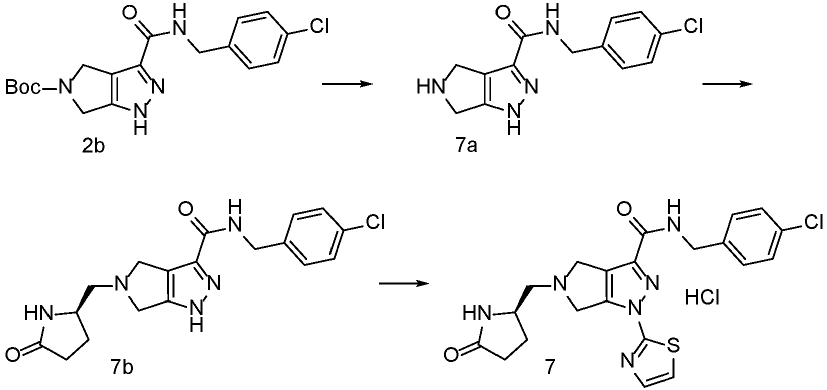

### Step A - synthesis of compound 7a

To a mixture of 2b (7.42 g, 19.7 mmol) in ethyl acetate (43 mL) at 0 °C, was added an HCl-saturated solution of ethyl acetate (36 mL). The resulting reaction was allowed to stir for 30 minutes, then allowed to warm to room temperature and stirred for an additional 65 hours. The reaction mixture was concentrated in vacuo to provide 7a as a thick oil, which was used without further purification. MS: *m*/*z* 277.1 and 279.1 = [M+H for ³⁵Cl and ³⁷Cl].

### Step B - synthesis of compound 7b

To a mixture of 7a (6.02 g, 19.2 mmol), and diisopropylethylamine (10.1 mL, 57.7 mmol) in DMF (77 mL) was added Int-3 (3.76 g, 21.1 mmol), and the resulting reaction was heated at 60 °C, and allowed to stir at this temperature for 8 hours. Additional Int-3 (342 mg, 1.92 mmol) was added, and stirring continued at the same temperature for an additional 52 hours. The reaction mixture was diluted with water, extracted with dichloromethane, and the organic extract washed with water, dried over Na₂SO₄, and concentrated in vacuo. The resulting residue was purified using silica gel chromatography eluting with 0-15% MeOH/CH₂Cl₂ to provide 7b as a foam. MS: *m*/*z* 374.2 and 376.2 = [M+H for ³⁵Cl and ³⁷Cl].

### Step C - synthesis of compound 7

Compound 7 was made from 7b, using the method described in Example 6, step E. The resulting crude product was purified using preparative HPLC (reverse-phase C-18), eluting with acetonitrile/water + 0.1% TFA 10-55%. Fractions containing the product were combined, basified with saturated, aqueous NaHCO₃, and extracted with ethyl acetate. The organic extract was washed with brine, dried over MgSO₄, filtered, and concentrated in vacuo to provide **7.** ¹H NMR (500 MHz, CDCl₃, ppm): δ 7.55 (d, *J* = 3.5 Hz, 1H), 7.31-7.37 (m, 4H), 7.20 (t, *J* = 6.5 Hz, 1H), 7.14 (d, *J* = 3.5 Hz, 1H), 6.01 (s, 1H), 4.61 (d, *J* = 6.0 Hz, 2H), 4.24-4.33 (m, 2H), 4.12-4.16 (m, 1H), 4.03-4.11 (m, 1H), 3.85-3.87 (m, 1H), 2.95 (dd, *J* = 12.5, 4.0 Hz, 1H), 2.82 (dd, *J* = 12.5, 5.5 Hz, 1H), 2.37-2.43 (m, 2H), 2.26-2.33 (m, 1H), 1.76-1.83 (m, 1H).

Compound 7 was subsequently converted to its HCl salt using the method described in Example 2, Step E. MS: *m*/*z* 457.4 = [M+H].

### Example 8

### Preparation of Compound 8

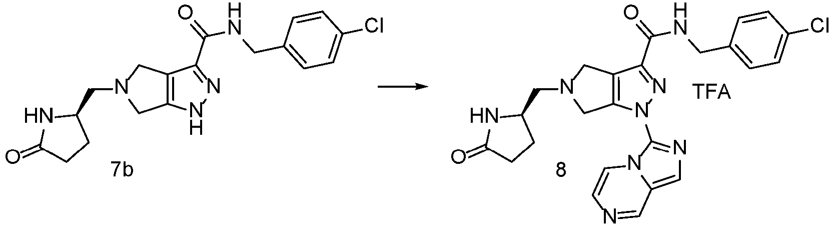

### Step A - synthesis of compound 8

To a mixture of 7b (44 mg, 0.12 mmol), cesium carbonate (139 mg, 0.427 mmol), and bis(2-isobutyrylcyclohexanone)copper (II) (9.0 mg, 0.023 mmol) in DMSO (350 µL) was added to 3-bromoimidazo[1,5-a]pyrazine (38 mg, 0.19 mmol), and the resulting reaction was heated at 100 ° C for about 15 hours. The reaction was filtered and purified using preparative HPLC (reverse-phase C-18), eluting with acetonitrile/water + 0.1% TFA 10-100%, to provide 8. MS: *m*/*z* 491.4 and 493.4 = [M+H for ³⁵Cl and ³⁷Cl]. ¹H NMR (500 MHz, DMSO-*d₆*, ppm): δ 9.45 (t, *J* = 5.5 Hz, 1H), 9.19 (d, *J =* 5.0 Hz, 1H), 7.96 (s, 1H), 7.79 (s, 1H), 7.74 (br s, 1H), 7.33-7.43 (m, 4H), 4.93 (br s, 1H), 4.68 (br s, 1H), 4.52 (d, *J* = 6.0 Hz, 2H), 3.99 (br s, 1H), ~3.43 (presumed m, masked by HDO peak, 1H), ~2.53 (presumed m, masked by DMSO peak, 2H), 2.24-2.30 (m, 2H), 2.12-2.19 (m, 1H), 1.75-1.80 (m, 1H).

The following compounds of the present invention were made using methodology described in Example 8 above, and substituting the appropriate reactants and/or reagents:

| Compound | Structure | MS [M+H] |
|---|---|---|
| 9 | 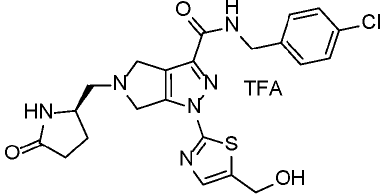 | 487.1 |
| 10 | 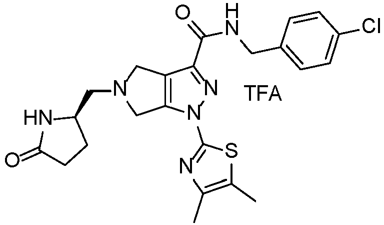 | 485.1 |
| 11 | 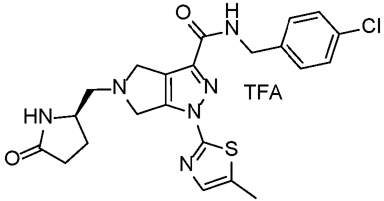 | 471.1 |
| 12 | 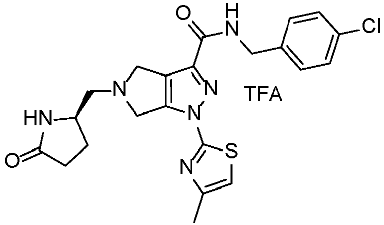 | 471.1 |
| 13 | 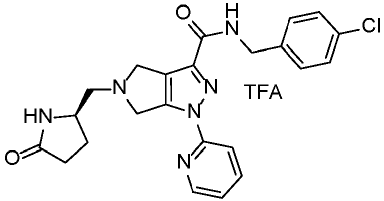 | 451.3 |
| 14 | 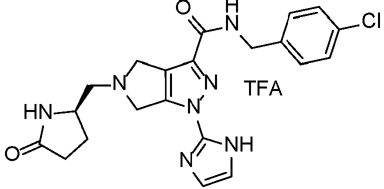 | 440.2 |
| 15 | 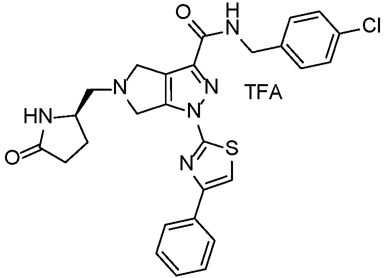 | 533.1 |
| 16 | 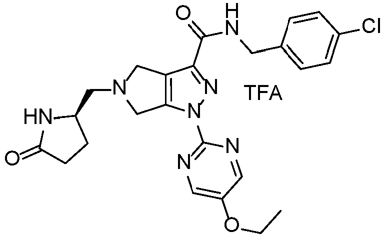 | 496.4 |
| 17 | 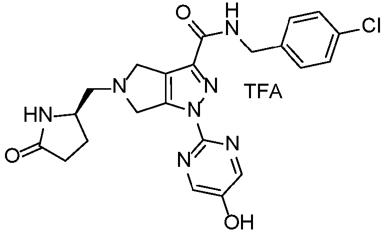 | 468.3 |
| 18 | 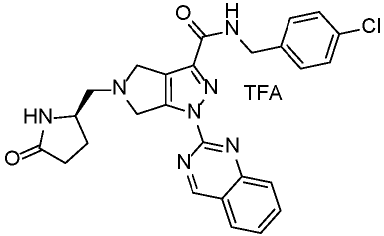 | 502.3 |
| 19 | 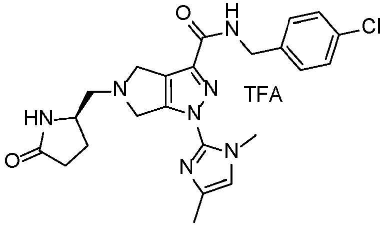 | 468.4 |
| 20 | 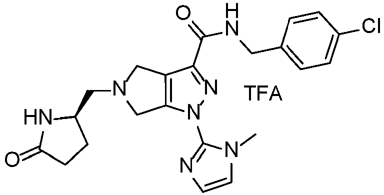 | 454.2 |
| 21 | 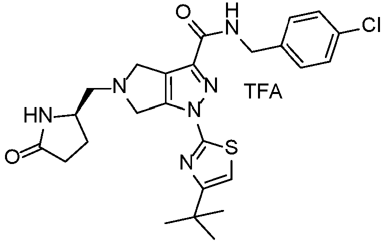 | 513.1 |

### Example 9

### Preparation of Compound 22

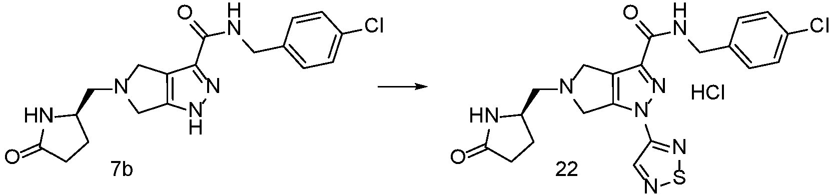

### Step A - synthesis of compound 22

A mixture of 7b (150 mg, 0.401 mmol), 3-bromo-1,2,5-thiadiazole (73.6 µL, 0.802 mmol), and tribasic potassium phosphate (256 mg, 1.204 mmol) in DMSO (2.51 mL) was purged subsurface with nitrogen. 2-Oxo-2-((perfluorophenyl)amino) acetic acid [CAS 1454681-04-9] (40.9 mg, 0.160 mmol), and copper (I) iodide (15 mg, 0.080 mmol) were added, purging continued a few minutes , and then the reaction was capped and heated at 85 °C for 24 hours. The reaction was filtered and purified using preparative HPLC (reverse-phase C-18), eluting with acetonitrile/water + 0.1% TFA 10-65%. Fractions containing the product were combined, basified with saturated, aqueous NaHCO₃, and extracted with ethyl acetate. The organic extract was washed with brine, dried over MgSO₄, and concentrated in vacuo to provide free-base 22. ¹H NMR (500 MHz, CDCl₃, ppm): δ 7.52 (d, *J* = 8.5 Hz, 1H), 7.31 (d, *J* = 8.0 Hz, 2H), 5.97 (s, 1H), 5.43 (s, 2H), 4.21 (d, *J* = 14.0 Hz, 1H), 4.12-4.16 (m, 1H), 4.08 (d, *J* = 13.0 Hz, 1H), 4.00 (dm *J* = 13.5 Hz, 1H), 3.86-3.92 (m, 1H), 2.94-2.97 (m, 1H), 2.85-2.89 (m, 1H), 2.41-2.46 (m, 2H), 2.29-2.36 (m, 1H), 1.78-1.85 (m, 1H).

The compound 22 free base was converted to an HCl salt using the methodology described in Example 2, Step E, and substituting the appropriate reactants and/or reagents, to provide 22. MS: *m*/*z* 456.1 and 458.1 = [M+H for ³⁵Cl and ³⁷Cl].

The following compounds of the present invention were made using methodology described in Example 9 above, and substituting the appropriate reactants and/or reagents, and utilizing the listed ligand in place of 2-oxo-2-((perfluorophenyl)amino)acetic acid:

| Compound | Ligand | Structure | MS [M+H] |
|---|---|---|---|
| 23 | 2-((2,6-difluorophenyl)amino)-2-oxoacetic acid | 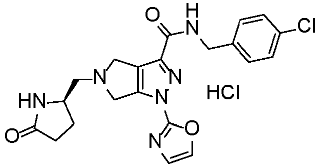 | 441.2 |
| 24 | 2-((3,5-bis(trifluoromethyl)phenyl)amino)-2-oxoacetic acid | 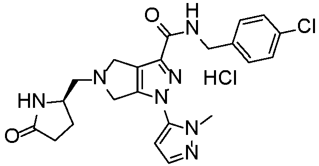 | 454.3 |

### Example 10

### Preparation of Compound 25

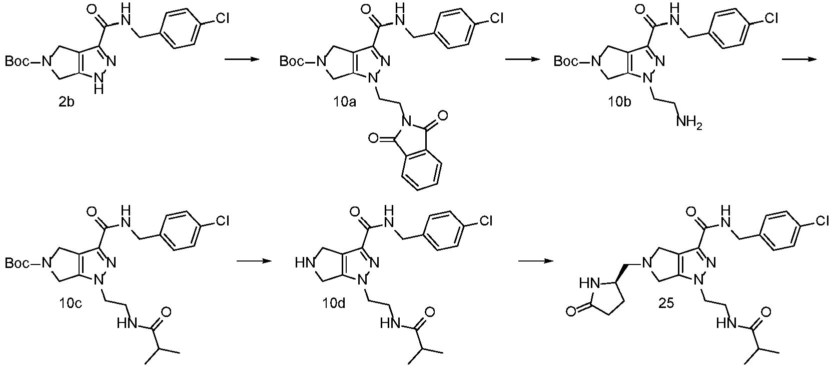

### Step A - synthesis of compound 10a

To a mixture of 2b (900 mg, 2.39 mmol) and potassium carbonate (990 mg, 7.16 mmol) in DMF (10 mL) was added 2-(2-bromoethyl)isoindoline-1,3-dione (1.20 g, 4.78 mmol). The resulting reaction mixture was stirred for 6 hours at 80 °C, then quenched with water (50 mL), extracted with ethyl acetate, washed with brine, dried over Na₂SO₄, and concentrated in vacuo. The resulting residue was purified using silica gel chromatography eluting with 0-10% MeOH/CH₂Cl₂ to provide 10a. MS: *m*/*z* 550.4 = [M+H].

### Step B - synthesis of compound 10b

To a mixture of 10a (420 mg, 0.764 mmol) in ethanol (5 mL) was added hydrazine hydrate (191 mg, 3.82 mmol), and the resulting reaction was allowed to stir for 4 hours at 50 °C. The reaction mixture was diluted with ethyl acetate, washed with water, dried over Na₂SO₄, and concentrated in vacuo to provide 10b, which was used without further purification. MS: *m*/*z* 420.3 = [M+H].

### Step C - synthesis of compound 10c

A mixture of triethylamine (53 mg, 0.52 mmol), 10b (220 mg, 0.524 mmol), and isobutyryl chloride (56 mg, 0.52 mmol) in dichloromethane (5 mL) was allowed to stir for 15 minutes at room temperature. The reaction mixture was concentrated in vacuo, and the resulting residue was purified using silica gel chromatography eluting with 0-10% MeOH/CH₂Cl₂ to provide 10a. MS: *m*/*z* 490.3 = [M+H].

### Step D - synthesis of compound 10d

To a mixture of 10c (180 mg, 0.367 mmol) in dichloromethane (2 mL) was added HCl (4 M in dioxane, 1 mL) at 0 °C, and the resulting reaction was allowed to stir for 3 hours at room temperature. The reaction mixture was concentrated in vacuo, and the resulting residue was diluted with saturated, aqueous Na₂CO₃ (20 mL), and extracted with dichloromethane. The resulting residue was washed with brine, dried over Na₂SO₄, filtered, and concentrated in vacuo to provide 10d, which was used without further purification. MS: *m*/*z* 390.3 = [M+H].

### Step E - synthesis of compound 25

To a mixture of 10d (90 mg, 0.23 mmol), and potassium carbonate (64 mg, 0.462 mmol) in acetonitrile (5 mL) was added a solution of Int-2 (62 mg, 0.23 mmol) in acetonitrile (1 mL), and the resulting reaction was allowed to stir for 16 hours at 80 °C. The reaction was filtered, and the filtrate was concentrated in vacuo. The resulting residue was purified using silica gel chromatography eluting with 0-10% MeOH/CH₂Cl₂ to provide 25. MS: *m*/*z* 487.3 = [M+H]. ¹H NMR (300 MHz, methanol-*d*₄, ppm): δ 7.33 (s, 4H), 4.51 (s, 2H), 4.22-4.17 (m, 2H), 3.97-3.84 (m, 5H), 3.61-3.57 (m, 2H), 2.88 (d, *J* = 6.6 Hz, 2H), 2.42-2.21 (m, 4H), 1.92-1.77 (m, 1H), 1.03 (d, *J* = 7.2 Hz, 6H).

The following compounds of the present invention were made using methodology described in Example 10 above, and substituting the appropriate reactants and/or reagents:

| Compound | Structure | MS [M+H for ³⁵Cl and ³⁷Cl] |
|---|---|---|
| 26 | 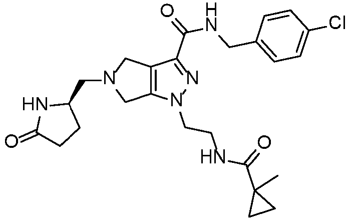 | 498.2 and 501.2 |
| 27 | 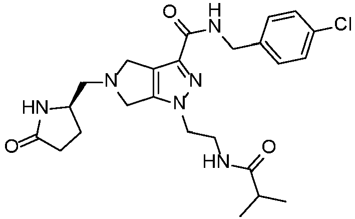 | 486.1 and 488.1 |

### Example 11

### Preparation of Compound 28

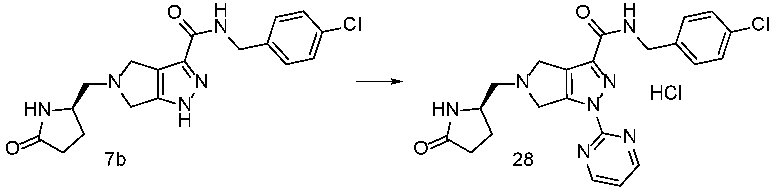

### Step A - synthesis of compound 28

A solution of 7b (35 mg, 0.094 mmol), and 2-bromopyrimidine (22.3 mg, 0.140 mmol) in NMP (312 µL) was added cesium carbonate (122 mg, 0.374 mmol), and allowed to stir at room temperature for about 15 hours. The reaction was filtered and purified using preparative HPLC (reverse-phase C-18), eluting with acetonitrile/water + 0.1% TFA 10-50%. Fractions containing the product were combined, basified with saturated, aqueous NaHCO₃, and extracted with ethyl acetate. The organic extract was washed with brine, dried over MgSO₄, filtered, and concentrated in vacuo to provide free-base 28. ¹H NMR (500 MHz, CDCl₃, ppm): δ 8.78 (d, *J* = 5.0 Hz, 2H), 7.52 (t, *J* = 6.0 Hz, 1H), 7.27-7.31 (m, 5H), 6.03 (s, 1H), 5.43 (s, 2H), 4.58 (d, *J* = 6.0 Hz, 2H), 4.32-4.38 (m, 2H), 4.14-4.17 (m, 1H), 4.04-4.07 (m, 1H), 3.84-3.90 (m, 1H), 2.96 (dd, *J* = 12.0, 3.5 Hz, 1H), 2.82 (dd, *J* = 12.0, 9.5 Hz, 1H), 2.37-2.45 (m, 2H), 2.27-2.34 (m, 1H), 1.76-1.84 (m, 1H). Compound 28 was subsequently converted to its HCl salt using the method described in Example 2, Step E, and substituting the appropriate reactants and/or reagents. MS: *m*/*z* 452.4 = [M+H].

The following compounds of the present invention were made using methodology described in Example 11 above, and substituting the appropriate reactants and/or reagents:

| Compound | Structure | MS [M+H for ³⁵Cl and ³⁷Cl] |
|---|---|---|
| 29 | 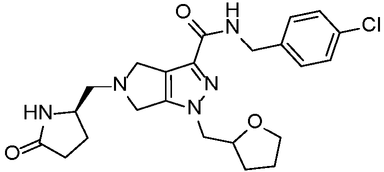 | 458.3 and 460.3 |
| 30 | 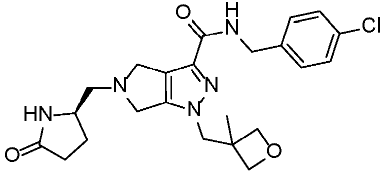 | 458.3 and 460.3 |

### Example 12

### Preparation of Int-6

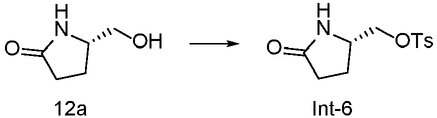

### Step A - synthesis of Int-6

To a mixture of 12a (50.0 g, 434 mmol), and 4-toluenesulfonyl chloride (91.0 g, 477 mmol) in dichloromethane (400 mL) was added triethylamine (57.1 g, 564 mmol), and 4-dimethylaminopyridine (5.31 g, 43.4 mmol), and the resulting reaction was allowed to stir at room temperature for 20 hours. The reaction mixture was concentrated in vacuo, diluted with HCl (0.5 N, 800 mL) and extracted with ethyl acetate. The organic extract was concentrated in vacuo to provide Int-6 as a solid, which was used without further purification. ¹H NMR (400 MHz, CDCl₃, ppm): δ 7.80 (d, *J* = 8.3 Hz, 2 H), 7.38 (d, *J* = 8.0 Hz, 2 H), 5.87 (br s, 1 H), 4.07 (dd, *J* = 9.6, 3.5 Hz, 1 H), 3.82-3.98 (m, 2 H), 2.47 (s, 3 H), 2.14 - 2.39 (m, 3 H), 1.71-1.84 (m, 1 H).

### Example 13

### Preparation of Compound 31

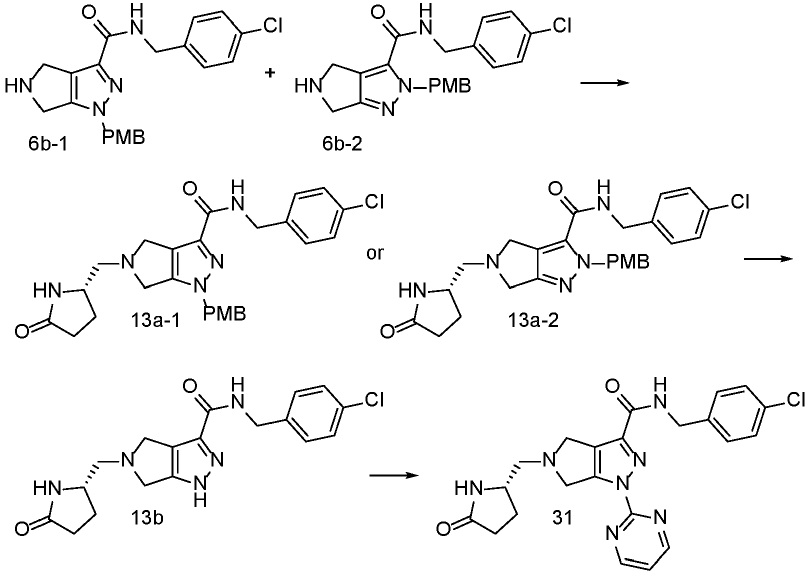

### Step A - synthesis of compound 13a-1 and 13a-2

A mixture of 6b-1 and 6b-2 (50.0 g, 125 mmol), Int-6 (37.3 g, 138 mmol), and cesium carbonate (61.5 g, 188 mmol) in acetonitrile (400 mL) was heated at 80°C for 1 hour. The reaction mixture was concentrated in vacuo and diluted with water (500 mL). The resulting suspension was filtered. The collected solid was slurried in acetonitrile (200 mL), and methyl-tert-butyl ether (100 mL) for 1 hour, then filtered. The collected solid was dried in vacuo to provide a mixture of 13a-1 and 13a-2 as a solid, which was used without further purification.

### Step B - synthesis of compound 13b

Compound 13b was made from the mixture of 13a-1 and 13a-2 using the method described in Example 6, step D, and substituting the appropriate reactants and/or reagents. The resulting residue was purified using preparative HPLC (reverse-phase C-18), eluting with acetonitrile/water + 0.1% TFA 10-100%. Fractions containing the product were combined, basified with saturated, aqueous NaHCO₃, and extracted with ethyl acetate. The organic extract was washed with brine, dried over Na₂SO₄, filtered, and concentrated in vacuo to provide 13b as a solid. ¹H NMR (400 MHz, DMSO-*d₆*, ppm): δ 13.01 (br d, *J* = 4.0 Hz, 1 H), 8.20-8.85 (m, 1 H), 7.68 (s, 1 H), 7.07-7.50 (m, 4 H), 4.19-4.64 (m, 2 H), 3.60-3.96 (m, 5 H), 2.61-2.84 (m, 2 H), 1.98-2.26 (m, 3 H), 1.60-1.85 (m, 1 H).

### Step C - synthesis of compound 31

Compound 31 was made from 13b, using the method described in Example 11, step A, and substituting the appropriate reactants and/or reagents. The reaction was filtered and purified using preparative HPLC (reverse-phase C-18), eluting with acetonitrile/water + 0.1% TFA 10-45%. Fractions containing the product were combined, basified with saturated, aqueous NaHCO₃, and extracted with ethyl acetate. The organic extract was washed with brine, dried over MgSO₄, and concentrated in vacuo to provide 31 as its free base. ¹H NMR (500 MHz, CDCl₃, ppm): δ 8.78 (d, *J* = 5.0 Hz, 2H), 7.50 (t, *J* = 6.0 Hz, 1H), 7.28-7.31 (m, 5H), 6.00 (s, 1H), 5.43 (s, 2H), 4.59 (d, *J* = 6.0 Hz, 2H), 4.32-4.39 (m, 2H), 4.13-4.18 (m, 1H), 4.04-4.08 (m, 1H), 3.85-3.90 (m, 1H), 2.97 (dd, *J* = 12.0, 3.5 Hz, 1H), 2.83 (dd, *J =* 12.0, 9.5 Hz, 1H), 2.37-2.45 (m, 2H), 2.27-2.34 (m, 1H), 1.77-1.84 (m, 1H). The compound 31 free base was converted to its HCl salt using the methodology described in Example 2, Step E, and substituting the appropriate reactants and/or reagents. MS: *m*/*z* 452.2 = [M+H].

### Example 14

### Preparation of Compound 32

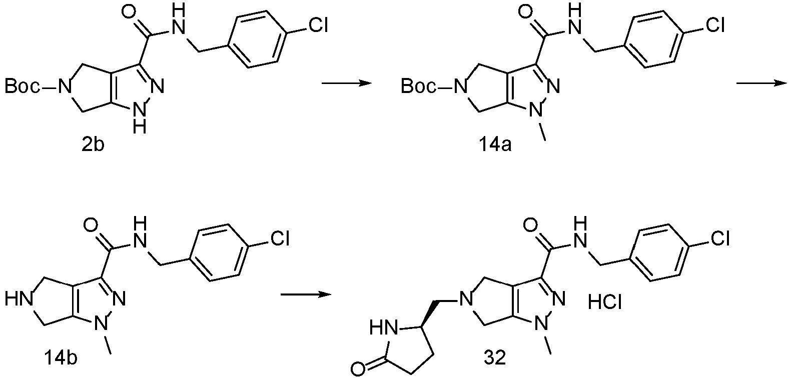

### Step A - synthesis of compound 14a

To a mixture of 2b (1.00 g, 2.26 mmol), and potassium carbonate (1.60 g, 11.3 mmol) in DMF (10 mL) was added iodomethane (0.96 g, 6.78 mmol) at 0 °C. The reaction was allowed to stir for 3 hours at room temperature, then was diluted with water (25 mL), and extracted with ethyl acetate. The organic extract was washed with brine, dried over Na₂SO₄, filtered, and concentrated in vacuo, and the resulting residue was purified using silica gel chromatography eluting with 0-10% MeOH/CH₂Cl₂ to provide 14a. MS: *m*/*z* 391.0 = [M+H].

### Step B - synthesis of compound 14b

Compound 14b was made from 14a, using the method described in Example 2, step C, and substituting the appropriate reactants and/or reagents, and used without further purification. MS: *m*/*z* 291.1 = [M+H].

### Step C - synthesis of compound 32

Compound 32 was made from 14b, using the method described in Example 10, step E, and substituting the appropriate reactants and/or reagents. The reaction mixture was filtered, and the filtrate was concentrated in vacuo. The resulting residue was purified using silica gel chromatography eluting with 1-10% MeOH/CH₂Cl₂ to provide 32 as its free base. MS: *m*/*z* 388.1 = [M+H]. ¹H NMR (400 MHz, methanol-*d*₄, ppm): δ 7.31 (s, 4H), 4.48 (s, 2H), 3.99-3.82 (m, 5H), 3.81 (s, 3H), 2.86 (d, *J* = 6.4 Hz, 2H), 2.40-2.25 (m, 3H), 1.87-1.83 (m, 1H).

The compound 32 free base was converted to its HCl salt using the method described in Example 2, Step E, and substituting the appropriate reactants and/or reagents. MS: *m*/*z* 388.3 = [M+H].

### Example 15

### Preparation of Compounds 33 and 34

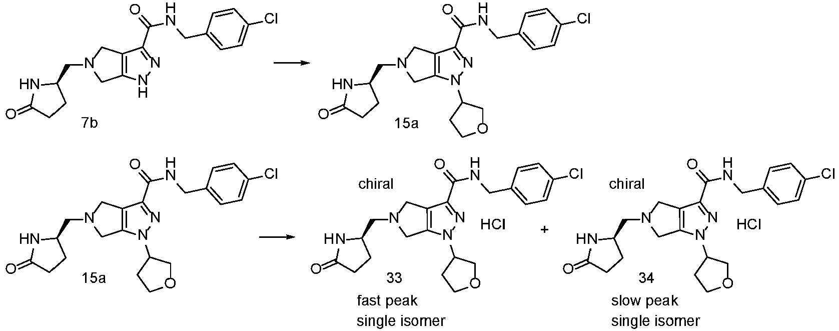

### Step A - synthesis of compound 15a

A mixture of 7b (135 mg, 0.361 mmol), and cesium carbonate (235 mg, 0.722 mmol) in DMF (2407 µL) was purged subsurface with nitrogen and treated dropwise with 3-iodotetrahydrofuran (74.1 µl, 0.722 mmol). The reaction was purged further with nitrogen, then heated to 80 °C, and allowed to stir at this temperature for about 15 hours. The reaction mixture was filtered, and the filtrate was directly purified using preparative HPLC (reverse-phase C-18), eluting with acetonitrile/water + 0.1% TFA 10-45%. Fractions containing the product were combined, basified with saturated, aqueous NaHCO₃, and extracted with ethyl acetate. The organic extract was washed with brine, dried over MgSO₄, and concentrated in vacuo to provide 15a as a foam (mixture of diastereomers). MS: *m*/*z* 444.4 = [M+H].

### Step B - resolution of compounds 33 and 34, and conversion to HCl salts

15a (mixture of diastereomer) was resolved using Prep-SFC using an AD-H (2x25 cm) column, eluting with 35% MeOH (+0.1% diethylamine) in CO₂ at 100 bar, 60 mL/min, to provide 33 (fast peak), and 34 (slow peak).

For 33: ¹H NMR (500 MHz, CDCl₃, ppm): δ 7.28-7.31 (m, 4H), 7.11 (br s, 1H), 6.00 (s, 1H), 4.86 (br s, 1H), 4.56 (d, *J* = 6.0 Hz, 2H), 3.85-4.12 (m, 7H), 3.82 (br s, 1H), 3.50 (s, 1H), 2.88-2.90 (m, 1H), 2.75-2.79 (m, 1H), 2.45-2.49 (m, 1H), 2.38-2.43 (m, 2H), 2.27 (br s, 2H), 1.78 (br s, 1H).

For 34: ¹H NMR (500 MHz, CDCl₃, ppm): δ 7.28-7.30 (m, 4H), 7.10 (br s, 1H), 5.97 (s, 1H), 4.86 (br s, 1H), 4.56 (d, *J* = 5.5 Hz, 2H), 3.95-4.09 (m, 5H), 3.85-3.89 (m, 1H), 3.82 (br s, 1H), 3.51 (s, 1H), 2.89-2.91 (m, 1H), 2.75-2.79 (m, 1H), 2.45-2.46 (m, 1H), 2.38-2.40 (m, 2H), 2.28 (br s, 2H), 1.79 (br s, 1H).

33 and 34 were converted to their HCl salts using the methodology described in Example 2, Step E, and substituting the appropriate reactants and/or reagents. MS: *m*/*z* 444.3 = [M+H] for both.

The following compound of the present invention was made using methodology described in Example 15, Step A above, and substituting the appropriate reactants and/or reagents:

| Compound | Structure | MS [M+H] |
|---|---|---|
| 35 | 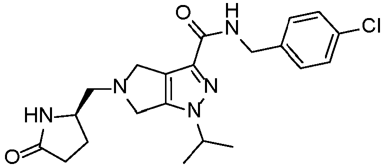 | 416.3 |

### Example 16

### Preparation of Compound 36

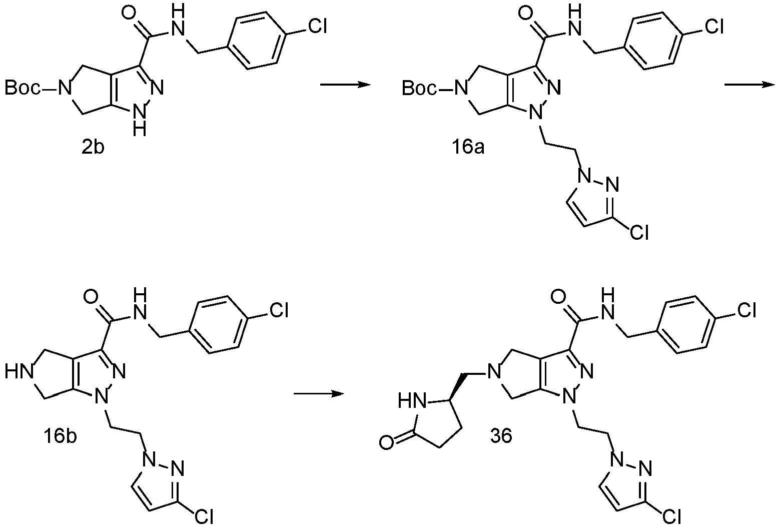

### Step A - synthesis of compound 16a

To a mixture of 2b (230 mg, 0.610 mmol), and tribasic potassium phosphate (259 mg, 1.22 mmol) in DMF (8 mL) was added 1-(2-bromoethyl)-3-chloro-1H-pyrazole (153 mg, 0.732 mmol), and the resulting reaction was allowed to stir for 3 hours at 60 °C. The reaction was quenched with water (80 mL), extracted with ethyl acetate, and the organic extract was washed with brine, dried over Na₂SO₄, filtered, and concentrated in vacuo. The resulting residue was purified using silica gel chromatography eluting with 0-15% EtOAc/petroleum ether to provide 16a. MS: *m*/*z* 505.3 = [M+H]

### Step B - synthesis of compound 16b

Compound 16b was made from 16a, using the method described in Example 2, step C, and substituting the appropriate reactants and/or reagents. MS: *m*/*z* 405.1 = [M+H].

### Step C - synthesis of compound 36

Compound 36 was made from 16b, using the method described in Example 4, step C, and substituting the appropriate reactants and/or reagents. The product was purified using preparative HPLC (reverse-phase C-18), eluting with acetonitrile/water + 10 mmol/L ammonium bicarbonate 30-95%. MS: *m*/*z* 502.4 = [M+H]. ¹H NMR (400 MHz, CDCl₃, ppm): δ 7.30 (s, 4H), 7.17 (br s, 1H), 6.93 (s, 1H), 6.34 (br s, 1H), 6.07 (s, 1H), 4.54 (s, 2H), 4.42-4.38 (m, 4H), 3.92-3.81 (m, 2H), 3.75-3.65 (m, 1H), 3.45-3.33 (m, 2H), 2.76-2.61 (m, 2H), 2.37-2.32 (m, 2H), 2.27-2.16 (m, 1H), 1.80-1.65 (m, 1H).

### Example 17

### Preparation of Int-7

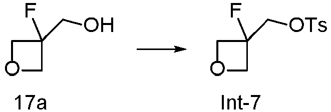

### Step A - synthesis of Int-7

A solution of 17a (200 mg, 1.885 mmol), and 4-toluenesulfonyl chloride (431 mg, 2.26 mmol) in dichloromethane (7.54 mL) was cooled at 0 °C and treated with triethylamine (315 µL, 2.26 mmol), followed by a catalytic amount of 4-dimethylaminopyridine (23 mg, 0.19 mmol). The reaction was allowed to warm to room temperature and stirred for 72 hours. The reaction mixture was diluted with water, extracted with dichloromethane, washed with brine, dried over Na₂SO₄, and concentrated in vacuo. The resulting residue was purified using silica gel chromatography eluting with 0-60% EtOAc/hexanes to provide Int-7 as a solid. MS: *m*/*z* 261.0 = [M+H].

### Example 18

### Preparation of Compound 37

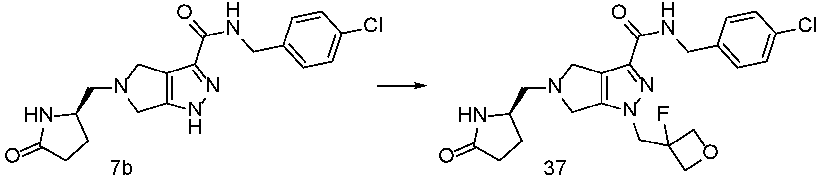

To a mixture of 7b (57 mg, 0.15 mmol), and cesium carbonate (99 mg, 0.31 mmol) in DMF (1.0 mL) was added Int-7 (59.5 mg, 0.229 mmol), and potassium iodide (2.5 mg, 0.015 mmol). The reaction was heated at 90 °C for 5 hours, then was quenched with water, and concentrated in vacuo. The resulting residue was taken up in dichloromethane, and the resulting solution washed with water, dried over Na₂SO₄, filtered, and concentrated in vacuo. The resulting residue was purified using silica gel chromatography eluting with 0-10% MeOH/CH₂Cl₂ to provide 37 as a solid. MS: *m*/*z* 462.2 and 464.2 = [M+H for ³⁵Cl and ³⁷Cl]. ¹H NMR (600 MHz, CDCl₃, ppm): δ 7.23-7.29 (m, 4H), 7.06 (br s, 1H), 6.01 (br s, 1H), 4.74 (d, *J* = 7.8 Hz, 1H), 4.71 (d, *J* = 8.4 Hz, 1H), 4.63-4.67 (m, 2H), 4.52 (d, *J* = 6.6 Hz, 2H), 4.49 (s, 1H), 4.45 (s, 1H), 4.03 (d, *J* = 11.4 Hz, 1H), 3.90 (d, *J* = 12.0 Hz, 1H), 3.81-3.84 (m, 2H), 3.75 (br s, 1H), 2.84 (dd, *J* = 12.0, 2.4 Hz, 1H), 2.69-2.73 (m, 1H), 2.31-2.35 (m, 2H), 2.20-2.26 (m, 1H), 1.69-1.75 (m, 1H).

### Example 19

### Preparation of Int-8

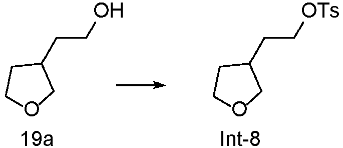

Int-8 was made from 19a, using the method described in Example 17, step A, and substituting the appropriate reactants and/or reagents. The resulting residue was purified using silica gel chromatography eluting with 0-100% MeOH/CH₂Cl₂ to provide Int-8. MS: *m*/*z 271.0* = [M+H].

### Example 20

### Preparation of Compound 38

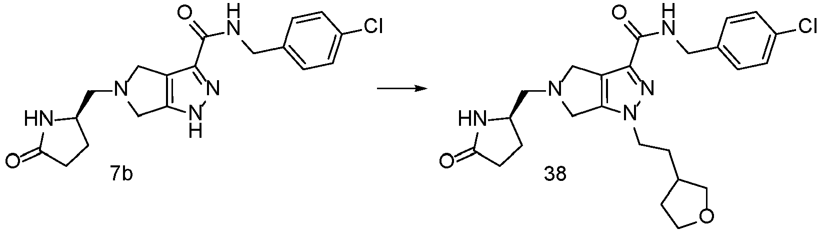

To a mixture of 7b (156 mg, 0.417 mmol), and tribasic potassium phosphate (266 mg, 1.25 mmol) in DMF (2 mL) was added Int-8 (113 mg, 0.417 mmol), and allowed to stir for 12 hours at 80 °C. The reaction was quenched with water (20 mL), extracted with ethyl acetate, and the organic extract was washed with brine, dried over Na₂SO₄, filtered, and concentrated in vacuo. The resulting residue was purified using silica gel chromatography eluting with 0-100% MeOH/CH₂Cl₂ to provide 38. MS: *m*/*z* 472.3 = [M+H]. ¹H NMR (400 MHz, methanol-*d*₄, ppm): δ 7.31 (s, 4H), 4.49 (s, 2H), 4.12 (t, *J* = 6.8 Hz, 2H), 3.98-3.89 (m, 4H), 3.88-3.80 (m, 3H), 3.74-3.67 (m, 1H), 3.33-3.30 (m, 1H), 2.87 (d, *J* = 6.0 Hz, 2H), 2.38-2.26 (m, 3H), 2.17-2.03 (m, 2H), 1.96-1.83 (m, 3H), 1.56-1.51 (m, 1H).

### Example 21

### Preparation of Int-9

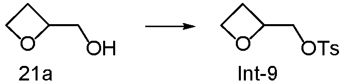

Int-9 was made from 21a, using the method described in Example 17, step A, and substituting the appropriate reactants and/or reagents. The resulting crude product was purified using silica gel chromatography eluting with 0-60% EtOAc/hexanes. MS: *m*/*z* 243.0 = [M+H].

### Example 22

### Preparation of Compound 39

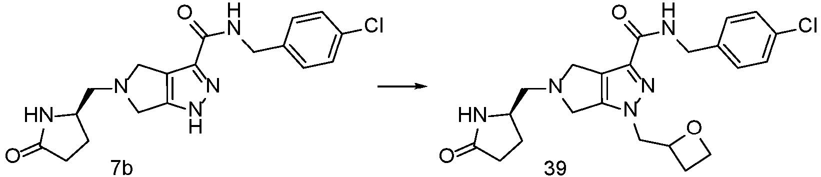

Compound 39 was made from 7b, using the method described in Example 18, step A, and substituting the appropriate reactants and/or reagents. The resulting crude product was purified using silica gel chromatography eluting with 0-10% MeOH/CH₂Cl₂. MS: *m*/*z* 444.2 and 446.2 = [M+H for ³⁵Cl and ³⁷Cl]. ¹H NMR (600 MHz, CDCl₃, ppm): δ 7.24-7.27 (m, 4H), 7.11 (br s, 1H), 6.11 (br s, 1H), 5.05 (br s, 1H), 4.54-4.59 (m, 1H), 4.47-4.53 (m, 2H), 4.20-4.28 (m, 1H), 4.12-4.19 (m, 2H), 4.03 (d, *J* = 10.8 Hz, 1H), 3.83-3.92 (m, 3H), 3.74 (br s, 1H), 2.83 (d, *J* = 12.0 Hz, 1H), 2.64-2.72 (m, 2H), 2.38-2.44 (m, 1H), 2.30-2.33 (m, 2H), 2.19-2.24 (m, 1H), 1.68-1.74 (m, 1H).

### Example 23

### Preparation of Int-10

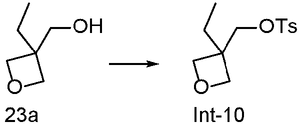

Int-10 was made from 23a, using the method described in Example 17, step A, and substituting the appropriate reactants and/or reagents. The resulting crude product was purified using silica gel chromatography eluting with 0-60% EtOAc/hexanes. MS: *m*/*z* 271.1 = [M+H].

### Example 24

### Preparation of Compound 40

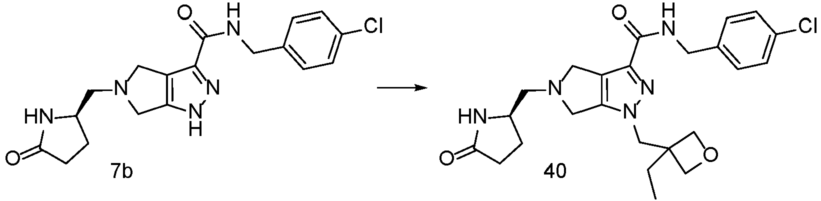

Compound 40 was made from 7b, using the method described in Example 18, step A, and substituting the appropriate reactants and/or reagents. The resulting crude product was purified using silica gel chromatography eluting with 0-10% MeOH/CH₂Cl₂. MS: *m*/*z* 472.3 and 474.3 = [M+H for ³⁵Cl and ³⁷Cl]. ¹H NMR (600 MHz, CDCl₃, ppm): δ 7.23-7.27 (m, 4H), 7.04 (br s, 1H), 6.16 (br s, 1H), 4.57 (t, *J* = 6.0 Hz, 2H), 4.51 (d, *J* = 6.0 Hz, 2H), 4.40 (d, *J* = 6.0 Hz, 2H), 4.18 (s, 2H), 4.03 (d, *J* = 12.0 Hz, 1H), 3.89 (d, *J* = 12.0 Hz, 1H), 3.83 (s, 2H), 3.75 (br s, 1H), 2.85 (dd, *J* = 12.6, 3.6 Hz, 1H), 2.71 (dd, *J* = 12.6, 9.6 Hz, 1H), 2.34 (t, *J* = 8.4 Hz, 2H), 2.20-2.26 (m, 1H), 1.69-1.75 (m, 1H), 1.58 (dd, *J* = 15.0, 7.2 Hz, 2H), 0.91 (t, *J* = 7.2 Hz, 3H).

### Example 25

### Preparation of Int-11

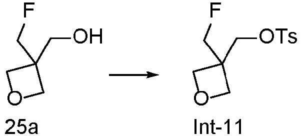

Int-11 was made from 25a, using the method described in Example 17, step A, and substituting the appropriate reactants and/or reagents. The resulting crude product was purified using silica gel chromatography eluting with 0-60% EtOAc/hexanes. MS: *m*/*z* 275.0 = [M+H].

### Example 26

### Preparation of Compound 41

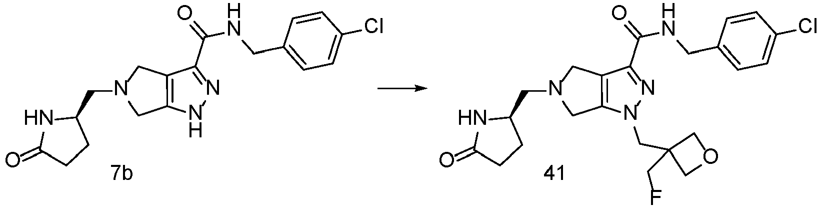

Compound 41 was made from 7b, using the method described in Example 18, step A, and substituting the appropriate reactants and/or reagents. The resulting crude product was purified using silica gel chromatography eluting with 0-10% MeOH/CH₂Cl₂. MS: *m*/*z* 476.2 and 478.2 = [M+H for ³⁵Cl and ³⁷Cl]. ¹H NMR (600 MHz, CDCl₃, ppm): δ 7.23-7.28 (m, 4H), 6.99 (br s, 1H), 6.09 (br s, 1H), 4.67-4.69 (m, 2H), 4.49-4.53 (m, 3H), 4.42-4.45 (m, 3H), 4.31 (s, 2H), 4.03 (d, *J=* 12.0 Hz, 1H), 3.89 (d, *J* = 12.0 Hz, 1H), 3.83 (s, 2H), 3.75 (br s, 1H), 2.85 (d, *J* = 12.0 Hz, 1H), 2.69-2.73 (m, 1H), 2.32-2.35 (m, 2H), 2.20-2.26 (m, 1H), 1.69-1.75 (m, 1H).

### Example 27

### Preparation of Compound 42

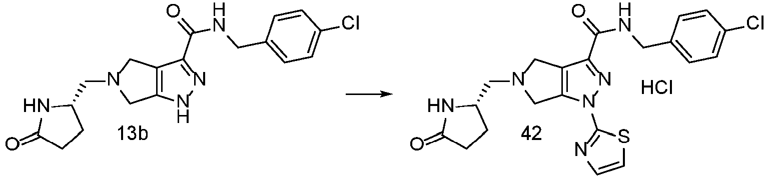

Compound 42 was made from 13b, using the method described in Example 6, step E. The resulting residue was purified using preparative HPLC (reverse-phase C-18), eluting with acetonitrile/water + 0.1% TFA 10-50%. Fractions containing the product were combined, basified with saturated, aqueous NaHCO₃, and extracted with ethyl acetate. The organic extract was washed with brine, dried over MgSO₄, and concentrated in vacuo to provide 42 as its free base. ¹H NMR (500 MHz, CDCl₃, ppm): δ 7.54 (d, *J*= 3.5 Hz, 1H), 7.31-7.35 (m, 4H), 7.23 (t, *J=* 6.0 Hz, 1H), 7.13 (d, *J= 3.0* Hz, 1H), 6.08 (s, 1H), 4.60 (d, *J* = 6.5 Hz, 2H), 4.23-4.31 (m, 2H), 4.10 (br s, 1H), 4.02-4.05 (m, 1H), 3.82-3.88 (m, 1H), 2.94 (dd, *J* = 12.0, 4.0 Hz, 1H), 2.79-2.83 (m, 1H), 2.40 (t, *J* = 9.0 Hz, 2H), 2.25-2.31 (m, 1H), 1.75-1.83 (m, 1H).

The compound 42 free base was converted to its HCl salt using the methodology described in Example 2, Step E, and substituting the appropriate reactants and/or reagents. MS: *m*/*z* 457.3 = [M+H].

### Example 28

### Preparation of Compounds 43 and 44

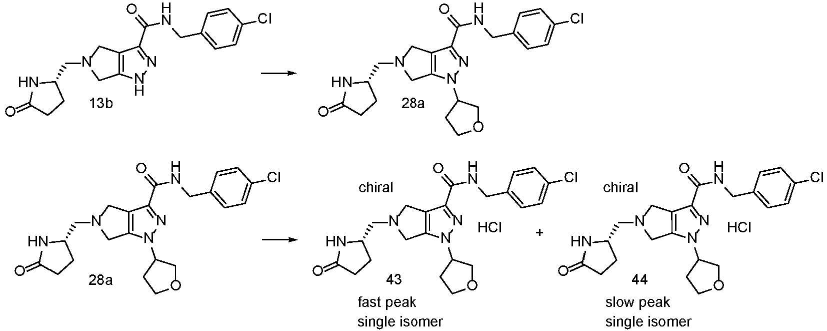

### Step A - synthesis of compound 28a

A mixture of 13b (135 mg, 0.361 mmol), and cesium carbonate (235 mg, 0.722 mmol) in DMF (2407 µL) was purged subsurface with nitrogen, and then 3-iodotetrahydrofuran (74.1 µl, 0.722 mmol) was added dropwise. The resulting reaction was purged further with nitrogen, then heated at 80 °C for about 15 hours. The reaction was filtered and purified using preparative HPLC (reverse-phase C-18), eluting with acetonitrile/water + 0.1% TFA 10-45%. Fractions containing the product were combined, basified with saturated, aqueous NaHCO₃, and extracted with ethyl acetate. The organic extract was washed with brine, dried over MgSO₄, and concentrated in vacuo to provide free-base 28a, a mixture of diastereomers, as a foam. MS: *m*/*z* 444.3 and 446.3 = [M+H for ³⁵Cl and ³⁷Cl].

### Step B - resolution of compounds 43 and 44, and conversion to HCl salts

The diastereomers were resolved using Prep-SFC using an OJ-H (2x25 cm) column, eluting with 25% MeOH (+0.1% diethylamine) in CO₂ at 100 bar, 70 mL/min, to provide free-base 43 (fast peak), and free-base 44 (slow peak).

For 43: ¹H NMR (500 MHz, CDCl₃, ppm): δ 7.30 (dd, *J* = 13.5, 9.0 Hz, 4H), 7.10 (t, *J* = 5.5 Hz, 1H), 5.91 (s, 1H), 4.84-4.88 (m, 1H), 4.56 (d, *J* = 6.0 Hz, 2H), 4.06-4.13 (m, 3H), 3.79-4.01 (m, 6H), 2.89 (dd, *J =* 12.5, 4.0 Hz, 1H), 2.77 (dd, *J* = 12.0, 9.5 Hz, 1H), 2.36-2.51 (m, 3H), 2.24-2.30 (m, 2H), 1.74-1.81 (br s, 1H).

For 44: ¹H NMR (500 MHz, CDCl₃, ppm): δ 7.30 (dd, *J* = 13.5, 8.5 Hz, 4H), 7.10 (t, *J* = 6.0 Hz, 1H), 5.89 (s, 1H), 4.85-4.87 (m, 1H), 4.56 (d, *J* = 6.0 Hz, 2H), 4.00-4.12 (m, 4H), 3.98 (s, 3H), 3.86-3.90 (m, 1H), 3.78-3.84 (m, 1H), 2.90 (dd, *J* = 12.5, 4.0 Hz, 1H), 2.77 (dd, *J* = 12.0, 9.5 Hz, 1H), 2.41-2.51 (m, 1H), 2.38-2.41 (m, 2H), 2.24-2.32 (m, 2H), 1.74-1.81 (br s, 1H).

The residues were converted to HCl salts using the methodology described in Example 2, Step E, and substituting the appropriate reactants and/or reagents, to provide 43 and 44. MS: *m*/*z* 444.4 and 446.4 = [M+H for ³⁵Cl and ³⁷Cl]] 43 and MS: *m*/*z* 444.4 = [M+H] for 44.

### Example 29

### Preparation of Compounds 45 and 46

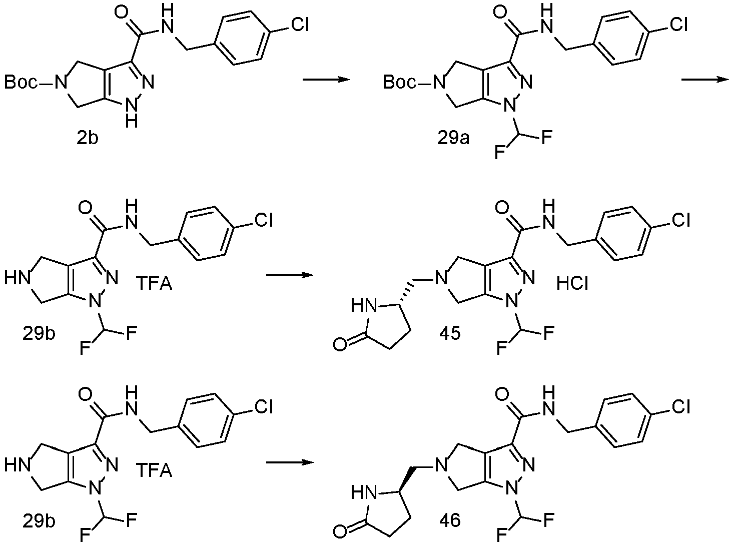

### Step A - synthesis of compound 29a

A mixture or 2-chloro-2,2-difluoroacetic acid, sodium salt (244 mg, 1.59 mmol), 2b (300 mg, 0.796 mmol), and cesium carbonate (519 mg, 1.59 mmol) in DMF (3300 µL), and the resulting reaction was heated to 110 °C for 20 minutes. Additional 2-chloro-2,2-difluoroacetic acid, sodium salt (244 mg, 1.592 mmol) was added, and the reaction was allowed to stir for an additional 30 minutes. The reaction mixture was diluted with water, extracted with ethyl acetate, and the organic extract was washed with brine, dried over MgSO₄, filtered and concentrated in vacuo. The resulting residue was purified using silica gel chromatography eluting with 0-50% EtOAc/hexanes to provide 29a as a solid. MS: *m*/*z* 427.3 and 429.2 = [M+H for ³⁵Cl and ³⁷Cl].

### Step B - synthesis of compound 29b

To a solution of 29a (127 mg, 0.298 mmol) in dichloromethane (1.0 mL) was added trifluoroacetic acid (200 µL, 2.60 mmol), and the resulting reaction was allowed to stir at room temperature for about 15 hours. The reaction mixture was directly concentrated in vacuo to provide 29b, which was used without further purification. MS: *m*/*z* 327.1 = [M+H].

### Step C - synthesis of compound 45

A mixture of 29b (60 mg, 0.142 mmol), (S)-5-(bromomethyl)pyrrolidin-2-one (37.8 mg, 0.212 mmol), and diisopropylethylamine (74.2 µL, 0.425 mmol) in DMF (708 µL) was heated to 80 °C and was allowed to stir at this temperature for about 15 hours. The reaction mixture was directly purified using preparative HPLC (reverse-phase C-18), eluting with acetonitrile/water + 0.1% TFA 10-100%. Fractions containing the product were combined, basified with saturated, aqueous NaHCO₃, and extracted with ethyl acetate. The organic extract was washed with brine, dried over MgSO₄, filtered, and concentrated in vacuo. The resulting residue was purified using silica gel chromatography eluting with 0-100% (3:1 EtOAc:EtOH)/hexanes to provide 45. MS: *m*/*z* 424.2 = [M+H]. ¹H NMR (600 MHz, CDCl₃, ppm): δ 7.27 (dd, *J* = 25.8, 8.4 Hz, 4H), 7.05 (t, *J =* 4.2 Hz, 1H), 7.02 (t, *J* = 60.6 Hz, 1H), 5.88 (s, 1H), 4.53 (d, *J* = 6.0 Hz, 2H), 4.01-4.09 (m, 3H), 3.93-3.95 (m, 1H), 3.76-3.81 (m, 1H), 2.89 (dd, *J* = 12.6, 4.2 Hz, 1H), 2.75 (dd, *J* = 12.6, 9.6 Hz, 1H), 2.33-2.40 (m, 2H) 2.22-2.28 (m, 1H), 1.71-1.78 (m, 1H).

Compound 45 was converted to its HCl salt using the methodology described in Example 2, Step E, and substituting the appropriate reactants and/or reagents. MS: *m*/*z* 424.2 = [M+H].

### Step C - synthesis of compound 46

Compound 46 (as its free base) was made using the method described in Step B (immediately above) and substituting (R)-5-(bromomethyl)pyrrolidin-2-one for (S)-5-(bromomethyl)pyrrolidin-2-one. MS: *m*/*z* 424.2 = [M+H]. ¹H NMR (600 MHz, CDCl₃, ppm): δ 7.27 (dd, *J =* 25.2, 8.4 Hz, 4H), 7.05 (t, *J* = 6.0 Hz, 1H), 7.02 (t, *J* = 60.6 Hz, 1H), 5.89 (s, 1H), 4.53 (d, *J* = 6.0 Hz, 2H), 4.01-4.06 (m, 3H), 3.93-3.95 (m, 1H), 3.76-3.81 (m, 1H), 2.89 (dd, *J* = 12.0, 4.2 Hz, 1H), 2.75 (dd, *J* = 12.6, 9.6 Hz, 1H), 2.34-2.40 (m, 2H) 2.23-2.28 (m, 1H), 1.72-1.78 (m, 1H).

### Example 30

### Preparation of Compound 47

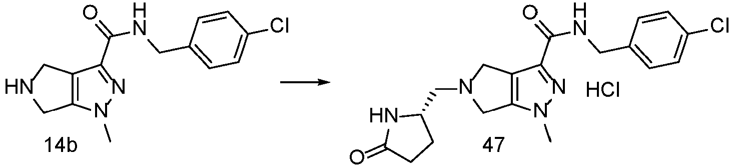

### Step A - synthesis of compound 47

Compound 47 was made from 14b, using the method described in Example 10, step E, and substituting the appropriate reactants and/or reagents. The crude product obtained was purified using silica gel chromatography eluting with 0-15% MeOH/CH₂Cl₂ to provide semi-pure material. The resulting residue was further purified using preparative HPLC (reverse-phase C-18), eluting with acetonitrile/water + 0.1% TFA 10-55%. Fractions containing the product were combined, basified with saturated, aqueous NaHCO₃, and extracted with ethyl acetate. The organic extract was washed with brine, dried over MgSO₄, and concentrated in vacuo to provide 47. MS: *m*/*z* 388.3 = [M+H]. ¹H NMR (500 MHz, CDCl₃, ppm): δ 7.28-7.32 (m, 4H), 7.09 (s, 1H), 5.91 (s, 1H), 4.56 (d, *J* = 6.0 Hz, 2H), 4.09 (d, *J* = 12.0 Hz, 1H), 3.95 (d, *J* = 12.0 Hz, 1H), 3.89 (br s, 2H), 3.79-3.84 (m, 1H), 3.79 (s, 3H), 2.91 (dd, *J* = 12.5, 4.0 Hz, 1H), 2.77 (dd, *J* = 12.0, 9.0 Hz, 1H), 2.36-2.42 (m, 2H), 2.25-2.31 (m, 1H), 1.75-1.82 (m, 1H).

Compound 47 was converted to its HCl salt using the methodology described in Example 2, Step E, and substituting the appropriate reactants and/or reagents. MS: *m*/*z* 388.3 and 390.3 = [M+H for ³⁵Cl and ³¹Cl].

### Example 31

### Preparation of Compound 48

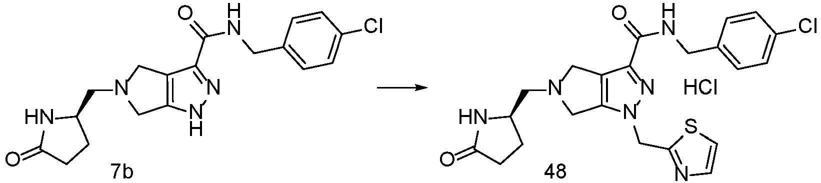

To a mixture of 7b (30 mg, 0.080 mmol), and tribasic potassium phosphate (68 mg, 0.32 mmol) in DMF (321 µL) was added 2-(chloromethyl)thiazole hydrochloride (20.5 mg, 0.120 mmol), and the resulting reaction was allowed to stir at room temperature for about 15 hours. The reaction mixture was filtered and purified using preparative HPLC (reverse-phase C-18), eluting with acetonitrile/water + 0.1% TFA 10-50%. Fractions containing the product were combined, basified with saturated, aqueous NaHCO₃, and extracted with ethyl acetate. The organic extract was washed with brine, dried over MgSO₄, filtered, and concentrated in vacuo to provide 48. ¹H NMR (500 MHz, CDCl₃, ppm): δ 7.81 (d, *J*= 3.5 Hz, 1H), 7.40 (d, *J* = 3.0 Hz, 1H), 7.27-7.33 (m, 4H), 7.15 (t, *J* = 6.0 Hz, 1H), 6.04 (s, 1H), 5.51 (s, 2H), 4.56 (d, *J* = 6.0 Hz, 2H), 4.07 (d, *J* = 12.0 Hz, 1H), 3.94 (d, *J =* 11.5 Hz, 1H), 3.82 (br s, 2H), 3.78 (br s, 1H), 2.87 (dd *J =* 12.0, 4.0 Hz, 1H), 2.73 (dd, *J* = 12.0, 9.5 Hz, 1H), 2.35-2.40 (m, 2H), 2.22-2.29 (m, 1H), 1.71-1.78 (m, 1H).

Compound 48 was converted to its HCl salt using the methodology described in Example 2, Step E, and substituting the appropriate reactants and/or reagents. MS: *m*/*z* 471.2 = [M+H].

### Example 32

### Preparation of Compound 49

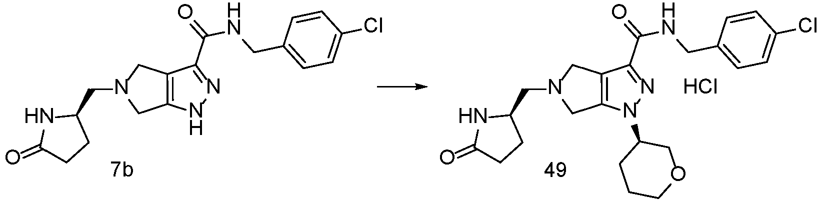

To stirring THF (3344 µL) was slowly added 7b (100 mg, 0.267 mmol) to prevent clumping. The mixture was allowed to stir for several minutes, until a clear solution was obtained, then (S)-tetrahydro-2h-pyran-3-ol (54.6 mg, 0.535 mmol) was added, followed by triphenylphosphine (140 mg, 0.535 mmol). The resulting suspension was allowed to stir for 15 minutes, then the reaction was cooled at 0 ° C and treated with diisopropyl azodicarboxylate (DIAD, 104 µL, 0.535 mmol, added quickly dropwise). The reaction was allowed to warm to room temperature and allowed to stir for 48 hours. Water was added, and the resulting mixture was concentrated in vacuo. The resulting residue was purified using preparative HPLC (reverse-phase C-18), eluting with acetonitrile/water + 0.1% TFA 10-75%. Fractions containing the product were combined, basified with saturated, aqueous NaHCO₃, and extracted with ethyl acetate. The organic extract was washed with brine, dried over MgSO₄, filtered, and concentrated in vacuo to provide 49. ¹H NMR (500 MHz, CDCl₃, ppm): δ 7.28-7.35 (m, 4H), 7.10 (t, *J* = 6.0 Hz, 1H), 6.02 (s, 1H), 4.48-4.61 (m, 2H), 4.13-4.22 (m, 2H), 4.07 (d, *J* = 12.5 Hz, 1H), 3.92-4.00 (m, 4H), 3.73-3.84 (m, 3H), 2.89 (dd, *J* = 12.5, 4.0 Hz, 1H), 2.76 (dd, *J* = 12.0, 9.5 Hz, 1H), 2.35-2.41 (m, 2H), 2.24-2.31 (m, 1H), 2.00-2.05 (m, 1H), 1.85-1.93 (m, 1H), 1.72-1.82 (m, 2H), 1.59-1.66 (m, 1H).

Compound 49 was converted to its HCl salt using the methodology described in Example 2, Step E, and substituting the appropriate reactants and/or reagents. MS: *m*/*z* 458.2 = [M+H].

The following compounds of the present invention were made using methodology described in Example 32 above (utilizing 7b or 13b as the starting material), and substituting the appropriate reactants and/or reagents:

| Compound | Structure | MS [M+H] |
|---|---|---|
| 50 | 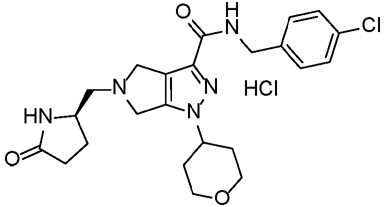 | 458.2 |
| 51 | 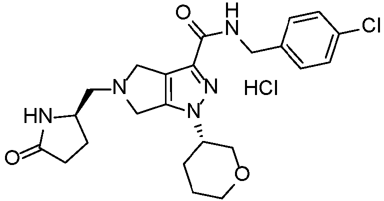 | 458.2 |
| 52 | 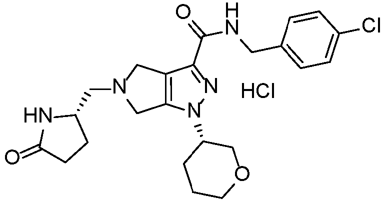 | 458.2 |
| 53 | 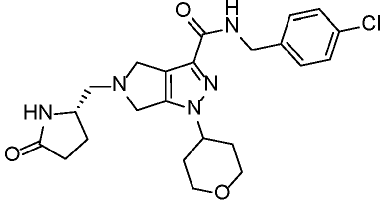 | 458.2 |

### Example 33

### Preparation of Compounds 54 and 55

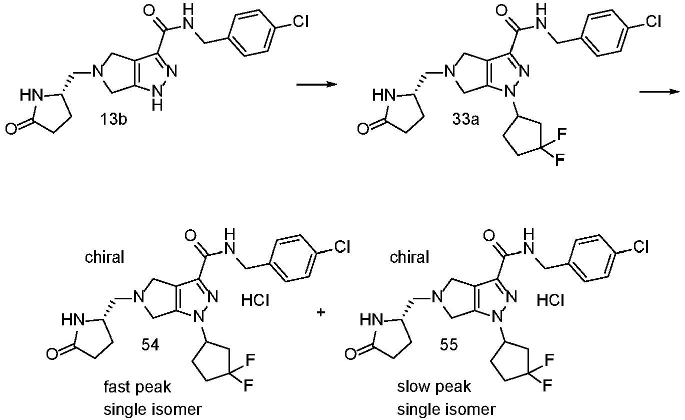

### Step A - synthesis of compound 33a

Compound 33a was made from 13b, using the method described in Example 32, and substituting the appropriate reactants and/or reagents. The resulting residue was purified using preparative HPLC (reverse-phase C-18), eluting with acetonitrile/water + 0.1% TFA 5-60%. Fractions containing the product were combined, basified with saturated, aqueous NaHCO₃, and extracted with ethyl acetate. The organic extract was washed with brine, dried over MgSO₄, and concentrated in vacuo to provide 33a, a mixture of diastereomers, as a foam. MS: *m*/*z* 478.3 and 480.3 = [M+H for ³⁵Cl and ³⁷Cl].

### Step B - resolution of compounds 54 and 55, and conversion to HCl salts

The compound 33a diastereomers were resolved using Prep-SFC using an AD-H (2x25 cm) column, eluting with 35% isopropanol (+0.1% diethylamine) in CO₂ at 100 bar, 65 mL/min, to provide free-base 54 (fast peak), and free-base 55 (slow peak).

For 54: ¹H NMR (500 MHz, CDCl₃, ppm): δ 7.32 (dd, *J* = 14.0, 8.5 Hz, 4H), 7.10 (t, *J* = 6.0 Hz, 1H), 6.00 (s, 1H), 4.62-4.68 (m, 1H), 4.57 (d, *J* = 6.5 Hz, 2H), 4.06-4.08 (m, 1H), 3.93-3.95 (m, 3H), 3.79-3.84 (m, 1H), 2.91 (dd, *J* = 12.5, 4.0 Hz, 1H), 2.78 (dd, *J* = 12.0, 9.0 Hz, 1H), 2.60-2.72 (m, 2H), 2.37-2.48 (m, 3H), 2.15-2.38 (m, 4H), 1.75-1.82 (m, 1H).

For 55: ¹H NMR (500 MHz, CDCl₃, ppm): δ 7.31 (dd, *J* = 14.0, 9.0 Hz, 4H), 7.10 (t, *J* = 6.0 Hz, 1H), 5.96 (s, 1H), 4.62-4.68 (m, 1H), 4.57 (d, *J* = 6.5 Hz, 2H), 4.05-4.09 (m, 1H), 3.94 (s, 2H), 3.92 (s, 1H), 3.79-3.85 (m, 1H), 2.91 (dd, *J* = 12.0, 4.0 Hz, 1H), 2.78 (dd, *J* = 12.0, 9.0 Hz, 1H), 2.62-2.73 (m, 2H), 2.38-2.48 (m, 3H), 2.14-2.38 (m, 4H), 1.75-1.82 (m, 1H).

Compounds 54 and 55 were converted to their HCl salts using the methodology described in Example 2, Step E, and substituting the appropriate reactants and/or reagents. MS: *m*/*z* 478.2 = [M+H] for both.

### Example 34

### Preparation of Compound 56

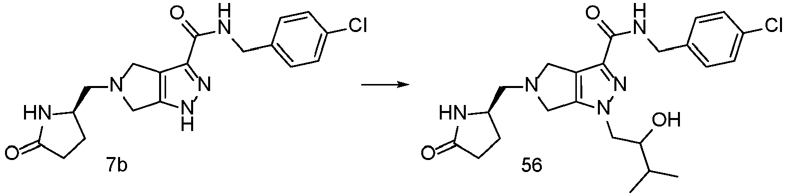

To a mixture of 7b (56 mg, 0.15 mmol), and cesium carbonate (98 mg, 0.30 mmol) in DMF (1 mL) was added 1,2-epoxy-3-methylbutane (25.8 mg, 0.300 mmol) dropwise. The reaction was allowed to stir at 80°C for about 15 hours. The reaction mixture was diluted with water, extracted with dichloromethane, and the organic extract was washed with brine, dried over Na₂SO₄, filtered and concentrated in vacuo. The resulting residue was purified using silica gel chromatography eluting with 0-5% MeOH/CH₂Cl₂ to provide 56 as a mixture of diastereomers. MS: *m*/*z* 460.3 and 462.3 = [M+H for ³⁵Cl and ³⁷Cl]. ¹H NMR (600 MHz, CDCl₃, ppm): δ 7.24-7.28 (m, 4H), 7.03 (br s, 1H), 6.05 (br s, 1H), 4.52 (t, *J* = 5.4 Hz, 3H), 3.99-4.07 (m, 4H), 3.89-3.93 (m, 3H), 3.75-3.85 (m, 2H), 3.76 (br s, 1H), 3.71 (br s, 1H), 3.04 (dd, *J* = 11.4, 6.6 Hz, < 1H), 2.89 (dd, *J* = 13.2, 3.0 Hz, < 1H), 2.83-2.86 (m, > 1H), 2.71 (br s, > 1H), 2.33-2.45 (m, 3H), 2.20-2.25 (m, 1H) 1.95-2.01 (m, 3H), 1.67-1.72 (m, 2H), 1.50-1.58 (m, 2H), 0.96-0.97 (m, 6H).

The following compounds of the present invention were made using methodology described in Example 34 above, and substituting the appropriate reactants and/or reagents:

| Compound | Structure | MS [M+H for ³⁵Cl and ³⁷Cl] |
|---|---|---|
| 57 | 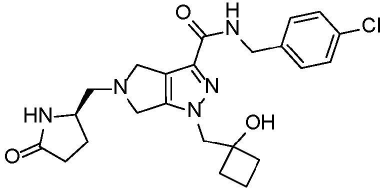 | 444.2 and 446.2 |
| 58 | 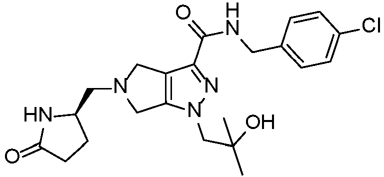 | 446.3 and 448.3 |

### Example 35

### Preparation of Compound 59

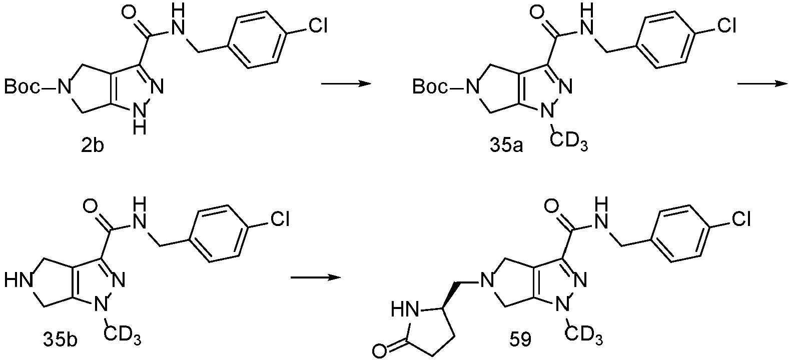

### Step A - synthesis of compound 35a

A mixture of 2b (101 mg, 0.268 mmol), and cesium carbonate (262 mg, 0.804 mmol) in DMF (2.7 mL) was cooled in an ice bath. Iodomethane-d₃ (58.3 mg, 0.402 mmol) was added dropwise, and the resulting reaction was allowed to warm to room temperature and stirred for about 15 hours. The reaction mixture was diluted with water and concentrated in vacuo, and the resulting residue was dissolved in dichloromethane. The organic extract was washed with water, dried over Na₂SO₄, filtered, and concentrated in vacuo, and the resulting residue was purified using silica gel chromatography eluting with 0-100% EtOAc/hexanes to provide 35a. MS: *m*/*z* 394.2 and 396.2 = [M+H for ³⁵Cl and ³⁷Cl].

### Step B - synthesis of compound 35b

Compound 35b was made from 35a, using the method described in Example 7, Step A, and substituting the appropriate reactants and/or reagents, and was used without further purification. MS: *m*/*z* 294.1 = [M+H].

### Step C - synthesis of compound 59

A mixture of 35b (38 mg, 0.13 mmol), potassium carbonate (21.5 mg, 0.155 mmol), and Int-3 (25.3 mg, 0.142 mmol) in acetonitrile (2.6 mL) was allowed to stir at 80 °C for about 15 hours. The reaction mixture was diluted with water and extracted with dichloromethane. The organic extract was washed with brine, dried over Na₂SO₄, filtered and concentrated in vacuo. The resulting residue was purified using silica gel chromatography eluting with 0-5% MeOH/CH₂Cl₂ to provide 59. MS: *m*/*z* 391.3 and 393.3 = [M+H for ³⁵Cl and ³⁷Cl]. ¹H NMR (600 MHz, CDCl₃, ppm): δ 7.26 (dd, *J* = 16.2, 8.4 Hz, 4H), 7.05 (br s, 1H), 5.87 (br s, 1H), 4.52 (d, *J* = 6.0 Hz, 2H), 4.05 (d, *J* = 12.0 Hz, 1H), 3.91 (d, *J* = 12.0 Hz, 1H), 3.86 (s, 2H), 3.77 (br s, 1H), 2.86 (dd, *J* = 12.0, 3.6 Hz, 1H), 2.73 (dd, *J* = 12.0, 9.6 Hz, 1H), 2.35-2.38 (m, 2H), 2.22-2.28 (m, 1H), 1.73-1.77 (m, 1H).

### Example 36

### Preparation of Int-12

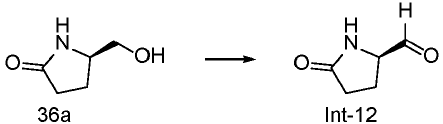

To a solution of 36a (300 mg, 2.61 mmol) in dichloromethane (3 mL) was added 1,1,1-triacetoxy-1,1-dihydro-1,2-benziodoxol-3(1*H*)-one (Dess-Martin periodinane, 1.22 g, 2.87 mmol), and the resulting reaction was allowed to stir for 2 hours at room temperature. The reaction mixture was concentrated in vacuo to provide Int-12, which was used without further purification. MS: *m*/*z* 113.9 = [M+H].

### Example 37

### Preparation of Compound 60

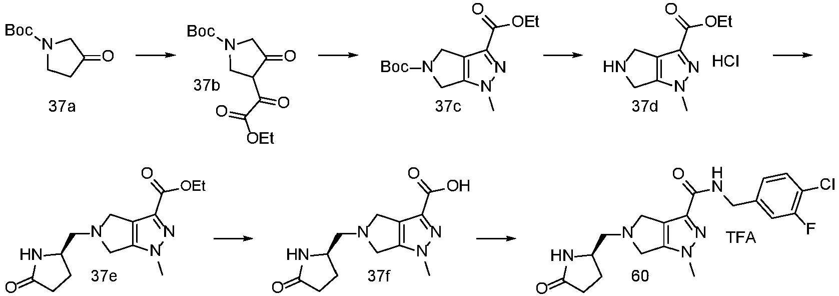

### Step A - synthesis of compound 37b

A mixture of 37a (50.0 g, 270 mmol) in tetrahydrofuran (150 mL) was cooled at -70 °C and treated with lithium bis(trimethylsilyl)amide (1 M in THF, 270 mL) over 5 minutes. The reaction was allowed to stir for 30 minutes at -70 ° C, and then a solution of diethyl oxalate (39.5 g, 270 mmol) in tetrahydrofuran (80 mL) was added dropwise at -70 ° C. The reaction was allowed to stir for 16 hours at 15 ° C, then quenched by addition of saturated, aqueous ammonium chloride (150 mL). The resulting mixture was diluted with water (200 mL), extracted with ethyl acetate, and the organic extract was washed with brine, dried over Na₂SO₄, filtered, and concentrated in vacuo to provide 37b as an oil which was used without further purification.

### Step B - synthesis of compound 37c

To a mixture of 37b (90.0 g, 315 mmol) in acetic acid (150 mL) was added methyl hydrazine (72.7 g, 631 mmol) dropwise over 5 minutes at 0 °C. The reaction was heated at 120 °C for 1.5 hours. The cooled reaction was quenched with water (100 mL), extracted with ethyl acetate, washed with saturated, aqueous NH₄HCO₃, dried over Na₂SO₄, and filtered. The filtrate was concentrated in vacuo and purified using silica gel chromatography eluting with 0-100% EtOAc/petroleum ether to provide 37c as a solid. MS: *m*/*z* 296.2 = [M+H].

### Step C - synthesis of compound 37d

A mixture of 37c (10.0 g, 33.9 mmol) in dichloromethane (50 mL) was saturated with hydrogen chloride gas and allowed to stir at 25 °C for 1.5 hours. The reaction mixture was concentrated in vacuo, and the resulting residue was slurried in diethyl ether and filtered. The collected solid was dried in vacuo to provide 37d as a solid, which was used without further purification. MS: *m*/*z* 196.2 = [M+H].

### Step D - synthesis of compound 37e

To a mixture of 37d (100 mg, 0.432 mmol) in 1,2-dichloroethane (5 mL) was added Int-12 (48.8 mg, 0.432 mmol), and the resulting reaction was allowed to stir at room temperature for 5 minutes. Sodium cyanotrihydroborate (54.2 mg, 0.863 mmol) was added, and the resulting reaction was allowed to stir for 1.5 hours. The reaction mixture was concentrated in vacuo, and the resulting residue was purified using silica gel chromatography eluting with 1-10% MeOH/CH₂Cl₂ to provide semi-pure material which was further purified using preparative HPLC (reverse-phase C-18), eluting with acetonitrile/water + 0.1% TFA 10-70% to provide 37e as a solid. MS: *m*/*z* 293.1 = [M+H].

### Step E - synthesis of compound 37f

A mixture of 37e (250 mg, 0.898 mmol), sodium hydroxide (1 N, 1.8 mL), methanol (1.8 mL), and tetrahydrofuran (1.8 mL) was allowed to stir at room temperature for about 15 hours. The reaction mixture was concentrated in vacuo to provide 37f, which was used without further purification.

### Step F - synthesis of compound 60

Compound 60 was made using the method described in Example 2, Step A, utilizing 37f, and substituting (4-chloro-3-fluorophenyl)methanamine for (4-chlorophenyl)methanamine. The resulting residue was purified using preparative HPLC (reverse-phase C-18), eluting with acetonitrile/water + 0.1% TFA 10-100% to provide 60. MS: *m*/*z* 406.4 and 408.4 = [M+H for ³⁵Cl and ³⁷Cl]. ¹H NMR (500 MHz, DMSO-*d₆*, ppm): δ 8.93 (s, 1H), 7.70 (s, 1H), 7.52 (t, *J* = 8.0 Hz, 1H), 7.26 (d, *J =* 10.0 Hz, 1H), 7.14 (d, *J* = 8.0 Hz, 1H), 4.37 (d, *J* = 11.0 Hz, 2H), 3.84 (s, 3H), 2.21-2.28 (m, 2H), 2.13-2.19 (m, 1H), 1.72-1.79 (m, 1H). Additional peaks were obscured by HDO and DMSO peaks.

The following compounds of the present invention were made using the methodology described in Example 37 above, and substituting the appropriate reactants and/or reagents:

| Compound | Structure | MS [M+H] or [M+H for ³⁵Cl and ³⁷Cl] |
|---|---|---|
| 61 | 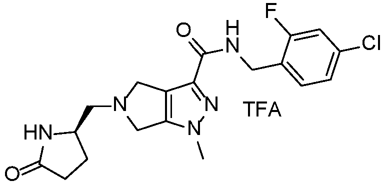 | 406.2 and 408.2 |
| 62 | 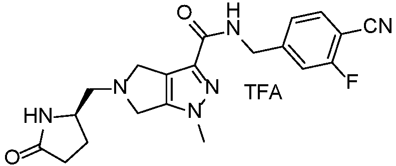 | 397.4 |

### Example 38

### Preparation of Compound 63

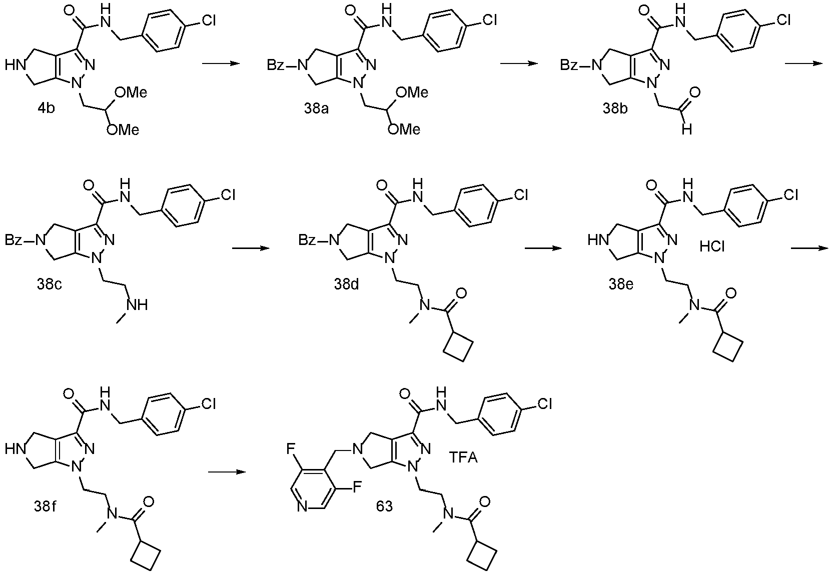

### Step A - synthesis of compound 38a

To a mixture of 4b (3.10 g, 4.25 mmol) in dichloromethane (40 mL) was added triethylamine (1.78 ml, 12.8 mmol), and then the mixture was cooled to 0 ° C. Benzoyl chloride (0.657 g, 4.67 mmol) was added, and the resulting reaction was allowed to stir at room temperature for 1 hour. The reaction mixture was diluted with water (20 mL), extracted with ethyl acetate, and the organic extract was washed with brine, dried over Na₂SO₄, and concentrated in vacuo. The resulting residue was purified using silica gel chromatography eluting with 30-100% EtOAc/petroleum ether to provide 38a as a solid. MS: *m*/*z* 469.2 = [M+H].

### Step B - synthesis of compound 38b

Compound 38b was made from 38a, using the method described in Example 4, step D, and substituting the appropriate reactants and/or reagents, and used without further purification. MS: *m*/*z* 441.3 = [M+H for hydrate].

### Step C - synthesis of compound 38c

To a mixture of methanamine (2.94 g, 28.4 mmol), and 38b (1.25 g, 2.84 mmol) in tetrahydrofuran (20 mL) was added acetic acid (1.30 ml, 22.7 mmol), adjusting the pH to 5. The resulting reaction was allowed to stir at room temperature for 0.5 hours, and sodium cyanoborohydride (0.445 g, 7.09 mmol) was added. The resulting reaction was allowed to stir at room temperature for 3 hours, then diluted with water (30 mL), and extracted with ethyl acetate. The organic extract was washed with brine, dried over Na₂SO₄, filtered, and concentrated in vacuo to provide 38c as a solid, which was used without further purification. MS: *m*/*z* 438.2 = [M+H].

### Step D - synthesis of compound 38d

To a mixture of cyclobutanecarboxylic acid (0.230 g, 2.30 mmol) in DMF (15 mL) was added HATU (0.875 g, 2.30 mmol), and the resulting mixture was allowed to stir at room temperature for 20 minutes. 38c (1.20 g, 1.54 mmol), and diisopropylethylamine (0.268 mL, 1.54 mmol) were added, and the resulting reaction was allowed to stir for 3 hours. The reaction mixture was diluted with water (50 mL) and extracted with ethyl acetate. The organic extract was washed with brine, dried over Na₂SO₄, filtered, and concentrated in vacuo. The resulting residue was purified using silica gel chromatography eluting with 1-10% MeOH/CH₂Cl₂ to provide 38d as a solid. MS: *m*/*z* 520.1 = [M+H].

### Step E - synthesis of compound 38e

To a mixture of 38d (310 mg, 0.596 mmol) in methanol (15 mL) was added hydrazine hydrate (50-60%, 30.0 µL, 0.596 mmol), and the resulting reaction was allowed to stir at 90 ° C for 16 hours. The reaction mixture was cooled to room temperature and filtered. The filtrate was concentrated in vacuo, and the resulting residue was purified using preparative HPLC (reverse-phase C-18), eluting with acetonitrile/water + 0.1% TFA 10-80%. HCl (1 N, 1.08 mL) to provide 38e as a solid.

### Step F - synthesis of compound 38f

To a mixture of 38e (168 mg, 0.371 mmol) in dichloromethane was added a solution of water: saturated aqueous NaHCO₃ (1:1), and the resulting reaction was allowed to stir for 10 minutes. The layers were separated, and the aqueous layer was extracted with dichloromethane and then with ethyl acetate. The combined organic extracts were dried over Na₂SO₄, filtered, and concentrated in vacuo to provide 38f, which was used without further purification. MS: *m*/*z* 416.1 and 418.0 = [M+H for ³⁵Cl and ³⁷Cl].

### Step G - synthesis of compound 63

To a solution (69 µL) of 38f (146 mg, 0.351 mmol) in 9:1 DMF:acetic acid (10 mL), in a microvial, was added a solution (12 µL) of 3,5-difluoroisonicotinaldehyde (28.6 mg, 0.200 mmol) in DMF (0.5 mL), followed by MP-triacetoxyborohydride (Biotage, 10 mg, 2.05 mmol/g). The resulting reaction was shaken at 10G on a Lab Ram for about 15 hours. The reaction mixture was then filtered, and concentrated in vacuo to provide 63, which was used without further purification. MS: *m*/*z* 543.7 = [M+H].

### Example 39

### Preparation of Compound 64

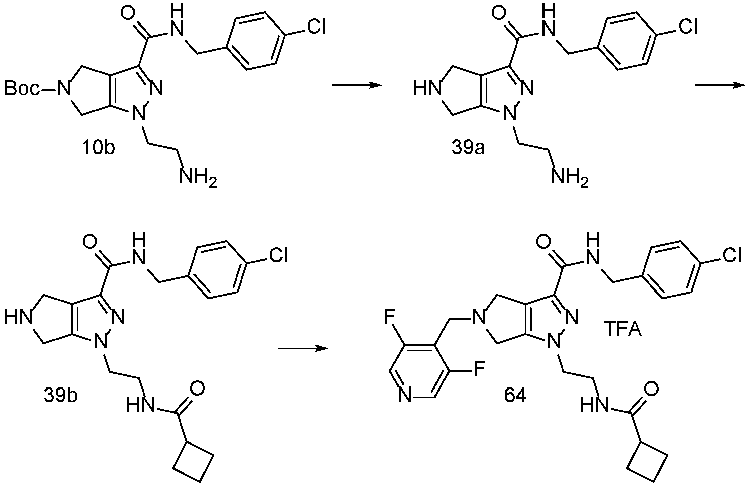

### Step A - synthesis of compound 39a

Compound 39a was made from 10b, using the method described in Example 2, step A, and substituting the appropriate reactants and/or reagents. The resulting residue was purified using silica gel chromatography eluting with 30-90% EtOAc/petroleum ether to provide 39a as a solid. MS: *m*/*z* 502.5 = [M+H].

### Step B - synthesis of compound 39b

Compound 39b was made from 39a, using the method described in Example 6, step B, and substituting the appropriate reactants and/or reagents to provide 39b as a solid, which was used without further purification. MS: *m*/*z* 402.3 = [M+H].

### Step C - synthesis of compound 64

Compound 64 was made from 39b, using the method described in Example 38, Step G, and substituting the appropriate reactants and/or reagents. MS: *m*/*z* 529.2 = [M+H].

The following compounds of the present invention were made using methodology described in Example 39 above, and substituting the appropriate reactants and/or reagents:

| Compound | Structure | MS [M+H] or [M+H for ³⁵Cl and ³⁷Cl] |
|---|---|---|
| 65 | 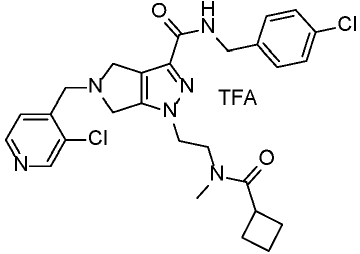 | 541.4 |
| 66 | 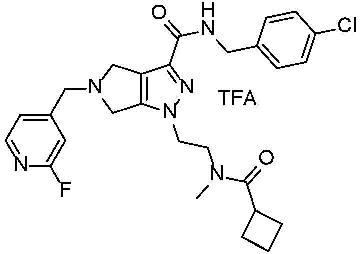 | 525.7 |
| 67 | 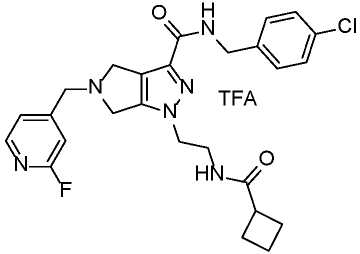 | 511.3 |
| 68 | 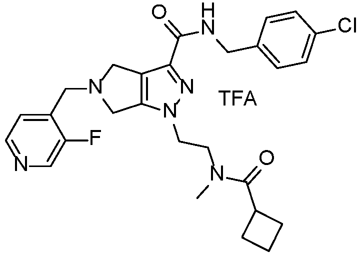 | 525.7 |
| 69 | 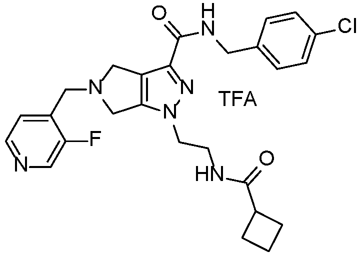 | 511.2 |
| 70 | 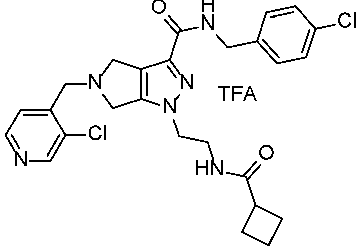 | 527.2 |

### Example 40

### Preparation of Compounds 71 and 72

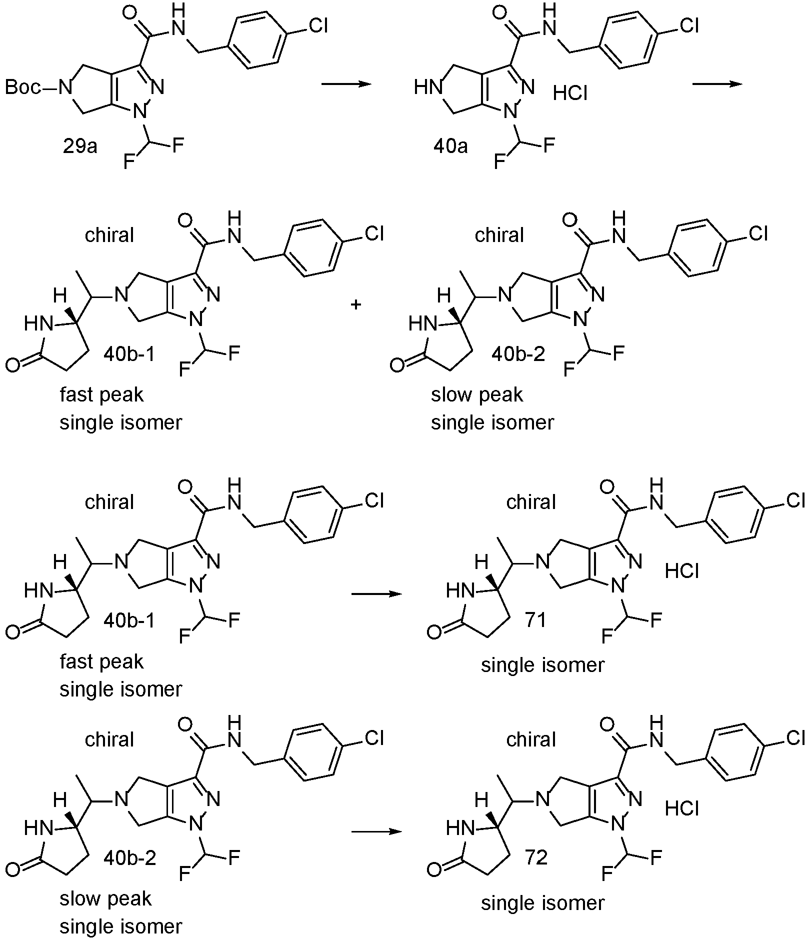

### Step A - synthesis of compound 40a

To a solution of 29a (460 mg, 1.08 mmol) in dichloromethane (5500 µL) was added HCl (4 M in dioxane, 5388 µL). The reaction mixture was allowed to stir at room temperature for 1.5 hours, then concentrated in vacuo to provide 40a, which was used without further purification. MS: *m*/*z* 368.2 and 370.2 = [M+H+MeCN for ³⁵Cl and ³⁷Cl].

### Step B - synthesis of compounds 40b-1 and 40b-2

Compounds 40b-1 and 40b-2 were made from 40a, using the method described in Example 6, Step C, utilizing Int-4 and substituting the appropriate reactants and/or reagents. The resulting residue was purified using silica gel chromatography eluting with 0-100% (3:1 EtOAc:EtOH)/hexanes, then by preparative HPLC (reverse-phase C-18), eluting with acetonitrile/water + 0.1% TFA 10-100%. Fractions containing the product were combined, basified with saturated, aqueous NaHCO₃, and extracted with ethyl acetate. The organic extract was washed with brine, dried over MgSO₄, and concentrated in vacuo to provide 40b-1 and 40b-2 (mixture of diastereomers).

The diastereomers were resolved using Prep-SFC using an OZ-H (2x25 cm) column, eluting with 45% EtOH (+0.1% diethylamine) in CO₂ at 100 bar, 50 mL/min, to provide **40b-1** (fast peak), and 40b-2 (slow peak). MS: *m*/*z* 438.2 = [M+H] for both.

### Step C - synthesis of compound 71

Compound 40b-1 was converted to its HCl salt using the methodology described in Example 2, Step E, and substituting the appropriate reactants and/or reagents, to provide 71. MS: *m*/*z* 438.2 = [M+H]. ¹H NMR (500 MHz, CD₃OD, ppm): δ 7.66 (t, *J* = 60.0 Hz, 1H), 7.34 (s, 4H), 4.98 (br s, 2H), 4.79 (br s, 2H), 4.53 (s, 2H), 4.13-4.15 (m, 1H), 3.85-3.89 (m, 1H), 2.38-2.49 (m, 3H), 2.04-2.09 (m, 1H), 1.43 (d, *J* = 6.5 Hz, 3H).

### Step D - synthesis of compound 72

Compound 40b-2 was converted to its HCl salt using the methodology described in Example 2, Step E, and substituting the appropriate reactants and/or reagents, to provide 72. MS: *m*/*z* 438.2 = [M+H]. ¹H NMR (500 MHz, CD₃OD, ppm): δ 7.66 (t, *J* = 60.0 Hz, 1H), 7.34 (s, 4H), 4.99 (br s, 2H), 4.80 (br s, 2H), 4.53 (s, 2H), 4.14-4.15 (m, 1H), 3.86-3.89 (m, 1H), 2.38-2.50 (m, 3H), 2.04-2.09 (m, 1H), 1.43 (d, *J* = 6.5 Hz, 3H).

### Example 41

### Preparation of Int-13 and Int-14

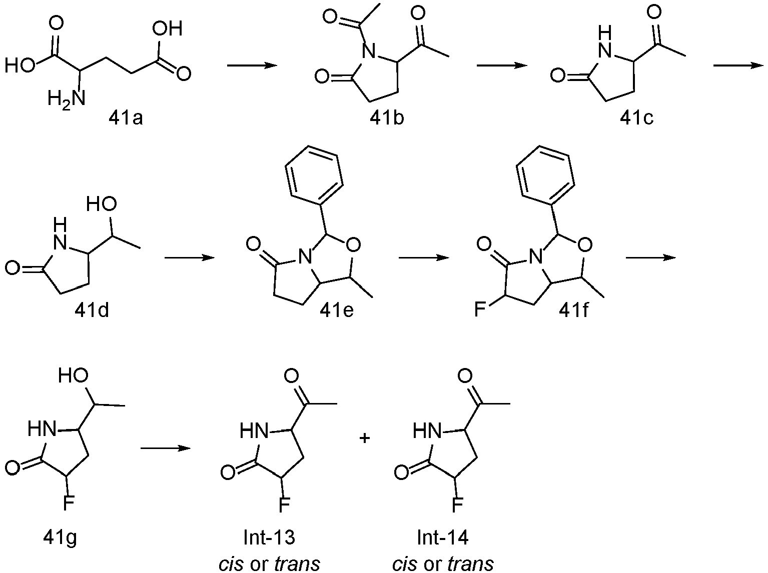

### Step A - synthesis of compound 41b

A mixture of 4-dimethylaminopyridine (1.66 g, 13.59 mmol), triethylamine (52.1 g, 515 mmol), and D,L-glutamic acid (14.2 mL, 136 mmol) in acetic anhydride (71.9 mL, 761 mmol) was allowed to stir for 16 hours at 60 °C. The reaction mixture was concentrated in vacuo, and the resulting residue was purified using silica gel chromatography eluting with 20-50% EtOAc/petroleum ether to provide 41b as an oil. MS: *m*/*z* 170.2 = [M+H].

### Step B - synthesis of compound 41c

A mixture of 41b (18.0 g, 106 mmol), and sodium carbonate (36.9 g, 348 mmol) in water (257 mL) was allowed to stir for 16 hours at room temperature. The reaction mixture was filtered, and the collected solid was washed with water. The filtrate was extracted with ethyl acetate, washed with brine, dried over Na₂SO₄, and concentrated in vacuo. The resulting residue was purified using silica gel chromatography eluting with 0-10% MeOH/CH₂Cl₂ to provide 41c as an oil.

### Step C - synthesis of compound 41d

To a mixture of 41c (5.00 g, 39.3 mmol) in methanol (80 mL) at 0 ° C, was added sodium borohydride (2.23 g, 59.0 mmol) in portions. The reaction was warmed to room temperature and stirred for 30 minutes, quenched by the addition of HCl (1 M, 30 mL), and concentrated in vacuo. The resulting residue was azeotroped with toluene (3 x 50 mL) to provide 41d as an oil. MS: *m*/*z* 130.1 = [M+H].

### Step D - synthesis of compound 41e

To a mixture of 41d (5.30 g, 41.0 mmol), and benzaldehyde (5.44 g, 51.3 mmol) in toluene (80 mL) was added 4-methylbenzenesulfonic acid (0.071 g, 0.410 mmol), and the reaction was allowed to stir at 120 ° C for 16 hours under a Dean-Stark trap. The reaction mixture was cooled to room temperature, and washed with NaHCO₃ (10 mL), and brine (10 mL). The collected organic phase was dried over Na₂SO₄, filtered, and concentrated in vacuo. The resulting residue was purified using silica gel chromatography eluting with 0-50% EtOAc/petroleum ether to provide 41e as an oil. MS: *m*/*z* 218.2 = [M+H].

### Step E - synthesis of compound 41f

To a mixture of diisopropylamine (2.93 g, 29.0 mmol) in tetrahydrofuran (15 mL) at -78 ° C, was added n-butyllithium (2.5 M in hexane, 11.3 mL, 28.2 mmol) dropwise under a nitrogen atmosphere. The reaction mixture was then allowed to warm to 0°C over approximately 30 minute, then again cooled to -78 ° C and 41e (4.2 g, 19.33 mmol) in tetrahydrofuran (20 mL) was added dropwise, The resulting reaction was allowed to stir for 45 minutes at -78 ° C. *N-*Fluorobenzenesulfonimide (6.40 g, 20.3 mmol) in tetrahydrofuran (15 mL) was added. The resulting reaction was allowed to stir for 30 minutes at -78 ° C, then warmed to room temperature and stirred for 10 minutes. The reaction was quenched by addition of water (50 mL) and extracted with ethyl acetate. The organic extract was washed with brine, dried over Na₂SO₄, filtered, and concentrated in vacuo. The resulting residue was purified using silica gel chromatography eluting with 0-50% EtOAc/petroleum ether to provide 41f as an oil. MS: *m*/*z* 236.1 = [M+H].

### Step F - synthesis of compound 41g

To a mixture of 41f (2.8 g, 11.90 mmol) in dichloromethane (20 mL) at 0 ° C, was added TFA (3.00 mL, 11.9 mmol). The resulting reaction was allowed to stir for 1 hour at room temperature, then concentrated in vacuo. The resulting residue was azeotroped with toluene (3 x 50 mL), and the residue obtained was residue was further purified using silica gel chromatography eluting with 1-9% MeOH/CH₂Cl₂ to provide 41g as an oil. MS: *m*/*z* 148.1 = [M+H].

### Step G - synthesis of Int-13 and Int-14

To a mixture of 41g (950 mg, 6.46 mmol) in dichloromethane (15 mL) at 0 ° C, was added Dess-Martin periodinane (5477 mg, 12.91 mmol). The reaction was allowed to stir for 16 hours at room temperature, then quenched by the addition of saturated, aqueous NaHCO₃ (15 mL). The resulting mixture was concentrated in vacuo, and the resulting residue was purified using silica gel chromatography eluting with 1-10% MeOH/CH₂Cl₂ to provide both Int-13 (major product) as a solid and Int-14 (minor product) as a solid. MS: *m*/*z* 146.1 = [M+H] for both.

### Example 42

### Preparation of Compounds 73, 74, 75, and 76

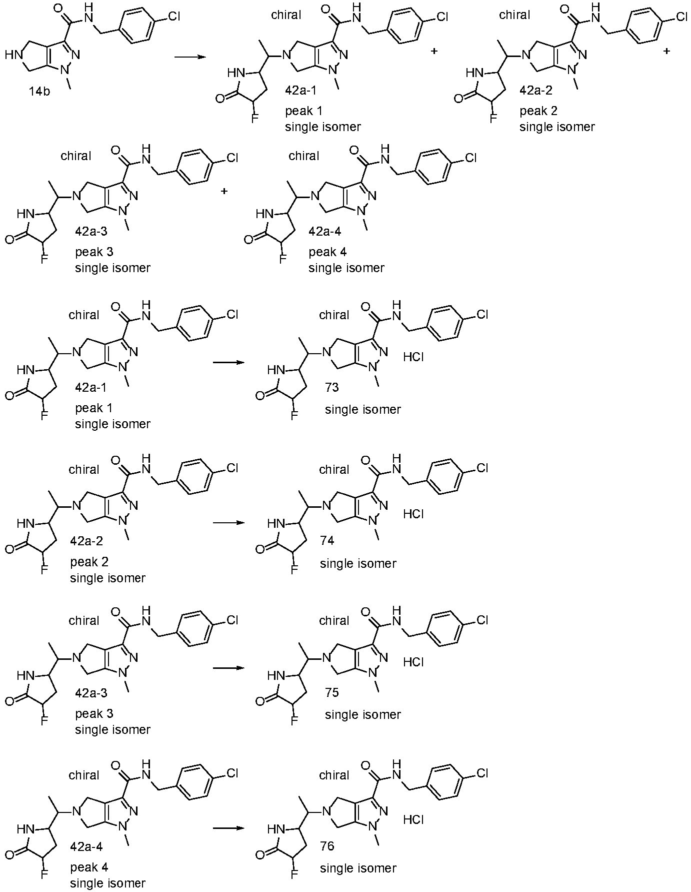

### Step A - synthesis of compounds 42a-1, 42a-2, 42a-3, and 42a-4

Compounds 42a-1, 42a-2, 42a-3, and 42a-4 were made separately using the method described in Example 6, step C, utilizing Int-13 as starting material, and substituting the appropriate reactants and/or reagents. The crude product obtained from each synthesis was purified using preparative HPLC (reverse-phase C-18), eluting with acetonitrile/water + 0.1% TFA 10-50%. Fractions containing the product were combined, basified with saturated, aqueous NaHCO₃, and extracted with ethyl acetate. The organic extract was washed with brine, dried over MgSO₄, and concentrated in vacuo to provide the product as a mixture of diastereomers.

The diastereomers were resolved using Prep-SFC using an AD-H (2x25 cm) column, eluting with 50% MeOH (+0.1% diethylamine) in CO₂ at 100 bar, 50 mL/min, to provide 42a-1 (peak 1), 42a-2 (peak 2), 42a-3 (peak 3), and 42a-4 (peak 4). MS: *m*/*z* 420.4 = [M+H] for all four.

### Step B - synthesis of compound 73

Compound 42a-1 was converted to the compound 73 HCl salt using the methodology described in Example 2, Step E, and substituting the appropriate reactants and/or reagents. MS: *m*/*z* 420.4 = [M+H]. ¹H NMR (500 MHz, CD₃OD, ppm): δ 7.33 (s, 4H), 5.15-5.28 (m, 1H), 4.72-4.78 (m, 2H), 4.50 (s, 2H), 4.23-4.25 (m, 1H), 3.93 (s, 3H), 3.80-3.83 (m, 1H), 3.67 (s, 2H), 2.49-2.56 (m, 2H), 1.39 (d, *J* = 6.5 Hz, 3H).

### Step C - synthesis of compound 74

Compound 42a-2 was converted to the compound 74 HCl salt using the methodology described in Example 2, Step E, and substituting the appropriate reactants and/or reagents. MS: *m*/*z* 420.4 = [M+H]. ¹H NMR (500 MHz, CD₃OD, ppm): δ 7.33 (s, 4H), 5.06-5.19 (m, 1H), 4.70-4.78 (m, 2H), 4.51 (s, 2H), 4.20 (s, 1H), 3.93 (s, 3H), 3.88 (s, 1H), 3.67 (s, 2H), 2.90-2.96 (m, 1H), 2.05-2.14 (m, 1H), 1.45 (d, *J* = 6.5 Hz, 3H).

### Step D - synthesis of compound 75

Compound 42a-3 was converted to the compound 75 HCl salt using the methodology described in Example 2, Step E, and substituting the appropriate reactants and/or reagents. MS: *m*/*z* 420.4 = [M+H]. ¹H NMR (500 MHz, CD₃OD, ppm): δ 7.33 (s, 4H), 5.09-5.23 (m, 1H), 4.72-4.81 (m, 2H), 4.51 (s, 2H), 4.12-4.16 (m, 1H), 3.94 (s, 3H), 3.89-3.92 (m, 1H), 3.67 (s, 2H), 2.77-2.86 (m, 1H), 2.12-2.23 (m, 1H), 1.42 (d, *J* = 6.5 Hz, 3H).

### Step E - synthesis of compound 76

Compound 42a-4 was converted to the compound 76 HCl salt using the methodology described in Example 2, Step E, and substituting the appropriate reactants and/or reagents. MS: *m*/*z* 420.4 = [M+H]. ¹H NMR (500 MHz, CD₃OD, ppm): δ 7.33 (s, 4H), 5.06-5.19 (m, 1H), 4.70-4.80 (m, 2H), 4.51 (s, 2H), 4.21 (s, 1H), 3.93 (s, 3H), 3.89 (s, 1H), 3.67 (s, 2H), 2.90-2.96 (m, 1H), 2.05-2.21 (m, 1H), 1.45 (d, *J* = 6.5 Hz, 3H).

### Example 43

### Preparation of Int-15

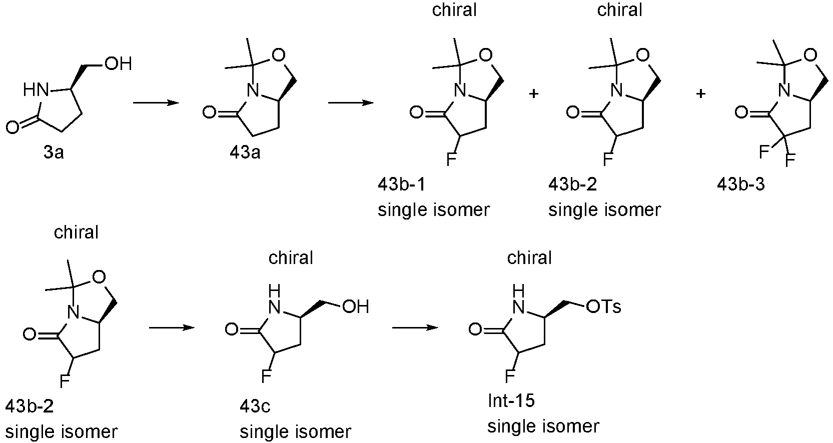

### Step A - synthesis of compound 43a

To a mixture of 3a (32.7 g, 284 mmol), and 4-methylbenzenesulfonic acid (0.98 g, 5.7 mmol) in toluene (100 mL) was added 2,2-dimethoxypropane (44.4 g, 426 mmol). The reaction was allowed to stir for 4 hours at 120 ° C, then the reaction mixture was concentrated in vacuo. The resulting residue was purified using silica gel chromatography eluting with 1-10% MeOH/CH₂Cl₂ to provide 43a. MS: *m*/*z* 156.3 = [M+H].

### Step B - synthesis of compounds 43b-1, 43b-2, and 43b-3

To a mixture of diisopropylamine (15.7 g, 155 mmol) in tetrahydrofuran (250 mL) at -78 °C under nitrogen atmosphere, was added n-butyllithium (2.5 M in hexane, 53.6 mL, 134 mmol). The reaction was allowed to stir for 1 hour at -78 °C then a -78 °C solution of 43a (16.0 g, 103 mmol) in tetrahydrofuran (150 mL) was added, and the reaction mixture was allowed to stir for 45 minutes at -78 ° C. N-Fluorobenzenesulfonimide (NFSI)(48.8 g, 155 mmol) was added, and the resulting reaction was allowed to stir for 1 hour at -78 °C. The reaction was quenched with saturated, aqueous ammonium chloride (200 mL), and extracted with ethyl acetate. The organic extract was washed with brine, dried over Na₂SO₄, and concentrated in vacuo, and the resulting residue was purified using preparative HPLC (reverse-phase C-18), eluting with acetonitrile/water + 10 mmol/L ammonium bicarbonate 10-45% to provide 43b-1 (Peak 1, solid), 43b-2 (Peak 2, solid), and 43b-3 (Peak 3, solid). MS: *m*/*z* 174.1 = [M+H] for 43b-1 and 43b-2. MS: *m*/*z* 192.1 = [M+H] for 43b-3.

### Step C - synthesis of compound 43c

A mixture of 4-methylbenzenesulfonic acid (0.994 g, 5.77 mmol), and 43b-2 (2.00 g, 11.6 mmol) in acetonitrile (10 mL), and water (10 mL) was allowed to stir for 16 hours at 80 °C. The reaction mixture was then concentrated in vacuo, and the resulting residue was purified using silica gel chromatography eluting with 1-10% MeOH/CH₂Cl₂ to provide 43c. MS: *m*/*z* 134.1 = [M+H].

### Step D - synthesis of Int-15

A mixture of 4-dimethylaminopyridine (9.2 mg, 0.075 mmol), triethylamine (228 mg, 2.25 mmol), 4-methylbenzene-1-sulfonyl chloride (286 mg, 1.50 mmol), 43c (100 mg, 0.751 mmol) in dichloromethane (5 mL) was allowed to stir for 2 hours at room temperature. The reaction was quenched with water (10 mL), extracted with ethyl acetate, and the organic extract was washed with brine, dried over Na₂SO₄, filtered, and concentrated in vacuo. The resulting residue was purified using silica gel chromatography eluting with 40-100% EtOAc/petroleum ether to provide Int-15. MS: m/z 288.2 = [M+H].

### Example 44

### Preparation of Compound 77

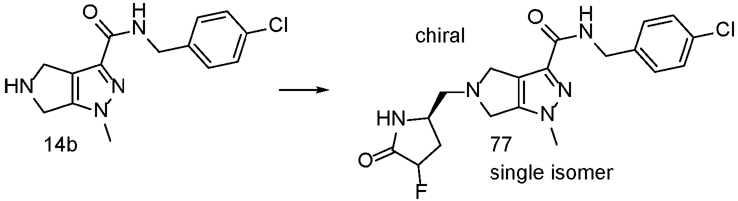

A mixture of Int-15 (109 mg, 0.378 mmol), 14b (110 mg, 0.378 mmol), and potassium carbonate (105 mg, 0.757 mmol) in acetonitrile (4 mL) was allowed to stir for 16 hours at 80 °C. The reaction was quenched with water (10 mL), extracted with dichloromethane, washed with brine, dried over MgSO₄, filtered, and concentrated in vacuo. The resulting residue was purified using silica gel chromatography eluting with 0-10% MeOH/CH₂Cl₂ to provide 77. MS: *m*/*z* 424.1 = [M+H]. ¹H NMR: (400 MHz, methanol-*d*₄, ppm): δ 7.33 (s, 4H), 5.07-5.24 (m, 1H), 4.50 (s, 2H), 3.88-4.01 (m, 5H), 3.87 (s, 3H), 2.84-2.95 (m, 2H), 2.35-2.43 (m, 2H).

### Example 45

### Preparation of Int-16

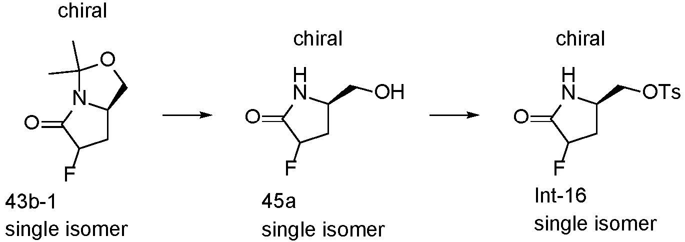

### Step A - synthesis of compound 45a

Compound 45a was made from 43b-1, using the method described in Example 43, step C, and substituting the appropriate reactants and/or reagent. The crude product obtained was purified using silica gel chromatography eluting with 0-10% MeOH/CH₂Cl₂ to provide 45a as a solid. MS: *m*/*z* 134.2 = [M+H].

### Step B - synthesis of Int-16

Int-16 was made from 45a, using the method described in Example 43, step D, and substituting the appropriate reactants and/or reagent. The crude product obtained was purified using silica gel chromatography eluting with 0-10% MeOH/CH₂Cl₂ to provide Int-16 as a solid. MS: *m*/*z* 288.2 = [M+H].

### Example 46

### Preparation of Compound 78

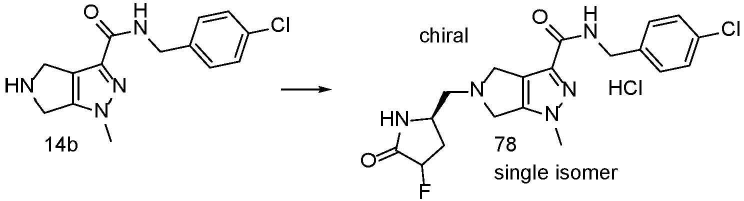

To a mixture of 14b (261 mg, 0.898 mmol), and Int-16 (388 mg, 1.35 mmol) in acetonitrile (11.2 mL) was added diisopropylethylamine (0.470 mL, 2.69 mmol), and the reaction was allowed to stir at 100 °C under a Vigreux condenser for about 15 hours. Additional diisopropylethylamine (0.250 mL), and acetonitrile (3 mL) were added, and stirring was continued for an additional 48 hours. Additional diisopropylethylamine (0.200 mL), and DMF (3 mL) were added, and heating continued for an additional 15 hours. The reaction mixture was then cooled to room temperature, concentrated in vacuo, and the resulting residue was diluted with water. The resulting solution was extracted with ethyl acetate, and the organic extract was washed with brine, dried over MgSO₄, and concentrated in vacuo. The resulting residue was purified using silica gel chromatography eluting with 0-10% MeOH/CH₂Cl₂ to provide semi-pure material which was further purified using preparative HPLC (reverse-phase C-18), eluting with acetonitrile/water + 0.1% TFA 10-50%. The product obtained was then basified with saturated, aqueous NaHCO₃, and extracted with ethyl acetate. The organic extract was washed with brine, dried over MgSO₄, filtered and concentrated in vacuo to provide 78 as a foam. MS: *m*/*z* 406.3 = [M+H]. ¹H NMR (500 MHz, CDCl₃, ppm): δ 7.27-7.31 (m, 4H), 7.16 (t, *J* = 6.0 Hz, 1H), 6.79 (br s, 1H),5.09 (dt, *J* = 52.5, 7.0 Hz, 1H), 4.54 (d, *J* = 6.5 Hz, 2H), 4.07-4.10 (m, 1H), 3.84-3.94 (m, 3H), 3.77 (s, 3H), 3.63-3.72 (m, 1H), 2.96 (dd, *J* = 12.5, 4.0 Hz, 1H), 2.81 (dd, *J =* 12.5, 9.5 Hz, 1H), 2.65-2.73 (m, 1H), 1.90-2.02 (m, 1H).

Compound 78 was converted to its HCl salt using the methodology described in Example 2, Step E, and substituting the appropriate reactants and/or reagents. MS: *m*/*z* 406.3 = [M+H].

### Example 47

### Preparation of Int-17

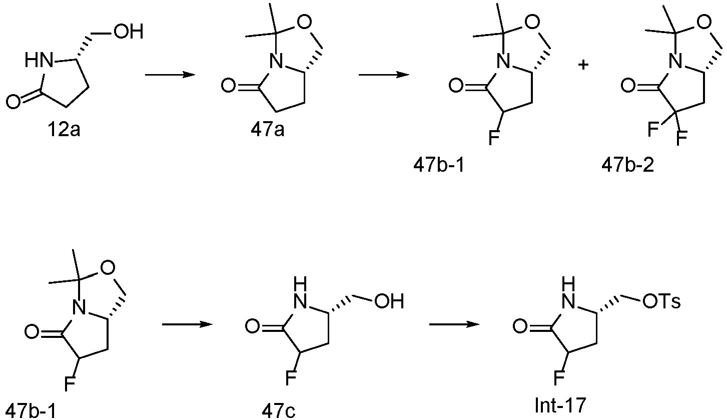

### Step A - synthesis of compound 47a

Compound 47a was made using the method described in Example 43, step A, and substituting the appropriate reactants and/or reagents. The crude product obtained was purified using silica gel chromatography eluting with 0-10% MeOH/CH₂Cl₂ to provide 47a as a solid. MS: *m*/*z* 156.1 = [M+H].

### Step B - synthesis of compounds 47b-1 and 47b-2

Compounds 47b-1 and 47b-2 were made using the method described in Example 43, step B, and substituting the appropriate reactants and/or reagents. The resulting residue was purified using silica gel chromatography eluting with 0-85% EtOAc/petroleum ether to provide 47b-1 (major product, mixture of diastereomers, solid), and 47b-2 (minor product, solid). MS: *m*/*z* 174.1 = [M+H] for 47b-1 and MS: *m*/*z* 192.1 = [M+H] for 47b-2.

### Step C - synthesis of compound 47c

Compound 47c was made from 47b-1, using the method described in Example 43, step C, and substituting the appropriate reactants and/or reagents. The resulting residue was purified using silica gel chromatography eluting with 1-10% MeOH/CH₂Cl₂ to provide 47c (mixture of diastereomers) as a solid. MS: *m*/*z* 175.1 = [M+H].

### Step D - synthesis of compound Int-17

Int-17 was made from 47c, using the method described in Example 43, step D, and substituting the appropriate reactants and/or reagents. The resulting residue was purified using silica gel chromatography eluting with 1-10% MeOH/CH₂Cl₂ to provide Int-17 (mixture of diastereomers) as a solid. MS: *m*/*z* 288.1 = [M+H].

### Example 48

### Preparation of Compounds 79 and 80

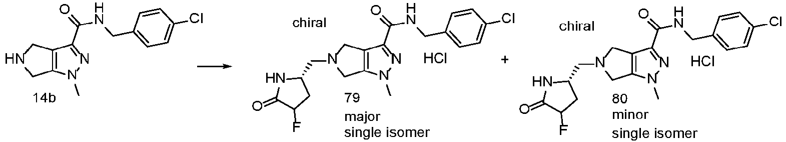

To a mixture of Int-17 (mixture of diastereomers, 217 mg, 0.757 mmol), and 14b (200 mg, 0.688 mmol) in acetonitrile (10 mL) was added potassium carbonate (380 mg, 2.75 mmol), and the resulting reaction was allowed to stir at 80 °C for 16 hours. The reaction mixture was diluted with water (10 mL), extracted with ethyl acetate, and the organic extract was washed with brine, dried over Na₂SO₄, filtered, and concentrated in vacuo. The resulting residue was purified using silica gel chromatography eluting with 0-5% MeOH/CH₂Cl₂ to provide 79 (major product, solid), and free-base 80 (minor product, solid).

Compound 79: MS: *m*/*z* 406.2 = [M+H]. ¹H NMR (300 MHz, methanol-*d*₄, ppm): δ 7.31 (s, 4H), 5.11 (dt, *J* = 53.4, 7.5 Hz, 1H), 4.57 (s, 2H), 3.86-3.97 (m, 5H), 3.81 (s, 3H), 2.86-2.94 (m, 2H), 2.30-2.43 (m, 2H).

Compound 80: MS: *m*/*z* 406.3 = [M+H]. ¹H NMR (300 MHz, methanol-*d*₄, ppm): δ 7.31 (s, 4H), 5.11 (dt, *J* = 53.1, 6.9 Hz, 1H), 4.49 (s, 2H), 3.87-4.01 (m, 4H), 3.82 (s, 3H), 3.74-3.81 (m, 1H), 2.84-2.94 (m, 2H), 2.66-2.79 (m, 1H), 1.94-2.03 (m, 1H).

Both compound 79 and 80 were converted to their HCl salts by addition of HCl (0.5 M, 200 uL) in water (2mL), and acetonitrile (1 mL), followed by lyophilization.

### Example 49

### Preparation of Int-18

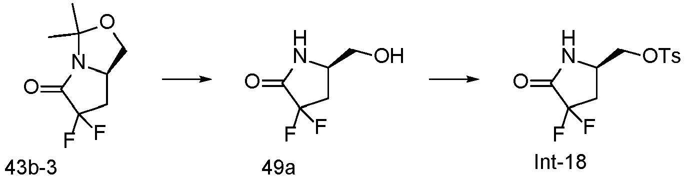

### Step A - synthesis of compound 49a

Compound 49a was made from 43b-3, using the method described in Example 43, step C, and substituting the appropriate reactants and/or reagents. The resulting residue was purified using silica gel chromatography eluting with 0-10% MeOH/CH₂Cl₂ to provide 49a as a solid.

### Step B - synthesis of Int-18

Int-18 was made from 49a, using the method described in Example 43, step D, and substituting the appropriate reactants and/or reagents. The resulting residue was purified using silica gel chromatography eluting with 0-10% MeOH/CH₂Cl₂ to provide Int-18 as a solid. ¹H NMR (300 MHz, CDCl₃, ppm): δ 7.84-7.75 (m, 2H), 7.39 (d, J = 8.0 Hz, 2H), 7.26 (s, 1H), 6.70 (s, 1H), 4.14 (dd, J = 9.8, 3.6 Hz, 1H), 4.02 (s, 1H), 4.03-3.88 (m, 1H), 2.80-2.56 (m, 1H), 2.47 (s, 3H), 2.35-2.12 (m, 1H), 1.26 (s, 1H), 0.88 (s, 1H).

### Example 50

### Preparation of Compound 81

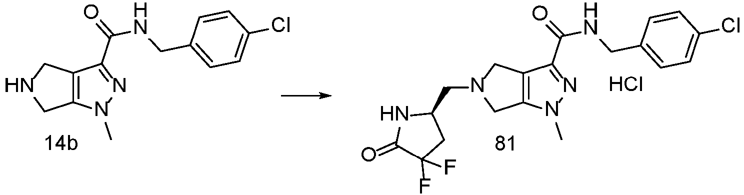

Compound 81 was made from 14b, using the method described in Example 4, step C, and substituting the appropriate reactants and/or reagents. The resulting residue was purified using preparative HPLC (reverse-phase C-18), eluting with acetonitrile/water + 0.1% TFA 40-60%. Fractions containing the product were treated with HCl (1 M, 3 drops per fraction), and concentrated in vacuo to provide the HCl salt of 81 as a solid. MS: *m*/*z* 424.1 = [M+H]. ¹H NMR (400 MHz, methanol-*d*₄, ppm): δ 7.33 (s, 4H), 4.78-4.91 (m, 4H), 4.51 (s, 2H), 4.27-4.28 (m, 1H), 3.93 (s, 3H), 3.66-3.79 (m, 2H), 2.95-3.05 (m, 1H), 2.49-2.56 (m, 1H).

### Example 51

### Preparation of Int-19

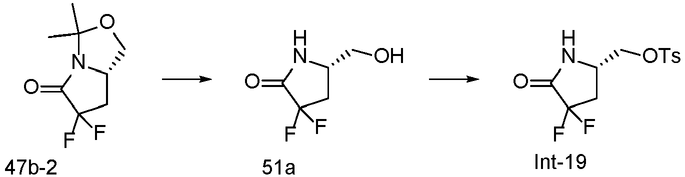

### Step A - synthesis of compound 51a

A mixture of 47b-2 (920 mg, 4.81 mmol) in acetic acid (16.8 mL), acetonitrile (3.6 mL), and water (3.6 mL) was heated to 92 °C and allowed to stir at this temperature for 16 hours. The reaction mixture was concentrated in vacuo, and the resulting residue was purified using silica gel chromatography eluting with 5-20% MeOH/CH₂Cl₂ to provide 51a as a solid. MS: *m*/*z* 152.1 = [M+H].

### Step B - synthesis of Int-19

Int-19 was made using the method described in Example 43, step D, and substituting the appropriate reactants and/or reagents. The resulting residue was purified using silica gel chromatography eluting with 1-10% MeOH/CH₂Cl₂ to provide Int-19 as a solid. MS: *m*/*z 323.1* = [M+H for hydrate].

### Example 52

### Preparation of Compound 82

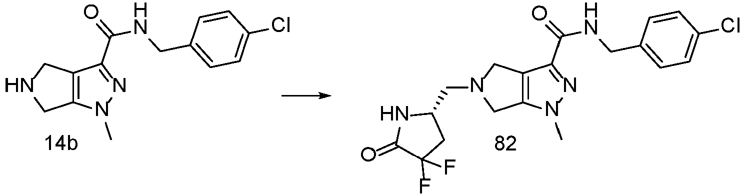

Compound 82 was made from 14b, using the method described in Example 10, step E, and substituting the appropriate reactants and/or reagents. The resulting residue was purified using silica gel chromatography eluting with 0-5% MeOH/CH₂Cl₂ to provide 82 as a solid. MS: *m*/*z* 424.1 = [M+H]. ¹H NMR (400 MHz, methanol-*d*₄, ppm): δ 7.33 (s, 4H), 4.73-4.92 (m, 4H), 4.51 (s, 2H), 4.25 (br s, 1H), 3.93 (s, 3H), 3.64-3.77 (m, 2H), 2.91-3.05 (m, 1H), 2.45-2.57 (m, 1H).

### Example 53

### Preparation of Int-20

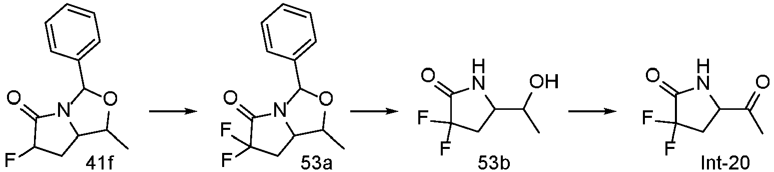

### Step A - synthesis of compound 53a

To a mixture of diisopropylamine (1.24 g, 12.2 mmol) in tetrahydrofuran (10 mL) at -78 °C under nitrogen atmosphere, was added n-butyllithium (2.5 M in hexanes, 4.9 mL). The reaction was warmed to 0 °C and stirred for 30 minutes, then the reaction was cooled to -78 °C. A -78 °C solution of 41f (2.40 g, 10.2 mmol) in tetrahydrofuran (50 mL) was added, and the resulting reaction was allowed to stir for 45 minutes at -78 °C. A -78 °C solution of N-Fluoro-N-(phenylsulfonyl)benzenesulfonamide (4.20 g, 13.3 mmol) in tetrahydrofuran (50 mL) was added, and the resulting reaction was allowed to stir for 30 minutes at -78 °C, then warmed to room temperature and stirred for 10 minutes . The reaction was quenched with saturated, aqueous ammonium chloride (100 mL), extracted with ethyl acetate, and the organic extract was washed with brine, dried over Na₂SO₄, filtered, and concentrated in vacuo. The resulting residue was purified using silica gel chromatography eluting with 0-100% EtOAc/petroleum ether to provide 53a. MS: *m*/*z* 254.1 = [M+H].

### Step B - synthesis of 53b

To a mixture of 53a (1.50 g, 5.92 mmol) in dichloromethane (10 mL) was added trifluoroacetic acid (3.0 mL, 5.9 mmol), and the reaction was allowed to stir for 8 hours at room temperature. The reaction mixture was concentrated in vacuo, and the resulting residue was diluted with toluene and concentrated three times. The resulting residue was purified using silica gel chromatography eluting with 0-10% MeOH/CH₂Cl₂ to provide 53b. MS: *m*/*z* 166.1 = [M+H].

### Step C - synthesis of Int-20

To a mixture of 53b (350 mg, 2.12 mmol) in dichloromethane (10 mL) was added 1,1,1-triacetoxy-1,1-dihydro-1,2-benziodoxol-3(1H)-one (Dess-Martin periodinane, 1.80 g, 4.24 mmol), and sodium bicarbonate (1.78 g, 21.2 mmol) at room temperature. The reaction mixture was stirred for 16 hours, then diluted with saturated, aqueous sodium thiosulfate (20 mL), and concentrated in vacuo. The resulting residue was purified using silica gel chromatography eluting with 30-80% EtOAc/petroleum ether to provide Int-20. MS: *m*/*z* 164.0 = [M+H].

### Example 54

### Preparation of Compounds 83, 84, 85, and 86

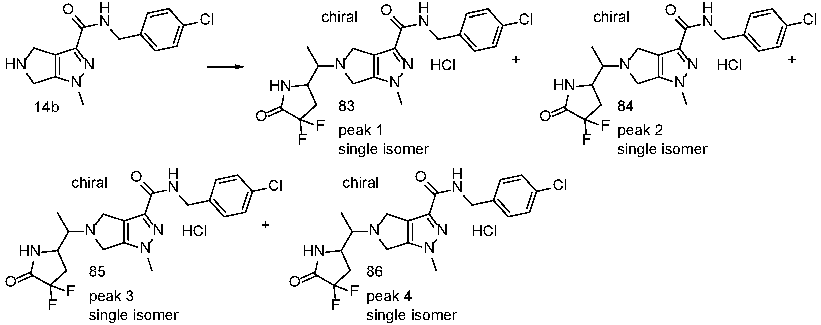

### Step A - synthesis of compounds 83, 84, 85, and 86

Compounds 83, 84, 85, and 86 were made as a mixture of diasteromers from 14b, using the method described in Example 6, step C, utilizing Int-20 and substituting the appropriate reactants and/or reagents. The resulting residue was purified using silica gel chromatography eluting with 0-10% MeOH/CH₂Cl₂ to provide a mixture of diastereomers. MS: *m*/*z* 438.1 = [M+H].

The diastereomers were resolved using chiral HPLC (column: CHIRALPAK IG), eluting with a gradient of hexane:dichloromethane:isopropyl alcohol of 3:1:4. Fractions for each of the four isolated peaks were combined with HCl (0.5 M, excess), and free-dried to provide 83, 84, 85, and 86 as their HCl salts.

For compound 83: MS: *m*/*z* 438.1 = [M+H]. ¹H NMR (400 MHz, methanol-*d*₄, ppm): δ 7.31 (s, 4H), 4.89-4.71 (m, 4H), 4.49 (s, 2H), 4.39-4.35 (m, 1H), 3.94-3.91 (m, 4H), 3.05-2.94 (m, 1H), 2.54-2.49 (m, 1H), 1.42 (d, J = 6.8 Hz, 3H).

For compound 84: MS: *m*/*z* 438.3 = [M+H]. ¹H NMR (400 MHz, methanol-*d*₄, ppm): δ 7.31 (s, 4H), 4.86-4.76 (m, 4H), 4.49 (s, 2H), 4.31-4.30 (m, 1H), 3.93-3.90 (m, 4H), 2.83-2.95 (m, 1H), 2.64-2.59 (m, 1H), 1.40 (d, J = 6.8 Hz, 3H).

For compound 85: MS: *m*/*z* 438.2 = [M+H]. ¹H NMR (400 MHz, methanol-*d*₄, ppm): δ 7.31 (s, 4H), 4.86-4.75 (m, 4H), 4.49 (s, 2H), 4.31-4.29 (m, 1H), 3.93-3.90 (m, 4H), 2.83-2.95 (m, 1H), 2.64-2.60 (m, 1H), 1.40 (d, J = 6.8 Hz, 3H).

For compound 86: MS: *m*/*z* 438.3 = [M+H]. ¹H NMR (400 MHz, methanol-*d*₄, ppm): δ 7.31 (s, 4H), 4.85-4.74 (m, 4H), 4.49 (s, 2H), 4.39-4.36 (m, 1H), 3.94-3.91 (m, 4H), 3.05-2.88 (m, 1H), 2.60-2.45 (m, 1H), 1.42 (d, J = 6.8 Hz, 3H).

### Example 55

### Preparation of Int-21

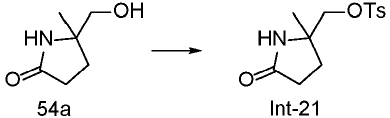

Int-21 was made from 54a, using the method described in Example 43, step D, and substituting the appropriate reactants and/or reagents. The resulting residue was purified using silica gel chromatography eluting with 0-5% MeOH/CH₂Cl₂ to provide Int-21 as a solid. MS: *m*/*z* 284.1 = [M+H].

### Example 56

### Preparation of Compounds 87 and 88

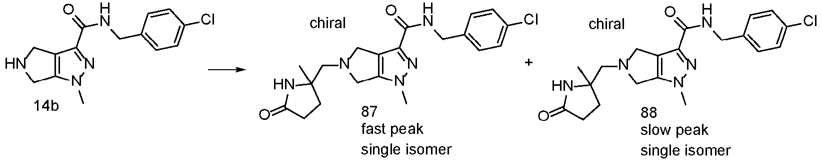

### Step A - synthesis of compounds 87 and 88

Compounds 87 and 88 were made from 14b, using the method described in Example 48, step A, and substituting the appropriate reactants and/or reagents and heating the reaction to 100 ° C. The resulting residue was purified using silica gel chromatography eluting with 0-5% MeOH/CH₂Cl₂ to provide a mixture of enantiomers as a foam. MS: *m*/*z* 402.2 = [M+H].

The enantiomers were resolved using Prep-SFC using an OD-H (2x25 cm) column, eluting with 25% EtOH in CO₂ at 100 bar, 65 mL/min, to provide 87 (fast peak), and 88 (slow peak).

For compound 87: MS: *m*/*z* 402.2 and 404.2 = [M+H for ³⁵Cl and ¹⁷Cl]. ¹H NMR (600 MHz, CDCl₃, ppm): δ 7.23 (dd, *J* = 13.8, 8.4 Hz, 4H), 7.09 (t, *J* = 6.6 Hz, 1H), 6.15-6.19 (m, 1H), 4.49 (d, *J* = 6.0 Hz, 2H), 4.09 (s, 2H), 3.96 (d, *J* = 12.0 Hz, 1H), 3.88 (d, *J* = 12.0 Hz, 1H), 3.69 (s, 3H), 2.81 (dd, *J=* 24.6, 13.8 Hz, 2H), 2.35-2.45 (m, 2H), 2.04-2.08 (m, 1H), 1.80-1.90 (m, 1H), 1.26 (s, 3H).

For compound 88: MS: *m*/*z* 402.2 and 404.2 = [M+H for ³⁵Cl and ³⁷Cl]. ¹H NMR (600 MHz, CDCl₃, ppm): δ 7.24 (dd, *J* = 13.8, 7.8 Hz, 4H), 7.06 (br s, 1H), 5.97 (br s, 1H), 4.50 (d, *J* = 6.0 Hz, 2H), 4.10 (s, 2H), 3.97 (d, *J* = 12.0 Hz, 1H), 3.88 (d, *J* = 12.0 Hz, 1H), 3.70 (s, 3H), 2.82 (dd, *J* = 22.8, 14.4 Hz, 2H), 2.36-2.46 (m, 2H), 2.04-2.09 (m, 1H), 1.81-1.86 (m, 1H), 1.27 (s, 3H).

### Example 57

### Preparation of Int-22

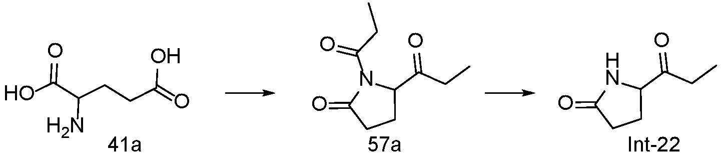

### Step A - synthesis of compound 57a

Compound 57a was made using the method described in Example 41, step A, utilizing propionic anhydride and substituting the appropriate reactants and/or reagents. The resulting residue was purified using silica gel chromatography eluting with 0-10% MeOH/CH₂Cl₂ to provide 57a as a brown oil. MS: *m*/*z* 198.1 = [M+H].

### Step B - synthesis of Int-22

Int-22 was made using the method described in Example 41, step B, and substituting the appropriate reactants and/or reagents. The resulting residue was purified using silica gel chromatography eluting with 1-10% MeOH/CH₂Cl₂ to provide Int-22 as a solid. MS: *m*/*z* 142.1 = [M+H].

### Example 58

### Preparation of Compounds 89, 90, 91, and 92

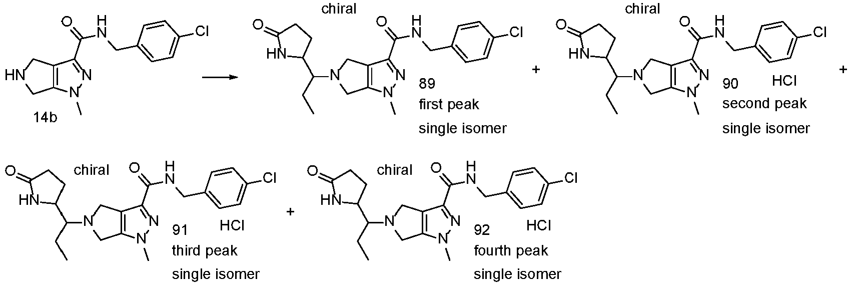

### Step A - synthesis of compounds 89, 90, 91, and 92

To a mixture of Int-22 (2.00 g, 10.3 mmol), 14b (1.00 g, 3.44 mmol), and sodium cyanoborohydride (1.10 g, 17.2 mmol) in 1,2-dichloroethane (15 mL) was added acetic acid (1.24 g, 20.6 mmol), and the resulting reaction was allowed to stir for 16 hours at 120°C in a sealed tube. The reaction was quenched with water (20 mL), extracted with ethyl acetate, and the organic extract was washed with brine, dried over Na₂SO₄, and concentrated in vacuo. The resulting residue was purified using silica gel chromatography eluting with 0-5% MeOH/CH₂Cl₂ to provide a mixture of diastereomers.

The diastereomers were resolved using chiral HPLC (column: CHIRALPAK ID-3), eluting with a gradient of hexane:dichloromethane:ethanol - 1:1:2. Fractions containing each of the 4 separated peaks were lyophilized and the residues were mixed in acetonitrile (1 mL), water (2 mL), and HCl (0.5 M, 880 µL), then lyophilized again to provide 89, 90, 91, and 92 as their HCl salts.

For compound 89: MS: *m*/*z* 416.2 = [M+H]. ¹H NMR: (300 MHz, methanol-*d*₄, ppm): δ 7.32 (s, 4H), 4.72-4.94 (m, 4H), 4.49 (s, 2H), 4.11-4.22 (m, 1H), 3.91 (s, 3H), 3.51-3.64 (m, 1H), 2.38-2.49 (m, 3H), 2.02-2.18 (m, 1H), 1.79-1.91 (m, 2H), 1.17 (t, J = 7.2 Hz, 3H).

For compound 90: MS: *m*/*z* 416.2 = [M+H]. ¹H NMR: (300 MHz, methanol-*d*₄, ppm): δ 7.31 (s, 4H), 4.72-4.97 (m, 4H), 4.49 (s, 2H), 4.13-4.22 (m, 1H), 3.91 (s, 3H), 3.58-3.68 (m, 1H), 2.34-2.52 (m, 3H), 2.03-2.16 (m, 1H), 1.83-1.91 (m, 2H), 1.17 (t, J = 7.5 Hz, 3H).

For compound 91: MS: *m*/*z* 416.2 = [M+H]. ¹H NMR: (300 MHz, methanol-*d*₄, ppm): δ 7.31 (s, 4H), 4.72-4.97 (m, 4H), 4.49 (s, 2H), 4.21-4.26 (m, 1H), 3.92 (s, 3H), 3.68-3.72 (m, 1H), 2.38-2.52 (m, 3H), 1.85-2.06 (m, 3H), 1.18 (t, J = 7.2 Hz, 3H).

For compound 92: MS: *m*/*z* 416.2 = [M+H]. ¹H NMR: (300 MHz, methanol-*d*₄, ppm): δ 7.31 (s, 4H), 4.72-4.97 (m, 4H), 4.49 (s, 2H), 4.21-4.26 (m, 1H), 3.91 (s, 3H), 3.68-3.72 (m, 1H), 2.41-2.52 (m, 3H), 1.85-2.06 (m, 3H), 1.17 (t, J = 7.5 Hz, 3H).

### Example 59

### Preparation of Int-23

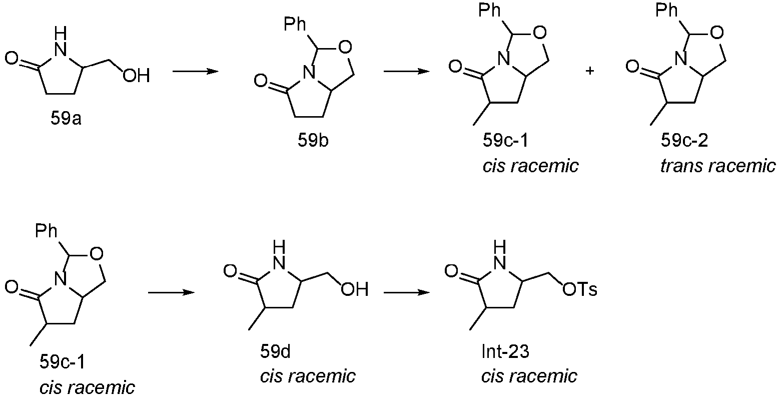

### Step A - synthesis of compound 59b

A mixture of 59a (7.04 g, 61.1 mmol), benzaldehyde (8.1 g, 76 mmol), and 4-methylbenzenesulfonic acid (133 mg, 0.772 mmol) in toluene (50 mL) was refluxed for 12 hours under a Dean-Stark trap. The reaction was cooled to room temperature and washed with saturated aqueous sodium bicarbonate (2 × 20 mL), and brine (20 mL), then the organic phase was dried over Na₂SO₄ and concentrated in vacuo. The resulting residue was purified using silica gel chromatography eluting with 30% EtOAc/petroleum ether to provide 59b. MS: *m*/*z* 204.2 = [M+H].

### Step B - synthesis of compound 59c-1

A solution of diisopropylamine (3.25 g, 32.1 mmol) in tetrahydrofuran (80 mL) was treated dropwise with n-butyllithium (2.5 M in hexane, 13.1 mL) at -10 °C under nitrogen. The reaction was allowed to stir for 15 minutes at -10 °C, then cooled to -78 °C, and a solution of 59b (6.00 g, 29.5 mmol) in tetrahydrofuran (16 mL) was added dropwise. The reaction was allowed to stir for 30 minutes at -78 °C, then treated with iodomethane (5.03 g, 35.4 mmol). The reaction was then allowed to stir for 20 minutes at -20°C and quenched with water (20 mL). The resulting solution was extracted with ethyl acetate, and the organic extract was washed with brine, dried over Na₂SO₄, and concentrated in vacuo. The resulting residue was purified using silica gel chromatography eluting with 0-30% EtOAc/petroleum ether to provide 59c-1 *(cis,* major species). MS: *m*/*z* 218.2 = [M+H]. ¹H NMR: (400 MHz, CDCl₃, ppm): δ 7.48-7.46 (m, 2H), 7.41-7.32 (m, 3H), 6.36 (s, 1H), 4.27-4.23 (m, 1H), 4.15-4.07 (m, 1H), 3.55 (t, J = 7.6 Hz, 1H), 3.02-2.92 (m, 1H), 2.67-2.60 (m, 1H), 1.60-1.52 (m, 1H), 1.26 (d, J = 7.2 Hz, 3H).

### Step C - synthesis of compound 59d

To a solution of 59c-1 (1.65 g, 7.59 mmol) in methanol (28.8 mL) and water (3.2 mL) was added 4-methylbenzenesulfonic acid (35 mg, 0.20 mmol). The reaction was heated at reflux for 16 hours, then the reaction mixture was cooled to room temperature, and concentrated in vacuo to provide 59d, which was used without further purification. MS: *m*/*z* 130.1 = [M+H].

### Step D - synthesis of Int-23

Int-23 was made from 59d, using the method described in Example 3, step A, and substituting the appropriate reactants and/or reagents. The resulting residue was purified using silica gel chromatography eluting with 30-80% EtOAc/petroleum ether to provide Int-23. MS: *m*/*z* 284.1 = [M+H].

### Example 60

### Preparation of Compounds 93 and 94

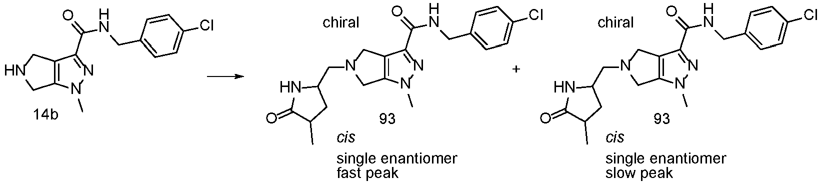

To a mixture of 14b (67 mg, 0.23 mmol), and potassium carbonate (64 mg, 0.461 mmol) in acetonitrile (2 mL) was added Int-23 (144 mg, 0.507 mmol), and the resulting reaction was allowed to stir for 8 hours at 80 °C. The reaction mixture was diluted with water (20 mL), extracted with ethyl acetate, and the organic extract was washed with brine, dried over Na₂SO₄, and concentrated in vacuo. The resulting residue was purified using silica gel chromatography eluting with 0-10% MeOH/CH₂Cl₂ to provide a mixture of *cis* enantiomers.

The enantiomers were resolved using Prep-SFC using an OJ-H (2x25 cm) column, eluting with 25% MeOH (+0.1% diethylamine) in CO₂ at 100 bar, 65 mL/min, to provide 93 (fast peak), and 94 (slow peak).

For compound 93: MS: *m*/*z* 402.3 = [M+H]. ¹H NMR (500 MHz, CDCl₃, ppm): δ 7.21-7.23 (m, 4H), 7.05 (br s, 1H), 5.97 (s, 1H), 4.49 (s, 2H), 4.01 (br s, 1H), 3.88 (br s, 1H), 3.81 (s, 2H), 3.71-3.72 (m, 3H), 3.66 (br s, 1H), 2.85-2.87 (m, 1H), 2.38-2.48 (m, 2H), 1.17 (s, 3H). One proton was obscured.

For compound 94: MS: *m*/*z* 402.3 = [M+H]. ¹H NMR (500 MHz, CDCl₃, ppm): δ 7.20-7.24 (m, 4H), 7.03 (br s, 1H), 5.92 (s, 1H), 4.49 (s, 2H), 4.01 (br s, 1H), 3.89 (br s, 1H), 3.81 (s, 2H), 3.70-3.72 (m, 3H), 3.66 (br s, 1H), 2.85-2.86 (m, 1H), 2.64-2.66 (m, 1H), 2.38-2.48 (m, 2H), 1.16 (s, 3H). One proton was obscured.

### Example 61

### Preparation of Int-24

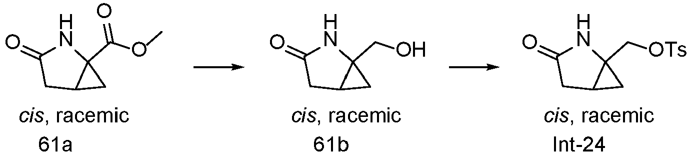

### Step A - synthesis of compound 61b

A solution of 61a (500 mg, 3.22 mmol) in tetrahydrofuran (23.60 mL) was cooled at 0 °C and treated with lithium aluminum hydride (1 M in THF, 3.38 mL), then allowed to warm to room temperature for 3 hours. The reaction mixture was diluted with water (128 µL), sodium hydroxide (15% aqueous, 128 µL), and then more water (384 µL). The resulting mixture was filtered over Celite, washing with THF. The filtrated was concentrated in vacuo to provide 61b which was used without further purification.

### Step B - synthesis of Int-24

Int-24 was made using the method described in Example 3, step A, and substituting the appropriate reactants and/or reagents. The resulting residue was purified using silica gel chromatography eluting with 0-100% EtOAc/hexanes to provide Int-24. MS: *m*/*z* 282.2 = [M+H].

### Example 62

### Preparation of Compound 95

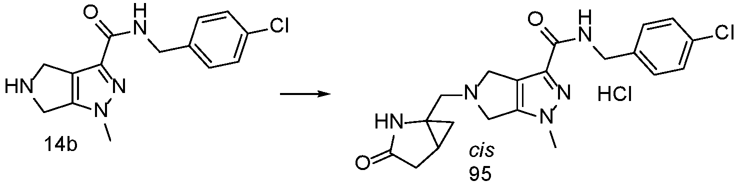

### Step A - synthesis of compound 95

A solution of 14b (70 mg, 0.24 mmol), and Int-24 (118 mg, 0.419 mmol) in acetonitrile (1204 µL) was added diisopropylethylamine (126 µL, 0.722 mmol), sealed, and heated at 100 °C for 48 hours. The reaction mixture was concentrated in vacuo, and the resulting residue was purified using preparative HPLC (reverse-phase C-18), eluting with acetonitrile/water + 0.1% TFA 10-45%. Fractions containing the product were combined, basified with saturated, aqueous NaHCO₃, and extracted with ethyl acetate. The organic extract was washed with brine, dried over MgSO₄, filtered, and concentrated in vacuo to provide free-base 95. MS: *m*/*z* 400.4 = [M+H].

The free base was converted to its HCl salt using the methodology described in Example 2, Step E, and substituting the appropriate reactants and/or reagents. MS: *m*/*z* 400.4 = [M+H]. ¹H NMR (500 MHz, CDCl₃, ppm): δ 7.28-7.32 (m, 4H), 7.12 (t, *J* = 6.0 Hz, 1H), 6.17 (s, 1H), 4.56 (d, *J* = 6.0 Hz, 2H), 4.10 (d, *J* = 6.5 Hz, 1H), 3.99 (d, *J* = 12.0 Hz, 1H), 3.84-3.94 (m, 2H), 3.80 (s, 3H), 3.29 (d, *J* = 12.5 Hz, 1H), 2.75-2.82 (m, 2H), 2.40 (d, *J* = 18.0 Hz, 1H), 1.46-1.50 (m, 1H), 0.97-1.00 (m, 1H), 0.70 (t, *J* = 5.5 Hz, 1H).

### Example 63

### Preparation of Int-25

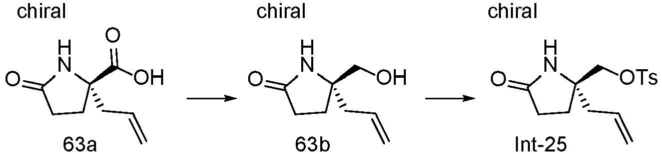

### Step A - synthesis of compound 63b

To a mixture of 63a (1 g, 5.91 mmol) in ethanol (2.57 mL) was treated quickly dropwise with thionyl chloride (0.518 mL, 7.09 mmol) at 0 °C and stirred for 2 hours and allowed to stir at room temperature for 96 hours. The reaction mixture was concentrated in vacuo, and the resulting residue was taken up in ethyl acetate, then stirred over a small amount of potassium carbonate and MgSO₄. The reaction was filtered, and the filtrate was concentrated in vacuo. The resulting residue was dissolved in tetrahydrofuran (10.3 mL), and treated with lithium chloride (0.501 g, 11.8 mmol), and ethanol (2.57 mL), followed by sodium borohydride (0.447 g, 11.8 mmol) at 0 °C. The resulting reaction was allowed to stir at room temperature for about 15 hours. Water (50 µL) was added, followed by additional sodium borohydride (0.447 g, 11.8 mmol), and ethanol (10 mL). After stirring for about 15 hours, the reaction was quenched by addition of citric acid (5%, aqueous). The resulting solution was allowed to stir for 1 hour, then was concentrated in vacuo, and slurried in 3:1 EtOAc:MeOH. After 6 hours, the mixture was filtered through a pad of Celite^{™}, and the Celite^{™} pad was washed with 3:1 EtOAc:MeOH. The filtrate was concentrated in vacuo to provide 63b, which was used without further purification. MS: *m*/*z* 311.3 = [2xM+H].

### Step B - synthesis of Int-25

Int-25 was made from 63a, using the method described in Example 3, step A, and substituting the appropriate reactants and/or reagents. The resulting residue was purified using silica gel chromatography eluting with 0-65% EtOAc/hexanes to provide Int-25. MS: *m*/*z 310.2* = [M+H].

### Example 64

### Preparation of Compound 96

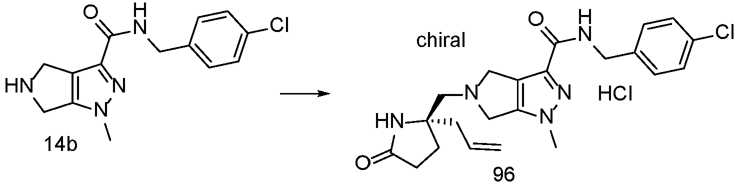

Compound 96 was made from 14b, using the method described in Example 62, step A, and substituting the appropriate reactants and/or reagents. The resulting residue was purified using silica gel chromatography eluting with 0-5% MeOH/CH₂Cl₂ and the product obtained was further purified using preparative HPLC (reverse-phase C-18), eluting with acetonitrile/water + 0.1% TFA 10-60%. Fractions containing the product were combined, basified with saturated, aqueous NaHCO₃, and extracted with ethyl acetate. The organic extract was washed with brine, dried over MgSO₄, and concentrated in vacuo to provide free-base 96. MS: *m*/*z* 428.4 = [M+H]. ¹H NMR (500 MHz, CDCl₃, ppm): δ 7.27-7.32 (m, 4H), 7.09 (t, *J =* 6.0 Hz, 1H), 5.77-5.85 (m, 2H), 5.18-5.21 (m, 2H), 4.55 (d, *J =* 6.5 Hz, 2H), 4.12-4.16 (m, 2H), 4.01-4.04 (m, 1H), 3.93 (dt, *J =* 12.0, 2.5 Hz, 1H), 3.76 (s, 3H), 2.90 (q, *J =* 14.5 Hz, 2H), 2.38-2.49 (m, 3H), 2.29-2.37 (m, 1H), 1.96-2.04 (m, 2H).

The free base was converted to its HCl salt using the methodology described in Example 2, Step E, and substituting the appropriate reactants and/or reagents. MS: *m*/*z* 428.3 = [M+H].

### Example 65

### Preparation of Int-26

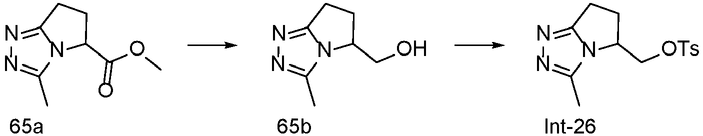

### Step A - synthesis of compound 65b

A mixture of 65a (400 mg, 2.21 mmol) in tetrahydrofuran (11 mL) was cooled to 0 °C and treated with lithium aluminum hydride (2 M in THF, 1.10 mL) dropwise. The reaction was allowed to stir at 0 °C for 2 hours. The reaction was quenched with water (84 µL), sodium hydroxide (15% aqueous, 84 µL), and additional water (252 µL). After stirring at room temperature for 20 minutes, the mixture was filtered, and the collected solid was washed with dichloromethane. The combined filtrate and washing were concentrated in vacuo to provide 65b, which was used without further purification.

### Step B - synthesis of Int-26

Int-26 was made from 65b, using the method described in Example 3, step A, and substituting the appropriate reactants and/or reagents. The crude product obtained was purified using silica gel chromatography eluting with 0-100% (3:1 EtOAc:EtOH)/hexanes. MS: *m*/*z* 308.2 = [M+H].

### Example 66

### Preparation of Compound 97

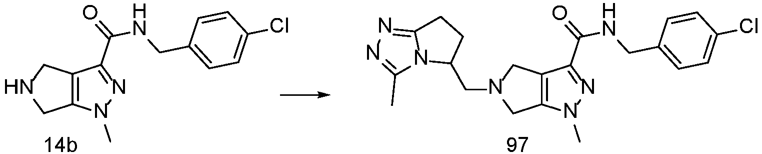

Compound 97 was made from 14b, using the method described in Example 62, step A, and substituting the appropriate reactants and/or reagents. The product obtained was purified using preparative HPLC (reverse-phase C-18), eluting with acetonitrile/water + 0.1% TFA 5-75%. Fractions containing the product were combined, basified with saturated, aqueous NaHCO₃, and extracted with ethyl acetate. The organic extract was washed with brine, dried over MgSO₄, and concentrated in vacuo to provide 97. MS: *m*/*z* 426.3 and 428.3 = [M+H for ³⁵Cl and ³⁷Cl]. ¹H NMR (600 MHz, CDCl₃, ppm): δ 7.24-7.28 (m, 4H), 7.06 (s, 1H), 4.52 (d, *J* = 6.6 Hz, 1H), 4.27 (br s, 1H), 4.03-4.10 (m, 2H), 3.66-3.75 (m, 5H), 3.03-3.04 (m, 2H), 2.93-2.97 (m, 1H), 2.84-2.91 (m, 2H), 2.52-2.54 (m, 1H), 2.44 (s, 3H).

### Example 67

### Preparation of Int-27

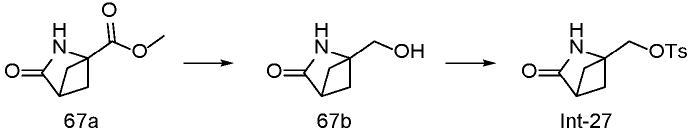

### Step A - synthesis of compound 67b

Compound 67b was made from 67a, using the method described in Example 65, step A, and substituting the appropriate reactants and/or reagents, and was used without further purification.

### Step B - synthesis of Int-27

Int-27 was made from 67b, using the method described in Example 3, step A, and substituting the appropriate reactants and/or reagents. The product obtained was purified using silica gel chromatography eluting with 0-100% EtOAc/hexanes. MS: *m*/*z* 282.3 = [M+H].

### Example 68

### Preparation of Compound 98

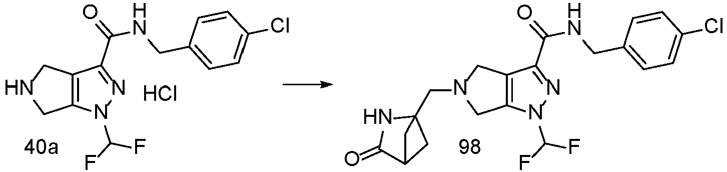

Compound 98 was made from 40a, using the method described in Example 62, step A, and substituting the appropriate reactants and/or reagents. The product obtained was purified using silica gel chromatography eluting with 0-100% (3:1 EtOAc:EtOH)/hexanes. MS: *m*/*z* 436.4 = [M+H]. ¹H NMR (500 MHz, CDCl₃, ppm): δ 7.28-7.34 (m, 4H), 7.09 (s, 1H), 7.06 (t, *J* = 60.5 Hz, 1H), 5.47 (s, 1H), 4.57 (d, *J =* 6.0 Hz, 2H), 4.04-4.08 (m, 4H), 3.11 (s, 2H), 2.81-2.82 (m, 1H), 2.46-2.48 (m, 4H).

### Example 69

### Preparation of Compound 99

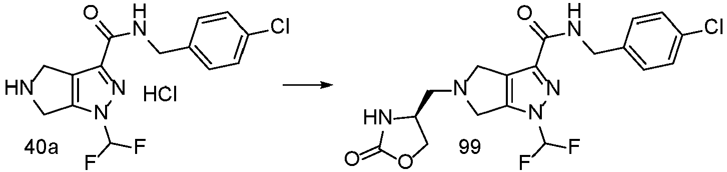

Compound 99 was made from 40a, using the method described in Example 7, step B, utilizing (R)-4-(bromomethyl)oxazolidin-2-one and substituting the appropriate reactants and/or reagents. The product obtained was purified using preparative HPLC (reverse-phase C-18), eluting with acetonitrile/water + 0.1% TFA 10-100%. Fractions containing the product were combined, basified with saturated, aqueous NaHCO₃, and extracted with ethyl acetate. The organic extract was washed with brine, dried over MgSO₄, and concentrated in vacuo. The resulting residue was further purified using silica gel chromatography eluting with 0-100% (3:1 EtOAc:EtOH)/hexanes. MS: *m*/*z* 426.1 = [M+H]. ¹H NMR (600 MHz, CDCl₃, ppm): δ 7.24-7.30 (m, 4H), 7.04 (s, 1H), 7.02 (t, *J =* 60.0 Hz, 1H), 5.25 (s, 1H), 4.49-4.54 (m, 3H), 4.12-4.14 (m, 1H), 4.04-4.09 (m, 3H), 3.95-3.98 (m, 2H), 2.93 (d, *J =* 6.0 Hz, 2H).

### Example 70

### Preparation of Compound 100

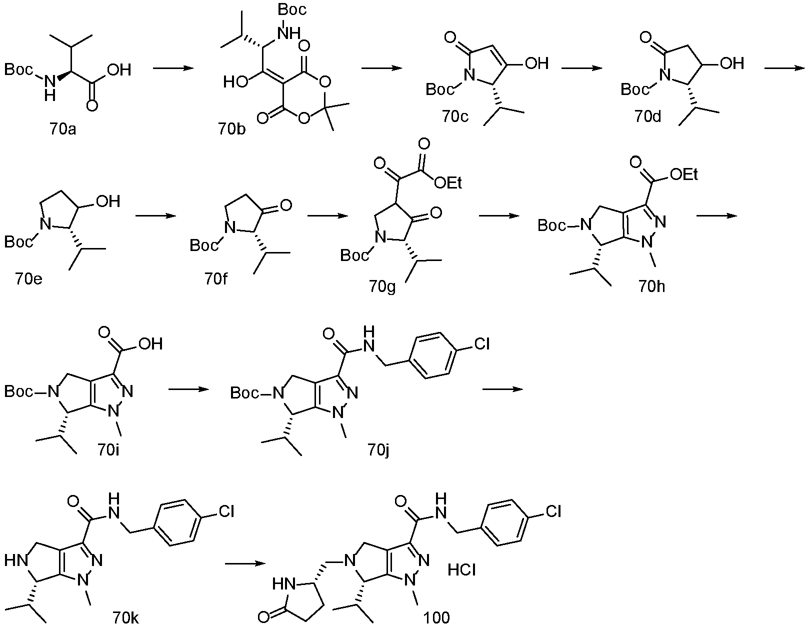

### Step A - synthesis of compound 70b

To a mixture of 70a (40.0 g, 184 mmol) in dichloromethane (1 L) was added 2,2-dimethyl-1,3-dioxane-4,6-dione (39.8 g, 276 mmol), and 4-dimethylaminopyridine (33.7 g, 276 mmol) at room temperature. N,N-Dicyclohexylcarbodiimide (57.0 g, 276 mmol) was added at 0 °C, and the resulting reaction was allowed to stir for 16 hours at room temperature. The reaction was filtered, and the filtrate was washed with saturated, aqueous potassium bisulfate (1 M, 200 mL), and concentrated in vacuo. The resulting residue was diluted with acetone (300 mL) and filtered. The filtrate was concentrated in vacuo to provide 70b which was used without further purification. MS: *m*/*z* 344.2 = [M+H].

### Step B - synthesis of compound 70c

To a mixture of 70b (70.0 g, 214 mmol) in ethyl acetate (500 mL) was allowed to stir for 3 hours at 70 °C. The reaction mixture was concentrated in vacuo, and the resulting residue was purified using silica gel chromatography eluting with 1-20% EtOAc/petroleum ether to provide 70c. MS: *m*/*z* 186.1 [M-tBu+H].

### Step C - synthesis of compound 70d

To a mixture of 70c (4.00 g, 16.6 mmol) in dichloromethane (30 mL) was added acetic acid (12.0 mL, 199 mmol), and sodium borohydride (3.8 g, 99 mmol) at 0 °C. The reaction was allowed to stir for 8 hours at 0 °C, then quenched with methanol (30 mL), and concentrated in vacuo. The resulting residue was purified using silica gel chromatography eluting with 0-10% MeOH/CH₂Cl₂ to provide 70d. MS: *m*/*z* 188.1 [M-tBu+H].

### Step D - synthesis of compound 70e

To a mixture of 70d (2.50 g, 10.3 mmol) in dichloromethane (5 mL) was added borane (1 M in tetrahydrofuran, 30 mL) at 0 °C. The reaction was allowed to stir for 1.5 hours at room temperature, then quenched with methanol (30 mL), and concentrated in vacuo. The resulting residue was purified using silica gel chromatography eluting with 0-5% MeOH/CH₂Cl₂ to provide **70e.** MS: *m*/*z* 174.2 [M-*^{t}*Bu+H].

### Step E - synthesis of compound 70f

To a mixture of 70e (1.50 g, 6.54 mmol), and sodium bicarbonate (4.40 g, 52.3 mmol) in dichloromethane (20 mL) was added 1,1,1-triacetoxy-1,1-dihydro-1,2-benziodoxol-3(1*H*)-one (Dess-Martin periodinane, 4.16 g, 9.81 mmol) at room temperature. The reaction was allowed to stir for 16 hours, quenched with saturated, aqueous sodium thiosulfate (50 mL), extracted with dichloromethane, washed with brine, dried over Na₂SO₄, and concentrated in vacuo. The resulting residue was purified using silica gel chromatography eluting with 0-5% MeOH/CH₂Cl₂ to provide 70f. MS: *m*/*z* 172.2 [M-*^{t}*Bu+H].

### Step F - synthesis of compound 70g

Compound 70g was made using the method described in Example 37, step A, and substituting the appropriate reactants and/or reagents. The resulting residue was purified using silica gel chromatography eluting with 0-5% MeOH/CH₂Cl₂ to provide 70g. MS: *m*/*z* 272.1 [M-*^{t}*Bu+H].

### Step G - synthesis of compound 70h

Compound 70h was made using the method described in Example 37, step B, and substituting the appropriate reactants and/or reagents. The resulting residue was purified using silica gel chromatography eluting with 0-100% EtOAc/petroleum ether to provide 70h. MS: *m*/*z* 338.2 [M + H].

### Step H - synthesis of compound 70i

To a mixture of 70h (600 mg, 1.78 mmol) in tetrahydrofuran (10 mL), and water (10 mL) was added lithium hydroxide (85.0 mg, 3.56 mmol), and stirred for 5 hours at 45 ° C. The reaction mixture was diluted with water (30 mL), and the pH was adjusted to 6 with hydrochloric acid (1 M) at 0 ° C. The mixture was extracted with ethyl acetate, washed with brine, dried over Na₂SO₄, and concentrated in vacuo to provide 70i which was used without further purification. MS: *m*/*z* 310.2 [M+H].

### Step I - synthesis of compound 70j

Compound 70j was made using the method described in Example 2, step A, and substituting the appropriate reactants and/or reagents. The resulting residue was purified using silica gel chromatography eluting with 0-10% MeOH/CH₂Cl₂ to provide 70j. MS: *m*/*z* 433.2 [M+H].

### Step J - synthesis of compound 70k

Compound 70k was made using the method described in Example 2, step C, and substituting the appropriate reactants and/or reagents to provide **70k** which was used without further purification. MS: *m*/*z* 333.2 [M+H].

### Step K - synthesis of compound 100

Compound 100 was made using the method described in Example 10, step E, substituting Int-6b and the appropriate reactants and/or reagents. The resulting residue was purified using silica gel chromatography eluting with 0-10% MeOH/CH₂Cl₂. Fractions containing the product were treated with HCl (1 M, 3 drops per fraction), and concentrated in vacuo to provide to provide 100. MS: *m*/*z* 430.2 [M-Cl]. ¹H NMR (400 MHz, methanol-*d*₄, ppm): δ 7.34-7.29 (m 4H), 4.99-4.86 (m, 2H), 4.60-4.50 (m, 1H), 4.49 (s, 2H), 4.24-4.40 (m, 1H), 3.95 (s, 3H), 3.64-3.59 (m, 2H), 2.57-2.56 (m, 1H), 2.45-2.36 (m, 3H), 1.91-1.90 (m, 1H), 1.29-1.15 (m, 6H).

### Example 71

### Preparation of Compounds 101 and 102

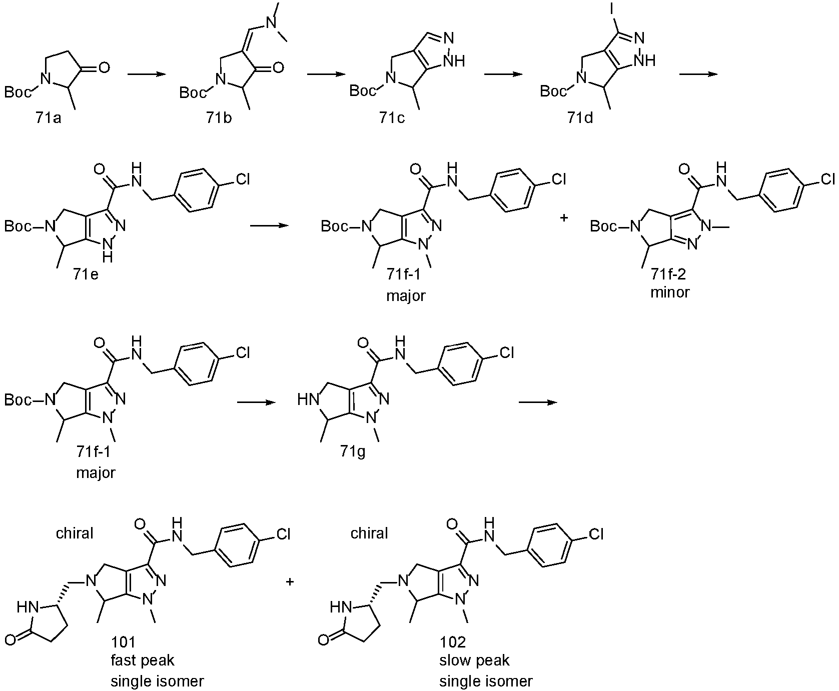

### Step A - synthesis of compound 71b

A mixture of 71a (1.90 g, 9.54 mmol), and N,N-dimethylformamide dimethyl acetal (20.0 mL, 9.54 mmol) was heated at 100 °C for 1 hour. The reaction mixture was concentrated in vacuo to provide 71b, which was used without further purification. MS: *m*/*z* 255.2 = [M+H].

### Step B - synthesis of compound 71c

To a mixture of 71b (2.41 g, 9.48 mmol) in ethanol (47.4 mL) was added hydrazine (3.0 mL, 95 mmol), and the reaction was allowed to stir at 60 ° C for about 15 hours. The reaction mixture was concentrated in vacuo, toluene (100 mL) was added, and the resulting reaction was heated at reflux for about 15 hours under a Dean-Stark trap. The reaction mixture was concentrated in vacuo to provide 71c, which was used without further purification. MS: *m*/*z* 224.1 = [M+H].

### Step C - synthesis of compound 71d

A mixture of 71c (2.39 g, 10.7 mmol), and N-iodosuccinimide (4.82 g, 21.4 mmol) in DMF (53.5 mL) was allowed to stir at 80 ° C for 2 hours. The reaction mixture was diluted with water (500 mL) and extracted with ethyl acetate. The organic extract was washed with Na₂S₂O₃ (10% aqueous, 50 mL), dried over Na₂SO₄, and concentrated in vacuo. The resulting residue was purified using silica gel chromatography eluting with 0-60% EtOAc/hexanes to provide 71d. MS: *m*/*z* 350.1 = [M+H].

### Step D - synthesis of compound 71e

To a mixture of 71d (2.00 g, 5.73 mmol) in DMSO (38.2 mL) was added 4-chlorobenzylamine (5.68 g, 40.1 mmol), triethylamine (3.99 ml, 28.6 mmol), and sodium *tert-*butoxide (0.550 g, 5.73 mmol). [1,1'-Bis(Diphenylphosphino)ferrocene]palladium (II) dichloride (PdCl₂(dppf), 0.42 g, 0.57 mmol) was then added, and the resulting reaction was allowed to stir at 95 ° C for 16 hours, under 10 atmospheres of carbon monoxide. The reaction mixture was diluted with ethyl acetate and washed with brine, dried over Na₂SO₄, and concentrated in vacuo. The resulting residue was purified using silica gel chromatography eluting with 0-100% EtOAc/hexanes to provide 71e. MS: *m*/*z* 391.2 = [M+H].

### Step E - synthesis of compounds 71f-1 and 71f-2

Compounds 71f-1 and 71f-2 were made from 71e, using the method described in Example 14, step A, and substituting the appropriate reactants and/or reagents. The resulting residue was purified using silica gel chromatography eluting with 0-55% EtOAc/hexanes to provide 71f-1 (major), and 71f-2 (minor). MS: *m*/*z* 405.2 = [M+H] for 71f-1.

### Step F - synthesis of compound 71g

Compound 71g was made from 71f-2, using the method described in Example 2, step C, and substituting the appropriate reactants and/or reagents to provide 71g which was used without further purification. MS: *m*/*z* 305.2 = [M+H].

### Step G - synthesis of compounds 101 and 102

To a mixture of 71g (120 mg, 0.394 mmol), and (S)-5-(bromomethyl)pyrrolidin-2-one (140 mg, 0.787 mmol) in acetonitrile (1575 µL) was added potassium carbonate (163 mg, 1.18 mmol). The reaction was allowed to stir at 110 °C for 5 hours, then filtered, and purified using preparative HPLC (reverse-phase C-18), eluting with acetonitrile/water + 0.1% TFA 5-95% to provide a mixture of diastereomers.

The diastereomers were resolved using Prep-SFC using an AS-H (2x25 cm) column, eluting with 35% MeOH (+0.1% diethylamine) in CO₂ at 100 bar, 60 mL/min, to provide 101 (fast peak), and 102 (slow peak).

For compound 101: MS: *m*/*z* 402.1 and 404.1 = [M+H for ³⁵Cl and ³⁷Cl]. ¹H NMR (600 MHz, CDCl₃, ppm): δ 7.24-7.27 (m, 4H), 7.05 (t, *J =* 6.0 Hz, 1H), 5.94 (s, 1H), 4.48-4.55 (m, 2H), 4.31 (dd, *J =* 12.0, 2.4 Hz, 1H), 3.84-3.86 (m, 1H), 3.75-3.80 (m, 1H), 3.75 (s, 3H), 3.56 (dd, *J* = 12.0, 1.8 Hz, 1H), 2.81 (dd, *J* = 12.6, 3.0 Hz, 1H), 2.63 (t, *J* = 11.4 Hz, 1H), 2.32-2.43 (m, 2H), 2.21-2.27 (m, 1H), 1.63-1.69 (m, 1H), 1.36 (d, *J* = 6.6 Hz, 3H).

For compound 102: MS: *m*/*z* 402.1 and 404.1 = [M+H for ³⁵Cl and ³⁷Cl]. ¹H NMR (600 MHz, CDCl₃, ppm): δ 7.24-7.28 (m, 4H), 7.04 (s, 1H), 5.73 (s, 1H), 4.48-4.56 (m, 2H), 4.19 (dd, *J* = 12.0, 2.4 Hz, 1H), 3.96-3.97 (m, 1H), 3.74-3.80 (m, 2H), 3.76 (s, 3H), 2.80-2.83 (m, 1H), 2.72-2.75 (m, 1H), 2.34 (t, *J =* 8.4 Hz, 1H), 2.22-2.28 (m, 2H), 1.80-1.86 (m, 1H), 1.35 (d, *J =* 6.0 Hz, 3H).

### Example 72

### Preparation of Compounds 103 and 104

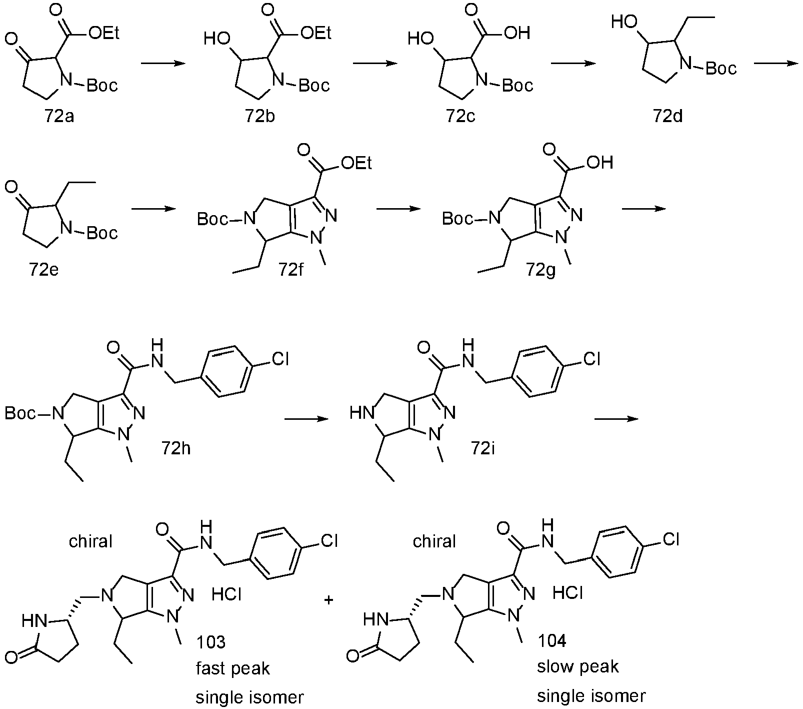

### Step A - synthesis of compound 72b

To a 0 °C mixture of 72a (7.00 g, 27.2 mmol) in tetrahydrofuran (50 mL) was added sodium borohydride (0.36 g, 9.5 mmol). The reaction was allowed to stir for 1 hour at 0 °C, then was quenched with saturated ammonium chloride (20 mL) and extracted with ethyl acetate. The organic extract was washed with brine, dried over Na₂SO₄, filtered, and concentrated in vacuo to provide 72b, which was used without further purification. MS: *m*/*z* 160.1 [M-Boc+H].

### Step B - synthesis of compound 72c

To a mixture of 72b (7.00 g, 27.0 mmol) in tetrahydrofuran (15 mL), and water (15 mL) was added lithium hydroxide monohydrate (2.30 g, 54.0 mmol). The resulting reaction was allowed to stir at room temperature for 1 hour, then was diluted with water (30 mL). The pH was adjusted to 7 with hydrochloric acid (1 M), and the solution was extracted with ethyl acetate. The organic phase was washed with brine, dried over Na₂SO₄, and concentrated in vacuo to provide 72c which was used without further purification. MS: *m*/*z* 176.1 [M-*^{t}*Bu+H].

### Step C - synthesis of compound 72d

To a mixture of **72c** (1.00 g, 4.32 mmol), bromoethane (0.94 g, 8.7 mmol), nickel (II) chloride-ethylene glycol dimethyl ether complex (9.5 mg, 0.043 mmol), 4,4'-di-tert-butyl-2,2'-bipyridine (0.012 g, 0.043 mmol), and potassium carbonate (1.20 g, 8.65 mmol) in water (1.56 mL, 86 mmol) and acetonitrile (15 mL), was added Ir[dF(CF₃)ppy]₂(dtbbpy)PF₆ (0.10 g, 0.086 mmol) under argon. The reaction was irradiated with blue LED (400 nm, 15 W) for 16 hours, under an active cooling fan to maintain room temperature. The reaction was quenched with water (30 mL), extracted with ethyl acetate, and the organic extract was washed with brine, dried over Na₂SO₄, and concentrated in vacuo. The resulting residue was purified using preparative HPLC (reverse-phase C-18), eluting with acetonitrile/water + 0.1% TFA 10-60% to provide 72d. MS: *m*/*z* 160.2 [M-*^{t}*Bu+H].

### Step D - synthesis of compound 72e

Compound 72e was made from 72d, using the method described in Example 41, step G, and substituting the appropriate reactants and/or reagents, and used without further purification. MS: *m*/*z* 158.2 [M-*^{t}*Bu+H].

### Step E - synthesis of compound 72f

Compound 72f was made from 72e, using the method described in Example 37, step A, and substituting the appropriate reactants and/or reagents. The product obtained was purified using silica gel chromatography eluting with 0-10% MeOH/CH₂Cl₂ to provide 72f. MS: *m*/*z* 324.3 [M+H].

### Step F - synthesis of compound 72g

Compound 72g was made from 72f, using the method described in Example 72, step B, and substituting the appropriate reactants and/or reagents, and was used without further purification. MS: *m*/*z* 296.3 [M+H].

### Step G - synthesis of compound 72h

Compound 72h was made from 72g, using the method described in Example 2, step A, and substituting the appropriate reactants and/or reagents. The product obtained was purified using silica gel chromatography eluting with 0-10% MeOH/CH₂Cl₂ to provide 72h. MS: *m*/*z* 419.3 [M+H].

### Step H - synthesis of compound 72i

Compound 72i was made from 72h, using the method described in Example 2, step C, and substituting the appropriate reactants and/or reagents, and was used without further purification. MS: *m*/*z* 319.2 [M+H].

### Step I - synthesis of compounds 103 and 104

Compounds 103 and 104 was made from 72i, using the method described in Example 13, step A, utilizing Int-6 and substituting the appropriate reactants and/or reagents to provide a mixture of diastereomers which were resolved using preparative chiral-HPLC (column: CHIRALPAK IF), eluting with a gradient of hexane: dichloromethane: ethanol - 3:1:4. HCl (1 M, 3 drops per fraction) was added to each target fraction and the fractions were concentrated in vacuo to provide 103 (fast peak), and 104 (slow peak).

For compound 103: MS: *m*/*z* 416.3 [M-Cl]. ¹H NMR: (400 MHz, methanol-*d*₄, ppm): δ 7.34 (s, 4H), 5.10 (s, 1H), 4.90 (s, 1H), 4.52 (s, 2H), 4.24 (s, 1H), 3.96 (s, 3H), 3.72-3.60 (m, 2H), 2.58-2.32 (m, 3H), 2.20-2.28 (m, 1H), 1.97 (s, 1H), 1.12 (s, 3H).

For compound 104: MS: *m*/*z* 416.3 [M-Cl]. ¹H NMR: (400 MHz, methanol-*d*₄, ppm): δ 7.33 (s, 4H), 5.05-5.01 (m, 2H), 4.62-4.51 (m, 1H), 4.51 (s, 2H), 4.27-4.23 (m, 1H), 3.95 (s, 3H), 3.72-3.60 (m, 2H), 2.52-2.27 (m, 5H), 1.97-1.90 (m, 1H), 1.23-1.18 (m, 3H).

### Example 73

### Preparation of Compounds 105 and 106

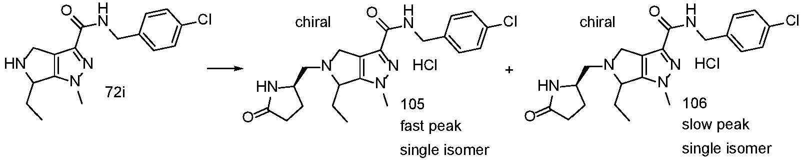

Compounds 105 and 106 were made from 72i, using the method described in Example 13, step A, utilizing Int-2, and substituting the appropriate reactants and/or reagents to provide a mixture of diastereomers which were resolved using silica gel chromatography eluting with 0-10% MeOH/CH₂Cl₂. HCl (1 M, 3 drops per fraction) was added to each target fraction and the fractions were concentrated in vacuo to provide 105 (fast peak) as its HCl salt, and 106 (slow peak) as its HCl salt.

For compound 105 HCl salt: MS: *m*/*z* 416.2 = [M+H]. ¹H NMR (400 MHz, methanol-*d₄*, ppm): δ 7.33 (s, 4H), 4.89-5.03 (m, 2H), 4.60-4.68 (m, 1H), 4.52 (s, 2H), 4.22 (br s, 1H), 3.96 (s, 3H), 3.62-3.70 (m, 2H), 2.40-2.56 (m, 3H), 2.27 (br s, 2H), 1.92 (br s, 1H), 1.19 (s, 3H).

For compound 106 HCl salt: MS: *m*/*z* 416.2 = [M+H]. ¹H NMR (400 MHz, methanol-*d₄*, ppm): δ 7.34 (s, 4H), 5.09 (s, 1H), 4.89 (s, 1H), 4.52 (s, 3H), 4.21 (br s, 1H), 3.96 (s, 3H), 3.60-3.64 (m, 2H), 2.35-2.54 (m, 3H), 2.21 (br s, 2H), 1.96 (br s, 1H), 1.15 (s, 3H).

### Example 74

### Preparation of Compounds 107 and 108

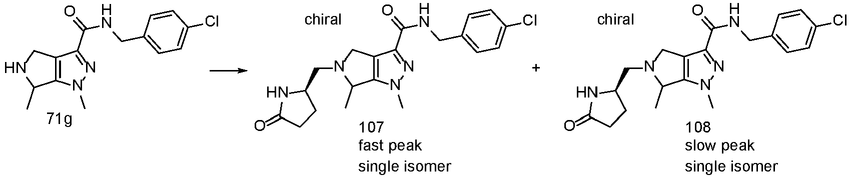

Compounds 107 and 108 were made from 72g, using the method described in Example 10, step E, utilizing (R)-5-(bromomethyl)pyrrolidin-2-one, and substituting the appropriate reactants and/or reagents. The products obtained were purified using preparative HPLC (reverse-phase C-18), eluting with acetonitrile/water + 0.1% TFA 5-95%. Fractions containing the products were combined, basified with saturated, aqueous NaHCO₃, and extracted with ethyl acetate. The organic extract was washed with brine, dried over MgSO₄, and concentrated in vacuo to provide a mixture of diastereomers which were resolved using Prep-SFC using an ASH (3x20 cm) column, eluting with 40% MeOH (+0.1% diethylamine) in CO₂ at 100 bar, 80 mL/min, to provide 107 (fast peak), and 108 (slow peak).

For compound 107: MS: *m*/*z* 402.2 and 404.2 = [M+H for ³⁵Cl and ³⁷Cl]. ¹H NMR (600 MHz, CDCl₃, ppm): δ 7.24-7.28 (m, 4H), 4.49-4.54 (m, 2H), 4.31 (dd, *J =* 12.0, 3.0 Hz, 1H), 3.84-3.87 (m, 1H), 3.75-3.80 (m, 1H), 3.76 (s, 3H), 3.56 (dd, *J =* 12.0, 2.4 Hz, 1H), 2.82 (dd, *J =* 12.0, 3.0 Hz, 1H), 2.62-2.66 (m, 1H), 2.32-2.43 (m, 2H), 2.22-2.27 (m, 1H), 1.63-1.70 (m, 1H), 1.35 (d, *J=* 6.0 Hz, 3H).

For compound 108: MS: *m*/*z* 402.2 and 404.2 = [M+H for ³⁵Cl and ³⁷Cl]. ¹H NMR (600 MHz, CDCl₃, ppm): δ 7.24-7.28 (m, 4H), 4.48-4.55 (m, 2H), 4.19 (dd, *J =* 12.0, 3.0 Hz, 1H), 3.95-3.98 (m, 1H), 3.75-3.80 (m, 1H), 3.76 (s, 3H), 3.73-3.76 (m, 1H), 2.79-2.82 (m, 1H), 2.72-2.75 (m, 1H), 2.31-2.38 (m, 2H), 2.21-2.27 (m, 1H), 1.80-1.86 (m, 1H), 1.34 (d, *J =* 6.6 Hz, 3H).

### Example 75

### Preparation of Int-28 and Int-29

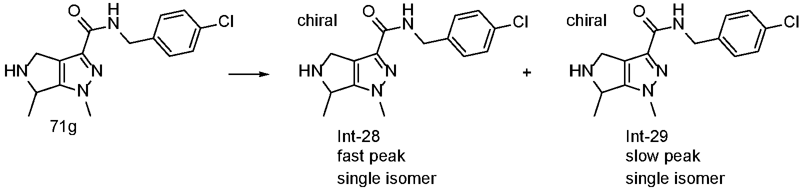

Compound 71g was resolved using Prep-SFC using an IC (2x25 cm) column, eluting with 25% EtOH (+0.2% diethylamine) in CO₂ at 100 bar, 75 mL/min, to provide Int-28 (fast peak), and Int-29 (slow peak). MS: *m*/*z* 305.1 = [M+H] for both.

### Example 76

### Preparation of Compounds 109, 110, 111, and 112

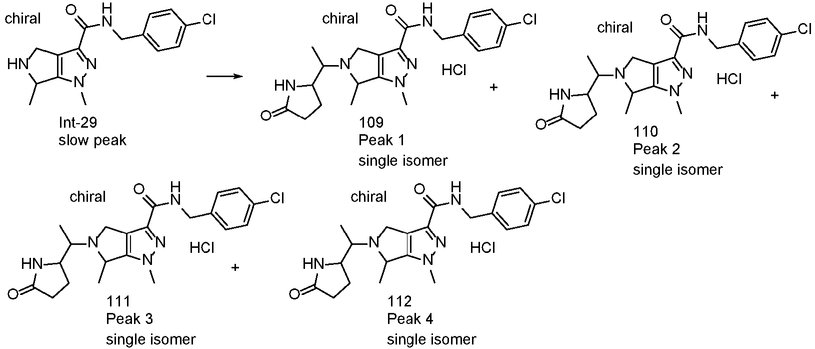

### Step A - synthesis of compounds 109, 110, 111, and 112

Compounds 109, 110, 111, and 112 were made using the method described in Example 6, step C, utilizing Int-1 and substituting the appropriate reactants and/or reagents. The resulting residue was purified using preparative HPLC (reverse-phase C-18), eluting with acetonitrile/water + 0.1% TFA 10-45%. Fractions containing the product were combined, basified with saturated, aqueous NaHCO₃, and extracted with ethyl acetate. The organic extract was washed with brine, dried over MgSO₄, and concentrated in vacuo to provide a mixture of diastereomers which were resolved using Prep-SFC using an AS-H (2x25 cm) column, eluting with 35% MeOH (+0.1% diethylamine) in CO₂ at 100 bar, 55 mL/min, to provide free-base 109 (peak 1), 110 (peak 2), 111 (peak 3), and 112 (peak 4).

For free-base 109: ¹H NMR (500 MHz, CDCl₃, ppm): δ 7.27-7.31 (m, 4H), 7.11 (t, *J=* 6.0 Hz, 1H), 6.19 (s, 1H), 4.55 (d, *J =* 6.0 Hz, 2H), 4.05-4.08 (m, 1H), 3.96 (d, *J =* 3.0 Hz, 2H), 3.79 (s, 3H), 3.49-3.57 (m, 1H), 2.65-2.71 (m, 1H), 2.44-2.52 (m, 1H), 2.33-2.41 (m, 1H), 2.20-2.26 (m, 1H), 1.63-1.72 (m, 1H), 1.38 (d, *J =* 6.5 Hz, 3H), 1.00 (d, *J =* 6.5 Hz, 3H).

For free-base 110: ¹H NMR (500 MHz, CDCl₃, ppm): δ 7.27-7.31 (m, 4H), 7.13 (t, *J=* 6.0 Hz, 1H), 6.23 (s, 1H), 4.55 (d, *J =* 6.0 Hz, 2H), 4.30-4.32 (m, 2H), 3.85 (d, *J =* 12.0 Hz, 1H), 3.77 (s, 3H), 3.56-3.31 (m, 1H), 2.68-2.73 (m, 1H), 2.34-2.48 (m, 2H), 2.18-2.24 (m, 1H), 1.63-1.72 (m, 1H), 1.34 (d, *J =* 6.0 Hz, 3H), 1.03 (d, *J =* 6.5 Hz, 3H).

For free-base 111: ¹H NMR (500 MHz, CDCl₃, ppm): δ 7.28-7.32 (m, 4H), 7.09 (t, *J =* 6.0 Hz, 1H), 5.89 (s, 1H), 4.52-4.60 (m, 2H), 4.29-4.33 (m, 2H), 3.85-3.89 (m, 2H), 3.79 (s, 3H), 2.87-2.92 (m, 1H), 2.33-2.41 (m, 2H), 2.22-2.29 (m, 1H), 1.94-2.01 (m, 1H), 1.35 (d, *J =* 6.0 Hz, 3H), 1.15 (d, *J =* 6.5 Hz, 3H).

For free-base 112: ¹H NMR (500 MHz, CDCl₃, ppm): δ 7.29-7.32 (m, 4H), 7.09 (t, *J =* 6.0 Hz, 1H), 6.01 (s, 1H), 4.52-4.60 (m, 2H), 4.10-4.14 (m, 1H), 3.96 (d, *J =* 3.0 Hz, 2H), 3.81 (s, 3H), 3.57-3.61 (m, 1H), 2.71-2.77 (m, 1H), 2.33-2.41 (m, 2H), 2.24-2.31 (m, 1H), 2.11-2.19 (m, 1H), 1.39 (d, *J* = 6.0 Hz, 3H), 1.10 (d, *J* = 6.5 Hz, 3H).

The compounds were converted to their respective HCl salts using the methodology described in Example 2, Step E, and substituting the appropriate reactants and/or reagents.

For compound 109 HCl salt: MS: *m*/*z* 416.3 = [M+H].

For compound 110 HCl salt: MS: *m*/*z* 416.3 and 418.3 = [M+H for ³⁵Cl and ³⁷Cl].

For compound 111 HCl salt: MS: *m*/*z* 416.4 and 418.3 = [M+H for ³⁵Cl and ³⁷Cl].

For compound 112 HCl salt: MS: *m*/*z* 416.3 = [M+H].

### Example 77

### Preparation of Compound 113

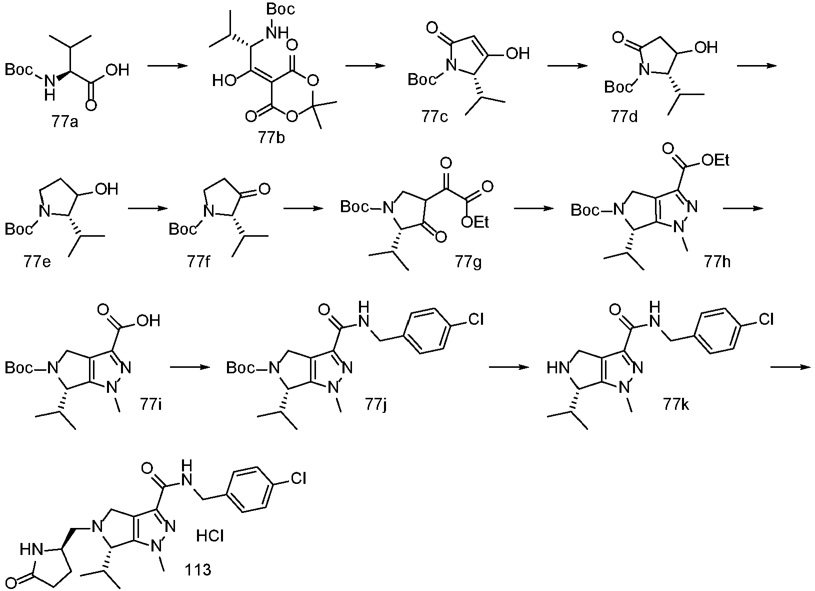

### Step A - synthesis of compound 77b

Compound 77b was made from 77a, using the method described in Example 70, step A, and substituting the appropriate reactants and/or reagents, and was used without further purification. MS: *m*/*z* 344.2 [M+H].

### Step B - synthesis of compound 77c

Compound 77c was made from 77b, using the method described in Example 70, step B, and substituting the appropriate reactants and/or reagents. The product obtained was purified using silica gel chromatography eluting with 1-20% EtOAc/petroleum ether. MS: *m*/*z* 186.1 [M-*^{t}*Bu+H].

### Step C - synthesis of compound 77d

Compound 77d was made from 77c, using the method described in Example 70, step C, and substituting the appropriate reactants and/or reagents. The product obtained was purified using silica gel chromatography eluting with 0-10% MeOH/CH₂Cl₂. MS: *m*/*z* 188.1 [M-*^{t}*Bu+H].

### Step D - synthesis of compound 77e

Compound 77e was made from 77d, using the method described in Example 70, step D, and substituting the appropriate reactants and/or reagents. The product obtained was purified using silica gel chromatography eluting with 0-5% MeOH/CH₂Cl₂. MS: *m*/*z* 174.2 [M-*^{t}*Bu+H].

### Step E - synthesis of compound 77f

Compound 77f was made from 77e, using the method described in Example 70, step E, and substituting the appropriate reactants and/or reagents. The product obtained was purified using silica gel chromatography eluting with 0-5% MeOH/CH₂Cl₂. MS: *m*/*z* 172.2 [M-*^{t}*Bu+H].

### Step F - synthesis of compound 77g

Compound 77g was made from 77f, using the method described in Example 37, step A, and substituting the appropriate reactants and/or reagents. The product obtained was purified using silica gel chromatography eluting with 0-5% MeOH/CH₂Cl₂. MS: *m*/*z* 272.1 [M-*^{t}*Bu+H].

### Step G - synthesis of compound 77h

Compound 77h was made from 77g, using the method described in Example 37, step B, and substituting the appropriate reactants and/or reagents. The resulting residue was purified using silica gel chromatography eluting with 0-100% EtOAc/petroleum ether. MS: *m*/*z* 338.2 [M+H].

### Step H - synthesis of compound 77i

Compound 77i was made from 77h, using the method described in Example 70, step H, and substituting the appropriate reactants and/or reagents, and was used without further purification. MS: *m*/*z* 310.2 [M+H].

### Step I - synthesis of compound 77j

Compound 77j was made from 77i, using the method described in Example 2, step A, and substituting the appropriate reactants and/or reagents. The resulting residue was purified using silica gel chromatography eluting with 0-10% MeOH/CH₂Cl₂. MS: *m*/*z* 433.2 [M+H].

### Step J - synthesis of compound 77k

Compound 77k was made from 77j, using the method described in Example 2, step C, and substituting the appropriate reactants and/or reagents, and was used without further purification. MS: *m*/*z* 333.2 [M+H].

### Step K - synthesis of compound 113

To a mixture of 77k (120 mg, 0.361 mmol) in DMF (5 mL) was added potassium carbonate (100 mg, 0.721 mmol), and Int-2 (107 mg, 0.397 mmol), and the resulting reaction was allowed to stir for 16 hours at 80 °C. The reaction was quenched with water (30 mL), extracted with ethyl acetate, and the organic extract was washed with brine, dried over Na₂SO₄, and concentrated in vacuo. The resulting residue was purified using silica gel chromatography eluting with 0-10% MeOH/CH₂Cl₂. HCl (1 M, 3 drops per fraction) was added to each target fraction and the fractions were concentrated in vacuo to provide 113 as its HCl salt. MS: *m*/*z* 430.2 [M+H]. ¹H NMR (400 MHz, methanol-*d*₄, ppm): δ 7.34-7.29 (m 4H), 4.99-4.86 (m, 2H), 4.60-4.50 (m, 1H), 4.49 (s, 2H), 4.24-4.40 (m, 1H), 3.95 (s, 3H), 3.64-3.59 (m, 2H), 2.57-2.56 (m, 1H), 2.45-2.36 (m, 3H), 1.91-1.90 (m, 1H), 1.29-1.15 (m, 6H).

The following compound of the present invention was made using methodology described in Example 77 above, using the enantiomer of 77a ((*tert*-butoxycarbonyl)-*D*-valine) as starting material, and substituting Int-6 (final step), and the appropriate reactants and/or reagents:

| Compound | Structure | MS [M+H] |
|---|---|---|
| 114 | 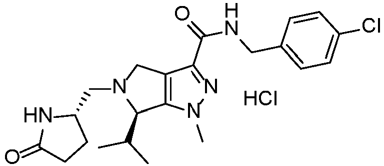 | 430.3 |

### Example 78

### Preparation of Compounds 115, 116, 117, and 118

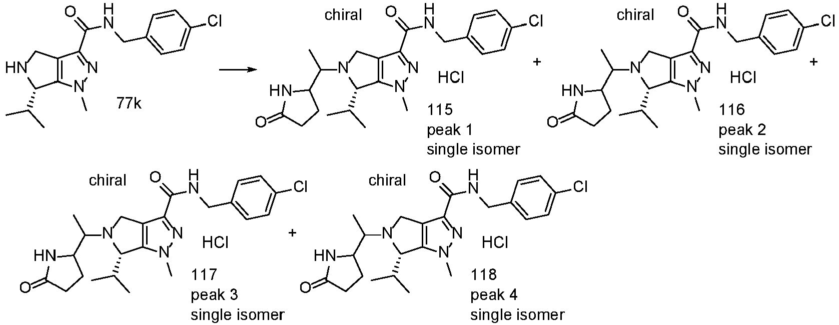

### Step A - synthesis of compounds 115, 116, 117, and 118

A mixture of Int-1 (220 mg, 1.50 mmol), 77k (50.0 mg, 0.150 mmol), acetic acid (54 mg, 0.90 mmol), and sodium cyanotrihydroborate (47 mg, 0.75 mmol) in 1,2-dichloroethane (1 mL) was allowed to stir for 1 hour at 120 °C in a sealed tube. The reaction mixture was diluted with methanol (20 mL), and concentrated in vacuo to provide 115, 116, 117, and **118** as a mixture of diastereomers. MS: *m*/*z* 444.4 [M+H].

The diastereomers were resolved using preparative HPLC, eluting with a gradient of acetonitrile: water (0.1% formic acid) - 3:7 to 6:4. HCl (0.5 M, few drops) was added to each target fraction and the fractions were concentrated in vacuo to provide 115 (first peak), 116 (second peak), 117 (third peak), and 118 (fourth peak).

For compound 115: MS: *m*/*z* 444.2 [M+H]. ¹H NMR (400 MHz, methanol-*d₄*, ppm): δ 7.34 (s, 4H), 5.15-5.19 (m, 1H), 4.91-4.96 (m, 1H), 4.58-4.62 (m, 1H), 4.52 (s, 2H), 4.22-4.26 (m, 1H), 4.00 (s, 3H), 3.82-3.86 (m, 1H), 2.33-2.55 (m, 4H), 1.93-2.07 (m, 1H), 1.10-1.41 (m, 9H).

For compound 116: MS: *m*/*z* 444.2 [M+H]. ¹H NMR (400 MHz, methanol-*d₄*, ppm): δ 7.32-7.39 (m, 4H), 5.23 (s, 1H), 4.93-4.99 (m, 1H), 4.93 (s, 1H), 4.52 (s, 3H), 4.20-4.24 (m, 1H), 4.00 (s, 3H), 3.78-3.83 (m, 1H), 2.05-2.51 (m, 5H), 1.18-1.28 (m, 6H), 1.05-1.12 (s, 3H).

For compound 117: MS: *m*/*z* 444.3 [M+H]. ¹H NMR (400 MHz, methanol-*d₄*, ppm): δ 7.32-7.37 (m, 4H), 5.11 (s, 1H), 4.82-4.84 (m, 1H), 4.52-4.59 (m, 3H), 4.02 (s, 3H), 3.89 (s, 1H), 2.34-2.55 (m, 4H), 1.87-1.93 (m, 1H), 1.10-1.32 (m, 9H).

For compound 118: MS: *m*/*z* 444.3 [M+H]. ¹H NMR (400 MHz, methanol-*d₄*, ppm): δ 7.32-7.37 (m, 4H), 5.12 (s, 1H), 4.87-4.91 (m, 1H), 4.67-4.70 (m, 1H), 4.52 (s, 2H), 4.12 (s, 1H), 3.98 (s, 3H), 3.81 (s, 1H), 2.05-2.59 (m, 4H), 1.18-1.34 (m, 9H).

### Example 79

### Preparation of Compounds 119 and 120

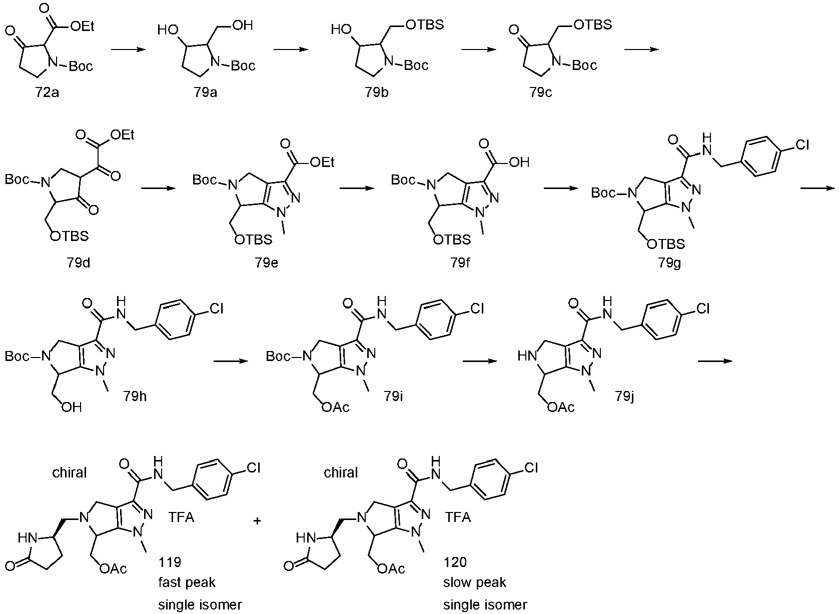

### Step A - synthesis of compound 79a

To a mixture of 72a (8.00 g, 31.1 mmol) in methanol (100 mL) at 0 °C, was added lithium borohydride (2.71 g, 124 mmol). The reaction was allowed to stir for 4 hours at room temperature, then was quenched with saturated, aqueous ammonium chloride (200 mL). The resulting solution was extracted with dichloromethane, and the organic extract was washed with brine, dried over MgSO₄, filtered and concentrated in vacuo. The resulting residue was purified using silica gel chromatography eluting with 10% MeOH/CH₂Cl₂ to provide 79a. MS: *m*/*z* 218.2 [M+H].

### Step B - synthesis of compound 79b

To a mixture of 79a (2.40 g, 11.1 mmol), and triethylamine (1.17 g, 11.6 mmol) in dichloromethane (30 mL) at 0 °C, was added *tert*-butyldimethylsilyl chloride (1.75 g, 11.6 mmol), and 4-dimethylaminopyridine (0.013 g, 0.11 mmol). The reaction was allowed to stir for 2 hours at room temperature then quenched with saturated, aqueous sodium bicarbonate (100 mL), extracted with dichloromethane, washed with brine, dried over MgSO₄, and concentrated in vacuo. The resulting residue was purified using silica gel chromatography eluting with 66% diethyl ether/petroleum ether to provide 79b. MS: *m*/*z* 332.2 [M+H].

### Step C - synthesis of compound 79c

Compound 79c was made using the method described in Example 53, step C, and substituting the appropriate reactants and/or reagents. The resulting residue was purified using silica gel chromatography eluting with 66% diethyl ether/petroleum ether to provide 79c. MS: *m*/*z* 330.1 [M+H].

### Step D - synthesis of compound 79d

Compound 79d was made using the method described in Example 37, step A, and substituting the appropriate reactants and/or reagents. The resulting residue was purified using silica gel chromatography eluting with 10% MeOH/CH₂Cl₂ to provide 79d. MS: *m*/*z* 430.3 [M+H].

### Step E - synthesis of compound 79e

Compound 79e was made using the method described in Example 37, step B, and substituting the appropriate reactants and/or reagents. The resulting residue was purified using silica gel chromatography eluting with 50% EtOAc/petroleum ether to provide 79e. MS: *m*/*z* 440.2 [M+H].

### Step F - synthesis of compound 79f

Compound 79f was made using the method described in Example 72, step B, and substituting the appropriate reactants and/or reagents to provide 79f which was used without further purification. MS: *m*/*z* 412.2 [M+H].

### Step G - synthesis of compound 79g

Compound 79g was made using the method described in Example 2, step A, and substituting the appropriate reactants and/or reagents. The resulting residue was purified using silica gel chromatography eluting with 20-80% EtOAc/petroleum ether to provide 79g. MS: *m*/*z* 535.2 [M+H].

### Step H - synthesis of compound 79h

To a mixture of 79g (350 mg, 0.654 mmol) in tetrahydrofuran (3 mL), water (3 mL), and methanol (3 mL) was added lithium hydroxide monohydrate (274 mg, 6.54 mmol), and stirred for 16 hours at room temperature. The reaction mixture was concentrated in vacuo. The pH was adjusted to 7 with hydrochloric acid (1 M), and the resulting reaction was extracted with ethyl acetate, washed with brine, dried over Na₂SO₄, and concentrated in vacuo to provide 79h which was used without further purification. MS: *m*/*z* 421.2 [M+H].

### Step I - synthesis of compound 79i

To a mixture of 79h (400 mg, 0.950 mmol) in pyridine (2 mL), and acetic anhydride (2 mL) was allowed to stir for 1 hour at 10 ° C. The reaction mixture was concentrated in vacuo, and the resulting residue was diluted with saturated, aqueous sodium bicarbonate (100 mL), extracted with ethyl acetate, washed with brine, dried over Na₂SO₄, and concentrated in vacuo. The resulting residue was purified using silica gel chromatography eluting with 20-60% EtOAc/petroleum ether to provide 79i. MS: *m*/*z* 463.2 [M+H].

### Step J - synthesis of compound 79j

Compound 79j was made using the method described in Example 2, step C, and substituting the appropriate reactants and/or reagents to provide 79j which was used without further purification. MS: *m*/*z* 363.2 [M+H].

### Step K - synthesis of compounds 119 and 120

Compounds 119 and 120 were made using the method described in Example 25, step E, and substituting the appropriate reactants and/or reagents. The resulting residue was purified using silica gel chromatography eluting with 0-10% MeOH/CH₂Cl₂ to provide a mixture of diastereomers. MS: *m*/*z* 460.2 [M+H]. The diastereomers were resolved using preparative chiral HPLC (column: CHIRALPAK IG), eluting with a gradient of hexane: dichloromethane: ethanol - 1:1:2 to provide free-base 119 (fast peak), and 120 (slow peak). Approximately 5 mg of each isolated compound was mixed with acetonitrile (1 mL), and trifluoroacetic acid (0.002 M in water, 5.44 mL, 10.87 µmol), and concentrated in vacuo to provide 119 and 120.

For compound 119: MS: *m*/*z* 460.3 [M+H]. ¹H NMR (400 MHz, methanol-*d₄*, ppm): δ 7.31 (s, 4H), 4.37-4.65 (m, 6H), 3.93-4.11 (m, 2H), 3.91 (s, 3H), 3.21-3.22 (m, 2H), 2.30-2.45 (m, 3H), 2.02 (s, 3H), 1.88-1.95 (m, 1H).

For compound 120: MS: *m*/*z* 460.3 [M+H]. ¹H NMR (400 MHz, methanol-*d₄*, ppm): δ 7.31 (s, 4H), 4.35-4.72 (m, 5H), 3.92-4.06 (m, 2H), 3.91 (s, 3H), 3.18-3.24 (m, 2H), 2.31-2.44 (m, 3H), 2.04 (s, 3H), 1.82-1.87 (m, 1H).

### Example 80

### Preparation of Compounds 121 and 122

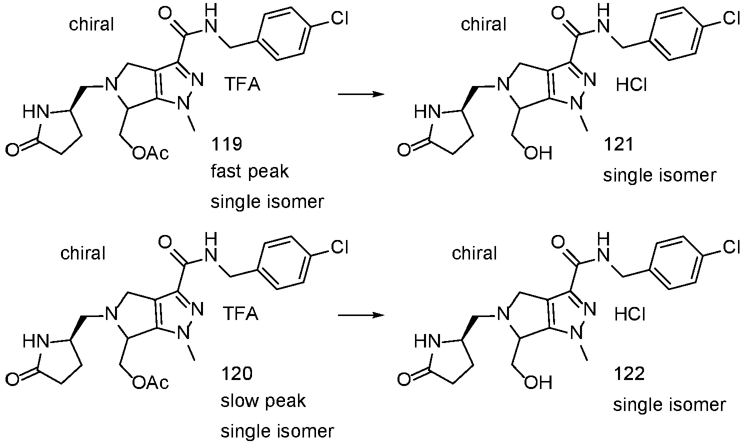

### Step A - synthesis of compound 121

To a mixture of 119 (60 mg, 0.130 mmol) in water (1 mL), and methanol (1 mL) was added lithium hydroxide monohydrate (54.7 mg, 1.31 mmol). The reaction was allowed to stir for 1 hour at room temperature. The reaction mixture was concentrated in vacuo, and the pH was adjusted to 7 with hydrochloric acid (0.1 M), extracted with ethyl acetate, and the organic extract was washed with brine, dried over Na₂SO₄, and concentrated in vacuo. The resulting residue was taken up in acetonitrile (1 mL), and treated with hydrochloric acid (0.002 M, 36 mL). The reaction mixture was concentrated in vacuo to provide 121. MS: *m*/*z* 418.2 [M+H]. ¹H NMR (400 MHz, methanol-*d₄*, ppm): δ 7.31 (s, 4H), 5.18-5.14 (m, 1H), 5.02-4.89 (m, 1H), 4.59-4.48 (m, 3H), 4.24-4.20 (m, 2H), 4.04-4.00 (m, 1H), 3.93 (s, 3H), 3.73-3.57 (m, 2H), 2.51-2.34 (m, 3H), 1.95-1.93 (m, 1H).

### Step B - synthesis of compound 122

Compound 122 was made from **120** using the method described in Step A, immediately above. MS: *m*/*z* 418.3 and 420.2 [M+H for ³⁵Cl and ³⁷Cl]. ¹H NMR (400 MHz, methanol-*d₄*, ppm): δ 7.31 (s, 4H), 5.12 (s, 1H), ~4.89-4.8 (m, 1H, partially obscured), 4.57-4.49 (m, 3H), 4.28-4.10 (m, 3H), 3.94 (s, 3H), 3.71-3.63 (m, 2H), 2.52-2.35 (m, 3H), 1.98-1.88 (m, 1H).

### Example 81

### Preparation of Compounds 123 and 124

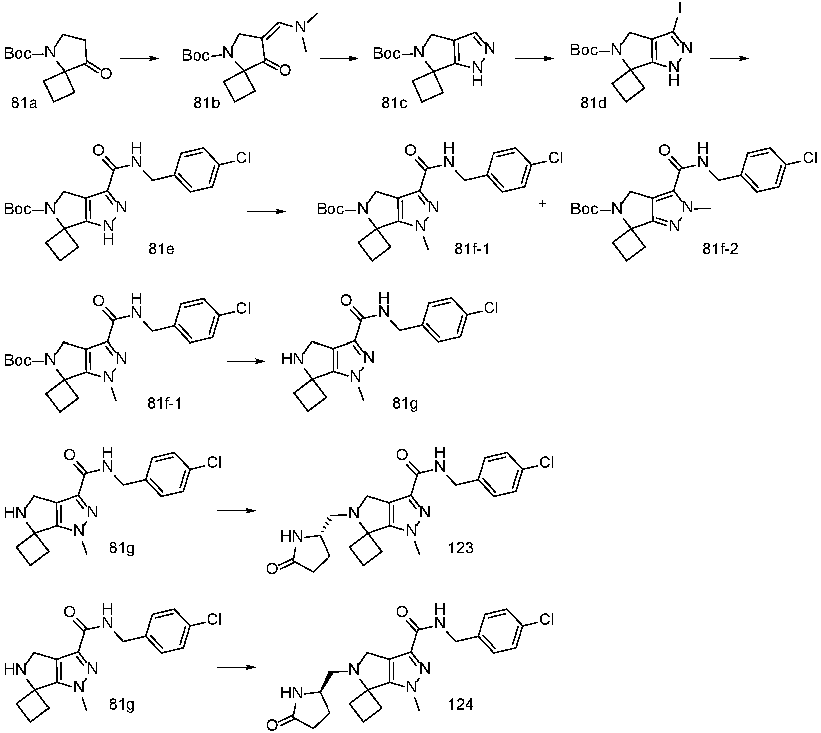

### Step A - synthesis of compound 81b

A mixture of 81a (2.00 g, 8.88 mmol), and 1,1-dimethoxy-N,N-dimethylmethanamine (20.0 mL, 8.88 mmol) was sealed and allowed to stir at 100 °C for 2 hours. The reaction mixture was cooled to room temperature, then concentrated in vacuo to provide 81b as a solid, which was used without further purification. MS: *m*/*z* 281.2 [M+H].

### Step B - synthesis of compound 81c

Compound 81c was made from 81b, using the method described in Example 71, step B, and substituting the appropriate reactants and/or reagents. The product obtained was purified using silica gel chromatography eluting with 0-100% (3:1 EtOAc:EtOH)/hexanes to provide 81c as a foam. MS: *m*/*z* 224.1 = [M+H].

### Step C - synthesis of compound 81d

Compound 81d was made from 81c, using the method described in Example 71, step C, and substituting the appropriate reactants and/or reagents. The product obtained was purified using silica gel chromatography eluting with 0-60% EtOAc/hexanes to provide 81d as an oil. MS: *m*/*z* 376.0 = [M+H].

### Step D - synthesis of compound 81e

Compound 81e was made from 81d, using the method described in Example 71, step D, substituting the appropriate reactants and/or reagents, and heating at 120 °C under 140 psi of carbon monoxide for 16 hours. The product obtained was purified using silica gel chromatography eluting with 0-100% EtOAc/hexanes to provide 81e as a solid. MS: *m*/*z* 417.1 and 419.1 = [M+H for ³⁵Cl and ³⁷Cl].

### Step E - synthesis of compounds 81f-1 and 81f-2

Compounds 81f-1 and 81f-2 were made from 81e, using the method described in Example 2, step B, and substituting the appropriate reactants and/or reagents. The mixture of products obtained was purified using silica gel chromatography eluting with 0-50% (3:1 EtOAc:EtOH)/hexanes and then by preparative HPLC (reverse-phase C-18), eluting with acetonitrile/water + 0.1% TFA 5-95% to provide 81f-1 as a solid. MS: *m*/*z* 431.1 and 433.2 = [M+H for ³⁵Cl and ³⁷Cl].

### Step F - synthesis of compound 81g

Compound 81g was made from 81f-1 using the method described in Example 2, step C, and substituting the appropriate reactants and/or reagents to provide 81g as a solid which was used without further purification. MS: *m*/*z* 331.1 and 333.3 = [M+H for ³⁵Cl and ³⁷Cl].

### Step G - synthesis of compound 123

To a mixture of 81g (80 mg, 0.24 mmol), and (S)-5-(bromomethyl)pyrrolidin-2-one (86 mg, 0.48 mmol) in acetonitrile (967 µL) was added potassium carbonate (100 mg, 0.725 mmol), and the reaction was allowed to stir at 110 °C for 5 hours. The reaction mixture was filtered and purified using preparative HPLC (reverse-phase C-18), eluting with acetonitrile/water + 0.1% TFA 5-95%. Fractions containing the product were combined, basified with saturated, aqueous NaHCO₃, and extracted with ethyl acetate. The organic extract was washed with brine, dried over Na₂SO₄, and concentrated in vacuo to provide 123. MS: *m*/*z* 428.2 = [M+H]. ¹H NMR (600 MHz, methanol-*d₄*, ppm): δ 7.29 (s, 4H), 4.46 (s, 2H), 4.03 (s, 3H), 3.96 (d, *J =* 12.0 Hz, 1H), 3.89-3.91 (m, 1H), 3.71 (d, *J =* 12.0 Hz, 1H), 2.95-2.99 (m, 1H), 2.87-2.90 (m, 1H), 2.63-2.69 (m, 1H), 2.54-2.60 (m, 1H), 2.22-2.44 (m, 5H), 2.01-2.06 (m, 1H), 1.85-1.97 (m, 2H).

### Step H - synthesis of compound 124

Compound 124 was made from 81g, using the method described in Step G above, and substituting (*R*)-5-(bromomethyl) pyrrolidin-2-one for (*S*)-5-(bromomethyl) pyrrolidine-2-one. MS: *m*/*z* 428.2 and 430.2 = [M+H for ³⁵Cl and ³⁷Cl]. ¹H NMR (600 MHz, methanol-*d₄*, ppm): δ 7.28 (s, 4H), 4.46 (s, 2H), 4.03 (s, 3H), 3.97 (d, *J =* 12.6 Hz, 1H), 3.88-3.92 (m, 1H), 3.71 (d, *J =* 11.4 Hz, 1H), 2.96-2.99 (m, 1H), 2.87-2.90 (m, 1H), 2.63-2.69 (m, 1H), 2.54-2.60 (m, 1H), 2.20-2.44 (m, 5H), 2.01-2.07 (m, 1H), 1.86-1.94 (m, 2H).

### Example 82

### Preparation of Compounds 125 and 126

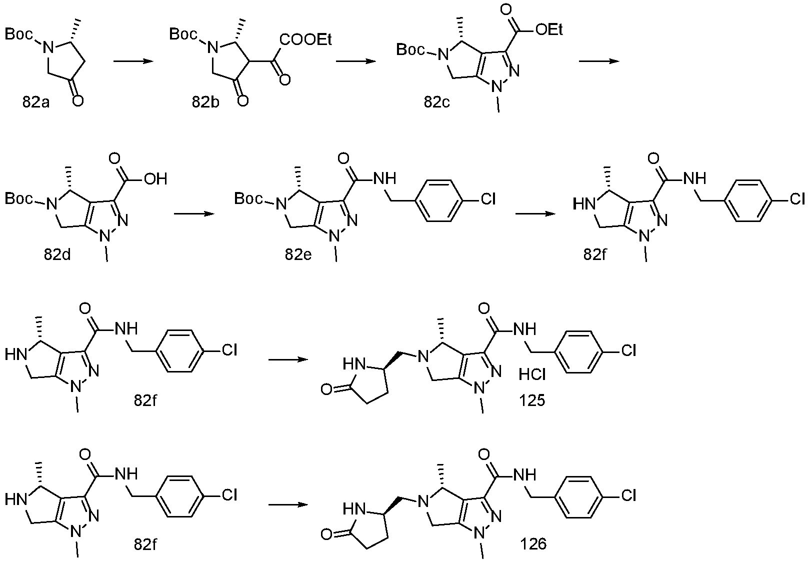

### Step A - synthesis of compound 82b

Compound 82b was made from 82a, using the method described in Example 37, step A, and substituting the appropriate reactants and/or reagents, and was used without further purification. MS: *m*/*z* 299.9 [M+H].

### Step B - synthesis of compound 82c

Compound 82c was made using the method described in Example 37, step B, and substituting the appropriate reactants and/or reagents. The product obtained was purified using silica gel chromatography eluting with 20-50% EtOAc/petroleum ether. MS: *m*/*z* 310.2 [M+H].

### Step C - synthesis of compound 82d

Compound 82d was made from 82c, using the method described in Example 72, step B, and substituting the appropriate reactants and/or reagents, and was used without further purification. MS: *m*/*z* 282.2 [M+H].

### Step D - synthesis of compound 82e

Compound 82e was made using the method described in Example 2, step A, and substituting the appropriate reactants and/or reagents. The product obtained was purified using silica gel chromatography eluting with 20-60% EtOAc/petroleum ether. MS: *m*/*z* 405.2 [M+H].

### Step E - synthesis of compound 82f

Compound 82f was made from 82e, using the method described in Example 6, step B, and substituting the appropriate reactants and/or reagents. The product obtained was purified using silica gel chromatography eluting with 0-10% MeOH/CH₂Cl₂. MS: *m*/*z* 305.1 [M+H].

### Step F - synthesis of compound 125

Compound 125 was made from 82f, using the method described in Example 81, step G, utilizing Int-2, and substituting the appropriate reactants and/or reagents. The product obtained was purified using silica gel chromatography eluting with 0-5% MeOH/CH₂Cl₂, and the purified product was taken up in dichloromethane (0.4 mL), and treated with HCl (concentrated in vacuo, 7.4 µL), and then concentrated in vacuo to provide 125 as its HCl salt. MS: *m*/*z* 402.2 [M+H]. ¹H NMR (600 MHz, methanol-*d*₄, ppm): δ 7.29 (s, 4H), 4.93 (br s, 1H), 4.59 (br s, 1H), 4.42-4.50 (m, 2H), 4.13 (br s, 1H), 3.88 (s, 3H), 3.57-3.66 (m, 2H), 2.42-2.48 (m, 2H), 2.35-2.39 (m, 1H), 1.88 (br s, 3H).

### Step G - synthesis of compound 126

Compound 126 was made from 82f using the method described in Step G above and utilizing Int-6 in place of Int-2. MS: *m*/*z* 402.2 [M+H]. ¹H NMR (400 MHz, methanol-*d*₄, ppm): δ 7.32 (s, 4H), 4.51 (d, *J =* 15.2 Hz, 1H), 4.43 (d, *J =* 15.2 Hz, 1H), 4.18 (dd, *J =* 12.4, 2.4 Hz, 1H), 3.99-4.01 (m, 1H), 3.77-3.80 (m, 4H), 3.64 (dd, *J =* 12.4, 2.0 Hz, 1H), 2.82-2.84 (m, 2H), 2.25-2.40 (m, 3H), 1.85-1.96 (m, 1H), 1.42 (d, *J =* 6.0 Hz, 3H).

### Example 83

### Preparation of Int-30

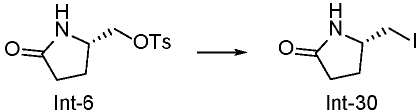

### Step A - synthesis of Int-30

To a mixture of Int-6 (5.00 g, 18.6 mmol) in acetone (80 mL) was added sodium iodide (14 g, 93 mmol). The reaction mixture was allowed to stir for 16 hours at 55 °C, then filtered, and the filtrate was concentrated in vacuo. The resulting residue was diluted with ethyl acetate (200 mL), and the organic extract was washed with saturated, aqueous sodium thiosulfate (100 mL), then dried over Na₂SO₄ filtered, and concentrated in vacuo. The resulting residue was purified using silica gel chromatography eluting with 30-80% EtOAc/petroleum ether to provide Int-30. MS: *m*/*z* 225.8 [M+H].

### Example 84

### Preparation of Compounds 127 and 128

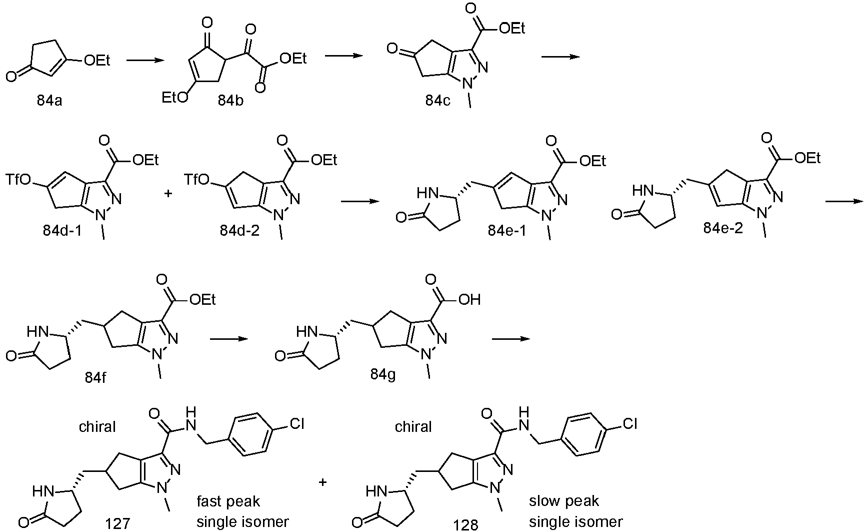

### Step A - synthesis of compound 84b

Compound 84b was made from 84a, using the method described in Example 37, step A, and substituting the appropriate reactants and/or reagents. The product obtained was purified using silica gel chromatography eluting with 0-100% EtOAc/petroleum. MS: *m*/*z* 227.0 [M+H].

### Step B - synthesis of compound 84c

Compound 84c was made from 84b, using the method described in Example 37, step B, and substituting the appropriate reactants and/or reagents. The product obtained was purified using silica gel chromatography eluting with 0-60% EtOAc/petroleum ether. MS: *m*/*z* 209.2 [M+H].

### Step C - synthesis of compounds 84d-1 and 84d-2

To a -78 ° C mixture of 84c (3.00 g, 14.4 mmol), and trifluoro-*N*-phenyl-*N-*((trifluoromethyl)sulfonyl)methanesulfonamide (6.70 g, 18.7 mmol) in tetrahydrofuran (125 mL) was added lithium bis(trimethylsilyl)amide (1 M in tetrahydrofuran, 22 mL) dropwise at -78 ° C under nitrogen. The reaction was warmed to 0 °C and allowed to stir at this temperature for 3 hours. The reaction was quenched with saturated, aqueous sodium bicarbonate (50 mL), and extracted with ethyl acetate. The organic extract was washed with brine, dried over Na₂SO₄, filtered and concentrated in vacuo. The resulting residue was purified using silica gel chromatography eluting with 0-40% EtOAc/petroleum ether to provide a mixture of 84d-1 and 84d-2. MS: *m*/*z* 341.0 [M+H] for the mixture.

### Step D - synthesis of compounds 84e-1 and 84e-2

To a mixture of zinc (0.990 g, 15.1 mmol) in dry DMF (3 mL) was added 1,2-dibromoethane (129 µL, 1.5 mmol) under nitrogen. The reaction was allowed to stir for 10 minutes at 60 °C, then chlorotrimethylsilane (150 µL, 1.20 mmol) was added, and the resulting reaction was allowed to stir for 30 minutes at room temperature. A solution of Int-30 (1.70 g, 7.53 mmol) in DMF (1 mL) was then added, and the reaction was allowed to stir for 3 hours at 0 °C. In a separate reaction flask, a mixture of tris(dibenzylideneacetone)dipalladium(0) (137 mg, 0.150 mmol), dicyclohexyl((2-[(2,6-dimethoxyphenyl)methyl]phenyl)methyl)phosphane (131 mg, 0.300 mmol), and 84d-1 and 84d-2 (0.85 g, 2.5 mmol) in DMF (5 mL) was prepared and cooled to 0 °C under nitrogen atmosphere, and to this was added the prepared zinc reagent at 0 °C under nitrogen. The resulting reaction was allowed to stir for 1 hour at 0 °C and then stirred for 16 hours at room temperature. The reaction was quenched with water (50 mL), extracted with ethyl acetate, and the organic extract was washed with brine, dried over Na₂SO₄, and concentrated in vacuo. The resulting residue was purified using silica gel chromatography eluting with 0-10% MeOH/CH₂Cl₂ to provide a mixture of 84e-1 and 84e-2. MS: *m*/*z* 290.1 [M+H] for both.

### Step E - synthesis of compound 84f

A mixture of 84e-1 and 84e-2 (250 mg, 0.864 mmol), and palladium on carbon (10 wt%, 92 mg, 0.086 mmol) in methanol (10 mL) was allowed to stir for 6 hours at room temperature under hydrogen (2 atm). The reaction mixture was filtered, and the filtrate was concentrated in vacuo to provide 84f, which was used without further purification. MS: *m*/*z* 292.2 [M+H].

### Step F - synthesis of compound 84g

Compound 84g was made from 84f, using the method described in Example 72, step B, and substituting the appropriate reactants and/or reagents, and was used without further purification. MS: *m*/*z* 264.1 [M+H].

### Step G - synthesis of compounds 127 and 128

Compounds 127 and 128 were made from 84g, using the method described in Example 2, step A, and substituting the appropriate reactants and/or reagents. The product obtained was purified using silica gel chromatography eluting with 0-10% MeOH/CH₂Cl₂ to provide a mixture of diastereomers. MS: *m*/*z* 387.2 [M+H].

The diastereomers were resolved using preparative chiral HPLC (column: CHIRALPAK IC), eluting with a gradient of methanol (0.1% formic acid):dichloromethane - 7:3 to provide **127** (fast peak), and 128 (slow peak).

For compound 127: MS: *m*/*z* 387.2 [M+H]. ¹H NMR (400 MHz, methanol-*d*₄, ppm): δ 8.38-8.41 (m, 1H), 7.31 (s, 4H), 4.49 (s, 2H), 3.79-3.73 (m, 4H), 3.13-2.91 (m, 3H), 2.49-2.40 (m, 2H), 2.38-2.28 (m, 3H), 1.90-1.70 (m, 3H).

For compound 128: MS: *m*/*z* 387.2 [M+H]. ¹H NMR (400 MHz, methanol-*d*₄, ppm): δ 7.31 (s, 4H), 4.49 (s, 2H), 3.83-3.76 (m, 4H), 3.10-2.90 (m, 3H), 2.47-2.41 (m, 2H), 2.36-2.29 (m, 3H), 1.89-1.69 (m, 3H).

### Example 85

### Preparation of Int-31

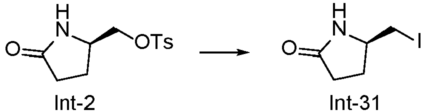

Int-31 was made from Int-2, using the method described in Example 83, step A, and substituting the appropriate reactants and/or reagents. The product obtained was purified using silica gel chromatography eluting with 3-5% MeOH/CH₂Cl₂.

### Example 86

### Preparation of Compounds 129 and 130

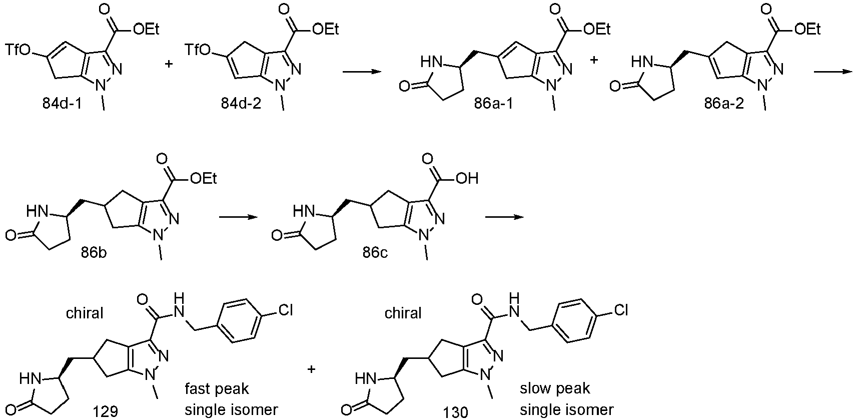

### Step A - synthesis of compounds 86a-1 and 86a-2

Compounds 86a-1 and 86a-2 were made using the method described in Example 84, step D, utilizing Int-31 and substituting the appropriate reactants and/or reagents. The resulting residue was purified using silica gel chromatography eluting with 0-10% MeOH/CH₂Cl₂ to provide a mixture of 86a-1 and 86a-2. MS: *m*/*z* 290.1 [M+H].

### Step B - synthesis of compound 86b

Compound 86b was made using the method described in Example 84, step E, and substituting the appropriate reactants and/or reagents to provide 86b which was used without further purification. MS: *m*/*z* 292.2 [M+H].

### Step C - synthesis of compound 86c

Compound 86c was made using the method described in Example 72, step B, and substituting the appropriate reactants and/or reagents to provide 86b which was used without further purification. MS: *m*/*z* 264.0 [M+H].

### Step D - synthesis of compounds 129 and 130

Compounds 129 and 130 were made using the method described in Example 2, step A, and substituting the appropriate reactants and/or reagents. The resulting residue was purified using silica gel chromatography eluting with 0-10% MeOH/CH₂Cl₂ to provide a mixture of diastereomers. MS: *m*/*z* 387.2 [M+H].

The diastereomers were resolved using chiral-Prep- HPLC (column: CHIRALPAK IC), eluting with a gradient of methanol (0.1% formic acid):dichloromethane - 7:3 to provide 129 (fast peak), and 130 (slow peak).

For compound 129: MS: *m*/*z* 387.2 [M+H]. ¹H NMR (300 MHz, methanol-*d*₄, ppm): δ 7.31 (s, 4H), 4.49 (s, 2H), 3.81-3.74 (m, 4H), 3.12-2.90 (m, 3H), 2.50-2.29 (m, 5H), 1.91-1.80 (m, 3H).

For compound 130: MS: *m*/*z* 387.2 [M+H]. ¹H NMR (300 MHz, methanol-*d*₄, ppm): δ 7.31 (s, 4H), 4.49 (s, 2H), 3.77-3.74 (m, 4H), 3.11-2.90 (m, 3H), 2.48-2.28 (m, 5H), 1.92-1.70 (m, 3H).

### Example 87

### Preparation of Compound 131

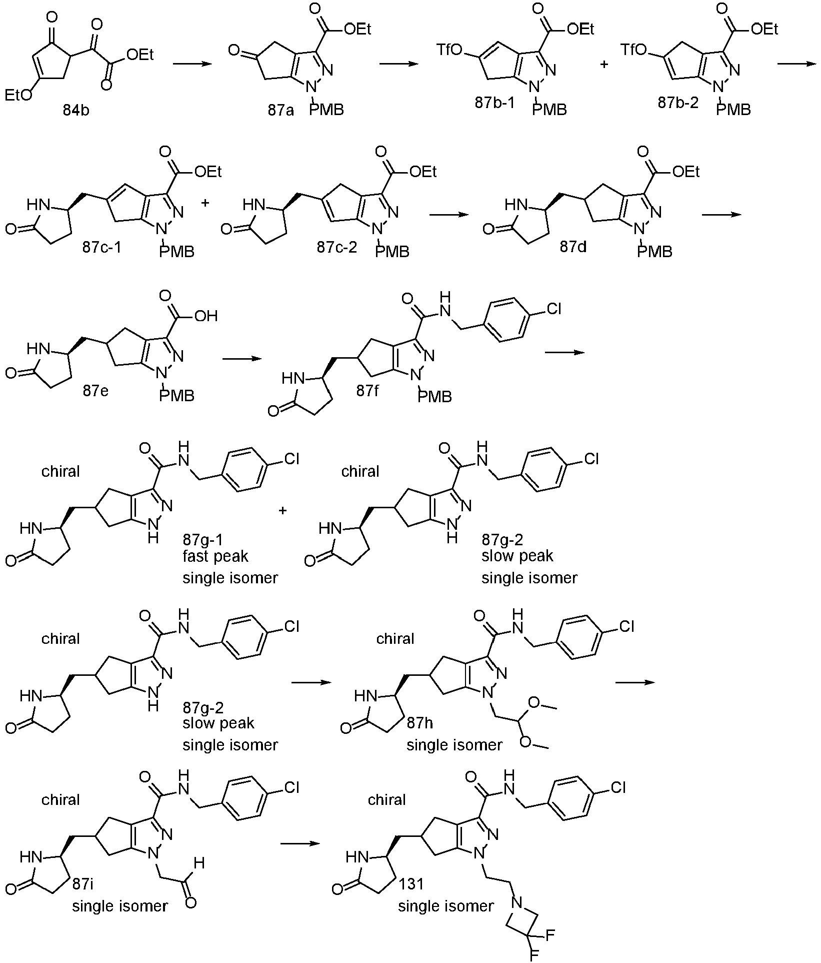

### Step A - synthesis of compound 87a

To a mixture of 84b (40.0 g, 177 mmol) in EtOH (587 mL) was added (4-methoxybenzyl)hydrazine (32.3 g, 212 mmol) over 5 minutes at room temperature, then the resulting solution was allowed to stir for an additional 5 minutes. Acetic acid (80 mL) was added dropwise, and the resulting reaction was allowed to stir for 1 hour at 70 °C. The reaction mixture was cooled to room temperature, and hydrochloric acid (6 M, 300 mL) was added dropwise. The reaction mixture was allowed to stir for 1 hour at room temperature, then concentrated in vacuo, and the resulting residue was diluted with saturated, aqueous sodium carbonate (500 mL), extracted with ethyl acetate, and the organic extract was washed with brine, dried over Na₂SO₄, and concentrated in vacuo. The resulting residue was purified using silica gel chromatography eluting with 0-60% EtOAc/petroleum ether to provide 87a as a solid. MS: *m*/*z* 315.1 = [M+H].

### Step B - synthesis of compounds 87b-1 and 87b-2

Compounds 87b-1 and 87b-2 were made from 87a, using the method described in Example 84, step C, and substituting the appropriate reactants and/or reagents. The product obtained was purified using silica gel chromatography eluting with 0-40% EtOAc/petroleum ether to provide a mixture of 87b-1 and 87b-2 as a solid. MS: *m*/*z* 447.3 [M+H] for both.

### Step C - synthesis of compounds 87c-1 and 87c-2

Compounds 87c-1 and 87c-2 were made from 87b-1 and 87b-2, using the method described in Example 84, step D, utilizing Int-31, and substituting the appropriate reactants and/or reagents. The product obtained was purified using silica gel chromatography eluting with 10% MeOH/CH₂Cl₂ to provide a mixture of 87c-1 and 87c-2 as a solid. MS: *m*/*z* 396.2 [M+H] for both.

### Step D - synthesis of compound 87d

Compound 87d was made from a mixture of 87c-1 and 87c-2, using the method described in Example 84, step E, running at 60 atm of hydrogen, and substituting the appropriate reactants and/or reagents to provide 87d as a solid. MS: *m*/*z* 398.3 [M+H].

### Step E - synthesis of compound 87e

Compound 87e was made from 87b, using the method described in Example 72, step B, and substituting the appropriate reactants and/or reagents to provide **87e** as a solid, which was used without further purification. MS: *m*/*z* 370.2 [M+H].

### Step F - synthesis of compound 87f

Compound 87f was made from 87e, using the method described in Example 2, step A, and substituting the appropriate reactants and/or reagents. The product obtained was purified using silica gel chromatography eluting with 30-60% EtOAc/petroleum ether to provide 87f as a solid. MS: *m*/*z* 493.5 [M+H].

### Step G - synthesis of compounds 87g-1 and 87g-2

Compounds 87g-1 and 87g-2 were made from 87f, using the method described in Example 6, step D, and substituting the appropriate reactants and/or reagents to provide a diasteromeric mixture of 87g-1 and 87g-2 as a solid. MS: *m*/*z* 373.2 [M+H] for both.

The diastereomers were resolved using Prep-SFC using an IC (33x100 mm) column, eluting with 50% MeOH (+0.1% diethylamine) in CO₂ at 100 bar, to provide 87g-1 (fast peak, minor), and 87g-2 (slow peak, major) as white solids.

For compound 87g-2: MS: *m*/*z* 373.1 [M+H].

### Step H - synthesis of compound 87h

A mixture of 87g-2 (180 mg, 0.483 mmol), tribasic potassium phosphate (307 mg, 1.45 mmol), and 2-bromo-1,1-dimethoxyethane (122 mg, 0.724 mmol) in DMF (3 mL) was allowed to stir at 85 °C for 12 hours. The reaction mixture was diluted with water (10 mL), extracted with ethyl acetate, and the organic extract was washed with saturated, aqueous NaHCO₃, dried over Na₂SO₄, and concentrated in vacuo. The resulting residue was purified using silica gel chromatography eluting with 0-10% MeOH/CH₂Cl₂ to provide 87h as an oil. MS: *m*/*z* 461.3 = [M+H].

### Step I - synthesis of compound 87i

Compound 87i was made from 87h, using the method described in Example 4, step D, and substituting the appropriate reactants and/or reagents to provide a solid which was used without further purification. MS: *m*/*z* 415.2 = [M+H].

### Step J - synthesis of compound 131

Compound 131 was made 87i, using the method described in Example 4, step E, and substituting the appropriate reactants and/or reagents. The product obtained was purified using preparative HPLC (reverse-phase C-18), eluting with acetonitrile/water + 0.1% ammonium bicarbonate 40-80% and the column fraction containing product were combined and lyophilized to provide 131. MS: *m*/*z* 492.2 = [M+H]. ¹H NMR (400 MHz, methanol-*d₄*, ppm): δ 7.31 (s, 4H), 4.49 (s, 2H), 4.08 (t, *J =* 6.0 Hz, 2H), 3.76-3.79 (m, 1H), 3.53 (t, *J =* 12.0 Hz, 4H), 2.95-3.31 (m, 5H), 2.43-2.52 (m, 2H), 2.31-2.37 (m, 3H), 1.84-1.91 (m, 1H), 1.76-1.80 (m, 2H).

The following compounds of the present invention were made using methodology described in Example 87, Step J above, and substituting the appropriate reactants and/or reagents:

| Compound | Structure | MS [M+H] |
|---|---|---|
| 132 | 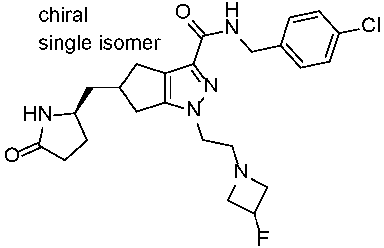 | 474.3 |
| 133 | 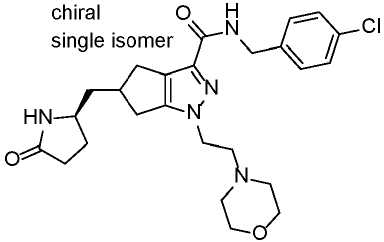 | 486.3 |

### Example 88

### Preparation of Compound 134

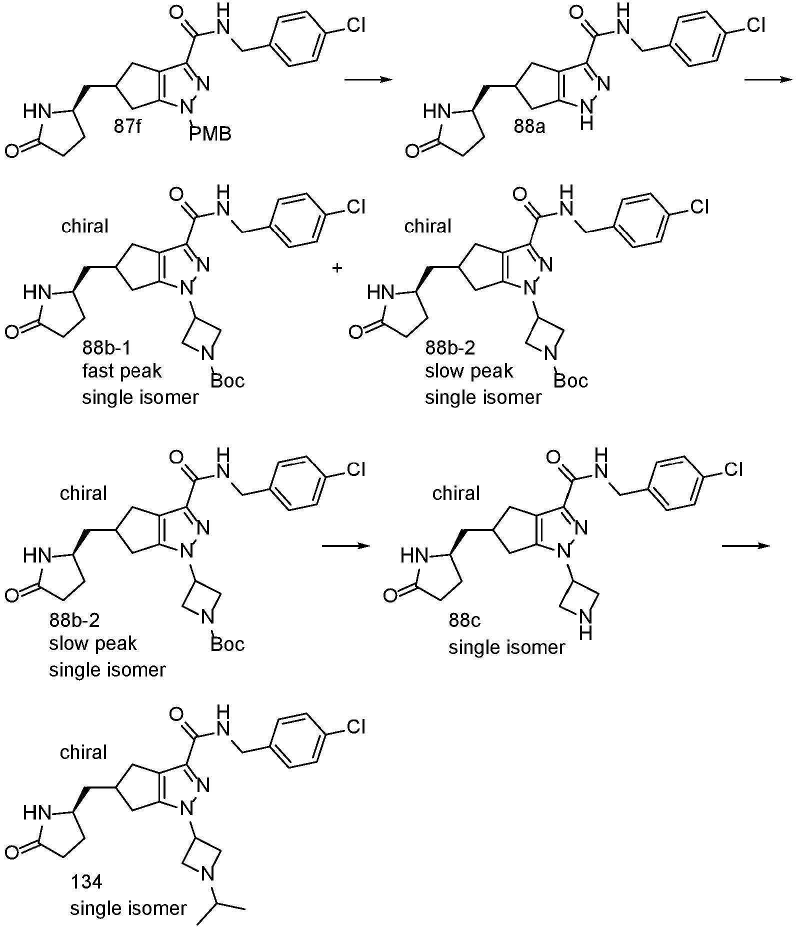

### Step A - synthesis of compound 88a

Compound 88a was made 87f, using the method described in Example 6, step D, and substituting the appropriate reactants and/or reagents to provide a solid, which was used without further purification. MS: *m*/*z* 373.2 = [M+H].

### Step B - synthesis of compounds 88b-1 and 88b-2

To a mixture of 88a (600 mg, 1.61 mmol) in DMF (10 mL) was added *tert*-butyl 3-(tosyloxy)azetidine-1-carboxylate (527 mg, 1.609 mmol), tribasic potassium phosphate (1.03 g, 4.83 mmol), and potassium iodide (267 mg, 1.609 mmol), and the resulting reaction was allowed to stir at 90 °C for 4 hours. The reaction mixture was diluted with water (50 mL), extracted with ethyl acetate, and the organic extract was washed with brine, dried over Na₂SO₄, and concentrated in vacuo. The resulting residue was purified using silica gel chromatography eluting with 0-10% MeOH/CH₂Cl₂ to provide a diastereomeric mixture of 88b-1b and 88b-2 as a solid. MS: *m*/*z* 528.5 = [M+H] for both.

The diastereomers were resolved using Prep-SFC using an OD (35x100 mm) column, eluting with 10% MeOH (+20 mmol NH₃), to provide 88b-1 (fast peak), and 88b-2 (slow peak) as oils.

### Step C - synthesis of compound 88c

Compound 88c was made from 88b-2, using the method described in Example 2, step C, and substituting the appropriate reactants and/or reagents to provide an oil, which was used without further purification. MS: *m*/*z* 428.3 = [M+H].

### Step D - synthesis of compound 134

A mixture of 88c (60 mg, 0.14 mmol), and acetone (16.3 mg, 0.280 mmol) in 1,2-dichloroethane (1 mL) was allowed to stir for 0.5 hours at 50 ° C. Sodium cyanotrihydroborate (44.1 mg, 0.701 mmol) was added, and the resulting reaction was allowed to stir for 1 hour at 50 °C. The reaction mixture was quenched with saturated, aqueous Na₂CO₃ (10 mL), extracted with ethyl acetate, and the organic extract was washed with brine, dried over Na₂SO₄, filtered, and concentrated in vacuo. The resulting residue was purified using preparative HPLC (reverse-phase C-18), eluting with acetonitrile/water + 10 mmol/L ammonium bicarbonate 45-60% to provide 134. MS: *m*/*z* 470.4 = [M+H]. ¹H NMR (300 MHz, methanol-*d₄*, ppm): δ 7.33 (s, 4H), 4.74-4.84 (m, 1H), 4.51 (s, 2H), 3.73-3.80 (m, 3H), 3.54-3.62 (m, 2H), 2.95-3.14 (m, 3H), 2.41-2.61 (m, 3H), 2.31-2.37 (m, 3H), 1.86-1.90 (m, 3H), 1.01-1.03 (m, 6H).

The following compound of the present invention was made using methodology described in Example 88, Step D above, and substituting the appropriate reactants and/or reagents:

| Compound | Structure | MS [M+H] |
|---|---|---|
| 135 | 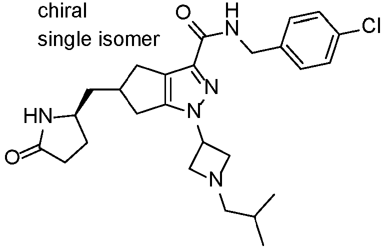 | 484.5 |

### Example 89

### Preparation of Compound 136

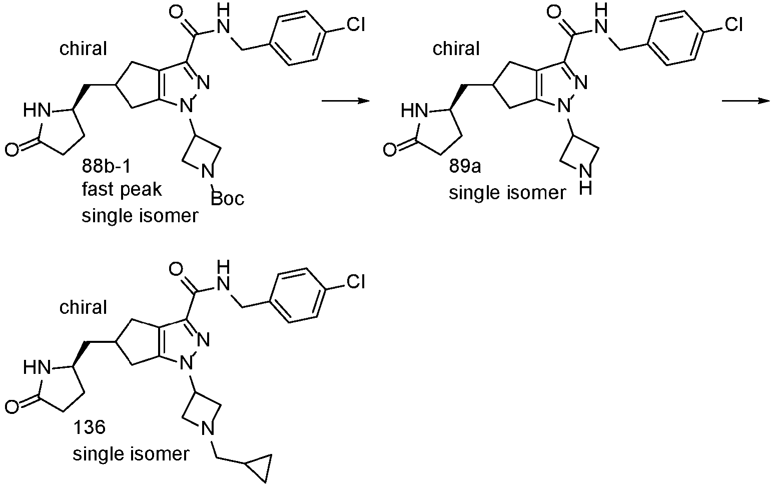

### Step A - synthesis of compound 89a

Compound 89a was made using the method described in Example 2, step C, utilizing 88b-2 and substituting the appropriate reactants and/or reagents to provide an oil, which was used without further purification. MS: *m*/*z* 428.3 = [M+H].

### Step B - synthesis of compound 136

Compound 136 was made from 89a, using the method described in Example 88, step D, and substituting the appropriate reactants and/or reagents. The product obtained was purified using silica gel chromatography eluting with 0-10% MeOH/CH₂Cl₂ to provide 136. MS: *m*/*z* 482.3 = [M+H]. ¹H NMR (400 MHz, methanol-*d₄*, ppm): δ 7.30-7.35 (m, 4H), 4.99-5.03 (m, 1H), 4.52 (s, 2H), 4.10 (s, 2H), 3.97 (s, 2H), 3.75-3.78 (m, 1H), 2.96-3.13 (m, 3H), 2.75-2.76 (m, 2H), 2.44-2.49 (m, 2H), 2.30-2.36 (m, 3H), 1.71-1.98 (m, 2-4H), 1.29 (s, 1H), 0.90-0.91 (m, 1H), 0.54-0.59 (m, 2H), 0.26-0.27 (m, 2H).

The following compound of the present invention was made using methodology described in Example 89, Step B above, and substituting the appropriate reactants and/or reagents:

| Compound | Structure | MS [M+H] |
|---|---|---|
| 137 | 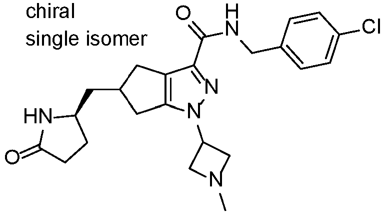 | 442.2 |

### Example 90

### Preparation of Compound 138

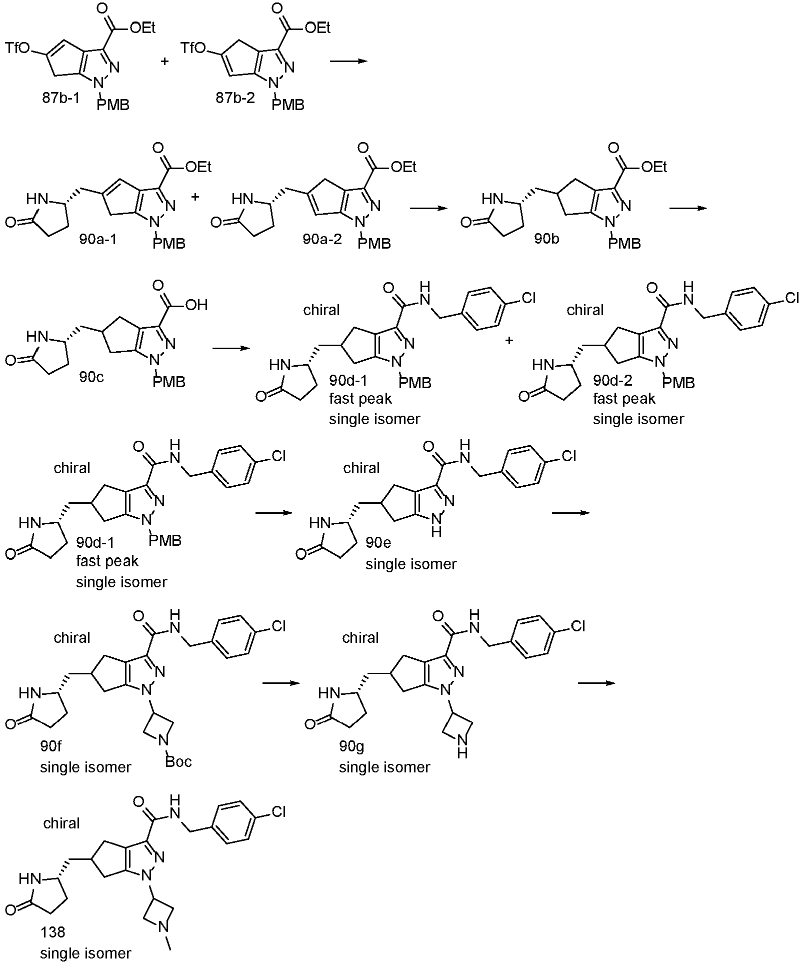

### Step A - synthesis of compounds 90a-1 and 90a-2

Compounds 90a-1 and 90a-2 were made from a mixture of 87b-1 and 87b-2, using the method described in Example 84, step D, and substituting the appropriate reactants and/or reagents. The product obtained was purified using silica gel chromatography eluting with 0-6% MeOH/CH₂Cl₂ to provide a mixture of 90a-1 and 90a2. MS: *m*/*z* 396.2 [M+H] for both.

### Step B - synthesis of compound 90b

Compound 90b was made from a mixture of 90a-1 and 90a2, using the method described in Example 84, step E, reacting at 50 °C for 72 hours, under 70 atmospheres of hydrogen and substituting the appropriate reactants and/or reagents to provide 90b as a solid, which was used without further purification. MS: *m*/*z* 398.2 [M+H].

### Step C - synthesis of compound 90c

Compound 90c was made from 90b, using the method described in Example 72, step B, and substituting the appropriate reactants and/or reagents to provide 90c as a solid, which was used without further purification. MS: *m*/*z* 370.2 [M+H].

### Step D - synthesis of compounds 90d-1 and 90d-2

Compounds 90d-1 and 90d-2 were made from 90c, using the method described in Example 2, step A, and substituting the appropriate reactants and/or reagents. The product obtained was purified using silica gel chromatography eluting with 30-60% EtOAc/petroleum ether to provide a diastereomeric mixture of 90d-1 and 90d-2 as a solid. MS: *m*/*z* 493.2 [M+H] for both.

The diastereomers were resolved using Prep-SFC using an Amylose-SA S (20x250 mm) column, eluting with 40% 1:1 IPA:DCM in CO₂ at 40 mL/min, to provide 90d-1 (fast peak), and 90d-2 (slow peak) as solids. MS: *m*/*z* 493.3 and 493.2 [M+H], respectively.

### Step E - synthesis of compound 90e

Compound 90e was made from 90d-1, using the method described in Example 6, step D, and substituting the appropriate reactants and/or reagents to provide 90e as a solid, which was used without further purification. MS: *m*/*z* 373.2 [M+H].

### Step F - synthesis of compound 90f

Compound 90f was made from 90e, using the method described in Example 88, step B, and substituting the appropriate reactants and/or reagents. The product obtained was purified using silica gel chromatography eluting with 10% MeOH/CH₂Cl₂ to provide 90f as a solid. MS: *m*/*z* 528.3 [M+H].

### Step G - synthesis of compound 90g

Compound 90g was made from 90f, using the method described in Example 2, step C, and substituting the appropriate reactants and/or reagents to provide 90g as a solid, which was used without further purification. MS: *m*/*z* 428.2 [M+H].

### Step H - synthesis of compound 138

Compound 138 was made from 90g, using the method described in Example 88, step D, and substituting the appropriate reactants and/or reagents. The product obtained was purified using preparative HPLC (reverse-phase C-18), eluting with acetonitrile/water + 10 mmol/L ammonium bicarbonate 30-50% to provide 138. MS: *m*/*z* 442.3 = [M+H]. ¹H NMR (400 MHz, methanol-*d₄*, ppm): δ 7.30-7.35 (m, 4H), 4.82-4.87 (m, 1H), 4.51 (s, 2H), 3.74-3.78 (m, 3H), 3.64-3.68 (m, 2H), 2.95-3.35 (m, 3H), 2.41-2.50 (m, 5H), 2.30-2.36 (m, 3H), 1.70-1.90 (m, 3H).

### Example 91

### Preparation of Compounds 139 and 140

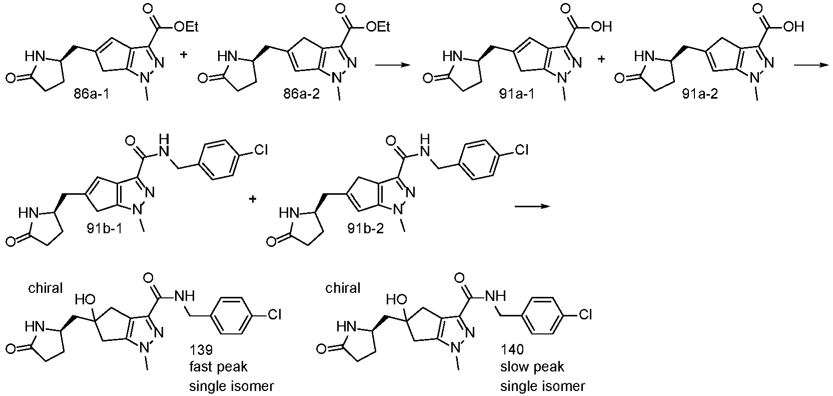

### Step A - synthesis of compounds 91a-1 and 91a-2

Compounds 91a-1 and 91a-2 were made from 86a-1 and 86a-2, using the method described in Example 72, step B, and substituting the appropriate reactants and/or reagents, and were used without further purification. MS: *m*/*z* 262 [M+H].

### Step B - synthesis of compounds 91b-1 and 91b-2

Compounds 91b-1 and 91b-2 were made from 91a-1 and 91a-2, using the method described in Example 2, step A, and substituting the appropriate reactants and/or reagents. The product obtained was purified using silica gel chromatography eluting with 0-5% MeOH/CH₂Cl₂ to provide a mixture of diastereomers. MS: *m*/*z* 385.2 [M+H] for both.

### Step C - synthesis of compounds 139 and 140

To a diasteromeric mixture of 91b-1 and 91b-2 (300 mg, 0.780 mmol) in tetrahydrofuran (20 mL) was added bis((4-oxopent-2-en-2-yl)oxy)cobalt (60 mg, 0.23 mmol) at room temperature, open to air. Phenylsilane (2.00 mL, 0.780 mmol) was added dropwise and the resulting reaction was allowed to stir for 16 hours at room temperature, then concentrated in vacuo. The resulting residue was purified using silica gel chromatography eluting with 0-8% MeOH/CH₂Cl₂ to provide a mixture of diastereomers. MS: *m*/*z* 403.2 [M+H] for both.

The diastereomers were resolved using chiral-HPLC (column: CHIRAL ART Cellulose-SBS), eluting with a gradient of hexane (8 mmol/L ammonia in methanol):ethanol - 1:1 to provide 139 (fast peak), and 140 (slow peak).

For compound 139: MS: *m*/*z* 403.2 [M+H]. ¹H NMR (400 MHz, methanol-*d*₄, ppm): δ 7.33 (s, 4H), 4.51 (s, 2H), 4.01-3.97 (m, 1H), 3.81 (s, 3H), 3.02-2.79 (m, 4H), 2.39-2.27 (m, 3H), 1.99 (d, *J =* 6.0 Hz, 2H), 1.87-1.77 (m, 1H).

For compound 140: MS: *m*/*z* 403.2 [M+H]. ¹H NMR (400 MHz, methanol-*d*₄, ppm): δ 7.34 (s, 4H), 4.51 (s, 2H), 4.00 (br s, 1H), 3.81 (s, 3H), 3.06-2.80 (m, 4H), 2.38-2.27 (m, 3H), 1.99 (s, 2H), 1.86-1.82 (m, 1H).

### Example 93

### HCMV and VZV polymerase assays

Human cytomegalovirus and varicella zoster virus DNA polymerases were expressed via baculovirus vector in SF21 cells and purified. Heterodimeric nucleic acid substrate used in the herpesvirus polymerase reactions was generated by annealing a 59-mer template to a 17-mer digoxigenin-labeled primer. Polymerase (HCMV final concentration of 0.2nM; VZV final concentration of 0.4nM) was combined with an inhibitor compound or DMSO in assay buffer (10 mM HEPES, pH 7.5, 25 mM KCl, 25 mM NaCl, 5 mM MgCl₂, 5% glycerol, 0.67 mg/ml bovine serum albuminutes, and 1mM tris(2-carboxyethyl)phosphine), and this mixture was preincubated for 30 minutes at room temperature in 384-well microtiter plates. The polymerization reaction was initiated by the addition of template/primer substrate (final concentration: 1.6 nM), and dNTPs (final concentration: 24 nM dCTP, 24 nMdGTP, 16nM dATP, 16 nM dTTP, and 0.8nM biotin-dUTP). After 60 minutes incubation at 37°C, reactions were terminated with quench buffer (25 mM HEPES pH 7.5, 100 mM NaCl, 0.25% Tween-20, 12 mM EDTA, and 1mg/ml bovine serum albumin). Incorporation of biotinylated UTP was detected with 2.5-5ug/ml anti-DIG AlphaLISA acceptor beads and 5-10 ug/ml streptavidin AlphaLISA donor beads (Perkin Elmer). Compound effects were normalized to the window defined by the controls (DMSO only and pre-quenched wells) and were fit using a 4-parameter algorithm to report an IC₅₀.

### Example 94

### Viral qPCR assays

### Viral qPCR assays (V0/V1)

MRC5 cells, Vero cells, and MeWo cells were obtained from ATCC and were maintained at 37°C/5% CO2/90% relative humidity in Minimal Essential Medium with 10% fetal bovine serum, 2.0 nM L-glutamine, 100 units/ml penicillin and 100ug/ml streptomycin. Assay plates were prepared by dispensing compounds dissolved in DMSO into wells of 384 well collagen-coated plates using an ECHO acoustic dispenser. Each compound was tested in a 10-point, serial 3-fold dilution. Controls included uninfected cells and infected cells treated only with DMSO. Assays were initiated by mixing selected cells, in suspension, with virus, and dispensing 50µl/well infected cells to pre-plated compounds. Plates were incubated at 37°C/5% CO2/90% relative humidity for ~72hrs to permit genomic replication, and infected cells were lysed by the addition of an equal volume of lysis buffer (10mM Tris-HCl, pH8, 50mM KCl, 2mM MgCl₂, 0.45% NP-40, 0.45% Tween-20, and 100µg/ml proteinase K). An aliquot of the lysate was transferred to a 384-well PCR plate and incubated at 56°C for 1 hour and then at 95°C for 10min. Levels of a viral gene and of the cellular control, PPIA (Thermo Fisher Assay ID = Hs04194521_s1), were measured in separate 10 µL qPCR assays using TaqMan^{®} Gene Expression Master Mix (Applied Biosystems), and an 7900HT Fast Real-Time PCR System with 384-Well Block Module. 7-point, serial 10-fold dilutions of a plasmid standard were run on each plate to generate a standard curve, and genome copies numbers were calculated by plotting experimental Ct onto linear regression of the standard curve. Viral genome copy numbers were normalized by cellular control copy number, and compound effects were normalized to the window defined by the controls. Calculated % effects were fit using a 4-parameter algorithm, and EC₅₀ was reported.

### Viral qPCR assays (V2/V3)

MRC5 cells, Vero cells, and MeWo cells were obtained from ATCC and were maintained at 37°C/5% CO2/90% relative humidity in Minimal Essential Medium with 10% fetal bovine serum, 2.0 nM L-glutamine, 100 units/ml penicillin and 100ug/ml streptomycin. Assay plates were prepared by dispensing compounds dissolved in DMSO into wells of 384 well collagen-coated plates using an ECHO acoustic dispenser. Each compound was tested in a 10-point, serial 3-fold dilution. Controls included uninfected cells and infected cells treated only with DMSO. Assays were initiated by mixing selected cells, in suspension, with virus, and dispensing 50µl/well infected cells to pre-plated compounds. Plates were incubated at 37°C/5% CO2/90% relative humidity for ~72hrs to permit genomic replication, and infected cells were lysed by the addition of an equal volume of lysis buffer (10mM Tris-HCl, pH8, 50mM KCl, 2mM MgCl₂, 0.45% NP-40, 0.45% Tween-20, and 100µg/ml proteinase K). An aliquot of the lysate was transferred to a 384-well PCR plate and incubated at 56°C for 1 hour and then at 95°C for 10min. Levels of a viral gene were measured in 10 µL qPCR assays using TaqMan^{®} Gene Expression Master Mix (Applied Biosystems), and an 7900HT Fast Real-Time PCR System with 384-Well Block Module. 7-point, serial 10-fold dilutions of a plasmid standard were run on each plate to generate a standard curve, and genome copies numbers were calculated by plotting experimental Ct onto linear regression of the standard curve. Compound effects on viral genome copy number were normalized to the window defined by the controls. Calculated % effects were fit using a 4-parameter algorithm, and EC₅₀ was reported.

HCMV: Strain AD169 Street was assayed in MRC-5 cells and was used at 0.05-0.1 pfu/cell. The assays were performed in either growth media or in the same media with 50% fetal bovine serum. Primer-probe set was Thermo Fisher Assay ID = AIFATFK.

VZV: Strain Street was maintained as an infected cell stock in MRC-5 cells. It was assayed in MeWo or MRC5 cells and was used at 0.04-0.1 pfu/cell. The assays were performed in growth medium. Primer-probe set was Thermo Fisher Assay ID = AIPAEXQ. HSV-1: Strain F Street was assayed in Vero or MRC5 cells and was used at 0.0005-0.004 pfu/cell in growth medium. Primer-probe set was Thermo Fisher Assay ID = AIBJZIB

Illustrative compounds of the present invention were tested in one or more of the above assays and results (in nM) are provided in the table below (blank boxes indicate data is not available):

| Compound | CMV^{a} (IC₅₀) | VZV^{a} (IC₅₀) | CMV MRC5 Cell^{b} (EC₅₀) | VZV MEWO Cell^{b} (IC₅₀) | VZV MRC5 Cell^{b} (IC₅₀) | HSV1 VERO Cell^{b} (EC₅₀) | HSV1 MRC5 Cell^{b} (EC₅₀) | HSV2 VERO Cell^{b} (EC₅₀) | HSV2 MRC5 Cell^{b} (EC₅₀) |
|---|---|---|---|---|---|---|---|---|---|
| 1 | 235.5 | N/A | 4477 | N/A | 7532 | N/A | 9979 | N/A | 6737 |
| 2 | 9.815 | N/A | 176.2 | N/A | 181.9 | N/A | 270.5 | N/A | 118.8 |
| 3 | 8.751 | 3.453 | 1255 | 342.5 | N/A | 33260 | N/A | 56920 | N/A |
| 4 | 52.59 | 32.63 | 2976 | N/A | N/A | 26120 | N/A | N/A | N/A |
| 5 | 5.304 | N/A | 391.9 | N/A | 405.4 | N/A | 247.7 | N/A | 247.8 |
| 6 | 957.5 | N/A | 5867 | N/A | N/A | N/A | N/A | N/A | N/A |
| 7 | 9.817 | N/A | 141.6 | N/A | 190.5 | N/A | 117.7 | N/A | 86.61 |
| 8 | 18.48 | N/A | 678.2 | N/A | 478.7 | N/A | 299.1 | N/A | 226.2 |
| 9 | 19.18 | N/A | 644.4 | N/A | N/A | N/A | N/A | N/A | N/A |
| 10 | 20.9 | N/A | 433.6 | N/A | 171.8 | N/A | 189.3 | N/A | 124.4 |
| 11 | 22.69 | N/A | 263.3 | N/A | 169.6 | N/A | 171.9 | N/A | 50.65 |
| 12 | 23.02 | N/A | 175.1 | N/A | 219.7 | N/A | 160.1 | N/A | 44.83 |
| 13 | 25.82 | N/A | 546.8 | N/A | 206.9 | N/A | 284.4 | N/A | 74.58 |
| 14 | 25.83 | N/A | 1030 | N/A | N/A | N/A | N/A | N/A | N/A |
| 15 | 30.96 | N/A | 1096 | N/A | N/A | N/A | N/A | N/A | N/A |
| 16 | 31.18 | N/A | 297.7 | N/A | 208.4 | N/A | 233.1 | N/A | 74.49 |
| 17 | 36.08 | N/A | 51440 | N/A | N/A | N/A | N/A | N/A | N/A |
| 18 | 71.11 | N/A | 554.8 | N/A | 368.1 | N/A | 229 | N/A | 108.1 |
| 19 | 90.59 | N/A | 398.7 | N/A | 534.7 | N/A | 650.4 | N/A | 332.8 |
| 20 | 114.8 | N/A | 448.4 | N/A | 309.3 | N/A | 449 | N/A | 142.9 |
| 21 | 157.2 | N/A | 2669 | N/A | N/A | N/A | N/A | N/A | N/A |
| 22 | 10.99 | N/A | 313 | N/A | 413.3 | N/A | 230.9 | N/A | 250.6 |
| 23 | 17.21 | N/A | 129.8 | N/A | 324.7 | N/A | 224 | N/A | 136.1 |
| 24 | 147.9 | N/A | 997.2 | N/A | N/A | N/A | N/A | N/A | N/A |
| 25 | 11.27 | 11.49 | 969.8 | 483.9 | 28980 | 99010 | 17420 | 99010 | 16360 |
| 26 | 16.19 | 10.16 | 2632 | N/A | N/A | 66070 | N/A | N/A | N/A |
| 27 | 45.93 | 13.12 | 5532 | N/A | N/A | 99010 | N/A | 99010 | N/A |
| 28 | 15.22 | N/A | 108.3 | N/A | 210.1 | N/A | 139.4 | N/A | 89.55 |
| 29 | 186.1 | 57.82 | 3685 | 258.5 | N/A | 5315 | N/A | 8934 | N/A |
| 30 | 244.7 | 65.92 | 5751 | 2348 | N/A | 11970 | N/A | 16380 | N/A |
| 31 | 26.88 | N/A | 434.6 | N/A | 1470 | N/A | 1037 | N/A | 1411 |
| 32 | 14.08 | 5.683 | 419.7 | N/A | 417.6 | 381 | 653.1 | 164.6 | 499.6 |
| 33 | 52.48 | N/A | 628.1 | N/A | 1964 | N/A | 1558 | N/A | 1408 |
| 34 | 51.64 | N/A | 1802 | N/A | 2053 | N/A | 789.6 | N/A | 1848 |
| 35 | 54.84 | 30.99 | 2674 | N/A | N/A | 1849 | N/A | 580.8 | N/A |
| 36 | 8.935 | 5.848 | 452.6 | 102.1 | 856.4 | 1193 | 1271 | 2841 | 960.3 |
| 37 | 23.32 | 60.75 | 3285 | N/A | N/A | 15540 | N/A | 23450 | N/A |
| 38 | 34.56 | 15.58 | 1238 | 500.5 | N/A | 2581 | N/A | 5828 | N/A |
| 39 | 54.91 | 45.16 | 1578 | N/A | N/A | 12380 | N/A | 16170 | N/A |
| 40 | 89.05 | 90.77 | 5297 | N/A | N/A | 23260 | N/A | 34520 | N/A |
| 41 | 110.9 | 23.69 | 6316 | N/A | N/A | 20380 | N/A | 30620 | N/A |
| 42 | 25.9 | N/A | 940.4 | N/A | 1054 | N/A | 996.2 | N/A | 1102 |
| 43 | 58.21 | N/A | 3292 | N/A | 2637 | N/A | 3551 | N/A | 3489 |
| 44 | 33.79 | N/A | 1100 | N/A | 2369 | N/A | 2499 | N/A | 2951 |
| 45 | 37.78 | N/A | 797.8 | N/A | 1027 | N/A | 1990 | N/A | 1041 |
| 46 | 69.71 | 23.02 | 706.1 | N/A | N/A | 211.4 | N/A | 529.8 | N/A |
| 47 | 38.14 | N/A | 328 | N/A | 656.6 | N/A | 878 | N/A | 611.1 |
| 48 | 69.67 | 118.1 | 4015 | N/A | N/A | N/A | N/A | N/A | N/A |
| 49 | 87.53 | N/A | 3541 | N/A | N/A | N/A | N/A | N/A | N/A |
| 50 | 124.9 | N/A | 1485 | N/A | N/A | N/A | N/A | N/A | N/A |
| 51 | 129.1 | N/A | 1795 | N/A | N/A | N/A | N/A | N/A | N/A |
| 12f | 139.5 | N/A | 3014 | N/A | N/A | N/A | N/A | N/A | N/A |
| 52 | 149.9 | N/A | 885.3 | N/A | N/A | N/A | N/A | N/A | N/A |
| 53 | 124.5 | N/A | 2090 | N/A | N/A | N/A | N/A | N/A | N/A |
| 54 | 146.3 | N/A | 1585 | N/A | N/A | N/A | N/A | N/A | N/A |
| 55 | 94.07 | 26.87 | 2709 | 477.9 | N/A | 5596 | N/A | 13080 | N/A |
| 56 | 129.1 | N/A | 1795 | N/A | N/A | N/A | N/A | N/A | N/A |
| 57 | 190.9 | 496.4 | 2084 | N/A | N/A | 36410 | N/A | 68290 | N/A |
| 58 | 120 | 18.95 | 1231 | 601.8 | N/A | 535.7 | N/A | 711.5 | N/A |
| 59 | 70.56 | 48.63 | 714 | N/A | 5211 | 1372 | 7101 | 3536 | 3177 |
| 60 | 143.3 | 111.9 | 969.5 | 1472 | 5956 | 2848 | 8350 | 5458 | 5568 |
| 61 | 345.7 | 192.2 | 1984 | N/A | N/A | 30830 | N/A | 37680 | N/A |
| 62 | 62.46 | N/A | N/A | N/A | N/A | N/A | N/A | N/A | N/A |
| 63 | 99.26 | 76.02 | N/A | N/A | N/A | N/A | N/A | N/A | N/A |
| 64 | 64.04 | N/A | N/A | N/A | N/A | N/A | N/A | N/A | N/A |
| 65 | 64.53 | N/A | N/A | N/A | N/A | N/A | N/A | N/A | N/A |
| 66 | 112.2 | 48.59 | N/A | N/A | N/A | N/A | N/A | N/A | N/A |
| 67 | 118.5 | N/A | N/A | N/A | N/A | N/A | N/A | N/A | N/A |
| 68 | 122.6 | 59.09 | N/A | N/A | N/A | N/A | N/A | N/A | N/A |
| 69 | 173.4 | 84.89 | N/A | N/A | N/A | N/A | N/A | N/A | N/A |
| 70 | 1711 | N/A | N/A | N/A | N/A | N/A | N/A | N/A | N/A |
| 71 | 76.23 | N/A | N/A | N/A | 1208 | N/A | 2084 | N/A | 1075 |
| 72 | 976.4 | N/A | N/A | N/A | N/A | N/A | N/A | N/A | N/A |
| 73 | 11.23 | N/A | 133.5 | N/A | 533.6 | N/A | 614 | N/A | 464.4 |
| 74 | 814.8 | N/A | 28100 | N/A | N/A | N/A | N/A | N/A | N/A |
| 75 | 70.86 | N/A | 2019 | N/A | N/A | N/A | N/A | N/A | N/A |
| 76 | 30.01 | 36.09 | 3612 | N/A | N/A | 1408 | N/A | 1588 | N/A |
| 77 | 49.83 | N/A | 304 | N/A | N/A | N/A | N/A | N/A | N/A |
| 78 | 251.8 | N/A | 2878 | N/A | 2870 | N/A | 3027 | N/A | 2218 |
| 79 | 69.65 | N/A | 232.1 | N/A | 1167 | N/A | 1183 | N/A | 935.4 |
| 80 | 168.2 | 101.8 | 2779 | N/A | 3889 | 1928 | 4002 | 2312 | 2834 |
| 81 | 557 | N/A | 1284 | N/A | N/A | N/A | N/A | N/A | N/A |
| 82 | 60.3 | N/A | 249.9 | N/A | 1453 | N/A | 2883 | N/A | 1017 |
| 83 | 13320 | N/A | 99010 | N/A | N/A | N/A | N/A | N/A | N/A |
| 84 | 13320 | N/A | 40020 | N/A | N/A | N/A | N/A | N/A | N/A |
| 85 | 1052 | N/A | 3365 | N/A | N/A | N/A | N/A | N/A | N/A |
| 86 | 18.19 | 37.04 | 616.2 | N/A | N/A | 1708 | N/A | 2364 | N/A |
| 87 | 12110 | 4055 | 33000 | N/A | N/A | 99010 | N/A | 99010 | N/A |
| 88 | 13320 | N/A | 33000 | N/A | N/A | N/A | N/A | N/A | N/A |
| 89 | 799.8 | N/A | 7640 | N/A | N/A | N/A | N/A | N/A | N/A |
| 90 | 31.73 | N/A | 212.3 | N/A | 1226 | N/A | 1319 | N/A | 1178 |
| 91 | 8609 | N/A | 99010 | N/A | N/A | N/A | N/A | N/A | N/A |
| 92 | 1182 | N/A | 65910 | N/A | N/A | N/A | N/A | N/A | N/A |
| 93 | 97.58 | N/A | 2160 | N/A | N/A | N/A | N/A | N/A | N/A |
| 94 | 120.4 | N/A | 1191 | N/A | N/A | N/A | N/A | N/A | N/A |
| 95 | 126.9 | N/A | 631.6 | N/A | 4546 | N/A | 3301 | N/A | 3723 |
| 96 | 144.4 | N/A | 1118 | N/A | N/A | N/A | N/A | N/A | N/A |
| 97 | 174.2 | N/A | N/A | N/A | N/A | N/A | N/A | N/A | N/A |
| 98 | 534.4 | N/A | 1401 | N/A | N/A | N/A | N/A | N/A | N/A |
| 99 | 5.069 | N/A | 60.46 | N/A | 53.15 | N/A | 79.46 | N/A | 43.19 |
| 100 | 7.617 | 4.842 | 222 | N/A | N/A | 217.2 | N/A | 196 | N/A |
| 101 | 530.7 | 391.1 | 32710 | N/A | N/A | 29060 | N/A | N/A | N/A |
| 102 | 5766 | N/A | 71170 | N/A | N/A | N/A | N/A | N/A | N/A |
| 103 | 17.41 | N/A | 156.9 | N/A | 104.4 | N/A | 171.9 | N/A | 62.98 |
| 104 | 1814 | N/A | 38680 | N/A | N/A | N/A | N/A | N/A | N/A |
| 105 | 23 | N/A | 479.4 | N/A | 289.1 | N/A | 335.2 | N/A | 273.9 |
| 106 | 108.4 | 30.48 | 1170 | N/A | N/A | 1058 | N/A | 1553 | N/A |
| 107 | 4541 | 1505 | 99010 | N/A | N/A | 73750 | N/A | 90340 | N/A |
| 108 | 701.7 | N/A | 1964 | N/A | N/A | N/A | N/A | N/A | N/A |
| 109 | 1528 | N/A | 5010 | N/A | N/A | N/A | N/A | N/A | N/A |
| 110 | 2896 | N/A | 22190 | N/A | N/A | N/A | N/A | N/A | N/A |
| 111 | 57.87 | N/A | 124.8 | N/A | 402.2 | N/A | 557.3 | N/A | 247.8 |
| 112 | 105 | N/A | 148.3 | N/A | 262.8 | N/A | 293.5 | N/A | 232.8 |
| 113 | 187.6 | N/A | 4829 | N/A | N/A | N/A | N/A | N/A | N/A |
| 114 | 77.6 | N/A | 1099 | N/A | N/A | N/A | N/A | N/A | N/A |
| 115 | 566.3 | N/A | 4239 | N/A | N/A | N/A | N/A | N/A | N/A |
| 116 | 10860 | N/A | 99010 | N/A | N/A | N/A | N/A | N/A | N/A |
| 117 | 679.9 | N/A | 4638 | N/A | N/A | N/A | N/A | N/A | N/A |
| 118 | 83.72 | N/A | 1601 | N/A | N/A | N/A | N/A | N/A | N/A |
| 119 | 7852 | N/A | 99010 | N/A | N/A | N/A | N/A | N/A | N/A |
| 120 | 120.7 | N/A | 642.5 | N/A | 1775 | N/A | 2741 | N/A | 2022 |
| 121 | 13320 | N/A | 99010 | N/A | N/A | N/A | N/A | N/A | N/A |
| 122 | 195 | 24.05 | 1307 | N/A | N/A | 860.3 | N/A | N/A | N/A |
| 123 | 1210 | 898.1 | 14650 | N/A | N/A | 13280 | N/A | N/A | N/A |
| 124 | 141.4 | N/A | 580.9 | N/A | 2159 | N/A | 3364 | N/A | 2353 |
| 125 | 1370 | N/A | 2978 | N/A | N/A | 4978 | N/A | 8043 | N/A |
| 126 | 522.4 | 159.1 | 6110 | N/A | N/A | 1193 | N/A | 1960 | N/A |
| 127 | 48.88 | 9.717 | 599 | 129.7 | 407.4 | 239.7 | 694.3 | 364.1 | 278 |
| 128 | 1060 | 288.9 | N/A | N/A | N/A | N/A | N/A | N/A | N/A |
| 129 | 74.23 | 9.097 | 1571 | 153.6 | 370.7 | 263.9 | 1021 | 422.4 | 383.5 |
| 130 | 4.44 | 4.44 | 1202 | N/A | N/A | 3840 | N/A | 4120 | N/A |
| 131 | 16.82 | 11.67 | 1577 | N/A | N/A | 7285 | N/A | 7019 | N/A |
| 132 | 21.57 | 12.8 | 2933 | N/A | N/A | 7164 | N/A | 9329 | N/A |
| 133 | 85.84 | 19.67 | 804.9 | N/A | 1214 | 1622 | 1190 | 1064 | 556.6 |
| 134 | 87.38 | 14.86 | 970 | N/A | 831.7 | 990.7 | 1170 | 584.3 | 544.9 |
| 135 | 89.32 | 26.14 | 769.1 | N/A | 633.3 | 1523 | 749.3 | 1973 | 589.3 |
| 136 | 118.9 | 16.54 | 979.5 | N/A | N/A | 3523 | N/A | 2517 | N/A |
| 137 | 173.6 | 50.05 | 7731 | N/A | N/A | 26100 | N/A | 55710 | N/A |
| 138 | 169.6 | N/A | 1868 | N/A | N/A | 4310 | N/A | 5897 | N/A |
| 139 | 50490 | N/A | 99010 | N/A | N/A | 99010 | N/A | 33000 | N/A |
| 140 | 63.24 | 26.47 | 2655 | N/A | N/A | 439.3 | N/A | 704.6 | N/A |

| | | | | | | | | | |
|---|---|---|---|---|---|---|---|---|---|
| N/A = not available a = data generated using the assay described in Example 94 b = data generated using the assay described in Example 93 | | | | | | | | | |

### Uses of the Fused Bicyclic Pyrazole Derivatives

The Fused Bicyclic Pyrazole Derivatives are useful in human and veterinary medicine for treating or preventing a viral infection in a patient. In one embodiment, the Fused Bicyclic Pyrazole Derivatives can be inhibitors of viral replication. In another embodiment, the Fused Bicyclic Pyrazole Derivatives can be inhibitors of herpesvirus replication. Accordingly, the Fused Bicyclic Pyrazole Derivatives are useful for treating viral infections, such as herpesvirus. In accordance with the invention, the Fused Bicyclic Pyrazole Derivatives can be administered to a patient in need of treatment or prevention of a viral infection.

Accordingly, in one embodiment, the invention provides methods for treating or preventing a viral infection in a patient comprising administering to the patient an effective amount of at least one Fused Bicyclic Pyrazole Derivative or a pharmaceutically acceptable salt thereof.

### Treatment or Prevention of Herpesvirus Infection

The Fused Bicyclic Pyrazole Derivatives are useful in the inhibition of herpesvirus replication, the treatment of herpesvirus infection and/or reduction of the likelihood or severity of symptoms of herpesvirus infection and the inhibition of herpesvirus viral replication and/or herpesvirus viral production in a cell-based system. For example, the Fused Bicyclic Pyrazole Derivatives are useful in treating infection by herpesvirus after suspected past exposure to herpesvirus by such means as blood transfusion, exchange of body fluids, bites, accidental needle stick, or exposure to patient blood during surgery or other medical procedures. Accordingly, in one embodiment, the invention provides a method for treating herpesvirus infection in a patient, the method comprising administering to the patient an effective amount of at least one Fused Bicyclic Pyrazole Derivative or a pharmaceutically acceptable salt thereof.

In one embodiment, the herpesvirus being treated or prevented is of the family α-herpesviridae. Herpesviruses of the family α-herpesviridae include, but are not limited to, herpes simplex virus 1 (HSV-1), herpes simplex 2 (HSV-2), and varicella zoster virus (VZV).

In another embodiment, the herpesvirus being treated or prevented is of the family β-herpesviridae. Herpesviruses of the family β-herpesviridae include, but are not limited to, human cytomegalovirus (CMV), human herpesvirus 6 (HHV6), and human herpesvirus 7 (HHV7).

In another embodiment, the herpesvirus being treated or prevented is of the family γ-herpesviridae. Herpesviruses of the family γ-herpesviridae include, but are not limited to, Epstein-Barr virus (EBV), human herpesvirus 4 (HHV4), and Kaposi's sarcoma-associated herpesvirus (KHSV), also known as human herpesvirus 8 (HHV8).

In one embodiment, the herpesvirus being treated or prevented is HSV-1.

In another embodiment, the herpesvirus being treated or prevented is HSV-2.

In another embodiment, the herpesvirus being treated or prevented is VZV.

In still another embodiment, the herpesvirus being treated or prevented is CMV.

In another embodiment, the herpesvirus being treated or prevented is HHV6.

In yet another embodiment, the herpesvirus being treated or prevented is HHV7.

In another embodiment, the herpesvirus being treated or prevented is EBV.

In a further embodiment, the herpesvirus being treated or prevented is HHV4.

In another embodiment, the herpesvirus being treated or prevented is KSHV.

In a specific embodiment, the amount administered is effective to treat or prevent infection by herpesvirus in the patient. In another specific embodiment, the amount administered is effective to inhibit herpesvirus viral replication and/or viral production in the patient.

The Fused Bicyclic Pyrazole Derivatives are also useful in the preparation and execution of screening assays for antiviral compounds. Furthermore, the Fused Bicyclic Pyrazole Derivatives are useful in establishing or determining the binding site of other antivirals to the herpesvirus polymerase.

The compositions and combinations of the present invention can be useful for treating a patient suffering from infection related to any herpesvirus infection. Herpesvirus types may differ in their antigenicity, level of viremia, severity of disease produced, and response to therapy. See Poole et al., Clinical Therapeutics, 40:8 (2018), 1282-1298.

### Combination Therapy

In another embodiment, the present methods for treating or preventing herpesvirus infection can further comprise the administration of one or more additional therapeutic agents which are not Fused Bicyclic Pyrazole Derivatives.

In one embodiment, the additional therapeutic agent is an antiviral agent. In another embodiment, the additional therapeutic agent is an anti-herpes agent.

Anti-herpes agents useful in the present compositions and methods include, but are not limited to, nucleoside polymerase inhibitors, such as acyclovir, valaciclovir, famciclovir, penciclovir, cidofovir, brincidofovir (CMX-001), valmanciclovir, ganciclovir, valganciclovir, and N-methanocarbathymidine (N-MCT); pyrophosphate polymerase inhibitors, such as foscarnet; CMV terminase inhibitors, such as letermovir; viral kinase inhibitors, such as maribavir; and helicase-primase inhibitors, such as pritelivir (AIC-316), and amenamevir (ASP-2151).

In another embodiment, the additional therapeutic agent is an immunomodulatory agent, such as an immunosuppressive agent. Immunosuppressant agents useful in the present compositions and methods include, but are not limited to, cytotoxic agents, such as cyclophosphamide and cyclosporin A; corticosteroids, such as hydrocortisone and dexamethasone, and non-steroidal anti-inflammatory agents (NSAID).

Accordingly, in one embodiment, the present invention provides methods for treating a herpesvirus infection in a patient, the method comprising administering to the patient: (i) at least one Fused Bicyclic Pyrazole Derivative, or a pharmaceutically acceptable salt thereof, and (ii) at least one additional therapeutic agent that is other than an Fused Bicyclic Pyrazole Derivative, wherein the amounts administered are together effective to treat or prevent the herpesvirus infection.

When administering a combination therapy of the invention to a patient, therapeutic agents in the combination, or a pharmaceutical composition or compositions comprising therapeutic agents, may be administered in any order such as, for example, sequentially, concurrently, together, simultaneously and the like. The amounts of the various actives in such combination therapy may be different amounts (different dosage amounts) or same amounts (same dosage amounts). Thus, for non-limiting illustration purposes, an Fused Bicyclic Pyrazole Derivative and an additional therapeutic agent may be present in fixed amounts (dosage amounts) in a single dosage unit (*e.g.,* a capsule, a tablet and the like).

In one embodiment, the at least one Fused Bicyclic Pyrazole Derivative is administered during a time when the additional therapeutic agent(s) exert their prophylactic or therapeutic effect, or vice versa.

In another embodiment, the at least one Fused Bicyclic Pyrazole Derivative and the additional therapeutic agent(s) are administered in doses commonly employed when such agents are used as monotherapy for treating a herpesvirus infection.

In another embodiment, the at least one Fused Bicyclic Pyrazole Derivative and the additional therapeutic agent(s) are administered in doses lower than the doses commonly employed when such agents are used as monotherapy for treating a herpesvirus infection.

In still another embodiment, the at least one Fused Bicyclic Pyrazole Derivative and the additional therapeutic agent(s) act synergistically and are administered in doses lower than the doses commonly employed when such agents are used as monotherapy for treating a herpesvirus infection.

In one embodiment, the at least one Fused Bicyclic Pyrazole Derivative and the additional therapeutic agent(s) are present in the same composition. In one embodiment, this composition is suitable for oral administration. In another embodiment, this composition is suitable for intravenous administration. In another embodiment, this composition is suitable for subcutaneous administration. In still another embodiment, this composition is suitable for parenteral administration.

The at least one Fused Bicyclic Pyrazole Derivative and the additional therapeutic agent(s) can act additively or synergistically. A synergistic combination may allow the use of lower dosages of one or more agents and/or less frequent administration of one or more agents of a combination therapy. A lower dosage or less frequent administration of one or more agents may lower toxicity of therapy without reducing the efficacy of therapy.

In one embodiment, the administration of at least one Fused Bicyclic Pyrazole Derivative and the additional therapeutic agent(s) may inhibit the resistance of a herpesvirus infection to these agents.

The doses and dosage regimen of the other agents used in the combination therapies of the present invention for the treatment or prevention of herpesvirus infection can be determined by the attending clinician, taking into consideration the approved doses and dosage regimen in the package insert; the age, sex and general health of the patient; and the type and severity of the viral infection or related disease or disorder. When administered in combination, the Fused Bicyclic Pyrazole Derivative(s), and the other agent(s) can be administered simultaneously (*i.e.,* in the same composition or in separate compositions one right after the other) or sequentially. This is particularly useful when the components of the combination are given on different dosing schedules, *e.g*., one component is administered once daily and another component is administered every six hours, or when the preferred pharmaceutical compositions are different, *e.g.,* one is a tablet and one is a capsule. A kit comprising the separate dosage forms is therefore advantageous.

In one embodiment, one or more compounds of the present invention are administered with one or more additional therapeutic agents selected from: an immunomodulator, an anti-herpes agent, a viral replication inhibitor, an antisense agent, a therapeutic vaccine, a virion production inhibitor, a viral entry inhibitor, a viral assembly inhibitor, an antibody therapy (monoclonal or polyclonal), and any agent useful for treating any type of herpesvirus infection.

### Compositions and Administration

Due to their activity, the Fused Bicyclic Pyrazole Derivatives are useful in veterinary and human medicine. As described above, the Fused Bicyclic Pyrazole Derivatives are useful for treating or preventing herpesvirus infection in a patient in need thereof.

Accordingly, in one embodiment, the present invention provides pharmaceutical compositions comprising an effective amount of a compound of formula(I), or a pharmaceutically acceptable salt thereof, and a pharmaceutically acceptable carrier.

In another embodiment, the present invention provides pharmaceutical compositions comprising (i) an effective amount of a compound of formula(I), or a pharmaceutically acceptable salt thereof, and a pharmaceutically acceptable carrier; and (ii) one or more additional therapeutic agents, wherein said additional therapeutic agents are selected from anti-herpes agents and immunomodulators.

When administered to a patient, the Fused Bicyclic Pyrazole Derivatives can be administered as a component of a composition that comprises a pharmaceutically acceptable carrier or vehicle. The present invention provides pharmaceutical compositions comprising an effective amount of at least one Fused Bicyclic Pyrazole Derivative and a pharmaceutically acceptable carrier. In the pharmaceutical compositions and methods of the present invention, the active ingredients will typically be administered in admixture with suitable carrier materials suitably selected with respect to the intended form of administration, *i.e.,* oral tablets, capsules (either solid-filled, semi-solid filled or liquid filled), powders for constitution, oral gels, elixirs, dispersible granules, syrups, suspensions, and the like, and consistent with conventional pharmaceutical practices. For example, for oral administration in the form of tablets or capsules, the active drug component may be combined with any oral non-toxic pharmaceutically acceptable inert carrier, such as lactose, starch, sucrose, cellulose, magnesium stearate, dicalcium phosphate, calcium sulfate, talc, mannitol, ethyl alcohol (liquid forms), and the like. Solid form preparations include powders, tablets, dispersible granules, capsules, cachets and suppositories. Powders and tablets may be comprised of from about 0.5 to about 95 percent inventive composition. Tablets, powders, cachets and capsules can be used as solid dosage forms suitable for oral administration.

Moreover, when desired or needed, suitable binders, lubricants, disintegrating agents and coloring agents may also be incorporated in the mixture. Suitable binders include starch, gelatin, natural sugars, corn sweeteners, natural and synthetic gums such as acacia, sodium alginate, carboxymethylcellulose, polyethylene glycol and waxes. Among the lubricants there may be mentioned for use in these dosage forms, boric acid, sodium benzoate, sodium acetate, sodium chloride, and the like. Disintegrants include starch, methylcellulose, guar gum, and the like. Sweetening and flavoring agents and preservatives may also be included where appropriate.

Liquid form preparations include solutions, suspensions and emulsions and may include water or water-propylene glycol solutions for parenteral or intravenous injection.

Also included are solid form preparations which are intended to be converted, shortly before use, to liquid form preparations for either oral or parenteral administration. Such liquid forms include solutions, suspensions and emulsions.

For preparing suppositories, a low melting wax such as a mixture of fatty acid glycerides or cocoa butter is first melted, and the active ingredient is dispersed homogeneously therein as by stirring. The molten homogeneous mixture is then poured into convenient sized molds, allowed to cool and thereby solidify.

Additionally, the compositions of the present invention may be formulated in sustained release form to provide the rate-controlled release of any one or more of the components or active ingredients to optimize therapeutic effects, *i.e.,* antiviral activity and the like. Suitable dosage forms for sustained release include layered tablets containing layers of varying disintegration rates or controlled release polymeric matrices impregnated with the active components and shaped in tablet form or capsules containing such impregnated or encapsulated porous polymeric matrices.

In one embodiment, the one or more Fused Bicyclic Pyrazole Derivatives are administered orally.

In another embodiment, the one or more Fused Bicyclic Pyrazole Derivatives are administered intravenously.

In still another embodiment, the one or more Fused Bicyclic Pyrazole Derivatives are administered sublingually.

In one embodiment, a pharmaceutical preparation comprising at least one Fused Bicyclic Pyrazole Derivative is in unit dosage form. In such form, the preparation is subdivided into unit doses containing effective amounts of the active components.

Compositions can be prepared according to conventional mixing, granulating or coating methods, respectively, and the present compositions can contain, in one embodiment, from about 0.1% to about 99% of the Fused Bicyclic Pyrazole Derivative(s) by weight or volume. In various embodiments, the present compositions can contain, in one embodiment, from about 1% to about 70% or from about 5% to about 60% of the Fused Bicyclic Pyrazole Derivative(s) by weight or volume.

The amount and frequency of administration of the Fused Bicyclic Pyrazole Derivatives will be regulated according to the judgment of the attending clinician considering such factors as age, condition and size of the patient as well as severity of the symptoms being treated. Generally, a total daily dosage of the at least one Fused Bicyclic Pyrazole Derivative(s) alone, or when administered as combination therapy, can range from about 1 to about 2500 mg per day, although variations will necessarily occur depending on the target of therapy, the patient and the route of administration. In one embodiment, the dosage is from about 10 to about 1000 mg/day, administered in a single dose or in 2-4 divided doses. In another embodiment, the dosage is from about 1 to about 500 mg/day, administered in a single dose or in 2-4 divided doses. In still another embodiment, the dosage is from about 1 to about 100 mg/day, administered in a single dose or in 2-4 divided doses. In yet another embodiment, the dosage is from about 1 to about 50 mg/day, administered in a single dose or in 2-4 divided doses. In another embodiment, the dosage is from about 500 to about 1500 mg/day, administered in a single dose or in 2-4 divided doses. In still another embodiment, the dosage is from about 500 to about 1000 mg/day, administered in a single dose or in 2-4 divided doses. In yet another embodiment, the dosage is from about 100 to about 500 mg/day, administered in a single dose or in 2-4 divided doses.

The compositions of the invention can further comprise one or more additional therapeutic agents, selected from those listed above herein. Accordingly, in one embodiment, the present invention provides compositions comprising: (i) at least one Fused Bicyclic Pyrazole Derivative or a pharmaceutically acceptable salt thereof; (ii) one or more additional therapeutic agents that are not an Fused Bicyclic Pyrazole Derivative; and (iii) a pharmaceutically acceptable carrier, wherein the amounts in the composition are together effective to treat herpesvirus infection.

In one embodiment, the present invention provides compositions comprising a Compound of Formula (I), and a pharmaceutically acceptable carrier.

In another embodiment, the present invention provides compositions comprising a Compound of Formula (I), a pharmaceutically acceptable carrier, and a second therapeutic agent selected from the group consisting of anti-herpes agents and immunomodulators.

In another embodiment, the present invention provides compositions comprising a Compound of Formula (I), a pharmaceutically acceptable carrier, and two additional therapeutic agents, each of which are independently selected from the group consisting of anti-herpes agents and immunomodulators.

### Kits

In one aspect, the present invention provides a kit comprising a therapeutically effective amount of at least one Fused Bicyclic Pyrazole Derivative, or a pharmaceutically acceptable salt, solvate, ester or prodrug of said compound and a pharmaceutically acceptable carrier, vehicle or diluent.

In another aspect the present invention provides a kit comprising an amount of at least one Fused Bicyclic Pyrazole Derivative, or a pharmaceutically acceptable salt, solvate, ester or prodrug of said compound and an amount of at least one additional therapeutic agent listed above, wherein the amounts of the two or more active ingredients result in a desired therapeutic effect. In one embodiment, the one or more Fused Bicyclic Pyrazole Derivatives and the one or more additional therapeutic agents are provided in the same container. In one embodiment, the one or more Fused Bicyclic Pyrazole Derivatives and the one or more additional therapeutic agents are provided in separate containers.

## Claims

1. A compound having the formula (I): 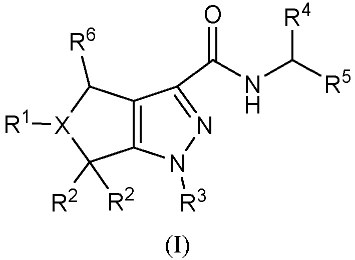
or a pharmaceutically acceptable salt thereof,
wherein:
X is N or C(R⁹);
R¹ is selected from -(C₁-C₆ alkylene)-(4 to 7-membered monocyclic heterocycloalkyl), - (C₁-C₆ alkylene)-(6 to 10-membered bicyclic heterocycloalkyl), and -(C₁-C₆ alkylene)-(5 or 6-membered monocyclic heteroaryl), wherein said 4 to 7-membered monocyclic heterocycloalkyl group, said 6 to 10-membered bicyclic heterocycloalkyl group, and said 5 or 6-membered monocyclic heteroaryl group, can each be optionally substituted with one or more R^{A} groups, which can be the same or different;
each R² is independently selected from H, C₁-C₆ alkyl, -(C₁-C₆ alkylene)ₘ-O-C(O)-(C₁-C₆ alkyl), and C₁-C₆ hydroxyalkyl, or both R² groups, together with the carbon atom to which they are attached, combine to form a spirocyclic C₃-C₆ cycloalkyl group;
R³ is selected from C₁-C₆ alkyl, , C₁-C₆ hydroxyalkyl, --CD³, -(C₁-C₆ alkylene)ₘ-C₃-C₆ monocyclic cycloalkyl -(C₁-C₆ alkylene)-N(R⁷)₂ C₁-C₆ haloalkyl, -(C₁-C₆ alkylene)ₘ-(3 to 7-membered monocyclic heterocycloalkyl), -(C₁-C₆ alkylene)ₘ-(5 or 6-membered monocyclic heteroaryl), and 9 or 10-membered bicyclic heteroaryl, wherein said C₃-C₆ monocyclic cycloalkyl group, said 3 to 7-membered monocyclic heterocycloalkyl group, said 5 or 6-membered monocyclic heteroaryl group, and said 9 or 10-membered bicyclic heteroaryl group can each be optionally substituted with one or more R^{B} groups, which can be the same or different;
each occurrence of R⁴ is independently H or C₁-C₆ alkyl;
R⁵ is phenyl, which can be optionally substituted with one or more groups, which can be the same or different, and are selected from: halo, CN, and NO₂;
R⁶ is H or C₁-C₆ alkyl;
each occurrence of R⁷ is independently selected from H, C₁-C₆ alkyl, and -C(O)R⁸;
each occurrence of R⁸ is independently selected from H, C₁-C₆ alkyl, and C₃-C₆ monocyclic cycloalkyl, wherein C₃-C₆ monocyclic cycloalkyl can be optionally substituted with a group selected from C₁-C₆ alkyl, halo, and -OH;
each occurrence of R⁹ is independently selected from H, C₁-C₆ alkyl and -OH;
each occurrence of R^{A} is independently selected from oxo, halo, C₁-C₆ alkyl, C₁-C₆ alkenyl and -C(O)-C₁-C₆ alkyl;
each occurrence of R^{B} is independently selected from C₁-C₆ alkyl, -OH, -O-(C₁-C₆ alkyl), halo, -C₁-C₆ haloalkyl, -C₁-C₆ hydroxyalkyl, phenyl, and -(C₁-C₆ alkylene)ₘ-(3 to 6-membered monocyclic cycloalkyl); and
occurrence of m is independently 0 or 1.

2. The compound of claim 1, or a pharmaceutically acceptable salt thereof, wherein:
(a) X is N; or
(b) X is CH.

3. The compound of claims 1 or 2, or a pharmaceutically acceptable salt thereof, wherein R¹ is selected from:

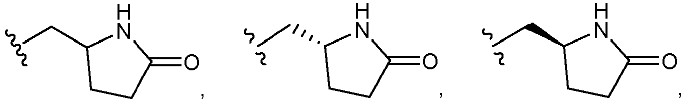

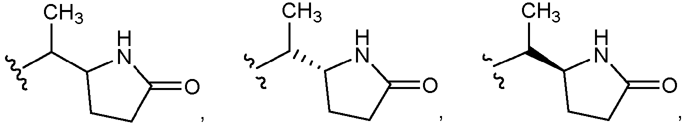

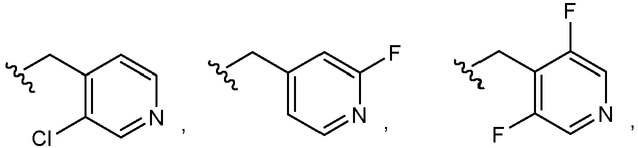

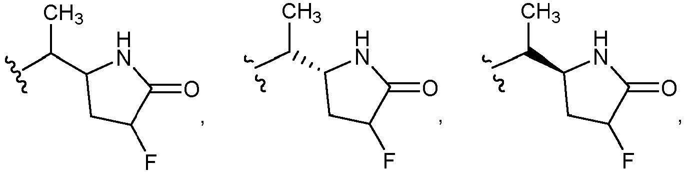

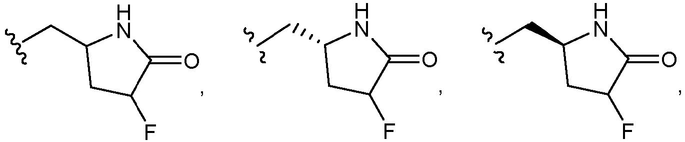

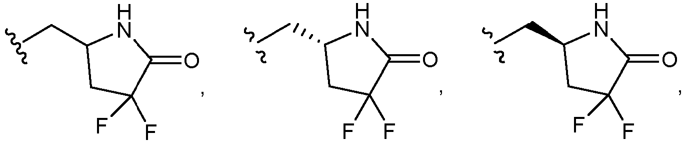

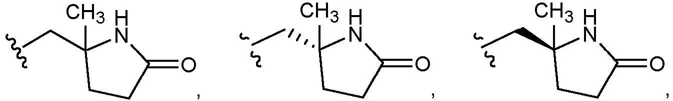

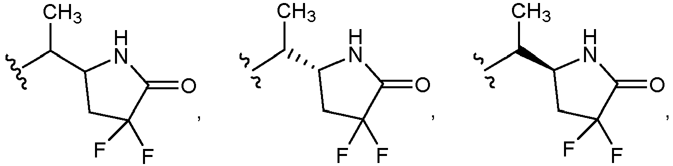

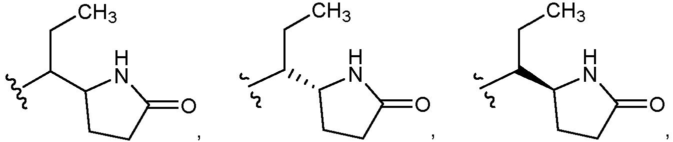

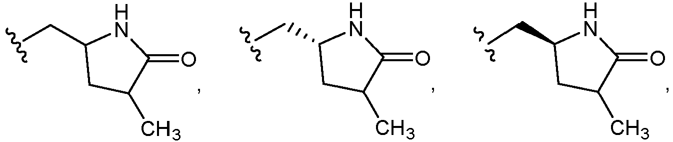

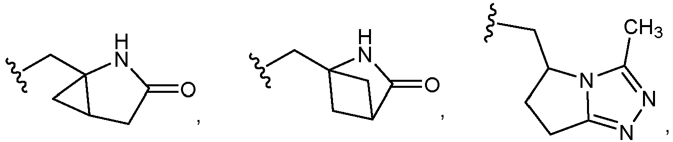

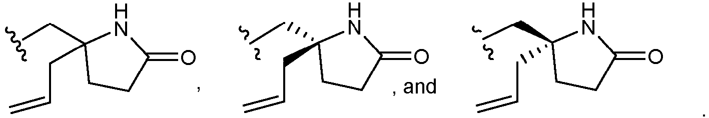

4. The compound of claim 3, or a pharmaceutically acceptable salt thereof, wherein R¹ is selected from

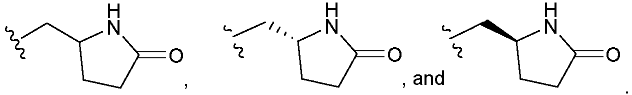

5. The compound of any of claims 1-4, or a pharmaceutically acceptable salt thereof, wherein one occurrence of R² is H, and the other occurrence of R² is selected from H, methyl, ethyl, isopropyl, -CH₂OC(O)CH₃, and -CH₂OH; or both R² groups, together with the carbon atom to which they are attached, combine to form a spirocyclic cyclobutyl group.

6. The compound of any of claims 1-5, or a pharmaceutically acceptable salt thereof, wherein R³ is selected from H, methyl, ethyl, isopropyl, -CHF₂, -CH₂CH(OH)CH(CH₃)₂, - CH₂C(OH)(CH₃)₂, -CD₃, -CH₂CH₂NHC(O)CH(CH₃)₂, thiazolyl, pyridyl, pyrimidinyl, indazolyl, 1,2,5-thiadiazolyl, oxazolyl, -CH₂CH₂-morpholinyl, pyrazolyl, -CH₂CH₂-pyrazolyl, thiazolyl, tetrahydrofuranyl, -CH₂CH₂-tetrahydrofuranyl, -CH₂-thiazolyl, tetrahydropyranyl, cyclopentanyl, azetidinyl, -CH₂-oxetanyl, -CH₂CH₂NHC(O)-cyclobutanyl, - CH₂CH₂N(CH₃)C(O)-cyclobutanyl, -CH₂CH₂N(CH₃)C(O)-cyclopropanyl, -CH₂CH₂-azetidinyl, and 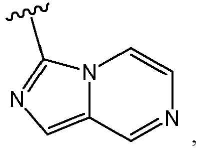 wherein said thiazolyl group, said pyridyl group, said pyrimidinyl group, said indazolyl group, said 1,2,5-thiadiazolyl group, said oxazolyl group, said pyrazolyl group, said tetrahydrofuranyl group, said -CH₂-thiazolyl group, said tetrahydropyranyl group, said cyclopentanyl group, said azetidinyl group, said oxetanyl group, said cyclobutanyl group, and said cyclopropanyl group can be optionally substituted with one or more of the following groups, which can be the same or different: F, Cl, methyl, ethyl, isopropyl, isobutyl, t-butyl, ethoxy, -OH, -CH₂OH, -CH₂F, -CH₂-cyclopropanyl, and phenyl.

7. The compound of claim 6, wherein R³ is selected from methyl,
- CH₂CH₂NHC(O)-cyclobutanyl, -CH₂CH₂N(CH₃)C(O)-cyclobutanyl, and
- CH₂CH₂N(CH₃)C(O)-cyclopropanyl.

8. The compound of any of claims 1-7, wherein R⁴ is H.

9. The compound of any of claims 1-8, or a pharmaceutically acceptable salt thereof, wherein R⁵ is phenyl substituted with one or more of the following groups, which can be the same or different: F, Cl and CN; optionally wherein R⁵ is:

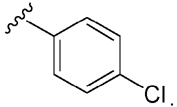

10. The compound of any of claims 1-9, or a pharmaceutically acceptable salt thereof, wherein R⁶ is H.

11. A pharmaceutical composition comprising the compound of any of claims 1-10, or a pharmaceutically acceptable salt thereof, and a pharmaceutically acceptable carrier.

12. The pharmaceutical composition of claim 11 further comprising one or more additional therapeutic agents, wherein said additional therapeutic agents are selected from anti-herpes agents, and immunomodulators; optionally wherein said additional therapeutic agents comprise letermovir.

13. The compound of any of claims 1-10, or a pharmaceutically acceptable salt thereof for use in treating herpesvirus.

14. A compound of any of claims 1-10, or a pharmaceutically acceptable salt thereof for use in therapy.

15. A combination comprising a compound of any of claims 1 to 10, or a pharmaceutically acceptable salt thereof and one or more additional therapeutic agents.

## Patentansprüche

1. Eine Verbindung der Formel (I): 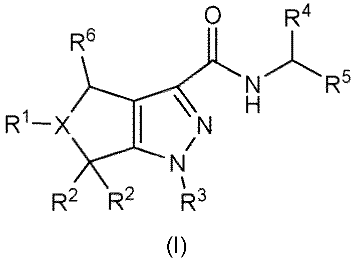
oder ein pharmazeutisch annehmbares Salz davon,
wobei:
X N oder C(R⁹) ist,
R¹ ausgewählt ist aus: -(C₁-C₆-Alkylen)-(4- bis 7-gliedriges monocyclisches Heterocycloalkyl), -(C₁-C₆-Alkylen)-(6- bis 10-gliedriges bicyclisches Heterocycloalkyl) und -(C₁-C₆-Alkylen)-(5- oder 6-gliedriges monocyclisches Heteroaryl), wobei die 4- bis 7-gliedrige monocyclische Heterocycloalkylgruppe, die 6- bis 10-gliedrige bicyclische Heterocycloalkylgruppe und die 5- oder 6-gliedrige monocyclische Heteroarylgruppe jeweils gegebenenfalls substituiert sein kann mit einer oder mehreren R^{A}-Gruppen, die gleich oder verschieden sein können,
jedes R² unabhängig ausgewählt ist aus: H, C₁-C₆-Alkyl, -(C₁-C₆-Alkylen)ₘ-O-C(O)-(C₁-C₆alkyl) und C₁-C₆-Hydroxyalkyl, oder beide R²-Gruppen zusammen mit dem Kohlenstoffatom, an das sie gebunden sind, gemeinsam eine spirocyclische C₃-C₆-Cycloalkylgruppe bilden,
R³ ausgewählt ist aus: C₁-C₆-Alkyl, C₁-C₆-Hydroxyalkyl, --CD³, -(C₁-C₆-Alkylen)ₘ-C₃-C₆monocyclisches Cycloalkyl, -(C₁-C₆-Alkylen)-N(R⁷)₂-C₁-C₆-Halogenalkyl, -(C₁-C₆-Alkylen)ₘ-(3-bis 7-gliedriges monocyclisches Heterocycloalkyl), -(C₁-C₆-Alkylen)ₘ-(5- oder 6-gliedriges monocyclisches Heteroaryl) und 9- oder 10-gliediges bicyclisches Heteroaryl, wobei die C₃-C₆monocyclische Cycloalkylgruppe, die 3- bis 7-gliedrige monocyclische Heterocycloalkylgruppe, die 5- oder 6-gliedrige monocyclische Heteroarylgruppe und die 9- oder 10-gliedrige bicyclische Heteroarylgruppe jeweils gegebenenfalls substituiert sein kann mit einer oder mehreren R^{B}-Gruppen, die gleich oder verschieden sein können,
jedes Vorkommen von R⁴ unabhängig H oder C₁-C₆-Alkyl ist,
R⁵ Phenyl ist, das gegebenenfalls substituiert sein kann mit einer oder mehreren Gruppen, die gleich oder verschieden sein können und ausgewählt sind aus: Halogen, CN und NO₂,
R⁶ H oder C₁-C₆-Alkyl ist,
jedes Vorkommen von R⁷ unabhängig ausgewählt ist aus: H, C₁-C₆-Alkyl und -C(O)R⁸,
jedes Vorkommen von R⁸ unabhängig ausgewählt ist aus: H, C₁-C₆-Alkyl und C₃-C₆monocyclisches Cycloalkyl, wobei das C₃-C₆-monocyclische Cycloalkyl gegebenenfalls substituiert sein kann mit einer Gruppe, ausgewählt aus C₁-C₆-Alkyl, Halogen und -OH,
jedes Vorkommen von R⁹ unabhängig ausgewählt ist aus: H, C₁-C₆-Alkyl und -OH,
jedes Vorkommen von R^{A} unabhängig ausgewählt ist aus: Oxo, Halogen, C₁-C₆-Alkyl, C₁-C₆-Alkenyl und -C(O)-C₁-C₆-Alkyl,
jedes Vorkommen von R^{B} unabhängig ausgewählt ist aus: C₁-C₆-Alkyl, -OH, -O-(C₁-C₆-Alkyl), Halogen, -C₁-C₆-Halogenalkyl, -C₁-C₆-Hydroxyalkyl, Phenyl und -(C₁-C₆-Alkylen)ₘ-(3- bis 6-gliedriges monocyclisches Cycloalkyl), und
das Vorkommen von m unabhängig 0 oder 1 ist.

2. Die Verbindung nach Anspruch 1 oder ein pharmazeutisch annehmbares Salz davon, wobei:
(a) X ist N oder
(b) X ist CH.

3. Die Verbindung nach Anspruch 1 oder 2 oder ein pharmazeutisch annehmbares Salz davon, wobei R¹ ausgewählt ist aus:

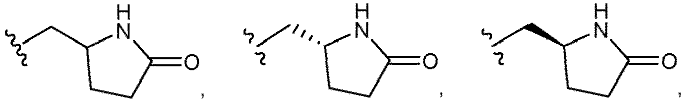

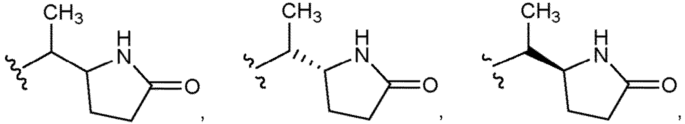

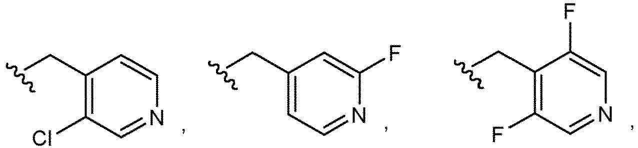

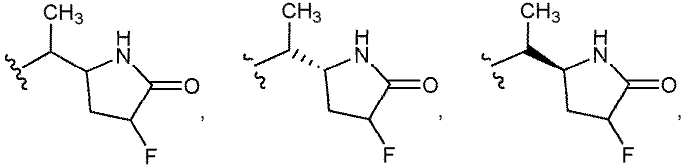

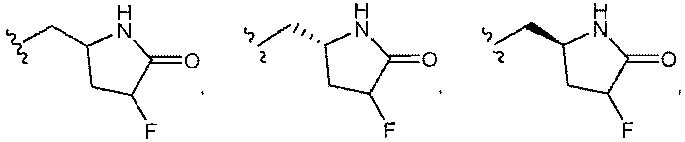

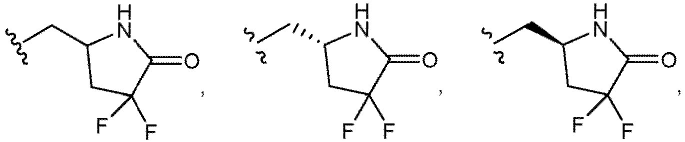

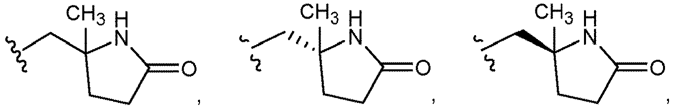

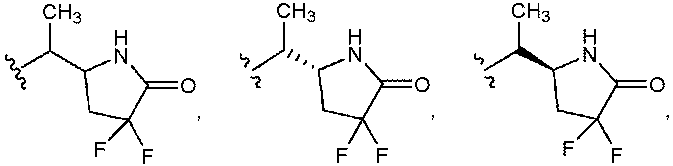

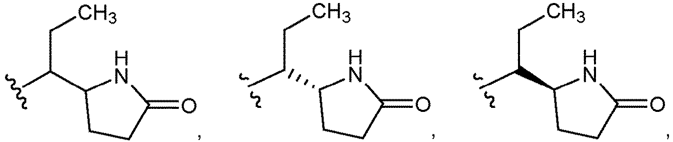

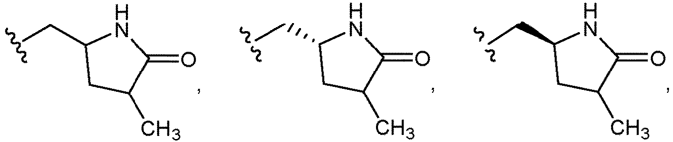

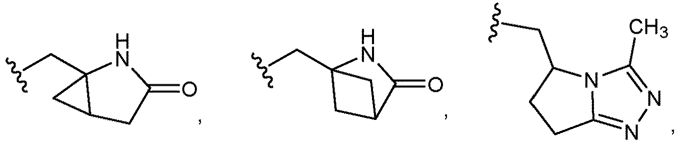

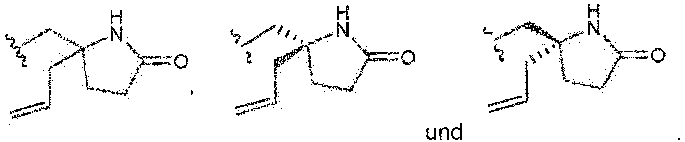

4. Die Verbindung nach Anspruch 3 oder ein pharmazeutisch annehmbares Salz davon, wobei R¹ ausgewählt ist aus

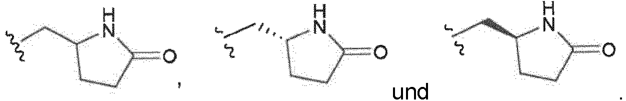

5. Die Verbindung nach einem der Ansprüche 1 - 4 oder ein pharmazeutisch annehmbares Salz davon, wobei ein Vorkommen von R² H ist und das andere Vorkommen von R² ausgewählt ist aus H, Methyl, Ethyl, Isopropyl, -CH₂OC(O)CH₃ und -CH₂OH, oder beide R²-Gruppen zusammen mit dem Kohlenstoffatom, an das sie gebunden sind, gemeinsam eine spirocyclische Cyclobutylgruppe bilden.

6. Die Verbindung nach einem der Ansprüche 1 - 5 oder ein pharmazeutisch annehmbares Salz davon, wobei R³ ausgewählt ist aus H, Methyl, Ethyl, Isopropyl, - CHF₂, -CH₂CH(OH)CH(CH₃)₂, -CH₂C(OH)(CH₃)₂, -CD₃, -CH₂CH₂NHC(O)CH(CH₃)₂, Thiazolyl, Pyridyl, Pyrimidinyl, Indazolyl, 1,2,5-Thiadiazolyl, Oxazolyl, -CH₂CH₂-Morpholinyl, Pyrazolyl, - CH₂CH₂-Pyrazolyl, Thiazolyl, Tetrahydrofuranyl, -CH₂CH₂-Tetrahydrofuranyl, -CH₂-Thiazolyl, Tetrahydropyranyl, Cyclopentanyl, Azetidinyl, -CH₂-Oxetanyl, -CH₂CH₂NHC(O)-Cyclobutanyl, -CH₂CH₂N(CH₃)C(O)-Cyclobutanyl, -CH₂CH₂N(CH₃)C(O)-Cyclopropanyl, - CH₂CH₂-Azetidinyl und 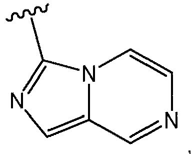 wobei die Thiazolylgruppe, die Pyridylgruppe, die Pyrimidinylgruppe, die Indazolylgruppe, die 1,2,5-Thiadiazolylgruppe, die Oxazolylgruppe, die Pyrazolylgruppe, die Tetrahydrofuranylgruppe, die -CH₂-Thiazolylgruppe, die Tetrahydropyranylgruppe, die Cyclopentanylgruppe, die Azetidinylgruppe, die Oxetanylgruppe, die Cyclobutanylgruppe und die Cyclopropanylgruppe gegebenenfalls substituiert sein können mit einer oder mehreren der folgenden Gruppen, die gleich oder verschieden sein können: F, Cl, Methyl, Ethyl, Isopropyl, Isobutyl, t-Butyl, Ethoxy, -OH, -CH₂OH, -CH₂F, -CH₂-Cyclopropanyl und Phenyl.

7. Die Verbindung nach Anspruch 6, wobei R³ ausgewählt ist aus Methyl, -CH₂CH₂NHC(O)-Cyclobutanyl, -CH₂CH₂N(CH₃)C(O)-Cyclobutanyl und -CH₂CH₂N(CH₃)C(O)-Cyclopropanyl.

8. Die Verbindung nach einem der Ansprüche 1 - 7, wobei R⁴ H ist.

9. Die Verbindung nach einem der Ansprüche 1 - 8 oder ein pharmazeutisch annehmbares Salz davon, wobei R⁵ Phenyl ist, substituiert mit einer oder mehreren der folgenden Gruppen, die gleich oder verschieden sein können: F, Cl und CN, gegebenenfalls wobei R⁵ ist:

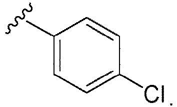

10. Die Verbindung nach einem der Ansprüche 1 - 9 oder ein pharmazeutisch annehmbares Salz davon, wobei R⁶ H ist.

11. Eine pharmazeutische Zusammensetzung, die die Verbindung nach einem der Ansprüche 1 - 10 oder ein pharmazeutisch annehmbares Salz davon und einen pharmazeutisch annehmbaren Träger umfasst.

12. Die pharmazeutische Zusammensetzung nach Anspruch 11, die ferner ein oder mehrere zusätzliche therapeutische Mittel umfasst, wobei die zusätzlichen therapeutischen Mittel ausgewählt sind aus Mittel gegen Herpes und Immunmodulatoren, gegebenenfalls wobei die zusätzlichen therapeutischen Mittel Letermovir umfassen.

13. Die Verbindung nach einem der Ansprüche 1 - 10 oder ein pharmazeutisch annehmbares Salz davon zur Verwendung bei der Behandlung des Herpesvirus.

14. Eine Verbindung nach einem der Ansprüche 1 - 10 oder ein pharmazeutisch annehmbares Salz davon zur Verwendung in der Therapie.

15. Eine Kombination, die eine Verbindung nach einem der Ansprüche 1 bis 10 oder ein pharmazeutisch annehmbares Salz davon und ein oder mehrere zusätzliche therapeutische Mittel umfasst.

## Revendications

1. Composé présentant la formule (I) : 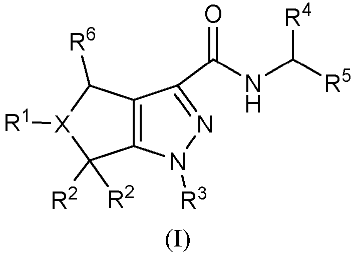 ou un sel pharmaceutiquement acceptable de celui-ci,
où :
X est N ou C(R⁹) ;
R¹ est choisi parmi : -(alkylène en C₁-C₆)-(hétérocycloalkyle monocyclique à 4 à 7 chaînons), -(alkylène en C₁-C₆)-(hétérocycloalkyle bicyclique à 6 à 10 chaînons) et -(alkylène en C₁-C₆)-(hétéroaryle monocyclique à 5 ou 6 chaînons), dans lequel ledit groupe hétérocycloalkyle monocyclique à 4 à 7 chaînons, ledit groupe hétérocycloalkyle bicyclique à 6 à 10 chaînons et ledit groupe hétéroaryle monocyclique à 5 ou 6 chaînons peuvent être chacun optionnellement substitués par un ou plusieurs groupes R^{A}, qui peuvent être identiques ou différents ;
chaque R² est indépendamment choisi parmi : H, alkyle en C₁-C₆, -(alkylène en C₁-C₆)ₘ-O-C(O)-(alkyle en C₁-C₆) et hydroxyalkyle en C₁-C₆ ou les deux groupes R², accompagnés de l'atome de carbone auquel ils sont attachés, se combinent pour former un groupe cycloalkyle en C₃-C₆ spirocyclique ;
R³ est choisi parmi : alkyle en C₁-C₆, hydroxyalkyle en C₁-C₆, -CD₃, -(alkylène en C₁-C₆)ₘ-(cycloalkyle en C₃-C₆ monocyclique), -(alkylène en C₁-C₆)-N(R⁷)₂ haloalkyle en C₁-C₆, - (alkylène en C₁-C₆)ₘ-(hétérocycloalkyle monocyclique à 3 à 7 chaînons), -(alkylène en C₁-C₆)ₘ-(hétéroaryle monocyclique à 5 ou 6 chaînons) et hétéroaryle bicyclique à 9 ou 10 chaînons, dans lequel ledit groupe cycloalkyle en C₃-C₆ monocyclique, ledit groupe hétérocycloalkyle monocyclique à 3 à 7 chaînons, ledit groupe hétéroaryle monocyclique à 5 ou 6 chaînons et ledit groupe hétéroaryle bicyclique à 9 ou 10 chaînons peuvent être chacun optionnellement substitués par un ou plusieurs groupes R^{B}, qui peuvent être identiques ou différents ;
chaque occurrence de R⁴ est indépendamment H ou un alkyle en C₁-C₆ ;
R⁵ est un phényle, qui peut être optionnellement substitué par un ou plusieurs groupes, qui peuvent être identiques ou différents et sont choisis parmi : halo, CN et NO₂ ;
R⁶ est H ou un alkyle en C₁-C₆ ;
chaque occurrence de R⁷ est indépendamment choisie parmi : H, alkyle en C₁-C₆ et - C(O)R⁸ ;
chaque occurrence de R⁸ est indépendamment choisie parmi : H, alkyle en C₁-C₆ et cycloalkyle en C₃-C₆ monocyclique, dans lequel le cycloalkyle en C₃-C₆ monocyclique peut être optionnellement substitué par un groupe choisi parmi : alkyle en C₁-C₆, halo et -OH ;
chaque occurrence de R⁹ est indépendamment choisie parmi : H, alkyle en C₁-C₆ et -OH ;
chaque occurrence de R^{A} est indépendamment choisie parmi : oxo, halo, alkyle en C₁-C₆, alcényle en C₁-C₆ et -C(O)-(alkyle C₁-C₆) ;
chaque occurrence de R^{B} est indépendamment choisie parmi : alkyle en C₁-C₆, -OH, -O-(alkyle en C₁-C₆), halo, -haloalkyle en C₁-C₆, -hydroxyalkyle en C₁-C₆, phényle et - (alkylène en C₁-C₆)ₘ-(cycloalkyle monocyclique à 3 à 6 chaînons) ; et
occurrence de m est indépendamment 0 ou 1.

2. Composé selon la revendication 1, ou un sel pharmaceutiquement acceptable de celui-ci, dans lequel :
(a) X est N ; ou
(b) X est CH.

3. Composé selon les revendications 1 ou 2, ou un sel pharmaceutiquement acceptable de celui-ci, dans lequel R¹ est choisi parmi :

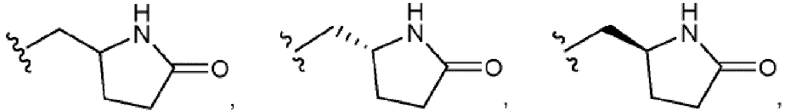

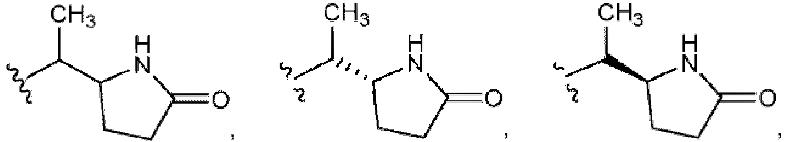

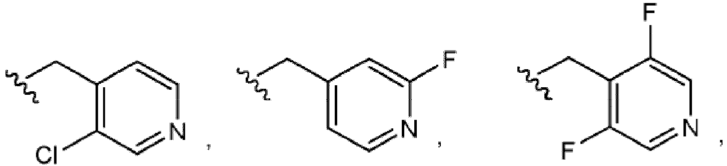

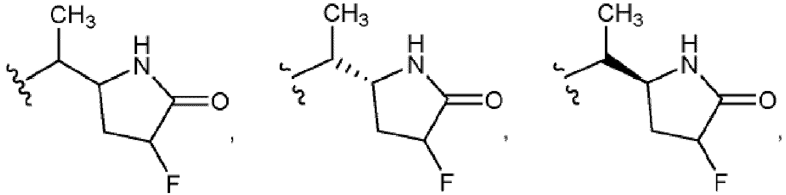

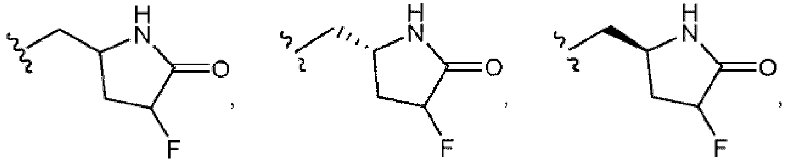

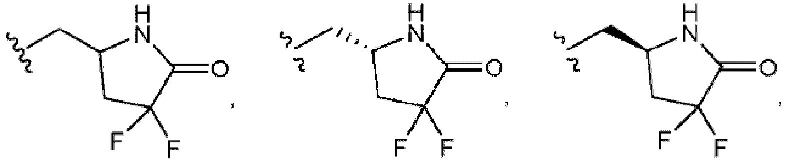

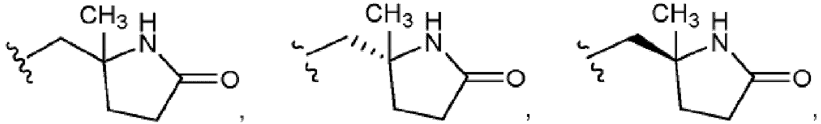

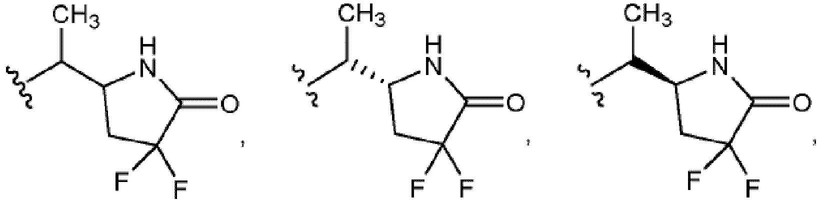

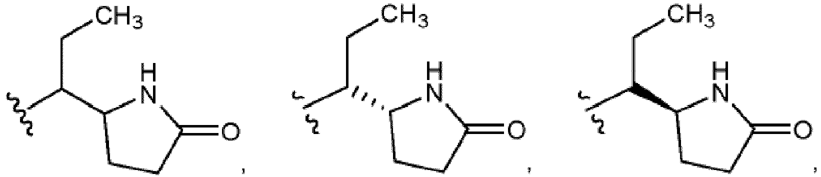

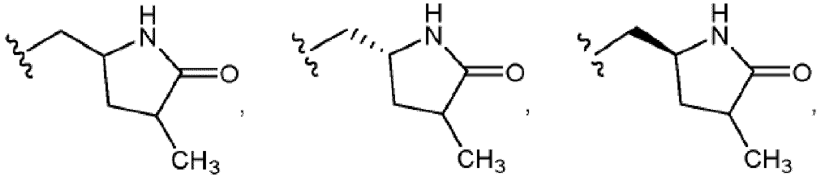

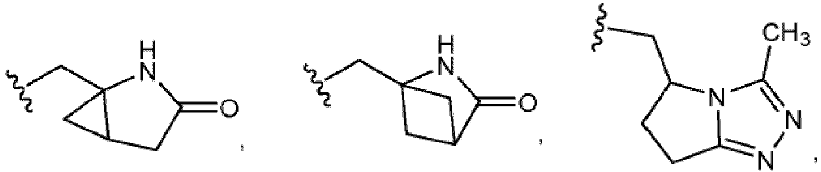

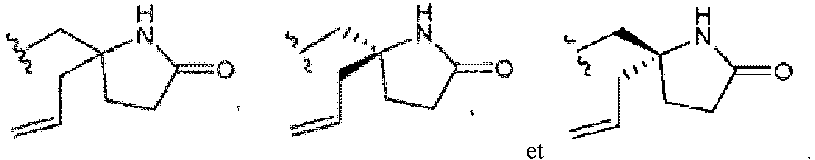

4. Composé selon la revendication 3, ou un sel pharmaceutiquement acceptable de celui-ci, dans lequel R¹ est choisi parmi :

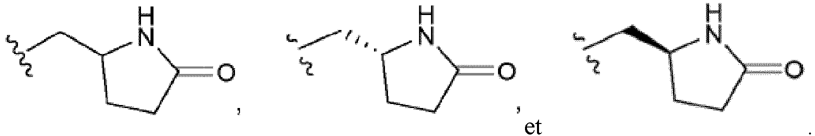

5. Composé selon l'une quelconque des revendications 1-4, ou un sel pharmaceutiquement acceptable de celui-ci, dans lequel une occurrence de R² est H et l'autre occurrence de R² est choisie parmi : H, méthyle, éthyle, isopropyle, -CH₂OC(O)CH₃ et -CH₂OH ; ou les deux groupes R², accompagnés de l'atome de carbone auquel ils sont liés, se combinent pour former un groupe cyclobutyle spirocyclique.

6. Composé selon l'une quelconque des revendications 1-5, ou un sel pharmaceutiquement acceptable de celui-ci, dans lequel R³ est choisi parmi : H, méthyle, éthyle, isopropyle, -CHF₂, - CH₂CH(OH)CH(CH₃)₂, -CH₂C(OH)(CH₃)₂, -CD₃, -CH₂CH₂NHC(O)CH(CH₃)₂, thiazolyle, pyridyle, pyrimidinyle, indazolyle, 1,2,5-thiadiazolyle, oxazolyle, -CH₂CH₂-morpholinyle, pyrazolyle, -CH₂CH₂-pyrazolyle, thiazolyle, tétrahydrofuranyle, -CH₂CH₂-tétrahydrofuranyle, -CH₂-thiazolyle, tétrahydropyranyle, cyclopentanyle, azétidinyle, -CH₂-oxétanyle, -CH₂CH₂NHC(O)-cyclobutanyle, - CH₂CH₂N(CH₃)C(O)-cyclobutanyle, -CH₂CH₂N(CH₃)C(O)-cyclopropanyle, -CH₂CH₂azétidinyle et 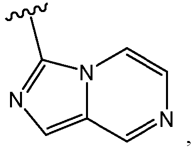 dans lequel ledit groupe thiazolyle, ledit groupe pyridyle, ledit groupe pyrimidinyle, ledit groupe indazolyle, ledit groupe 1,2,5-thiadiazolyle, ledit groupe oxazolyle, ledit groupe pyrazolyle, ledit groupe tétrahydrofuranyle, ledit groupe -CH₂-thiazolyle, ledit groupe tétrahydropyranyle, ledit groupe cyclopentanyle, ledit groupe azétidinyle, ledit groupe oxétanyle, ledit groupe cyclobutanyle et ledit groupe cyclopropanyle peuvent être optionnellement substitués par un ou plusieurs des groupes suivants, qui peuvent être identiques ou différents : F, Cl, méthyle, éthyle, isopropyle, isobutyle, t-butyle, éthoxy, -OH, -CH₂OH, -CH₂F, -CH₂-cyclopropanyle et phényle.

7. Composé selon la revendication 6, dans lequel R³ est choisi parmi : méthyle, -CH₂CH₂NHC(O)-cyclobutanyle, -CH₂CH₂N(CH₃)C(O)-cyclobutanyle, et -CH₂CH₂N(CH₃)C(O)-cyclopropanyle.

8. Composé selon l'une quelconque des revendications 1-7, dans lequel R⁴ est H.

9. Composé selon l'une quelconque des revendications 1-8, ou un sel pharmaceutiquement acceptable de celui-ci, dans lequel R⁵ est un phényle substitué par un ou plusieurs des groupes suivants, qui peuvent être identiques ou différents : F, Cl et CN ; optionnellement dans lequel R⁵ est :

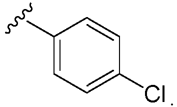

10. Composé selon l'une quelconque des revendications 1-9, ou un sel pharmaceutiquement acceptable de celui-ci, dans lequel R⁶ est H.

11. Composition pharmaceutique comprenant le composé selon l'une quelconque des revendications 1-10, ou un sel pharmaceutiquement acceptable de celui-ci, et un véhicule pharmaceutiquement acceptable.

12. Composition pharmaceutique selon la revendication 11 comprenant en outre un ou plusieurs agents thérapeutiques supplémentaires, où lesdits agents thérapeutiques supplémentaires sont choisis parmi : agents anti-herpès et immunomodulateurs ; optionnellement où lesdits agents thérapeutiques supplémentaires comprennent le létermovir.

13. Composé selon l'une quelconque des revendications 1-10, ou un sel pharmaceutiquement acceptable de celui-ci, pour utilisation dans le traitement d'un virus herpétique.

14. Composé selon l'une quelconque des revendications 1-10, ou un sel pharmaceutiquement acceptable de celui-ci, pour utilisation en thérapie.

15. Combinaison comprenant un composé selon l'une quelconque des revendications 1-10, ou un sel pharmaceutiquement acceptable de celui-ci, et un ou plusieurs agents thérapeutiques supplémentaires.
